# EUROPEAN PATENT SPECIFICATION

(11) **EP 1 519 932 B1**
(45) Date of publication and mention of the grant of the patent: **03.10.2007**
(21) Application number: 03738323.9
(22) Date of filing: 07.07.2003
(51) Int. Cl.: C07D 333/42, C07D 333/34, C07D 409/04

(54) **BISARYLSULFONAMIDE COMPOUNDS AND THEIR USE IN CANCER THERAPY**
BISARYLSULFONAMID-DERIVATE UND IHRE VERWENDUNG FÜR KREBS THERAPIE
COMPOSES DE BISARYLSULFONAMIDE ET LEUR UTILISATION DANS LE TRAITEMENT DU CANCER

(30) Priority: 05.07.2002 GB 0215650
(43) Date of publication of application: 06.04.2005
(73) Proprietor: Cyclacel Limited, London N1 7JQ (GB)
(72) Inventor: WANG, Shudong, Scotland DD8 2RZ (GB); GIBSON, Darren, Scotland DD5 3UN (GB); DUNCAN, Kenneth, Stirlingshire Scotland FK7 9RA (GB); BAILEY, Kevin, Scotland EH7 5PL (GB); THOMAS, Mark, Scotland DD2 1SG (GB); MacCALLUM, David, Scotland DD8 1QA (GB); ZHELEVA, Daniella, Scotland DD6 8NR (GB); TURNER, Nicholas John, Scotland EH3 6QR (GB); FISCHER, Peter Martin, Scotland DD11 2EN (GB)
(74) Representative: Clyde-Watson, Zöe
(86) International application number: PCT/GB2003/002923
(87) International publication number: WO 2004/005278

(56) References cited:
- EP-A- 1 258 252
- WO-A-00/15657
- WO-A-01/12593
- US-A- 2 402 623
- US-A- 3 034 955
- US-A- 5 721 246
- C. A. OBAFEMI: "Studies in the Heterocyclic Compounds V. Some Reactions of 5-Chloro-2-thiophenesulfonyl Derivatives." PHOSPHORUS AND SULFUR, vol. 13, 1982, pages 119-31, XP009011599
- M. ONO ET. AL.: "Pyrimidines. V. Smiles Rearrangement of N'-(p-Nitrobenzenesulfonyl)-N1-N2-diarylac etamides. " CHEMICAL AND PHARMACEUTICAL BULLETIN, vol. 38, no. 5, 1990, pages 1373-8, XP001155293
- V. N. MIKHAILOVA ET. AL.: "Synthesis and Properties of some Arenesulfonamides." JOURNAL OF GENERAL CHEMISTRY OF THE USSR /(ENGLISH TRANS.), , vol. 46, 1976, pages 876-7, XP009018771
- I. KH. FELDMAN ET. AL. : "Amidosulfones and Aminosulfones XXVI. Synthesis of Some Derivatives of 2-Methyl-5-nitro-and 4-Methyl-3-nitro-Benzenesulfonic Acids. " JOURNAL OF GENERAL CHEMISTRY OF THE USSR (ENGLISH TRANS) , vol. 33, 1963, pages 33-37, XP009018772
- G. DAUPHIN ET. AL.: "Application de l'étude de la dissociation acids de sulfonamides à des problèmes de structure." BULLETIN OF THE CHEMICAL SOCIETY OF FRANCE , vol. 1967, 1967, pages 3395-3404, XP001031257
- B. H. CHASE ET. AL.: "The Synthesis of Some Potential Antibacterial Agents. " JOURNAL OF PHARMACY AND PHARMACOLOGY, vol. 16, 1964, pages 163-73, XP001040339
- N. NEYKOVA ET. AL.: "Benzoxalone-5-sulfonamides, 1-(Benzoxazolone-5'-sulfonyl)-benzotriazol es and 4-Hydroxy-3,2'-diaminobenzenesulfonamides with Antiviral Activity" ARZNEIMITTEL FORSCHUNG, vol. 31, 1981, pages 747-52, XP001155294
- W.M. LINFIELD ET.AL.: "Antibacterially Active Substituted Anilides of Carboxylic and Sulfonic Acids" JOURNAL OF MEDICINAL CHEMISTRY, vol. 26, no. 12, 1983, pages 1741-6, XP001155296
- G. P. ELLIS ET. AL.: "One-step Synthesis and Spectral Study of Some 1-Methylbenzimidazoles including Use of a Lanthanide Shift Reagent. " JOURNAL OF THE CHEMICAL SOCIETY, PERKIN TRANS. I,, vol. 1974, 1974, pages 903-9, XP000655190
- G. DAUPHIN ET. AL. : "Etude de la dissociation acide de quelques sulfonamides" BULLETIN OF THE CHEMICAL SOCIETY OF FRANCE, vol. 1961, 1961, pages 486-92, XP009018774
- A. B. DE MILO ET. AL.: "Insect Chemosterilants. Analogues of 2,5-Dichloro-N-(2,4-dinitrophenyl)-benzene sulfonamide. " JOURNAL OF AGRICULTURAL AND FOOD CHEMISTRY, vol. 25, no. 1, 1977, pages 81-3, XP009018762
- F. BELL: "Investigations in the Diphenyl Series. Part IX. Further experiments with Sulphonamides. " JOURNAL OF THE CHEMICAL SOCIETY, vol. 1930, 1930, pages 1071-7, XP009018773
- C. NAEGLI ET. AL. : "Pyridon-2-sulfonsäure-5-amid und Einige Abkömmlinge" HELVETICA CHIMICA ACTA, vol. 22, 1939, pages 912-20, XP009020342
- D. F. ELLIOTT ET. AL. : "Studies in the Indole Series. Part IV. Sulphonamides Derived from Indole. " JOURNAL OF THE CHEMICAL SOCIETY, vol. 1944, 1944, pages 632-3, XP009020348
- S. WILKINSON: "5-Methoxy-N-methyltryptamine. A New Indole Alkaloid from Phalaris arundinacea" JOURNAL OF THE CHEMICAL SOCIETY, vol. 1958, 1958, pages 2079-81, XP009020347
- T. HOSHINO ET. AL. : "Synthese des d,l-Eseräthols" JUSTUS LIEBIGS ANNALEN DER CHEMIE, vol. 520, 1935, page 11-17 XP009020349
- A. SPINELLI ET. AL.: "Effects of Processing on the Amine Content of Pork Bellies " JOURNAL OF AGRICULTURAL AND FOOD CHEMISTRY, vol. 22, 1974, pages 1026-8, XP009020346
- G. PALMISANO ET. AL.: "Synthetic Studies on Indole Alkaloids. A Stereocontrolled Entry to the Cuanzine Structural Unit " TETRAHEDRON, vol. 45, 1989, pages 3583-96, XP002029549
- E. ARRANZ ET. AL.: "Novel 1,1,3-Trioxo-2H-4H-thieno[4,3-e]thiadiazin e Derivatives as Non Nuclotide Reverse Transcriptase Inhibitors That Inhibit HIV Type 1 Replication " JOURNAL OF MEDICINAL CHEMISTRY, vol. 41, 1998, pages 4109-17, XP002179390

## Description

### FIELD OF INVENTION

The present invention relates to bisarylsulfonamide compounds. In particular, the invention relates to bisarylsulfonamide compounds that are capable of binding to the oncoprotein HDM2 and modulating the HDM2-dependent regulation of the tumour suppressor p53 and/or E2F transcription factors in living cells. Further aspects of the invention relate to pharmaceutical preparations comprising such compounds, and the use thereof, e.g. in the therapeutic treatment of humans or animals.

### BACKGROUND

The *MDM2* oncogene was first cloned as an amplified gene on a *m*urine *d*ouble-*m*inute chromosome; the encoded proto-oncoprotein is designated MDM2 and the human equivalent protein is known as HDM2 [Zhang, Wang H; Curr. Pharmaceut. Design 2000; 6: 393-416]. The connection between HDM2 and human cancer is well established. Thus HDM2 is overexpressed in a variety of tumours due to gene amplification or increased transcription or translation [Momand J, Jung D, Wilczynski S, Niland J; Nucleic Acids Res. 1998; 26: 3453-3459].

Inactivation of the p53 tumour suppressor protein is a frequent event in human neoplasia [Lane DP, Lain S; Trends Molec. Med. 2002; 8: S38-S42.]. Apart from inactivation due to mutational defects, this may result, for example, from the binding of HDM2. Interaction of HDM2 with p53 *in vitro* or *in vivo* results in inhibition of p53-mediated transactivation. Formation of HDM2/p53 complexes favours nucleoplasmic transformation because of the loss of the p53 tumour suppressor effects upon complexation. HDM2 forms a negative autoregulatory loop with p53 by binding to its *N*-terminal activation domain thereby inhibiting the functions of p53 and promoting the proteolytic degradation of p53. Interference with this regulatory loop can be used to increase the concentration of active p53 in cells.

The HDM2-binding site on p53 was identified with the aid of a set of overlapping synthetic peptides [Picksley SM, Voijtesek B, Sparks A, Lane DP; Oncogene 1994; 9: 2523-2529] and was mapped to the sequence ¹⁸TFSDLW²³. Although longer peptides encompassing this sequence were potent inhibitors of p53/HDM2 complex formation, the hexapeptide p53(18-23) itself had little affinity [Böttger A, Böttger V, Garcia-Echeverria C, Chène P, Hochkeppel HK, Sampson W, Ang K, Howard SF, Picksley SM, Lane DP; J. Molec. Biol. 1997; 269: 744-756].

Screening of phage-displayed peptide libraries also revealed sequences containing the HDM2-binding motif [Böttger V, Böttger A, Howard SF, Picksley SM, Chene P, Garcia-Echeverria C, Hochkeppel HK, Lane DP; Oncogene 1996; 13: 2141-2147]. Here the starting 12mer peptide MPRFMDYWEGLN had sub-micromolar affinity and was 23-fold more potent than the corresponding wild-type p53-derived peptide ¹⁶QETFSDLWKLLF²⁷. Substitution and truncation studies revealed that the 8mer peptide FMDYWEGL was the minimal active sequence retaining micromolar affinity for HDM2 [Böttger A *et al, ibid*]*.* Based on the known binding mode of the corresponding p53 sequence [Kussie PH, Gorina S, Marechal V, Elenbaas B, Moreau J, Levine AJ, Pavletich NP; Science 1996; 274: 948-953], the helical structure of this peptide was stabilised by introduction of α,α-disubstituted amino acid residues α-aminoisobutyric (Aib) acid and 1-aminocyclopropanecarboxylic acid (Ac₃c) in place of the Asp and Gly residues, respectively. Molecular modelling suggested proximity of the Tyr side chain to the ε-amino group of the HDM2 Lys⁹⁴ residue and a phosphonomethylphenylalanine (Pmp) residue was used to replace Tyr. The resulting peptide was about 7-fold more potent, suggesting that the hypothetical stabilising salt bridge between the phosphonate and amino groups was in fact operating. Finally, inspection of the binding pocket for Trp²³ showed incomplete occupancy, suggesting substituents at the indole 6-position would improve binding. This was the case and substantial potency gain was obtained. Thus starting with the wild-type p53 12mer sequence the affinity was increased by > 1,700-fold.

The crystal structure of the p53/HDM2 complex, as well as the peptide optimisation work discussed above, show that the main contacts between p53 and the hydrophobic cleft in HDM2 involve only three p53 residues (Phe¹⁹, Trp²³, and Leu²⁶). The molecular mass of the three side chains in question amounts to *ca.* 300 Da, suggesting that modulation of the p53/HDM2 protein-protein interaction with non-peptidic small molecules may in fact be feasible. The validity of this hypothesis has already been confirmed to some extent.

Screening microbial extracts for the presence of inhibitors of the p53/HDM2 interaction, a fungal metabolite known as chlorofusin was identified as a micromolar inhibitor [Duncan SJ, Grueschow S, Williams DH, McNicholas C, Purewal R, Hajek M, Gerlitz M, Martin S, Wrigley SK, Moore M; J. Am. Chem. Soc. 2001; 123: 554-560]. Chalcones (1,3-diphenyl-2-propen-1-ones) have long been known to possess anti-tumour effects [De Vincenzo R, Ferlini C, Distefano M, Gaggini C, Riva A, Bombardelli E, Morazzoni P, Valenti P, Belluti F, Ranelletti FO, Mancuso S, Scambia G; Cancer Chemotherapy Pharmacol. 2000; 46: 305-312]. Certain chalcone derivatives, in particular a compound referred to as "B-1" {4-[3-(3,4-dichloro-phenyl)-acryloyl]-phenoxy}-acetic acid, and some of its analogues, were shown to inhibit the p53/HDM2 complex with high micromolar affinity [Stoll R, Renner C, Hansen S, Palme S, Klein C, Belling A, Zeslawski W, Kamionka M, Rehm T, Muehlhahn P, Schumacher R, Hesse F, Kaluza B, Voelter W, Engh RA, Holak TA; Biochemistry 2001; 40: 336-344]: Using multidimensional NMR techniques, evidence for direct binding of the chalcone derivatives to the Trp²³-binding pocket sub-site of the p53 binding cleft of HDM2 was presented.

Finally, peptidomimetic design starting from p53-derived HDM2-binding peptides led to acyltryptophanylpiperazides, p53/HDM2 antagonists with low micromolar affinity [Luke RWA, Hudson K, Hayward CF, Fielding C, Cotton R, Best R, Giles MB, Veldman MH, Griffiths LA, Jewsbury PJ, Breeze AL, Embrey KJ; Proc. Amer. Assoc. Cancer Res. 1999; 40: #4099; Luke RWA, Jewsbury PJ, Cotton R; PCT Int. Patent Appl. Publ. WO 00/15657; Zeneca Ltd., UK, 2000].

WO 01/12593 (Tularik Inc) discloses various N-phenyl-arylsulfonamides which are useful pharmacological agents for lowering plasma chlesterol and inhibiting abnormal cell proliferation.

US 5,721,246 (Eisai Co Ltd) discloses heterobicyclic sulfonamide derivatives, including indol-7-yl derivatives, that are useful as antitumour agents.

The present invention seeks to provide therapeutic agents that are useful in the treatment of cancer and other proliferative disorders.

### STATEMENT OF INVENTION

A first aspect of the invention relates to the use of a compound of formula I, 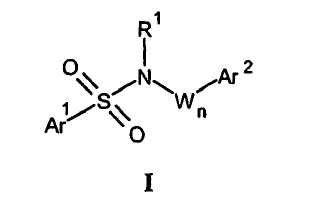 wherein
W is a C₁₋₅ branched or unbranched alkylene group or a C₂₋₅ alkenylene group; ,
n is 0 or 1;
R¹ is H, a C₁₋₈ branched or unbranched alkyl group, a C₂₋₈ alkenyl group, or an aryl or aralkyl group, each of which may be optionally substituted by one or more halogen or CF₃ groups;
Ar¹ is 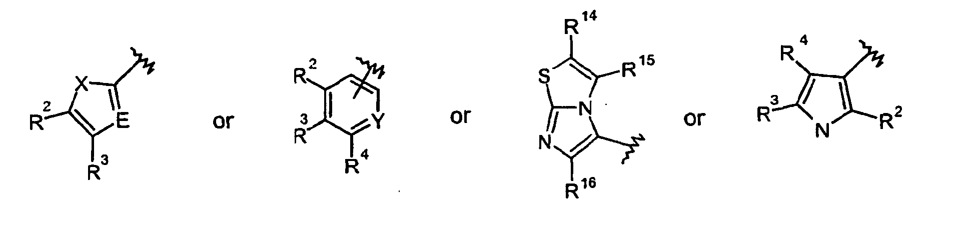 wherein
   X is S, O, NH or NR' where R' is a C₁₋₃ alkyl group;
   Y is CH or N;
   E is N or CR⁴;
   R², R³, R⁴, and R¹⁴⁻¹⁶ are each independently (A)ₚB, wherein A is C₁₋₃ alkyl, p is 0 or 1, and B is H, halogen, C₁₋₅ alkyl, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, an alicyclic group optionally containing one or more heteroatoms, optionally substituted by one or more OH, COR^{u}, halogen or CF₃ groups, or a heteroaryl group optionally substituted by one or more C₁₋₅ alkyl, halogen, SRⁱ or CF₃ groups; or
   R² and R³ are linked to form a saturated or unsaturated ring system, optionally containing one or more heteroatoms, and optionally substituted by one or more halogen, OH or CF₃ groups;
   Ar^{a-f} are each independently aryl groups optionally substituted by one or more C₁₋₅ alkyl, halogen or CF₃ groups;
   R^{a-i}, R^{s}, R^{t} and R^{u} are each independently C₁₋₅ alkyl groups optionally substituted by one or more alkoxy, halogen or CF₃ groups;
   and with the proviso that at least one of R², R³ and R⁴ is other than H;
Ar² is 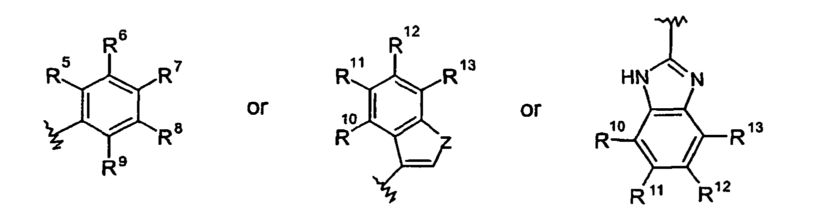 wherein
   Z is S, O, NH or NR" where R" is C₁₋₃ alkyl;
   R⁵, R⁶, R⁷, R⁸ and R⁹ are each independently (L)_{q}M wherein L is C₁₋₃ alkyl, q is 0 or 1, M is H, C₁₋₅ alkyl, halogen, NO₂, OH, NH₂, NHAr^{g}, NHR^{j}, NR^{k}R^{l}, SO₃H, SO₂NH₂, SO₂NHAr^{h}, SO₂NHR^{m}, SO₂Arⁱ, SO₂Rⁿ, CF₃, CN, COOH, COOR^{p}, CONH₂, CONHAr^{j}, CONHR^{q}, OR^{v}, COAr^{k} or COR^{r};
   R^{j-r}, R^{v} are each independently C₁₋₅ alkyl groups;
   Ar^{g-k} are each independently aryl groups;
   and with the proviso that at least one of the substituents R⁵, R⁶, R⁷, R⁸ and R⁹ is other than H;
   R¹⁰, R¹¹, R¹² and R¹³ are each independently H, C₁₋₅ alkyl, halogen, NO₂, OH, NH₂ or CF₃;
in the preparation of a medicament for treating a proliferative disorder.

A second aspect of the invention relates to the use of a compound as defined hereinabove for treating a proliferative disorder.

A third aspect of the invention relates to compounds of formula Ia, Ib, Ic and Id as outlined in more detail below.

A fourth aspect of the invention relates to a pharmaceutical composition comprising a compound according to the invention admixed with one or more pharmaceutically acceptable diluents, excipients or carriers.

A fifth aspect of the invention relates to the use of a compound of the invention in an assay for determining binding to HDM2.

A sixth aspect of the invention provides a method of detecting the binding of a ligand to HDM2, said method comprising the steps of:
(i) contacting a ligand with HDM2 in the presence of a p53-derived peptide; and
(ii) detecting any change in the interaction between HDM2 and said p53-derived peptide;
and wherein said ligand is a compound according to the invention.

A seventh aspect of the invention relates to a combination comprising at least one compound of the invention and at least one cytotoxic agent.

An eighth aspect relates to a pharmaceutical composition comprising at least one compound of the invention, and one or more cytotoxic agents, admixed with a pharmaceutically acceptable diluent, excipient or carrier.

A ninth aspect of the invention provides a method of treating a proliferative disorder, said method comprising administering to a subject at least one compound of the invention, consecutively, simultaneously or sequentially with one or more other cytotoxic agents.

A tenth aspect of the invention relates to a method of treating a proliferative disorder, said method comprising administering to a subject at least one compound of the invention consecutively, simultaneously or sequentially with radiotherapy.

### DETAILED DESCRIPTION

As mentioned, a first aspect of the invention relates to the use of a compound of formula I as defined hereinabove in the preparation of a medicament form treating proliferative disorders.

As used herein the phrase "preparation of a medicament" includes the use of a compound of the invention directly as the medicament in addition to its use in a screening programme for the identification of further agents or in any stage of the manufacture of such a medicament. Such a screening programme may for example include an assay for determining the binding to HDM2 and determining whether a candidate substance is capable of mimicking the activity of a compound of formula I.

The present invention relates to bisarylsulfonamides as therapeutic agents in the treatment of proliferative disorders.

In one preferred embodiment, Ar¹ is 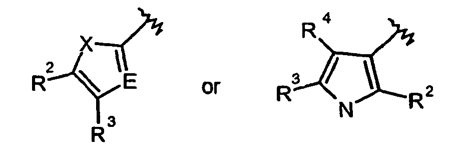 and Ar² is

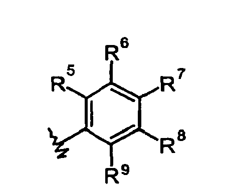

Preferably, X is S and E is CR⁴, or X is S and E is N.

In an alternative preferred embodiment, Ar¹ is 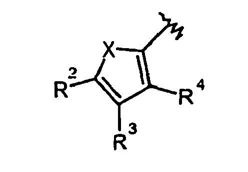 and Ar² is

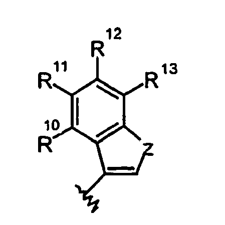

Preferably, Z is N.

In another preferred embodiment, Ar¹ is 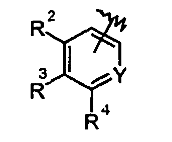 and Ar² is

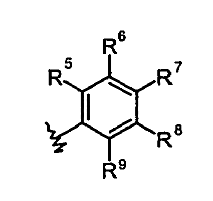

In another preferred embodiment, Ar¹ is 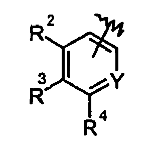 and Ar² is

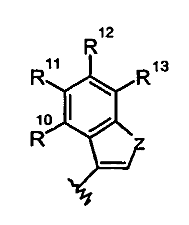

Preferably, Z is N.

In yet another preferred embodiment, Ar¹ is 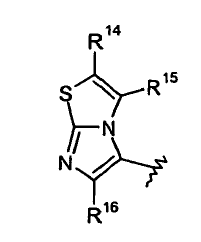 and Ar² is

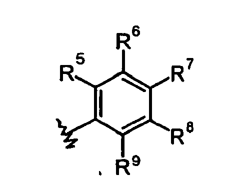

Preferably, R¹⁴⁻¹⁶ are all H.

In another preferred embodiment, Ar¹ is 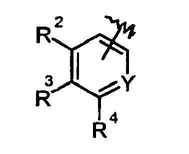 and Ar² is

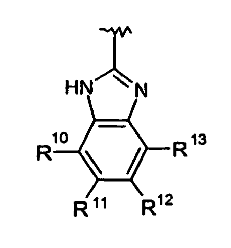

For all of the above embodiments, preferably,
- R², R³ and R⁴ are each independently (A)ₚB, wherein A is C₁₋₅ alkyl, p is 0 or 1, and B is H, F, Cl, Br, I, C₁₋₅ alkyl, NO₂, OH, NH₂, NHR^{a}, NR^{b}R^{c}, SO₃H, SO₂NH₂, NHPh, SO₂NHAr^{b}, SO₂NHR^{d}, SO₂Ph, SO₂R^{e}, CF₃, CN, COOH, COOR^{f} ; CONH₂, COONHPh, CONHR^{g}, S(CO)R^{s}, OR^{t}, OAr^{f}, COPh, COR^{h}, a morpholino, piperazino or piperidino group each of which may be optionally substituted by one or more OH or COR^{u} groups, or a heteroaryl group selected from pyridyl, pyrimidyl, oxazolyl, thiazolyl and pyrazolyl, each of which may be optionally substituted by one or more C₁₋₅ alkyl, halogen, SRⁱ or CF₃ groups, or R² and R³ together form a saturated 6-membered ring or an unsaturated 5-membered ring, each of which optionally contain one or more heteroatoms; and
- R⁵, R⁶, R⁷, R⁸, and R⁹ are each independently (L)_{q}M wherein L is C₁₋₅ alkyl, q is 0 or 1, M is H, C₁₋₅ alkyl, halogen, NO₂, OH, NH₂, NHPh, NHR^{j}, NR^{k}R^{l}, SO₃H, SO₂NH₂, SO₂NHPh, SO₂NHR^{m}, SO₂Ph, SO₂Rⁿ, CF₃, CN, COOH, COOR^{p}, CONH₂, CONHPh, CONHR^{q}, OR^{v}, COPh or COR^{r}.

More preferably, R², R³ and R⁴ are each independently (A)ₚB, wherein A is C₁₋₅ alkyl, p is 0 or 1, and B is H, F, Cl, Br, I, C₁₋₅ alkyl, NO₂, OH, NH₂, NHR^{a}, NR^{b}R^{c}, SO₃H, SO₂NH₂, NHPh, SO₂NHPh, SON₂HR^{d}, SO₂Ph, SO₂R^{e}, CF₃, CN, COOH, COOR^{f}, CONH₂, COONHPh, CONHR^{g}, S(CO)R^{s}, OR^{t}, OAr^{f}, COPh, COR^{h}, pyridyl, pyrimidyl, 2-methylsulfanylpyrimid-5-yl, oxazol-2-yl, thiazol-2-yl, 1-methyl-5-trifluoromethyl-1*H*-pyrazol-4-yl, morpholin-4-yl, 4-acetyl-piperazin-1-yl, 3-hydroxy-piperidin-1-yl, or R² and R³ together form

-OCH₂CH₂O-

-N-S-N-

or
a phenyl group optionally substituted by one or more halogens.

More preferably still, R², R³ and R⁴ are each independently H, halogen, NO₂, SO₂Ph, S(CO)Me, COOH, COOEt, OPh, OMe, NHCH₂CH₂OMe, 1-methyl-5-trifluoromethyl-1*H*-pyrazol-4-yl, 2-methylsulfanyl pyrimid-5-yl, N-(4-fluorophenyl)sulfonamido, N-(4-trifluoromethylphenyl)-sulfonamido, oxazol-2-yl, C₁₋₅ alkyl, NH₂, morpholin-4-yl, 4-acetyl-piperazin-1-yl, 3-hydroxy-piperidin-1-yl, or R² and R³ together form

OCH₂CH₂O-

-N-S-N-

or
a phenyl group optionally substituted by one or more halogens.

In one particularly preferred embodiment, Ar¹ is 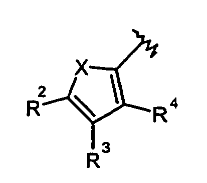
X is S or N, preferably S;
R², R³ and R⁴ are each independently C₁₋₅ alkyl, S(CO)Me, COOH, NHCH₂CH₂OMe, COOEt, H, halogen, NO₂, SO₂Ph, SO₂NH-(4-chlorophenyl), 1-methyl-5-trifluoromethyl-1*H*-pyrazol-4-yl, morpholin-4-yl, 2-methylsulfanylpyrimid-5-yl, N-(4-fluorophenyl)sulfonamido, N-(4-trifluoro-methylphenyl)-sulfonamido, 3-hydroxypiperidin-1-yl, pyridin-2-yl; or R² and R³ form a phenyl group optionally substituted by one or more halogens.

More preferably, for this embodiment,
- R² is halogen, SO₂Ph, NO₂, Et, SOMe, morpholin-4-yl, NHCH₂CH₂OMe, 3-hydroxy-piperidin-1-yl, 1-methyl-5-trifluoromethyl-1*H*-pyrazol-4-yl or 2-methylsulfanyl-pyrimid-5-yl;
- R³ is halogen, SO₂NH-(4-chlorophenyl), H, NO₂, N-(4-fluorophenyl)sulfonamido or N-(4-trifluoro methylphenyl)-sulfonamido; and
- R⁴ is H.

In another particularly preferred embodiment, Ar¹ is 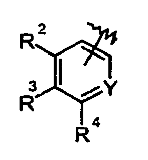
Y is CH or N; and
R², R³ and R⁴ are each independently H, OH, COOH, CF₃, OPh, OMe, NO₂, 4-acetylpiperazin-1-yl, NH₂, halogen, pyrazol-1-yl, oxazol-2-yl or C₁₋₅ alkyl, or R² and R³ together form -OCH₂CH₂O- or -N-S-N- .

More preferably, for this embodiment,
when Y is CH
   - R² is H, NO₂ or Cl;
   - R³ is NO₂, NH₂, Cl, CF₃, COOH, 4-acetyl-piperazin-1-yl; and
   - R⁴ is H, Cl, oxazol-2-yl, OH, NO₂, NH₂, OMe or Me; or
when Y is N
   - R² is H;
   - R³ is Br;
   - R⁴ is Cl or OPh.

In another preferred embodiment of the invention, Ar¹ is

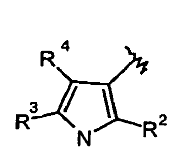

R² and R⁴ are C₁₋₅ alkyl, and R³ is COOH or COOEt.

In one preferred embodiment,
- R⁵, R⁶, R⁷, R⁸ and R⁹ are each independently H, halogen, OMe, NO₂, C₁₋₅ alkyl, CF₃ or OH; and
- R¹⁰, R¹¹, R¹² and R¹³ are all H.

More preferably still,
- R⁵ is H, C₁₋₅ alkyl, or halogen;
- R⁶ is H, halogen, NO₂, or CF₃;
- R⁷ is H, halogen, OMe, NO₂, OH or CF₃;
- R⁸ is H, halogen or CF₃;
- R⁹ is H.

In one preferred embodiment of the invention,
- W is CH₂, CH₂CH₂ or CH(CH₃)CH₂; and
- R¹ is H, CH₂Ph, CH₂CH(Me)₂, 3-(trifluoromethyl)benzyl, or Me.

In an especially preferred embodiment of the first aspect of the invention, the compound of formula I is selected from the following:
5-Chloro-4-nitrothiophene-2-sulfonic acid (3-trifluoromethylphenyl)amide [1];
5-Chloro-4-nitrothiophene-2-sulfonic acid (4-chlorophenyl)-amide [2];
5-Chloro-4-nitrothiophene-2-sulfonic acid (4-fluorophenyl)amide [3];
4-Bromo-5-chlorothiophene-2-sulfonic acid (4-fluorophenyl)amide [4];
5-Chloro-4-nitrothiophene-2-sulfonic acid (4-hydroxyphenyl)amide [5];
5-Chloro-4-nitrothiophene-3-sulfonic acid (4-trifluoromethylphenyl)amide [6];
5-Chloro-4-nitrothiophene-2-sulfonic acid (4-fluorophenyl)methylamide [7];
4,5-Dibromothiophene-2-sulfonic acid (3,5-bis-trifluoromethylphenyl)amide [8];
5-Chlorothiophene-2-sulfonic acid (4-trifluoromethylphenyl)amide [9];
5-Chlorothiophene-2-sulfonic acid (4-chlorophenyl)amide [10];
5-Chlorothiophene-2-sulfonic acid (3,5-bis-trifluoromethylphenyl)amide [11];
5-(2-Methylsulfanyl-pyrimidin-5-yl)-thiophene-2-sulfonic acid (3,5-dichlorophenyl)-amide [12];
4-Oxazol-2-yl-N-(4-trifluoromethylphenyl)benzenesulfonamide [13];
N-(3,5-Bis-trifluoromethylphenyl)-4-oxazol-2-yl-benzenesulfonamide [14];
4-Bromo-5-chlorothiophene-2-sulfonic acid (4-trifluoromethylphenyl)amide [15];
5-Bromothiophene-2-sulfonic acid (4-chlorophenyl)amide [16];
5-Bromothiophene-2-sulfonic acid (3,5-dichlorophenyl)amide [17];
5-Bromothiophene-2-sulfonic acid (3,5-bis-trifluoromethylphenyl)amide [18];
N-(4-Chlorophenyl)-3-nitrobenzenesulfonamide [19];
3-Nitro-N-(4-trifluoromethylphenyl)benzenesulfonamide [20];
N-(3,5-Bis-trifluoromethylphenyl)-3-nitrobenzenesulfonamide [21];
N-(2,4-Dichlorophenyl)-3-nitrobenzenesulfonamide [22];
5-Benzenesulfonylthiophene-2-sulfonic acid (4-trifluoromethylphenyl)-amide [23];
5-Benzenesulfonylthiophene-2-sulfonic acid (4-chlorophenyl)amide [24];
5-Benzenesulfonylthiophene-2-sulfonic acid (3,5-dichlorophenyl)amide [25];
5-Chlorothiophene-2-sulfonic acid (3,4-dichlorophenyl)amide [26];
4,5-Dibromothiophene-2-sulfonic acid (3-trifluoromethylphenyl)amide [27];
4,5-Dibromothiophene-2-sulfonic acid (3,4-dichlorophenyl)amide [28];
N-(3,5-Bis-trifluoromethylphenyl)-4-chloro-3-nitrobenzenesulfonamide [29];
4-Chloro-N-(3,4-dichlorophenyl)-3-nitrobenzenesulfonamide [30];
S-(1-Methyl-5-trifluoromethyl-1H-pyrazol-4-yl)-thiophene-2-sulfonic acid (4-trifluoromethylphenyl)-amide [31];
5-Chlorothiophene-2,4-disulfonic acid bis-[(4-fluorophenyl)-amide] [32];
5-Chlorothiophene-2,4-disulfonic acid bis-[(4-trifluoro-methyl-phenyl)-amide] [33];
4-Methyl-3-nitro-N-(4-trifluoromethylphenyl)benzenesulfonamide [34];
4-Chloro-3-nitro-N-(4-trifluoromethylphenyl)benzenesulfonamide [35];
3-Amino-4-methyl-N-(4-trifluoromethyl-phenyl)benzenesulfonamide [36];
N-(4-Chlorophenyl)-4-methyl-3-nitrobenzenesulfonamide [37];
4-Chloro-N-(4-chlorophenyl)-3-nitro-benzenesulfonamide [38];
5-Chloro-4-nitrothiophene-2-sulfonic acid (3,5-dichlorophenyl)-amide [39];
5-Chloro-4-nitrothiophene-2-sulfonic acid (3,5-difluorophenyl)-amide [40];
5-Bromo-6-chloropyridine-3-sulfonic acid (4-trifluoromethylphenyl)amide [41];
5-Bromo-6-chloropyridine-3-sulfonic acid (3,5-bis-trifluoromethylphenyl)amide [42];
5-(1-Methyl-5-trifluoromethyl-1H-pyrazol-4-yl)-thiophene-2-sulfonic acid (3,5-bistrifluoromethylphenyl)amide [43];
5-Chloro-4-nitrothiophene-2-sulfonic acid (3,5-bis-trifluoromethyl-phenyl)-amide [44];
5-Chloro-4-nitrothiophene-2-sulfonic acid 4-fluorobenzylamide [45];
5-Chloro-4-nitrothiophene-2-sulfonic acid 4-trifluoromethylbenzylamide [46];
4-Chloro-N-(3,5-dichlorophenyl)-3-nitro-benzenesulfonamide [47];
5-Chloro-4-nitrothiophene-2-sulfonic acid [2-(1H-indol-3-yl)-ethyl]amide [48];
5-Chloro-4-nitrothiophene-2-sulfonic acid [2-(1H-indol-3-yl)-1-methylethyl]amide [49];
5-Chloro-4-nitrothiophene-2-sulfonic acid methyl-(4-trifluoromethylphenyl)amide [50];
5-Chloro-4-nitrothiophene-2-sulfonic acid (4-chloro-phenyl)-methylamide [51]; and
5-Chloro-4-nitrothiophene-2-sulfonic acid methyl-(4-trifluoromethylbenzyl)amide [52];
5-Chloro-4-nitro-thiophene-2-sulfonic acid benzyl-(4-fluoro-benzyl)-amide [53];
5-Chloro-4-nitro-thiophene-2-sulfonic acid 3,5-dichloro-benzylamide [54];
5-Chloro-4-nitro-thiophene-3-sulfonic acid 3,5-difluoro-benzylamide [55];
5-Chloro-4-nitro-thiophene-2-sulfonic acid 4-chloro-benzylamide [56];
5-Chloro-4-nitro-thiophene-2-sulfonic acid [1-(4-fluoro-phenyl)-ethyl]-amide [57];
5-Chloro-4-nitro-thiophene-2-sulfonic acid (4-chloro-phenyl)-isobutyl-amide [58];
5-Chloro-4-nitro-thiophene-2-sulfonic acid (1H-benzo-imidazol-2-yl)-amide [59];
5-Chloro-4-nitro-thiophene-2-sulfonic acid [2-(6-chloro-1H-indol-3-yl)-ethyl]-amide [60];
5-Chloro-4-nitro-thiophene-2-sulfonic acid (4-methoxy-phenyl)-amide [61];
5-Chloro-4-nitro-thiophene-2-sulfonic acid p-tolylamide [63];
5-Chloro-4-nitro-thiophene-2-sulfonic acid benzyl-(4-chloro-phenyl)-amide [65];
5-Chloro-4-nitro-thiophene-2-sulfonic acid benzyl-(4-methoxy-phenyl)-amide [66];
5-Chloro-4-nitro-thiophene-2-sulfonic acid (4-chloro-phenyl)-(3-trifluoromethyl-benzyl)-amide [67];
5-Nitro-thiophene-2-sulfonic acid (4-chloro-phenyl)-amide [68];
4-Nitro-thiophene-2-sulfonic acid (4-chloro-phenyl)-amide [69];
5-Chloro-thiophene-2,4-disulfonic acid bis-[(4-chloro-phenyl)-amide] [70];
5-Ethyl-4-nitro-thiophene-2-sulfonic acid (4-chloro-phenyl)-amide [71];
Thioacetic acid S-[5-(4-chloro-phenylsulfamoyl)-3-nitro-thiophen-2-yl] ester [72];
5-Methyl-4-nitro-thiophene-2-sulfonic acid (4-chloro-phenyl)-amide [73];
5-Methyl-thiophene-2,4-disulfonic acid bis-[(4-chloro-phenyl)-amide] [74];
5-Chloro-4-nitro-thiophene-2-sulfonic acid (3-trifluoro-methyl-benzyl)-(4-trifluoromethylbenzyl)-amide [75];
4-Nitro-thiophene-2-sulfonic acid (4-trifluoromethyl-phenyl)-amide [76];
4-Nitro-thiophene-2-sulfonic acid [2-(1H-indol-3-yl)-ethyl]-amide [77];
5-(1-Methyl-5-tri-fluoromethyl-1H-pyrazol-3-yl)-thiophene-2-sulfonic acid (3,5-bis-trifluoromethyl-phenyl)-(3-trifluoro-methyl-benzyl)-amide [78];
5-Morpholin-4-yl-4-nitro-thiophene-2-sulfonic acid (4-chloro-phenyl)-amide [79];
5-(2-Methoxy-ethylamino)-4-nitro-thiophene-2-sulfonic acid (4-chloro-phenyl)-amide [80];
4-Chloro-N-[2-(5-chloro-1H-indol-3-yl)-ethyl]-3-nitro-benzenesulfonamide [81];
N-[2-(5-Chloro-1H-indol-3-yl)-ethyl]-4-methyl-3-nitro-benzenesulfonamide [82];
N-(1H-Benzoimidazol-2-yl)-4-chloro-3-nitro-benzenesulfonamide [83];
6-Chloro-imidazo[2,1-b]thiazole-5-sulfonic acid (3,5-bis-trifluoro-methyl-phenyl)-amide [84];
2,3-Dihydro-benzo[1,4]dioxine-6-sulfonic acid (4-chloro-phenyl)-amide [85];
3,3-Dihydro-benzo[1,4]dioxine-6-sulfonic acid (3,5-bis-trifluoromethyl-phenyl)-amide [86];
6-Phenoxy-pyridine-3-sulfonic acid (4-chloro-phenyl)-amide [87];
5-Chloro-3-methyl-benzo[b]thiophene-2-sulfonic acid (4-chloro-3-nitro-phenyl)-amide [88];
N-(3,5-Bis-trifluoromethyl-phenyl)-4-pyrazol-1-yl-benzenesulfonamide [89];
4-(4-Chloro-phenylsulfamoyl)-3,5-dimethyl-1H-pyrrole-2-carboxylic acid ethyl ester [90];
4-(3,5-Bis-trifluoromethyl-phenylsulfamoyl)-3,5-dimethyl-1H-pyrrole- 2-carboxylic acid [91];
4-(4-Chloro-phenylsulfamoyl)-3,5-dimethyl-1H-pyrrole-2-carboxylic acid [92];
2-(4-Chloro-phenylsulfamoyl)-4-methyl-thiazole-5-carboxylic acid ethyl ester [93];
3,5-Dichloro-N-(4-chloro-phenyl)-4-hydroxy-benzenesulfonamide [94];
N-(3,5-Bis-trifluoromethyl-phenyl)-3,5-dichloro-4-hydroxy-benzenesulfonamide [95];
3,5-Dichloro-4-hydroxy-N-(4-trifluoromethyl-phenyl)-benzenesulfonamide [96];
N-(4-Chloro-phenyl)-4-nitro-benzene-sulfonamide [97];
N-(3,5-Bis-trifluoromethyl-phenyl)-4-nitro-benzenesulfonamide [98];
4-Amino-N-(3,5-bis-trifluoromethyl-phenyl)-3-chloro-benzenesulfonamide [99];
3-Nitro-N-(4-trifluoromethyl-phenyl)-benzenesulfonamide [100];
3,5-Dichloro-N-(3,5-dichloro-phenyl)-4-hydroxy-benzenesulfonamide [101];
4-Amino-3-chloro-N-(4-chloro-phenyl)-benzenesulfonamide [102];
3-Chloro-N-(4-chloro-phenyl)-4-methoxy-benzenesulfonamide [103];
N-(3,5-Bis-trifluoromethyl-phenyl)-3-chloro-4-methoxy-benzene-sulfonamide [104];
N-(3-Chloro-4-nitro-phenyl)-3,5-bis-trifluoromethyl-benzenesulfonamide [105];
3-(4-Acetyl-piperazin-1-yl)-N-(3,5-bis-trifluoromethyl-phenyl)-4-nitrobenzenesulfonamide[106];
N-(3,5-Bis-trifluoromethyl-phenyl)-2-nitro-benzenesulfonamide [107];
3-(3,5-Bis-trifluoromethyl-phenylsulfamoyl)-benzoic acid [108];
3,5-Dichloro-N-(4-chloro-benzyl)-4-hydroxy-benzenesulfonamide [109];
3,5-Dichloro-4-hydroxy-N-(4-trifluoromethyl-benzyl)-benzenesulfonamide [110];
3,5-Dichloro-4-hydroxy-N-[2-(IH-indol-3-yl)-ethyl]-benzenesulfonamide [111];
4,5-Dibromo-thiophene-2-sulfonic acid (3,5-dichloro-phenyl)-amide [112];
N-(3,5-Dichloro-phenyl)-4-oxazol-2-yl-benzenesulfonamide [113];
4-Bromo-5-chloro-thiophene-2-sulfonic acid (3,5-bis-trifluoromethyl-phenyl)-amide [114];
4-Bromo-5-chloro-thiophene-2-sulfonic acid (3,5-dichloro-phenyl)-amide [115];
5-Bromo-thiophene-2-sulfonic acid (4-trifluoromethyl-phenyl)-amide [116];
5-Benzenesulfonyl-thiophene-2-sulfonic acid (3,5-bis-trifluoromethyl-phenyl)-amide [117];
5-Benzenesulfonyl-thiophene-2-sulfonic acid (2,4-dichloro=phenyl)-amide [118];
5-Chloro-3-methyl-benzo[b]thiophene-2-sulfonic acid (3,5-bis-trifluoromethyl-phenyl)-amide [119];
Benzo[b]thiophene-2-sulfonic acid (3,3-bis-thfluoromethyl-phcnyl)-amide [120];
Benzo[1,2,5]thiadiazole-5-sulfonic acid (4-chloro-phenyl)-amide [121];
Benzo[1,2,5]thiadiazole-5-sulfonic acid (3,5-bis-trifluoromethyl-phenyl)-amide [122];
Benzo[1,2,5]thiadiazole-5-sulfonic acid (4-trifluoromethyl-phenyl)-amide [123];
5-Pyridin-2-yl-thiophene-2-sulfonic acid (3,5-bis-trifluoromethyl-phenyl)-amide [124];
4,5-Dibromo-thiophene-2-sulfonic acid (4-chloro-phenyl)-amide [125];
4,5-Dibromo-thiophene-2-sulfonic acid (4-trifluoromethyl-phenyl)-amide [126];
3,5-Dichloro-N-(4-fluoro-benzyl)-4-hydroxy-benzenesulfonamide [127];
N-(3,5-Bis-trifluoromethyl-phenyl)-2,6-dichloro-benzenesulfonamide [128];
5-Chloro-4-nitro-thiophene-2-sulfonic acid (4-methoxy-2-methyl-phenyl)-amide [129];
5-(3-Hydroxy-piperidin-1-yl)-4-nitro-thiophene-2-sulfonic acid (4-chloro-phenyl)-amide [130]; and
5-Chloro-4-nitro-thiophene-2-sulfonic acid (4-nitro-phenyl)-amide [131].

In a further preferred embodiment, the compound of formula I is selected on the basis that it inhibits human tumour cell proliferation *in vitro* (using a standard 72-h MTT assay as described in Example 5) with an IC₅₀ value equal to or less than 20 µM.

In a particularly preferred embodiment, the compound of formula I is selected from the following:
5-Chloro-4-nitrothiophene-2-sulfonic acid (4-chlorophenyl)amide [2];
5-Chloro-4-nitrothiophene-2-sulfonic acid (4-fluorophenyl)amide [3];
5-Chloro-4-nitrothiophene-2-sulfonic acid (4-hydroxyphenyl)amide [5];
5-Chloro-4-nitrothiophene-2-sulfonic acid (4-trifluoromethylphenyl)amide [6];
5-Chloro-4-nitrothiophene-2-sulfonic acid (4-fluorophenyl)methylamide [7];
4-Chloro-3-nitro-N-(4-trifluoromethylphenyl)-benzene-sulfonamide [35];
4-Chloro-N-(4-chlorophenyl)-3-nitrobenzene-sulfonamide [38];
5-Chloro-4-nitrothiophene-2-sulfonic acid (3,5-dichlorophenyl)amide [39];
5-Chloro-4-nitrothiophene-2-sulfonic acid (3,5-difluorophenyl)amide [40];
5-(1-Methyl-5-trifluoromethyl-1H-pyrazol-4-yl)-thiophene-2-sulfonic acid (3,5-bistrifluoromethylphenyl)amide [43];
5-Chloro-4-nitrothiophene-2-sulfonic acid (3,5-bis-trifluoromethyl-phenyl)-amide [44];
5-Chloro-4-nitrothiophene-2-sulfonic acid 4-fluorobenzylamide [45];
5-Chloro-4-nitrothiophene-2-sulfonic acid 4-trifluoromethylbenzylamide [46];
4-Chloro-N-(3,5-dichlorophenyl)-3-nitro-benzenesulfonamide [47];
5-Chloro-4-nitrothiophene-2-sulfonic acid [2-(1H-indol-3-yl)-ethyl]amide [48];
5-Chloro-4-nitrothiophene-2-sulfonic acid [2-(1H-indol-3-yl)-1-methylethyl]amide [49];
5-Chloro-4-nitrothiophene-2-sulfonic acid (4-chlorophenyl)methylamide [51];
5-Chloro-4-nitro-thiophene-2-sulfonic acid benzyl-(4-fluoro-benzyl)-amide [53];
5-Chloro-4-nitro-thiophene-2-sulfonic acid 3,5-dichloro-benzylamide [54];
5-Chloro-4-nitro-thiophene-2-sulfonic acid 3,5-difluoro-benzylamide [55];
5-Chloro-4-nitro-thiophene-2-sulfonic acid 4-chloro-benzylamide [56];
5-Chloro-4-nitro-thiophene-2-sulfonic acid [1-(4-fluoro-phenyl)-ethyl]-amide [57];
5-Chloro-4-nitro-thiophene-2-sulfonic acid (4-methoxy-phenyl)-amide [61];
5-Chloro-4-nitro-thiophene-2-sulfonic acid p-tolylamide [63];
5-Chloro-4-nitro-thiophene-2-sulfonic acid benzyl-(4-chloro-phenyl)-amide [65];
5-Chloro-4-nitro-thiophene-2-sulfonic acid benzyl-(4-methoxy-phenyl)-amide [66];
5-Chloro-4-nitro-thiophene-2-sulfonic acid (4-chloro-phenyl)-(3-trifluoromethyl-benzyl)-amide [67];
4-Nitro-thiophene-2-sulfonic acid (4-chloro-phenyl)-amide [69];
5-Chloro-4-nitro-thiophene-2-sulfonic acid (3-trifluoro-methyl-benzyl)-(4-trifluoromethyl-benzyl)-amide [75]; and
N-(1H-Benzoimidazol-2-yl)-4-chloro-3-nitro-benzenesulfonamide [83].

More preferably, the compound of formula I is selected from the following: [2], [3], [5], [6], [7], [35], [39], [40], [44]-[46], [48], [49], [53]-[57], [61], [63], [65]-[67], [75] and [83].

Even more preferably, the compound of formula I is selected from the following: [2], [3], [5], [6], [39], [40], [46], [48], [49], [53], [56], [57], [61], [63], [66] and [83].

More preferably still, the compound of formula I is selected from the following: [2], [3], [46], [49], [61], [63] and [83].

In another preferred embodiment, the compound of formula I is selected on the basis that it inhibits p53-HDM2 interaction (using a competitive p53-derived peptide-HDM2 binding assay as described in Example 3) with an IC₅₀ value equal to or less than 200 µM.

Thus, in an especially preferred embodiment of the invention, the compound of formula I is selected from the following:
5-Chloro-4-nitrothiophene-2-sulfonic acid (4-chlorophenyl)amide [2];
5-Chloro-4-nitrothiophene-2-sulfonic acid (4-fluorophenyl)amide [3];
5-Chloro-4-nitrothiophene-2-sulfonic acid (4-hydroxyphenyl)amide [5];
5-Chloro-4-nitrothiophene-2-sulfonic acid (4-trifluoromethylphenyl)amide [6];
5-Chloro-4-nitrothiophene-2-sulfonic acid (4-fluorophenyl)methylamide [7];
5-Chlorothiophene-2-sulfonic acid (4-trifluoromethylphenyl)amide [9];
5-Bromothiophene-2-sulfonic acid (3,5-bis-trifluoromethylphenyl)amide [18];
5-Benzenesulfonyl-thiophene-2-sulfonic acid (4-trifluoromethylphenyl)amide [23];
4,5-Dibromothiophene-2-sulfonic acid (3-trifluoromethylphenyl)amide [27];
4,5-Dibromothiophene-2-sulfonic acid (3,4-dichlorophenyl)amide [28];
*N*-(3,5-Bis-trifluoromethylphenyl)-4-chloro-3-nitrobenzene-sulfonamide [29];
5-Chlorothiophene-2,4-disulfonic acid bis-[(4-trifluoromethylphenyl)amide] [33];
4-Chloro-3-nitro-N-(4-trifluoromethylphenyl)benzene-sulfonamide [35];
5-Chloro-4-nitrothiophene-2-sulfonic acid (3,5-dichlorophenyl)amide [39];
5-Chloro-4-nitrothiophene-2-sulfonic acid (3,5-difluorophenyl)amide [40];
5-(1-Methyl-5-trifluoromethyl-1*H*-pyrazol-4-yl)-thiophene-2-sulfonic acid (3,5-bistrifluoromethylphenyl)amide [43];
5-Chloro-4-nitrothiophene-2-sulfonic acid (3,5-bis-trifluoromethylphenyl)amide [44];
5-Chloro-4-nitrothiophene-2-sulfonic acid 4-fluorobenzylamide [45];
5-Chloro-4-nitrothiophene-2-sulfonic acid 4-trifluoromethylbenzylamide [46];
5-Chloro-4-nitrothiophene-2-sulfonic acid [2-(1*H*-indol-3-yl)-ethyl]amide [48];
5-Chloro-4-nitrothiophene-2-sulfonic acid [2-(1*H*-indol-3-yl)-1-methylethyl]amide [49];
5-Chloro-4-nitrothiophene-2-sulfonic acid methyl-(4-trifluoromethylphenyl) amide [50];
5-Chloro-4-nitrothiophene-2-sulfonic acid (4-chlorophenyl)methylamide [51]; and
5-Chloro-4-nitrothiophene-2-sulfonic acid methyl (4-trifluoromethylbenzyl)amide [52]
5-Chloro-4-nitro-thiophene-2-sulfonic acid benzyl-(4-fluoro-benzyl)-amide [53];
5-Chloro-4-nitro-thiophene-2-sulfonic acid 3,5-dichloro-benzylamide [54];
5-Chloro-4-nitro-thiophene-2-sulfonic acid 3,5-difluoro-benzylamide [55];
5-Chloro-4-nitro-thiophene-2-sulfonic acid 4-chloro-benzylamide [56];
5-Chloro-4-nitro-thiophene-2-sulfonic acid [1-(4-fluoro-phenyl)-ethyl]-amide [57];
5-Chloro-4-nitro-thiophene-2-sulfonic acid (1H-benzo-imidazol-2-yl)-amide [59];
5-Chloro-4-nitro-thiophene-2-sulfonic acid [2-(6-chloro-1H-indol-3-yl)-ethyl]-amide [60];
5-Chloro-4-nitro-thiophene-2-sulfonic acid (4-methoxy-phenyl)-amide [61];
5-Chloro-4-nitro-thiophene-2-sulfonic acid p-tolylamide [63];
5-Chloro-4-nitro-thiophene-2-sulfonic acid benzyl-(4-chloro-phenyl)-amide [65];
5-Chloro-4-nitro-thiophene-2-sulfonic acid benzyl-(4-methoxy-phenyl)-amide [66];
5-Chloro-4-nitro-thiophene-2-sulfonic acid (4-chloro-phenyl)-(3-trifluoromethyl-benzyl)-amide [67];
4-Nitro-thiophene-2-sulfonic acid (4-chloro-phenyl)-amide [69];
5-Chloro-thiophene-2,4-disulfonic acid bis-[(4-chloro-phenyl)-amide] [70];
Thioacetic acid S-[5-(4-chloro-phenylsulfamoyl)-3-nitro-thiophen-2-yl] ester [72];
5-Methyl-4-nitro-thiophene-2-sulfonic acid (4-chloro-phenyl)-amide [73];
5-Methyl-thiophene-2,4-disulfonic acid bis-[(4-chloro-phenyl)-amide] [74];
5-Chloro-4-nitro-thiophene-2-sulfonic acid (3-trifluoro-methyl-benzyl)-(4-trifluoromethylbenzyl)-amide [75];
5-(2-Methoxy-ethylamino)-4-nitro-thiophene-3-sulfonic acid (4-chloro-phenyl)-amide [80];
N-(1H-Benzoimidazol-2-yl)-4-chloro-3-nitro-benzenesulfonamide [83];
5-Chloro-3-methyl-benzo[b]thiophene-2-sulfonic acid (4-chloro-3-nitro-phenyl)-amide [88];
N-(3,5-Bis-trifluoromethyl-phenyl)-3-chloro-4-methoxy-benzene-sulfonamide [104];
N-(3-Chloro-4-nitro-phenyl)-3,5-bis-trifluoromethyl-benzenesulfonamide [105];
4,5-Dibromo-thiophene-2-sulfonic acid (3,5-dichloro-phenyl)-amide [112];
5-Bromo-thiophene-2-sulfonic acid (4-trifluoromethyl-phenyl)-amide [116];
5-Benzenesulfonyl-thiophene-2-sulfonic acid (3,5-bis-trifluoromethyl-phenyl)-amide [117];
5-Chloro-3-methyl-benzo[b]thiophene-2-sulfonic acid (3,5-bis-trifluoromethyl-phenyl)-amide [119];
Benzo[b]thiophene-2-sulfonic acid (3,5-bis-trifluoromethyl-phenyl)-amide [120];
Benzo[1,2,5]thiadiazole-5-sulfonic acid (3,5-bis-trifluoromethyl-phenyl)-amide [122];
4,5-Dibromo-thiophene-2-sulfonic acid (4-chloro-phenyl)-amide [125];
4,5-Dibromo-thiophene-2-sulfonic acid (4-trifluoromethyl-phenyl)-amide [126];
N-(3,5-Bis-trifluoromethyl-phenyl)-2,6-dichloro-benzenesulfonamide [128];
5-(3-Hydroxy-piperidin-1-yl)-4-nitro-thiophene-2-sulfonic acid (4-chloro-phenyl)-amide [130]; and
5-Chloro-4-nitro-thiophene-2-sulfonic acid (4-nitro-phenyl)-amide [131].

More preferably, the compound of formula I is selected from the following: [2], [3], [6], [7], [33], [39], [40], [44]-[46], [48]-[57], [59]-[61], [63], [65]-[67], [72], [75], [130] and [131].

Even more preferably, the compound of formula I is selected from the following: [7], [45], [46], [50]-[53], [65]-[67], [72] and [75].

More preferably still, the compound of formula I is selected from the following: [50], [51], [65], [67] and [75].

Another preferred embodiment of the invention relates to the use of a compound of formula Ic, 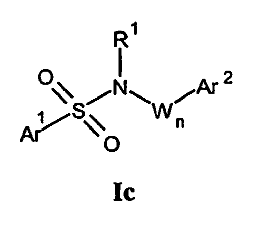 wherein
W is a C₁₋₅ branched or unbranched alkyl group or a C₂₋₅ alkenyl group;
n is 0 or 1;
R¹ is H, a C₁₋₈ branched or unbranched alkyl group, a C₂₋₈ alkenyl group, or an aryl or aralkyl group;
Ar¹ is 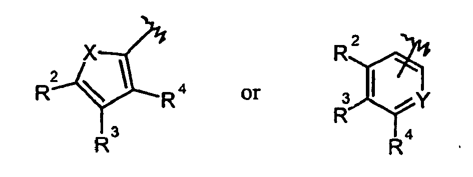 wherein
   X is S, O, NH or NR' where R' is a C₁₋₃ alkyl group;
   Y is CH or N;
   R², R³ and R⁴ are each independently (A)ₚB, wherein A is C₁₋₃ alkyl, p is 0 or 1, and B is H, halogen, C₁₋₅ alkyl, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, or a heteroaryl group optionally substituted by one or more C₁₋₅ alkyl, halogen, SRⁱ or CF₃ groups;
   Ar^{a-e} are each independently aryl groups optionally substituted by one or more C₁₋₅ alkyl, halogen or CF₃ groups;
   R^{a-i} are each independently C₁₋₅ alkyl groups;
   and with the proviso that at least one of R², R³ and R⁴ is other than H;
Ar² is 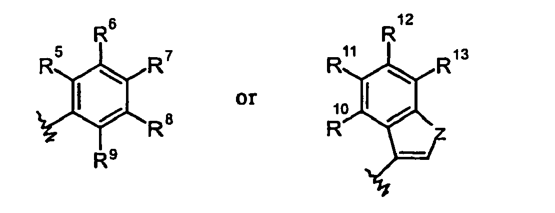 wherein
   Z is S, O, NH or NR" where R" is C₁₋₃ alkyl;
   R⁵, R⁶, R⁷, R⁸ and R⁹ are each independently (L)_{q}M wherein L is C₁₋₃ alkyl, q is 0 or 1, M is H, C₁₋₅ alkyl, halogen, NO₂, OH, NH₂, NHAr, NHR^{j}, NR^{k}R^{l}, SO₃H, SO₂NH₂, SO₂NHAr, SO₂NHR^{m}, SO₂Ar, SO₂Rⁿ, CF₃, CN, COOH, COOR^{p}, CONH₂, CONHAr, CONHR^{q}, COAr or COR^{r};
   R^{i-r} are each independently C₁₋₅ alkyl groups;
   and with the proviso that at least one of the substituents R⁵, R⁶, R⁷, R⁸ and R⁹ is other than H;
   R¹⁰, R¹¹, R¹² and R¹³ are each independently H, C₁₋₅ alkyl, halogen, NO₂, OH, NH₂ or CF₃;
in the preparation of a medicament for treating a proliferative disorder.

In one preferred embodiment, Ar¹ is

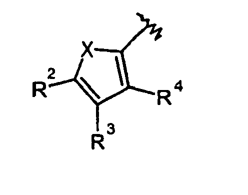

In another preferred embodiment, Ar¹ is

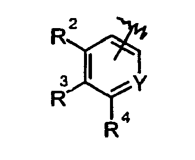

In one preferred embodiment, Ar² is

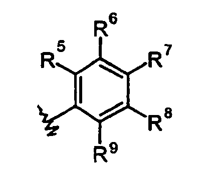

In another preferred embodiment, Ar² is

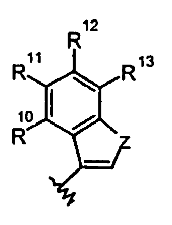

In one preferred embodiment, Ar¹ is 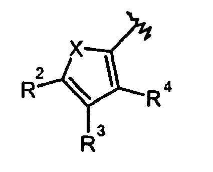 and Ar² is

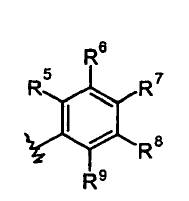

Preferably,
- R², R³ and R⁴ are each independently (A)ₚB, wherein A is C₁₋₅ alkyl, p is 0 or 1, and B is H, F, Cl, Br, I, C₁₋₅ alkyl, NO₂, OH, NH₂, NHR^{a}, 14R^{b}R^{c}, SO₃H, SO₂NH₂, NHPh, SO₂NHAr, SO₂NHR^{d}, SO₂Ph, SO₂R^{e}, CF₃, CN, COOH, COOR^{f}, CONH₂, COONHPh, CONHR^{g}, COPh, COR^{h}, or a heteroaryl group selected from pyridyl, pyrimidyl, oxazolyl, thiazolyl and pyrazolyl, each of which may be optionally substituted by one or more C₁₋₅ alkyl, halogen, SRⁱ or CF₃ groups; and
- R⁵, R⁶, R⁷, R⁸, and R⁹ are each independently (L)_{q}M wherein L is C₁₋₅ alkyl, q is 0 or 1, M is H, C₁₋₅ alkyl, halogen, NO₂, OH, NH₂, NHPh, NHR^{j}, NR^{k}R^{l}, SO₃H, SO₂NH₂, SO₂NHPh, SO₂NHR, SO₂Ph, SO₂R^{m}, CF₃, CN, COOH, COORⁿ, CONH₂, CONHPh, CONHR^{p}, COPh or COR^{q}.

More preferably, R², R³ and R⁴ are each independently (A)ₚB, wherein A is C₁₋₅ alkyl, p is 0 or 1, and B is H, F, Cl, Br, I, C₁₋₅ alkyl, NO₂, OH, NH₂, NHR^{a}, NR^{b}R^{c}, SO₃H, SO₂NH₂, NHPh, SO₂NHPh, SO₂NHR^{d}, SO₂Ph, SO₂R^{e}, CF₃, CN, COOH, COOR^{f}, CONH₂, COONHPh, CONHR^{g}, COPh, COR^{h}, pyridyl, pyrimidyl, 2-methylsulfanylpyrimid-5-yl, oxazol-2-yl, thiazol-2-yl, or 1-methyl-5-trifluoromethyl-1*H*-pyrazol-4-yl.

More preferably still, R², R³ and R⁴ are each independently H, halogen, NO₂, SO₂Ph, 1-methyl-5-trifluoromethyl-1*H*-pyrazol-4-yl, 2-methylsulfanyl pyrimid-5-yl, N-(4-fluorophenyl)sulfonamido, N-(4-trifluoromethylphenyl)-sulfonamido, cxazol-2-yl, C₁₋₅ alkyl or NH₂.

In one preferred embodiment, Ar¹ is 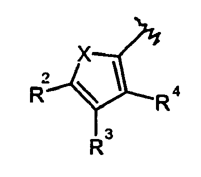
X is S;
R², R³ and R⁴ are each independently H, halogen, NO₂, SO₂Ph, 1-methyl-5-trifluoromethyl-1*H*-pyrazol-4-yl, 2-methylsulfanylpyrimid-5-yl, N-(4-fluorophenyl) sulfonamido or N-(4-trifluoromethylphenyl)-sulfonamido.

Preferably,
- R² is halogen, SO₂Ph, 1-methyl-5-trifluoromethyl-1*H*-pyrazol-4-yl or 2-methylsulfanyl-pyrimid-5-yl;
- R³ is halogen, H, NO₂, N-(4-fluorophenyl)sulfonamido or N-(4-trifluoro methylphenyl)-sulfonamido; and
- R⁴ is H.

More preferably, Ar¹ is 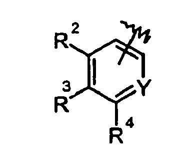
Y is CH or N; and
R², R³ and R⁴ are each independently H, NO₂, NH₂, halogen, oxazol-2-yl or C₁₋₅ alkyl.

More preferably,
when Y is CH
- R² is H;
- R³ is NO₂ or NH₂; and
- R⁴ is H, Cl, oxazol-2-yl or Me; or
when Y is N
- R² is H;
- R³ is Br;
- R⁴ is Cl.

Preferably,
- R⁵, R⁶, R⁷, R⁸ and R⁹ are each independently H, halogen, CF₃ or OH; and
- R¹⁰, R¹¹, R¹² and R¹³ are all H.

More preferably,
- R⁵ is H or halogen;
- R⁶ is H, halogen or CF₃;
- R⁷ is H, halogen, OH or CF₃;
- R⁹ is H.

Preferably,
- W is CH₂, CH₂CH₂ or CH(CH₃)CH₂; and
- R¹ is H or Me.

### THERAPEUTIC USE

The compounds of the invention have been found to possess anti-proliferative activity and are therefore believed to be of use in the treatment of proliferative disorders, such as cancers, leukaemias or other disorders associated with uncontrolled cellular proliferation such as psoriasis and restenosis.

As defined herein, an anti-proliferative effect within the scope of the present invention may be demonstrated by the ability to inhibit cell proliferation in an *in vitro* whole cell assay, for example using any of the cell lines AGS, H1299 or SJSA-1, or by showing inhibition of the interaction between HDM2 and p53 in an appropriate assay. These assays, including methods for their performance, are described in more detail in the accompanying Examples. Using such assays it may be determined whether a compound is anti-proliferative in the context of the present invention.

One aspect of the present invention therefore relates to the use of one or more compounds of the invention in the treatment of proliferative disorders.

The term "proliferative disorder" is used herein in a broad sense to include any disorder that requires control of the cell cycle, for example cardiovascular disorders such as restenosis and cardiomyopathy, auto-immune disorders such as glomerulonephritis and rheumatoid arthritis, dermatological disorders such as psoriasis, anti-inflammatory, anti-fungal, antiparasitic disorders such as malaria, emphysema and alopecia. In these disorders, the compounds of the present invention may induce apoptosis or maintain stasis within the desired cells as required. Preferably, the proliferative disorder is a cancer or leukaemia.

In one preferred embodiment of the invention, the compound of the invention is administered in an amount sufficient to modulate the interaction between HDM2 and p53.

Even more preferably, the compound of the invention is administered in an amount sufficient to inhibit the interaction between HDM2 and p53.

It is known in the art that HDM2 forms a negative autoregulatory loop with p53 by binding to its *N*-terminal activation domain thereby inhibiting the functions of p53 and promoting the proteolytic degradation of p53. Interference with this regulatory loop can be used to increase the concentration of active p53 in cells. Thus in tumours with wild-type p53, the equilibrium concentration of active p53 can be increased by antagonising the interaction between HDM2 and p53. This will result in restoration of the p53-mediated pro-apoptotic and anti-proliferative effects in such tumour cells. In tumour types sensitive to increases in functional p53 [Hansen R, Reddel R, Braithwaite A; Oncogene 1995; 11: 2535-2545], it is expected that the compounds of the present invention will be sufficient to induce apoptosis.

The negative regulation of p53 by HDM2 may limit the magnitude of p53 activation by DNA damaging agents currently used (chemotherapy and radiotherapy), thereby limiting their therapeutic effectiveness. Thus if the HDM2 feed-back inhibition of p53 is interrupted, an increase in functional p53 levels will increase the therapeutic effectiveness of such agents by restoring the wild-type p53 function that leads to apoptosis and/or by reversing p53-associated drug resistance. It was thus demonstrated that combining HDM2 inhibition [Wang H, Zeng X, Oliver P, Le LP, Chen J, Chen L, Zhou W, Agrawal S, Zhang R; Int. J. Oncol. 1999; 15: 653-660] and DNA-damaging treatments *in vivo* led to synergistic anti-tumour effects. Therefore, the compounds of the present invention also have therapeutic applications in sensitising tumour cells for chemotherapy and radiotherapy.

The oncogenic potential of HDM2 is not only determined by its ability to suppress p53, but also by its ability to regulate other tumour suppressor proteins, in particular the retinoblastoma protein pRb and the closely associated E2F1 transcription factor.

Thus, in a further preferred embodiment of the invention, the compound of the invention is administered in an amount sufficient to modulate the interaction between HDM2 and E2F transcription factors.

Even more preferably, the compound of the invention is administered in an amount sufficient to inhibit the interaction between HDM2 and E2F transcription factors.

HDM2 has been shown to interact directly with the pRb-regulated transcription factor E2F1/DP1 [Martin K, Trouche D, Hagemeier C, Sorensen TS, La Thangue NB, Kouzarides T; Nature 1995; 375: 691-694], whose activation capacity it stimulates. Thus overexpression of HDM2 increases E2F1-mediated transactivation [Daujat S, Neel H, Piette J; Trends Genet. 2001; 17: 459-464]. In the absence of p53, HDM2 inhibits the pro-apoptotic effect of E2F1/DP1 by favouring degradation of the heterodimer [Kowalik TF, Degregori J, Leone G, Jakoi L, Nevins JR; Cell Growth Differ. 1998; 9: 113-118]. Simultaneously, however, HDM2 still stimulates DNA synthesis in co-operation with E2F1/DP1. These apparently contradictory observations were reconciled by the proposal that high E2F1/DP1 levels in tumour cells are reduced by HDM2 to appropriate levels for the G1-S phase transition [Loughran 0, La Thangue NB; Mol. Cell. Biol. 2000; 20: 2186-2197]. In any case, the anti-apoptotic and growth-promoting activities of HDM2 seem to converge on a single target, E2F1, which could be crucial for the p53-independent oncogenic activities of HDM2 [Daujat S *et al, ibid*].
A domain of E2F1 (amino acids 390 to 406) shows striking similarity to the HDM2-binding domain of p53. Since the interactions of HDM2 with both p53 and E2F1 locate to the same binding site on HDM2, it can be expected that HDM2/p53 antagonists, such as the compounds of the present invention, will not only activate cellular p53 but also suppress E2F1 activities, which are commonly deregulated in tumour cells. Furthermore, it can be expected that the compounds of the invention will exhibit anti-proliferative effects in tumour cells, even if such cells are devoid of functional p53.

### COMPOUNDS

Another aspect of the invention relates to compounds of formula Ia, 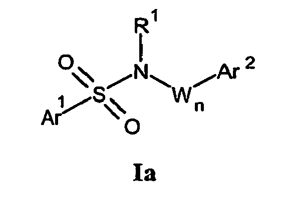 wherein
W is a C₁₋₅ branched or unbranched alkylene group or a C₂₋₅ alkenylene group;
n is 0 or 1;
R¹ is H, a C₁₋₈ branched or unbranched alkyl group, a C₂₋₈ alkenyl group, or an aryl or aralkyl group, each of which may be optionally substituted by one or more halogen or CF₃ groups;
Ar¹ is 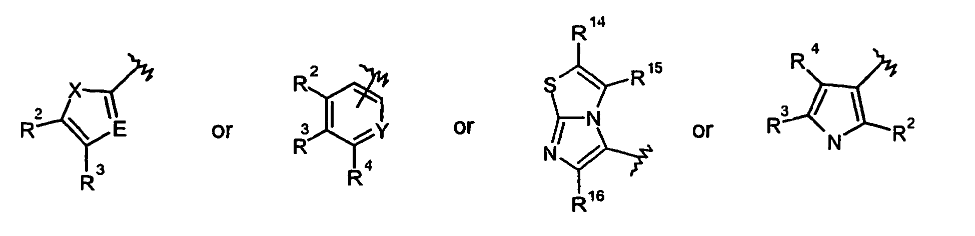 wherein
   X is O, NH or NR' where R' is a C₁₋₃ alkyl group;
   E is N or CR⁴;
   Y is N;
   R², R³, R⁴, and R¹⁴⁻¹⁶ are each independently (A)ₚB, wherein A is C₁₋₃ alkyl, p is 0 or 1, and B is H, halogen, C₁₋₅ alkyl, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f} an alicyclic group optionally containing one or more heteroatoms, optionally substituted by one or more OH, COR^{u}, halogen or CF₃ groups, or a heteroaryl group optionally substituted by one or more C₁₋₅ alkyl, halogen, SRⁱ or CF₃ groups; or
   R² and R³ are linked to form a saturated or unsaturated ring system, optionally containing one or more heteroatoms, and optionally substituted by one or more halogen, OH or CF₃ groups;
   Ar^{a-f} are each independently aryl groups optionally substituted by one or more C₁₋₅ alkyl, halogen or CF₃ groups;
   R^{a-i}, R^{s}, R^{t} and R^{u} are each independently C₁₋₅ alkyl groups optionally substituted by one or more alkoxy, halogen or CF3 groups;
   and with the proviso that at least one of R², R³ and R⁴ is other than H;
Ar² is 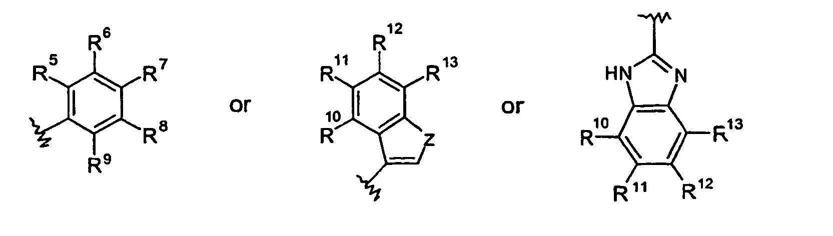 wherein
   Z is S, O, NH or NR" where R" is C₁₋₃ alkyl;
   R⁵, R⁶, R⁷, R⁸ and R⁹ are each independently (L)_{q}M wherein L is C₁₋₃ alkyl, q is 0 or 1, M is H, C₁₋₅ alkyl, halogen, NO₂, OH, NH₂, NHAr^{g}, NHR^{j}, NR^{k}R^{l}, SO₃H, SO₂NH₂, SO₂NHAr^{h}, SO₂NHR^{m}, SO₂Arⁱ, SO₂Rⁿ, CF₃, CN, COOH, COOR^{p}, CONH₂, CONHAr^{j}, CONHR^{q}, OR^{v}, COAr^{k} or COR^{r};
   R^{j-r}, R^{v} are each independently C₁₋₅ alkyl groups;
   Ar^{g-k} are each independently aryl groups;
   and with the proviso that at least one of the substituents R⁵, R⁶, R⁷, R⁸ and R⁹ is other than H;
   R¹⁰, R¹¹, R¹² and R¹³ are each independently H, C₁₋₅ alkyl, halogen, NO₂, OH, NH₂ or CF₃;
with the proviso that said compound is other than 5-[(4-chlorophenyl)amino]sulfonyl-2-furancarboxylic acid.

Another aspect of the invention relates to compounds of formula **Ib,** 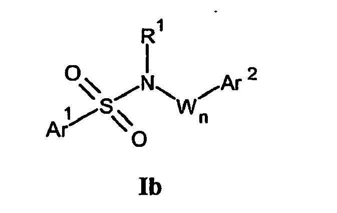 wherein
W is a C₁₋₅ branched or unbranched alkylene group or a C₂₋₅ alkenylene group;
n is 0 or 1;
R¹ is H, a C₁₋₈ branched or unbranched alkyl group, a C₂₋₈ alkenyl group, or an aryl or aralkyl group, each of which may be optionally substituted by one or more halogen or CF₃ groups;
Ar¹ is 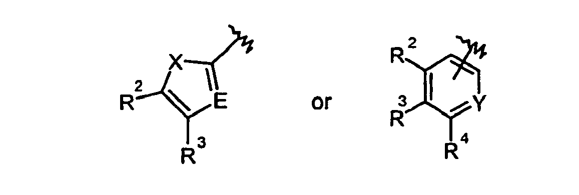 wherein
   X is S, O, NH or NR' where R' is a C₁₋₃ alkyl group;
   E is N or CR⁴;
   Y is CH or N;
   R², R³ and R⁴ are each independently (A)ₚB, wherein A is C₁₋₃ alkyl, p is 0 or 1, and B is H, halogen, C₁₋₅ alkyl, NO₂, OH, NH₂, NHR^{a}, NR^{b}R^{c}, SO₃H, SO₂NH₂, NHAr^{a}, SO₂NHAr^{h}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, an alicyclic group optionally containing one or more heteroatoms, optionally substituted by one or more OH, COR^{u}, halogen or CF₃ groups, or a heteroaryl group optionally substituted by one or more C₁₋₅ alkyl, halogen, SRⁱ or CF₃ groups; or
   R² and R³ are linked to form a saturated or unsaturated ring system, optionally containing one or more heteroatoms, and optionally substituted by one or more halogen, OH or CF₃ groups;
   Ar^{a-f} are each independently aryl groups optionally substituted by one or more C₁₋₅ alkyl, halogen or CF₃ groups;
   R^{a-i}, R^{s}, R^{t} and R^{u} are each independently C₁₋₅ alkyl groups optionally substituted by one or more alkoxy, halogen or CF₃ groups;
   and with the proviso that at least one of R², R³ and R⁴ is other than H;
Ar² is 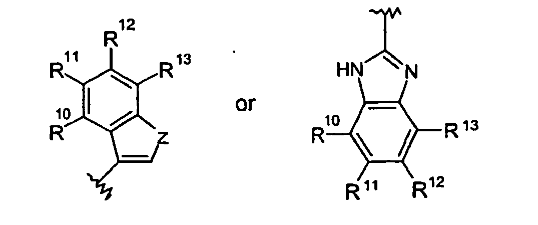 wherein
   Z is S, O, NH or NR" where R" is C₁₋₃ alkyl;
   R⁵, R⁶, R⁷, R⁸ and R⁹ are each independently (L)_{q}M wherein L is C₁₋₃ alkyl, q is 0 or 1, M is H, C₁₋₅ alkyl, halogen, NO₂, OH, NH₂, NHAr^{g}, NHR^{j}, NR^{k}R^{l}, SO₃H, SO₂NH₂, SO₂NHAr^{h}, SO₂NHR^{m}, SO₂Arⁱ, SO₂Rⁿ, CF₃, CN, COOH, COOR^{p}, CONH₂, CONHAr^{j}, CONHR^{q}, OR^{v}, COAr^{k} or COR^{r};
   R^{j-r}, R^{v} are each independently C₁₋₅ alkyl groups;
   Ar^{g-k} are each independently aryl groups;
   and with the proviso that at least one of the substituents R⁵, R⁶, R⁷, R⁸ and R⁹ is other than H;
   R¹⁰, R¹¹, R¹² and R¹³ are each independently H, C₁₋₅ alkyl, halogen, NO₂, OH, NH₂ or CF₃.

Another aspect of the invention relates to compounds of formula **Id**, 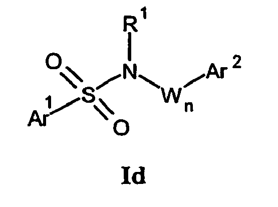 wherein
W is a C₁₋₅ branched or unbranched alkyl group or a C₂₋₅ alkenyl group;
n is 0 or 1;
R¹ is H, a C₁₋₈ branched or unbranched alkyl group, a C₂₋₈ alkenyl group, or an aryl or aralkyl group;
Ar¹ is 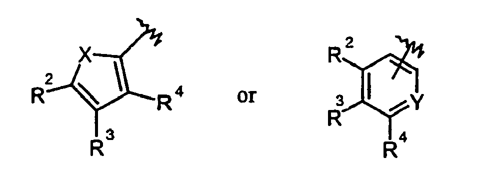 wherein
   X is O, NH or NR' where R' is a C₁₋₃ alkyl group;
   Y is CH or N;
   R², R³ and R⁴ are each independently (A)ₚB, wherein A is C₁₋₃ alkyl, p is 0 or 1, and B is H, halogen, C₁₋₅ alkyl, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, or a heteroaryl group optionally substituted by one or more C₁₋₅ alkyl, halogen, SRⁱ or CF₃ groups;
   Ar^{a-e} are each independently aryl groups optionally substituted by one or more C₁₋₅ alkyl, halogen or CF₃ groups;
   R^{a-i} are each independently C₁₋₅ alkyl groups;
   and with the proviso that at least one of R², R³ and R⁴ is other than H;
Ar² is 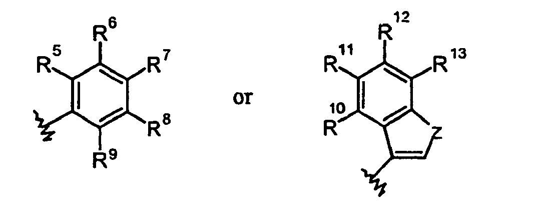 wherein
   Z is S, O, NH or NR" where R" is C₁₋₃ alkyl;
   R⁵, R⁶, R⁷, R⁸ and R⁹ are each independently (L)_{q}M wherein L is C₁₋₃ alkyl, q is 0 or 1, M is H, C₁₋₅ alkyl, halogen, NO₂, OH, NH₂, NHAr, NHR^{j}, NR^{k}R^{l}, SO₃H, SO₂NH₂, SO₂NHAr, SO₂NHR^{m}, SO₂Ar, SO₂Rⁿ, CF₃, CN, COOH, COOR^{p}, CONH₂, CONHAr, CONHR^{q}, COAr or COR^{r};
   R^{i-r} are each independently C₁₋₅ alkyl groups;
   and with the proviso that at least one of the substituents R⁵, R⁶, R⁷, R⁸ and R⁹ is other than H;
   R¹⁰, R¹¹, R¹² and R¹³ are each independently H, C₁₋₅ alkyl, halogen, NO₂, OH, NH₂ or CF₃;
with the proviso that said compound is other than
5-[(4-chlorophenyl)amino]sulfonyl-2-furancarboxylic acid;
N-[3,5-bis(trifluoromethyl)phenyl]-4-chloro-3-nitrobenzensulfonamide;
2,6-dichloro-N-[4-chloro-3-(3-diethylaminopropyl)-phenyl]4-trifluoromethylbenzene-sulfonamide; or
2,6-dichloro-N-[4-chloro-3-(3-dimethylaminopropyl)-phenyl]4-trifluoromethylbenzene-sulfonamide.

Preferred embodiments in respect of compounds of formula 1a, 1b and 1d are as defined above for compounds of formula I.

Another aspect of the invention relates to a compound selected from the following:
5-Chloro-4-nitrothiophene-2-sulfonic acid (3-trifluoromethylphenyl)amide [1];
5-Chloro-4-nitrothiophene-2-sulfonic acid (4-chlorophenyl)-amide [2];
5-Chloro-4-nitrothiophene-2-sulfonic acid (4-fluorophenyl)amide [3];
4-Bromo-5-chlorothiophene-2-sulfonic acid (4-fluorophenyl)amide [4];
5-Chloro-4-nitrothiophene-2-sulfonic acid (4-hydroxyphenyl)amide [5];
5-Chloro-4-nitrothiophene-2-sulfonic acid (4-trifluoromethylphenyl)amide [6];
5-Chloro-4-nitrothiophene-2-sulfonic acid (4-fluorophenyl)methylamide [7];
4,5-Dibromothiophene-2-sulfonic acid (3,5-bis-trifluoromethylphenyl)amide [8];
5-Chlorothiophene-2-sulfonic acid (3,5-bis-trifluoromethylphenyl)amide [11];
5-(2-Methylsulfanyl-pyrimidin-5-yl)-thiophene-2-sulfonic acid (3,5-dichlorophenyl)-amide [12];
4-Oxazol-2-yl-N-(4-trifluoromethylphenyl)benzenesulfonamide [13];
N-(3,5-Bis-trifluoromethylphenyl)-4-oxazol-2-yl-benzenesulfonamide [14];
4-Bromo-5-chlorothiophene-2-sulfonic acid (4-trifluoromethylphenyl)amide [15];
5-Bromothiophene-2-sulfonic acid (4-chlorophenyl)amide [16];
5-Bromothiophene-2-sulfonic acid (3,5-dichlorophenyl)amide [17];
5-Bromothiophene-2-sulfonic acid (3,5-bis-trifluoromethylphenyl)amide [18];
N-(4-Chlorophenyl)-3-nitrobenzenesulfonamide [19];
3-Nitro-N-(4-trifluoromethylphenyl)benzenesulfonamide [20];
N-(3,5-Bis-trifluoromethylphenyl)-3-nitrobenzenesulfonamide [21];
N-(2,4-Dichlorophenyl)-3-nitrobenzenesulfonamide [22];
5-Benzenesulfonylthiophene-2-sulfonic acid (4-trifluoromethylphenyl)-amide [23];
5-Benzenesulfonylthiophene-2-sulfonic acid (4-chlorophenyl)amide [24];
5-Benzenesulfonylthiophene-2-sulfonic acid (3,5-dichlorophenyl)amide [25];
5-Chlorothiophene-2-sulfonic acid (3,4-dichlorophenyl)amide [26];
4,5-Dibromothiophene-2-sulfonic acid (3-trifluoromethylphenyl)amide [27];
4,5-Dibromothiophene-2-sulfonic acid (3,4-dichlorophenyl)amide [28];
4-Chloro-N-(3,4-dichlorophenyl)-3-nitrobenzenesulfonamide [30];
5-(1-Methyl-5-trifluoromethyl-1H-pyrazol-4-yl)-thiophene-2-sulfonic acid (4-trifluoromethylphenyl)-amide [31];
5-Chlorothiophene-2,4-disulfonic acid bis-[(4-fluorophenyl)-amide] [32];
5-Chlorothiophene-2,4-disulfonic acid bis-[(4-trifluoro-methyl-phenyl)-amide] [33];
4-Methyl-3-nitro-N-(4-trifluoromethylphenyl)benzenesulfonamide [34];
4-Chloro-3-nitro-N-(4-trifluoromethylphenyl)benzenesulfonamide [35];
3-Amino-4-methyl-N-(4-trifluoromethyl-phenyl)benzenesulfonamide [36];
N-(4-Chlorophenyl)-4-methyl-3-nitrobenzenesulfonamide [37];
4-Chloro-N-(4-chlorophenyl)-3-nitro-benzenesulfonamide [38];
5-Chloro-4-nitrothiophene-2-sulfonic acid (3,5-dichlorophenyl)-amide [39];
5-Chloro-4-nitrothiophene-2-sulfonic acid (3,5-difluorophenyl)-amide [40];
5-Bromo-6-chloropyridine-3-sulfonic acid (4-trifluoromethylphenyl)amide [41];
5-Bromo-6-chloropyridine-3-sulfonic acid (3,5-bis-trifluoromethylphenyl)amide [42];
5-(1-Methyl-5-trifluoromethyl-1H-pyrazol-4-yl)-thiophene-2-sulfonic acid (3,5-bistrifluoromethylphenyl)amide [43];
5-Chloro-4-nitrothiophene-2-sulfonic acid (3,5-bis-trifluoromethyl-phenyl)-amide [44];
5-Chloro-4-nitrothiophene-2-sulfonic acid 4-fluorobenzylamide [45];
5-Chloro-4-nitrothiophene- 2-sulfonic acid 4-trifluoromethylbenzylamide [46];
4-Chloro-N-(3,5-dichlorophenyl)-3-nitro-benzenesulfonamide [47];
5-Chloro-4-nitrothiophene-2-sulfonic acid [2-(1H-indol-3-yl)-ethyl]amide [48];
5-Chloro-4-nitrothiophene-2-sulfonic acid [2-(1H-indol-3-yl)-1-methylethyl]amide [49];
5-Chloro-4-nitrothiophene- 2-sulfonic acid methyl-(4-trifluoromethylphenyl)amide [50];
5-Chloro-4-nitrothiophene-2-sulfonic acid (4-chloro-phenyl)-methylamide [51]; and
5-Chloro-4-nitrothiophene-2-sulfonic acid methyl-(4-trifluoromethylbenzyl)amide [52];
5-Chloro-4-nitro-thiophene-2-sulfonic acid benzyl-(4-fluoro-benzyl)-amide [53];
5-Chloro-4-nitro-thiophene-2-sulfonic acid 3,5-dichloro-benzylamide [54];
5-Chloro-4- nitro-thiophene- 2-sulfonic acid 3,5-difluoro-benzylamide [55];
5-Chloro-4-nitro-thiophene-2-sulfonic acid 4-chloro-benzylamide [56];
5-Chloro-4-nitro-thiophene-2-sulfonic acid [1-(4-fluoro-phenyl)-ethyl]-amide [57];
5-Chloro-4-nitro-thiophene-2-sulfonic acid (4-chloro-phenyl)-isobutyl-amide [58];
5-Chloro-4-nitro-thiophene-2-sulfonic acid (1H-benzo-imidazol-2-yl)-amide [59];
5-Chloro-4-nitro-thiophene-2-sulfonic acid [2-(6-chloro-1H-indol-3-yl)-ethyl]-amide [60];
5-Chloro-4-nitro-thiophene-2-sulfonic acid (4-methoxy-phenyl)-amide [61];
5-Chloro-4-nitro-thiophene-2-sulfonic acid phenylamide [62];
5-Chloro-4-nitro-thiophene-2-sulfonic acid p-tolylamide [63];
5-Chloro-4-nitro-thiophene-2-sulfonic acid benzylamide [64];
5-Chloro-4-nitro-thiophene-2-sulfonic acid benzyl-(4-chloro-phenyl)-amide [65];
5-Chloro-4-nitro-thiophene-2-sulfonic acid benzyl-(4-methoxy-phenyl)-amide [66];
5-Chloro-4-nitro-thiophene-2-sulfonic acid (4-chloro-phenyl)-(3-trifluoromethyl-benzyl)-amide [67];
5-Nitro-thiophene-2-sulfonic acid (4-chloro-phenyl)-amide [68];
4-Nitro-thiophene-2-sulfonic acid (4-chloro-phenyl)-amide [69];
5-Chloro-thiophene-2,4-disulfonic acid bis-[(4-chloro-phenyl)-amide] [70];
5-Ethyl-4-nitro-thiophene-2-sulfonic acid (4-chloro-phenyl)-amide [71];
Thioacetic acid S-[5-(4-chloro-phenylsulfamoyl)-3-nitro-thiophen-2-yl] ester [72];
5-Methyl-4-nitro-thiophene-2-sulfonic acid (4-chloro-phenyl)-amide [73];
5-Methyl-thiophene-2,4-disulfonic acid bis-[(4-chloro-phenyl)-amide] [74];
5-Chloro-4-nitro-thiophene-2-sulfonic acid (3-trifluoro-methyl-benzyl)-(4-trifluoromethylbenzyl)-amide [75];
4-Nitro-thiophene-2-sulfonic acid (4-trifluoromethyl-phenyl)-amide [76];
4-Nitro-thiophene-2-sulfonic acid [2-(1H-indol-3-yl)-ethyl]-amide [77];
5-(1-Methyl-5-tri-fluoromethyl-1H-pyrazol-3-yl)-thiophene-2-sulfonic acid (3,5-bis-trifluoromethyl-phenyl)-(3-trifluoro-methyl-benzyl)-amide [78];
5-Morpholin-4-yl-4-nitro-thiophene-2-sulfonic acid (4-chloro-phenyl)-amide [79];
5-(2-Methoxy-ethylamino)-4-nitro-thiophene-2-sulfonic acid (4-chloro-phenyl)-amide [80];
4-Chloro-N-[2-(5-chloro-1H-indol-3-yl)-ethyl]-3-nitro-benzenesulfonamide [81];
N-[2-(5-Chloro-1H-indol-3-yl)-ethyl]-4-methyl-3-nitro-benzenesulfonamide [82];
N-(1H-Benzoimidazol-2-yl)-4-chloro-3-nitro-benzenesulfonamide [83];
6-Chloro-imidazo[2,1-b]thiazole-5-sulfonic acid (3,5-bis-trifluoro-methyl-phenyl)-amide [84];
2,3-Dihydro-benzo[1,4]dioxine-6-sulfonic acid (4-chloro-phenyl)-amide [85];
2,3-Dihydro-benzo[1,4]dioxine-6-sulfonic acid (3,5-bis-trifluoromethyl-phenyl)-amide [86];
6-Phenoxy-pyridine-3-sulfonic acid (4-chloro-phenyl)-amide [87];
5-Chloro-3-methyl-benzo[b]thiophene-2-sulfonic acid (4-chloro-3-nitro-phenyl)-amide [88];
N-(3,5-Bis-trifluoromethyl-phenyl)-4-pyrazol-1-yl-benzenesulfonamide [89];
4-(4-Chloro-phenylsulfamoyl)-3,5-dimethyl-1H-pyrrole-2-carboxylic acid ethyl ester [90];
4-(3,5-Bis-trifluoromethyl-phenylsulfamoyl)-3,5-diinethyl-1H-pyrrole-2-carboxylic acid [91];
4-(4-Chloro-phenylsulfamoyl)-3,5-dimethyl-1H-pyrrole-2-carboxylic acid [92];
2-(4-Chloro-phenylsulfamoyl)-4-methyl-thiazole-5-carboxylic acid ethyl ester [93];
3,5-Dichloro-N-(4-chloro-phenyl)-4-hydroxy-benzenesuifonamide [94];
N-(3,5-Bis-trifluoromethyl-phenyl)-3,5-dichloro-4-hydroxy-benzenesulfonamide [95];
3,5-Dichloro-4-hydroxy-N-(4-trifluoromethyl-phenyl)-benzenesulfonamide [96];
N-(4-Chloro-phenyl)-4-nitro-benzene-sulfonamide [97];
N-(3,5-Bis-trifluoromethyl-phenyl)-4-nitro-benzenesulfonamide [98];
4-Amino-N-(3,5-bis-trifluoromethyl-phenyl)-3-chloro-benzenesulfonamide [99];
3-Nitro-N-(4-trifluoromethyl-phenyl)-benzenesulfonamide [100];
3,5-Dichloro-N-(3,5-dichloro-phenyl)-4-hydroxy-benzenesulfonamide [101];
4-Amino-3-chloro-N-(4-chloro-phenyl)-benzenesulfonamide [102];
3-Chloro-N-(4-chloro-phenyl)-4-methoxy-benzenesulfonamide [103];
N-(3,5-Bis-trifluoromethyl-phenyl)-3-chloro-4-methoxy-benzene-sulfonamide [104];
N-(3-Chloro-4-nitro-phenyl)-3,5-bis-trifluoromethyl-benzenesulfonamide [105];
3-(4-Acetyl-piperazin-1-yl)-N-(3,5-bis-trifluoromethyl-phenyl)-4-nitrobenzenesulfonamide [106];
N-(3,5-Bis-trifluoromethyl-phenyl)-2-nitro-benzenesulfonamide [107];
3-(3,5-Bis-trifluoromethyl-phenylsulfamoyl)-benzoic acid [108];
3,5-Dichloro-N-(4-chloro-benzyl)-4-hydroxy-benzenesulfonamide [109];
3,5-Dichloro-4-hydroxy-N-(4-trifluoromethyl-benzyl)-benzenesulfonamide [110];
3,5-Dichloro-4-hydroxy-N-[2-(1H-indol-3-yl)-ethyl]-benzenesulfonamide [111];
4,5-Dibromo-thiophene-2-sulfonic acid (3,5-dichloro-phenyl)-amide [112];
N-(3,5-Dichloro-phenyl)-4-oxazol-2-yl-benzenesulfonamide [113];
4-Bromo-5-chloro-thiophene-2-sulfonic acid (3,5-bis-trifluoromethyl-phenyl)-amide [114];
4-Bromo-5-chloro-thiophene-2-sulfonic acid (3,5-dichloro-phenyl)-amide [115];
5-Benzenesulfonyl-thiophene-2-sulfonic acid (3,5-bis-trifluoromethyl-phenyl)-amide [117];
5-Benzenesulfonyl-thiophene-2-sulfonic acid (2,4-dichloro-phenyl)-amide [118];
5-Chloro-3-methyl-benzo[b]thiophene-2-sulfonic acid (3,5-bis-trifluoromethyl-phenyl)-amide [119];
Benzo[b]thiophene-2-sulfonic acid (3,5-bis-trifluoromethyl-phenyl)-amide [120];
Benzo[1,2,5]thiadiazole-5-sulfonic acid (4-chloro-phenyl)-amide [121];
Benzo[1,2,5]thiadiazole-5-sulfonic acid (3,5-bis-trifluoromethyl-phenyl)-amide [122];
Benzo[1,2,5]thiadiazole-5-sulfonic acid (4-trifluoromethyl-phenyl)-amide [123];
5-Pyridin-2-yl-thiophene-2-sulfonic acid (3,5-bis-trifluoromethyl-phenyl)-amide [124];
4,5-Dibromo-thiophene-2-sulfonic acid (4-chloro-phenyl)-amide [125];
4,5-Dibromo-thiophene-2-sulfonic acid (4-trifluoromethyl-phenyl)-amide [126];
3,5-Dichloro-N-(4-fluoro-benzyl)-4-hydroxy-benzenesulfonamide [127];
N-(3,5-Bis-trifluoromethyl-phenyl)-2,6-dichloro-benzenesulfonamide [128];
5-Chloro-4-nitro-thiophene-2-sulfonic acid (4-methoxy-2-methyl-phenyl)-amide [129];
5-(3-Hydroxy-piperidin-1-yl)-4-nitro-thiophene-2-sulfonic acid (4-chloro-phenyl)-amide [130]; and
5-Chloro-4-nitro-thiophene-2-sulfonic acid (4-nitro-phenyl)-amide [131].

Preferably, the compound is selected from the following:
5-Chloro-4-nitrothiophene-2-sulfonic acid (4-chlorophenyl)amide [2];
5-Chloro-4-nitrothiophene-2-sulfonic acid (4-fluorophenyl)amide [3];
5-Chloro-4-nitrothiophene-2-sulfonic acid (4-hydroxyphenyl)amide [5];
5-Chloro-4-nitrothiophene-2-sulfonic acid (4-trifluoromethylphenyl)amide [6];
5-Chloro-4-nitrothiophene-2-sulfonic acid (4-fluorophenyl)methylamide [7];
4-Chloro-3-nitro-N-(4-trifluoromethylphenyl)-benzene-sulfonamide [35];
4-Chloro-N-(4-chlorophenyl)-3-nitrobenzene-sulfonamide [38];
5-Chloro-4-nitrothiophene-2-sulfonic acid (3,5-dichlorophenyl)amide [39];
5-Chloro-4-nitrothiophene-2-sulfonic acid (3,5-difluorophenyl)amide [40];
5-(1-Methyl-5-trifluoromethyl-1H-pyrazol-4-yl)-thiophene-2-sulfonic acid (3,5-bistrifluoromethylphenyl)amide [43];
5-Chloro-4-nitrothiophene-2-sulfonic acid (3,5-bis-trifluoromethyl-phenyl)-amide [44];
5-Chloro-4-nitrothiophene-2-sulfonic acid 4-fluorobenzylamide [45];
5-Chloro-4-nitrothiophene-2-sulfonic acid 4-trifluoromethylbenzylamide [46];
5-Chloro-4-nitrothiophene-2-sulfonic acid [2-(1H-indol-3-yl)-ethyl]amide [48];
5-Chloro-4-nitrothiophene-2-sulfonic acid [2-(1H-indol-3-yl)-1-methylethyl]amide [49];
5-Chloro-4-nitrothiophene-2-sulfonic acid (4-chlorophenyl)methylamide [51];
S-Chloro-4-nitro-thiophene-2-sulfonic acid benzyl-(4-fluoro-benzyl)-amide [53];
5-Chloro-4-nitro-thiophene-2-sulfonic acid 3,5-dichloro-benzylamide [54];
5-Chloro-4-nitro-thiophene-2-sulfonic acid 3,5-difluoro-benzylamide [55];
5-Chloro-4-nitro-thiophene-2-sulfonic acid 4-chloro-benzylamide [56];
5-Chloro-4-nitro-thiophene-2-sulfonic acid [1-(4-fluoro-phenyl)-ethyl]-amide [57];
5-Chloro-4-nitro-thiophene-2-sulfonic acid (4-methoxy-phenyl)-amide [61];
5-Chloro-4-nitro-thiophene-2-sulfonic acid p-tolylamide [63];
5-Chloro-4-nitro-thiophene-2-sulfonic acid benzylamide [64];
5-Chloro-4-nitro-thiophene-2-sulfonic acid benzyl-(4-chloro-phenyl)-amide [65];
5-Chloro-4-nitro-thiophene-2-sulfonic acid benzyl-(4-methoxy-phenyl)-amide [66];
5-Chloro-4-nitro-thiophene-2-sulfonic acid (4-chloro-phenyl)-(3-trifluoromethyl-benzyl)-amide [67];
4-Nitro-thiophene-2-sulfonic acid (4-chloro-phenyl)-amide [69];
5-Chloro-4-nitro-thiophene-2-sulfonic acid (3-trifluoro-methyl-benzyl)-(4-trifluoromethyl-benzyl)-amide [75]; and
N-(1H-Benzoimidazol-2-yl)-4-chloro-3-nitro-benzenesulfonamide [83].

More preferably, the compound of formula I is selected from the following: [2], [3], [5], [6], [7], [35], [39], [40], [44]-[46], [48], [49], [53]-[57], [61], [63]-[67], [75] and [83].

Even more preferably, the compound of formula I is selected from the following: [2], [3], [5], [6], [39], [40], [46], [48], [49], [53], [56], [57], [61], [63], [64], [66] and [83].

More preferably still, the compound of formula I is selected from the following: [2], [3], [46], [49], [61], [63] and [83].

In another preferred embodiment, the compound is selected from the following:
5-Chloro-4-nitrothiophene-2-sulfonic acid (4-chlorophenyl)amide [2];
5-Chloro-4-nitrothiophene-2-sulfonic acid (4-fluorophenyl)amide [3];
5-Chloro-4-nitrothiophene-2-sulfonic acid (4-hydroxyphenyl)amide [5];
5-Chloro-4-nitrothiophene-2-sulfonic acid (4-trifluoromethylphenyl)amide [6];
5-Chloro-4-nitrothiophene-2-sulfonic acid (4-fluorophenyl)methylamide [7];
5-Bromothiophene-2-sulfonic acid (3,5-bis-trifluoromethylphenyl)amide [18];
5-Benzenesulfonyl-thiophene-2-sulfonic acid (4-trifluoromethylphenyl)amide [23];
4,5-Dibromothiophene-2-sulfonic acid (3-trifluoromethylphenyl)amide [27];
4,5-Dibromothiophene-2-sulfonic acid (3,4-dichlorophenyl)amide [28];
5-Chlorothiophene-2,4-disulfonic acid bis-[(4-trifluoromethylphenyl)amide] [33];
4-Chloro-3-nitro-N-(4-trifluoromethylphenyl)benzene-sulfonamide [35];
5-Chloro-4-nitrothiophene-2-sulfonic acid (3,5-dichlorophenyl)amide [39];
5-Chloro-4-nitrothiophene-2-sulfonic acid (3,5-difluorophenyl)amide [40];
5-(1-Methyl-5-trifluoromethyl-1*H*-pyrazol-4-yl)-thiophene-2-sulfonic acid (3,5-bistrifluoromethylphenyl)amide [43];
5-Chloro-4-nitrothiophene-2-sulfonic acid (3,5-bis-trifluoromethylphenyl)amide [44];
5-Chloro-4-nitrothiophene-2-sulfonic acid 4-fluorobenzylamide [45];
5-Chloro-4-nitrothiophene-2-sulfonic acid 4-trifluoromethylbenzylamide [46];
5-Chloro-4-nitrothiophene-2-sulfonic acid [2-(1*H*-indol-3-yl)-ethyl]amide [48];
5-Chloro-4-nitrothiophene-2-sulfonic acid [2-(1*H*-indol-3-yl)-1-methylethyl]amide [49];
5-Chloro-4-nitrothiophene-2-sulfonic acid methyl-(4-trifluoromethylphenyl) amide [50];
5-Chloro-4-nitrothiophene-2-sulfonic acid (4-chlorophenyl)methylamide [51]; and
5-Chloro-4-nitrothiophene-2-sulfonic acid methyl (4-trifluoromethylbenzyl)amide [52]
5-Chloro-4-nitro-thiophene-2-sulfonic acid benzyl-(4-fluoro-benzyl)-amide [53];
5-Chloro-4-nitro-thiophene-2-sulfonic acid 3,5-dichloro-benzylamide [54];
5-Chloro-4-nitro-thiophene-2-sulfonic acid 3,5-difluoro-benzylamide [55];
5-Chloro-4-nitro-thiophene-2-sulfonic acid 4-chloro-benzylamide [56];
5-Chloro-4-nitro-thiophene-2-sulfonic acid [1-(4-fluoro-phenyl)-ethyl]-amide [57];
5-Chloro-4-nitro-thiophene-2-sulfonic acid (1H-benzo-imidazol-2-yl)-amide [59];
5-Chloro-4-nitro-thiophene-2-sulfonic acid [2-(6-chloro-1H-indol-3-yl)-ethyl]-amide [60];
5-Chloro-4-nitro-thiophene-2-sulfonic acid (4-methoxy-phenyl)-amide [61];
5-Chloro-4-nitro-thiophene-2-sulfonic acid phenylamide [62];
5-Chloro-4-nitro-thiophene-2-sulfonic acid p-tolylamide [63];
5-Chloro-4-nitro-thiophene-2-sulfonic acid benzylamide [64];
5-Chloro-4-nitro-thiophene-2-sulfonic acid benzyl-(4-chloro-phenyl)-amide [65];
5-Chloro-4-nitro-thiophene-2-sulfonic acid benzyl-(4-methoxy-phenyl)-amide [66];
5-Chloro-4-nitro-thiophene-2-sulfonic acid (4-chloro-phenyl)-(3-trifluoromethyl-benzyl)-amide [67];
4-Nitro-thiophene-2-sulfonic acid (4-chloro-phenyl)-amide [69];
5-Chloro-thiophene-2,4-disulfonic acid bis-[(4-chloro-phenyl)-amide] [70];
Thioacetic acid S-[5-(4-chloro-phenylsulfamoyl)-3-nitro-thiophen-2-yl] ester [72];
5-Methyl-4-nitro-thiophene-2-sulfonic acid (4-chloro-phenyl)-amide [73];
5-Methyl-thiophene-2,4-disulfonic acid bis-[(4-chloro-phenyl)-amide] [74];
5-Chloro-4-nitro-thiophene-2-sulfonic acid (3-trifluoro-methyl-benzyl)-(4-trifluoromethylbenzyl)-amide [75];
5-(2-Methoxy-ethylamino)-4-nitro-thiophene-2-sulfonic acid (4-chloro-phenyl)-amide [80];
N-(1H-Benzoimidazol-2-yl)-4-chloro-3-nitro-benzenesulfonamide [83];
5-Chloro-3-methyl-benzo[b]thiophene-2-sulfonic acid (4-chloro-3-nitro-phenyl)-amide [88];
N-(3,5-Bis-trifluoromethyl-phenyl)-3-chloro-4-methoxy-benzene-sulfonamide [104];
N-(3-Chloro-4-nitro-phenyl)-3,5-bis-trifluoromethyl-benzenesulfonamide [105];
4,5-Dibromo-thiophene-2-sulfonic acid (3,5-dichloro-phenyl)-amide [112];
5-Benzenesulfonyl-thiophene-2-sulfonic acid (3,5-bis-trifluoromethyl-phenyl)-amide [117];
5-Chloro-3-methyl-benzo[b]thiophene-2-sulfonic acid (3,5-bis-trifluoromethyl-phenyl)-amide [119];
Benzo[b]thiophene-2-sulfonic acid (3,5-bis-trifluoromethyl-phenyl)-amide [120];
Benzo[1,2,5]thiadiazole-5-sulfonic acid (3,5-bis-trifluoromethyl-phenyl)-amide [122];
4,5-Dibromo-thiophene-2-sulfonic acid (4-chloro-phenyl)-amide [125];
4,5-Dibromo-thiophene-2-sulfonic acid (4-trifluoromethyl-phenyl)-amide [126];
N-(3,5-Bis-trifluoromethyl-phenyl)-2,6-dichloro-benzenesulfonamide [128];
5-(3-Hydroxy-piperidin-1-yl)-4-nitro-thiophene-2-sulfonic acid (4-chloro-phenyl)-amide [130]; and
5-Chloro-4-nitro-thiophene-2-sulfonic acid (4-nitro-phenyl)-amide [131].

More preferably, the compound is selected from the following: [2], [3], [6], [7], [33], [39], [40], [44]-[46], [48]-[57], [59]-[67], [72], [75], [130] and [131].

Even more preferably, the compound is selected from the following: [7], [45], [46], [50]-[53], [64]-[67], [72] and [75].

More preferably still, the compound is selected from the following: [50], [51], [65], [67] and [75].

The invention also relates to the use of the above-mentioned compounds in the preparation of a medicament for treating a proliferative disorder.

### PHARMACEUTICAL COMPOSITIONS

A fourth aspect of the invention relates to a pharmaceutical composition comprising a compound of formula I as defined for said first and third aspects admixed with one or more pharmaceutically acceptable diluents, excipients or carriers. Even though the compounds of the present invention (including their pharmaceutically acceptable salts, esters and pharmaceutically acceptable solvates) can be administered alone, they will generally be administered in admixture with a pharmaceutical carrier, excipient or diluent, particularly for human therapy. The pharmaceutical compositions may be for human or animal usage in human and veterinary medicine.

Examples of such suitable excipients for the various different forms of pharmaceutical compositions described herein may be found in the "Handbook of Pharmaceutical Excipients, 2nd Edition, (1994), Edited by A Wade and PJ Weller.

Acceptable carriers or diluents for therapeutic use are well known in the pharmaceutical art, and are described, for example, in Remington's Pharmaceutical Sciences, Mack Publishing Co. (A. R. Gennaro edit. 1985).

Examples of suitable carriers include lactose, starch, glucose, methyl cellulose, magnesium stearate, mannitol, sorbitol and the like. Examples of suitable diluents include ethanol, glycerol and water.

The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s).

Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol.

Examples of suitable lubricants include sodium oleate, sodium stearate, magnesium stearate, sodium benzoate, sodium acetate, sodium chloride and the like.

Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

### SALTS/ESTERS

The compounds of the present invention can be present as salts or esters, in particular pharmaceutically acceptable salts or esters.

Pharmaceutically acceptable salts of the compounds of the invention include suitable acid addition or base salts thereof. A review of suitable pharmaceutical salts may be found in Berge et al, J Pharm Sci, 66, 1-19 (1977). Salts are formed, for example with strong inorganic acids such as mineral acids, e.g. sulphuric acid, phosphoric acid or hydrohalic acids; with strong organic carboxylic acids, such as alkanecarboxylic acids of 1 to 4 carbon atoms which are unsubstituted or substituted (e.g., by halogen), such as acetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane- or p-toluene sulfonic acid.

Esters are formed either using organic acids or alcohols/hydroxides, depending on the functional group being esterified. Organic acids include carboxylic acids, such as alkanecarboxylic acids of 1 to 12 carbon atoms which are unsubstituted or substituted (e.g., by halogen), such as acetic acid; with saturated or unsaturated dicarboxylic acid, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane- or p-toluene sulfonic acid. Suitable hydroxides include inorganic hydroxides, such as sodium hydroxide, potassium hydroxide, calcium hydroxide, aluminium hydroxide. Alcohols include alkanealcohols of 1-12 carbon atoms which may be unsubstituted or substituted, e.g. by a halogen).

### ENANTIOMERS/TAUTOMERS

In all aspects of the present invention previously discussed, the invention includes, where appropriate all enantiomers and tautomers of compounds of the invention. The man skilled in the art will recognise compounds that possess an optical properties (one or more chiral carbon atoms) or tautomeric characteristics. The corresponding enantiomers and/or tautomers may be isolated/prepared by methods known in the art.

### STEREO AND GEOMETRIC ISOMERS

Some of the compounds of the invention may exist as stereoisomers and/or geometric isomers - e.g. they may possess one or more asymmetric and/or geometric centres and so may exist in two or more stereoisomeric and/or geometric forms. The present invention contemplates the use of all the individual stereoisomers and geometric isomers of those agents, and mixtures thereof. The terms used in the claims encompass these forms, provided said forms retain the appropriate functional activity (though not necessarily to the same degree).

The present invention also includes all suitable isotopic variations of the agent or a pharmaceutically acceptable salt thereof. An isotopic variation of an agent of the present invention or a pharmaceutically acceptable salt thereof is defined as one in which at least one atom is replaced by an atom having the same atomic number but an atomic mass different from the atomic mass usually found in nature. Examples of isotopes that can be incorporated into the agent and pharmaceutically acceptable salts thereof include isotopes of hydrogen, carbon, nitrogen, oxygen, phosphorus, sulphur, fluorine and chlorine such as ²H, ³H, ¹³C, ¹⁴C, ¹⁵N, ¹⁷O, ¹⁸O, ³¹P, ³²P, ³⁵S, ¹⁸F and ³⁶Cl, respectively. Certain isotopic variations of the agent and pharmaceutically acceptable salts thereof, for example, those in which a radioactive isotope such as ³H or ¹⁴C is incorporated, are useful in drug and/or substrate tissue distribution studies. Tritiated, i.e., ³H, and carbon-14, i.e., ¹⁴C, isotopes are particularly preferred for their ease of preparation and detectability. Further, substitution with isotopes such as deuterium, i.e., ²H, may afford certain therapeutic advantages resulting from greater metabolic stability, for example, increased in vivo half-life or reduced dosage requirements and hence may be preferred in some circumstances. Isotopic variations of the agent of the present invention and pharmaceutically acceptable salts thereof of this invention can generally be prepared by conventional procedures using appropriate isotopic variations of suitable reagents.

### SOLVATES

The present invention also includes the use of solvate forms of the compounds of the present invention. The terms used in the claims encompass these forms.

### POLYMORPHS

The invention furthermore relates to the compounds of the present invention in their various crystalline forms, polymorphic forms and (an)hydrous forms. It is well established within the pharmaceutical industry that chemical compounds may be isolated in any of such forms by slightly varying the method of purification and or isolation form the solvents used in the synthetic preparation of such compounds.

### PRODRUGS

The invention further includes the compounds of the invention in prodrug form. Such prodrugs are generally compounds wherein one or more appropriate groups have been modified such that the modification may be reversed upon administration to a human or mammalian subject. Such reversion is usually performed by an enzyme naturally present in such subject, though it is possible for a second agent to be administered together with such a prodrug in order to perform the reversion in vivo. Examples of such modifications include ester (for example, any of those described above), wherein the reversion may be carried out be an esterase etc. Other such systems will be well known to those skilled in the art.

### ADMINISTRATION

The pharmaceutical compositions of the present invention may be adapted for oral, rectal, vaginal, parenteral, intramuscular, intraperitoneal, intraarterial, intrathecal, intrabronchial, subcutaneous, intradermal, intravenous, nasal, buccal or sublingual routes of administration.

For oral administration, particular use is made of compressed tablets, pills, tablets, gellules, drops, and capsules. Preferably, these compositions contain from 1 to 250 mg and more preferably from 10-100 mg, of active ingredient per dose.

Other forms of administration comprise solutions or emulsions which may be injected intravenously, intraarterially, intrathecally, subcutaneously, intradermally, intraperitoneally or intramuscularly, and which are prepared from sterile or sterilisable solutions. The pharmaceutical compositions of the present invention may also be in form of suppositories, pessaries, suspensions, emulsions, lotions, ointments, creams, gels, sprays, solutions or dusting powders.

An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required.

Injectable forms may contain between 10 - 1000 mg, preferably between 10 - 250 mg, of active ingredient per dose.

Compositions may be formulated in unit dosage form, i.e., in the form of discrete portions containing a unit dose, or a multiple or sub-unit of a unit dose.

### DOSAGE

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this invention.

Depending upon the need, the agent may be administered at a dose of from 0.01 to 30 mg/kg body weight, such as from 0.1 to 10 mg/kg, more preferably from 0.1 to 1 mg/kg body weight.

In an exemplary embodiment, one or more doses of 10 to 150 mg/day will be administered to the patient for the treatment of malignancy.

### COMBINATIONS

A further aspect of the present invention relates to a combination comprising at least one compound of the invention as defined above and at least one cytotoxic agent.

Preferably, the combination is a synergistic combination. Thus, preferably, the compound of the invention is capable of synergistically interacting with the one or more other cytotoxic agents, for example, to enhance the cytotoxic effect of the other agent.

In one preferred embodiment, the cytotoxic agent is a chemotherapeutic agent.

In a particularly preferred embodiment, the chemotherapeutic agent is cisplatin or etoposide. Even more preferably, the compound of the invention is capable of exhibiting a chemosensitisation effect, for example, by interacting synergistically to increase the cytotoxic effects of cisplatin and etoposide. In other words, the combined action of the compound of the invention and the cytotoxic agent produces a greater effect than would be expected from adding the individual effects of each component. Further details regarding the synergistic effect may be found in the accompanying examples.

Another aspect of the invention relates to a pharmaceutical composition comprising at least one compound of the invention as defined above, and one or more cytotoxic agents, admixed with a pharmaceutically acceptable diluent, excipient or carrier.

In a particularly preferred embodiment, the one or more compounds of the invention are administered in combination with one or more other anticancer agents, for example, existing anticancer drugs available on the market. In such cases, the compounds of the invention may be administered consecutively, simultaneously or sequentially with the one or more other anticancer agents.

Thus, one aspect of the invention provides a method of treating a proliferative disorder, said method comprising administering to a subject at least one compound of the invention as defined above consecutively, simultaneously or sequentially with one or more other cytotoxic agents.

Anticancer drugs in general are more effective when used in combination. In particular, combination therapy is desirable in order to avoid an overlap of major toxicities, mechanism of action and resistance mechanism(s). Furthermore, it is also desirable to administer most drugs at their maximum tolerated doses with minimum time intervals between such doses. The major advantages of combining chemotherapeutic drugs are that it may promote additive or possible synergistic effects through biochemical interactions and also may decrease the emergence of resistance in early tumor cells which would have been otherwise responsive to initial chemotherapy with a single agent. An example of the use of biochemical interactions in selecting drug combinations is demonstrated by the administration of leucovorin to increase the binding of an active intracellular metabolite of 5-fluorouracil to its target, thymidylate synthase, thus increasing its cytotoxic effects.

Numerous combinations are used in current treatments of cancer and leukemia. A more extensive review of medical practices may be found in "Oncologic Therapies" edited by E. E. Vokes and H. M. Golomb, published by Springer.

Beneficial combinations may be suggested by studying the activity of the test compounds with agents known or suspected of being valuable in the treatment of a particular cancer initially or cell lines derived from that cancer. This procedure can also be used to determine the order of administration of the agents, i.e. before, simultaneously, or after delivery. Such scheduling may be a feature of all the cycle acting agents identified herein.

The compounds of the present invention may also be used in combination with radiotherapy treatment. Thus, another aspect of the invention provides a method of treating a proliferative disorder, said method comprising administering to a subject at least one compound of formula I as defined above consecutively, simultaneously or sequentially with radiotherapy.

### ASSAYS

A fifth aspect of the invention relates to the use of a compound of formula I, as defined in the above-mentioned first aspect, in an assay for determining binding to HDM2.

Preferably, the assay is capable of identifying candidate compounds that influence the activity of HDM2 on p53 and/or E2F.

More preferably still, the assay is capable of identifying candidate compounds that inhibit the interaction between HDM2 and p53 and/or E2F.

Even more preferably, the assay is a competitive binding assay.

Preferably, the competitive binding assay comprises contacting a compound of formula I as defined in the above-mentioned first aspect of the invention with HDM2 in the presence of a p53-derived peptide and detecting any change in the interaction between HDM2 and said p53-derived peptide.

In a particularly preferred embodiment, said p53-derived peptide is a fluorescently labelled or biotinylated p53-derived peptide.

A sixth aspect of the invention provides a method of detecting the binding of a ligand to HDM2, said method comprising the steps of:
(i) contacting a ligand with HDM2 in the presence of a p53-derived peptide; and
(ii) detecting any change in the interaction between HDM2 and said p53-derived peptide;
and wherein said ligand is a compound according to the above-mentioned first aspect of the invention.

One aspect of the invention relates to a process comprising the steps of:
(a) performing an assay method described hereinabove;
(b) identifying one or more ligands capable of binding to a ligand binding domain; and
(c) preparing a quantity of said one or more ligands.

Another aspect of the invention provides a process comprising the steps of:
(a) performing an assay method described hereinabove;
(b) identifying one or more ligands capable of binding to a ligand binding domain; and
(c) preparing a pharmaceutical composition comprising said one or more ligands.

Another aspect of the invention provides a process comprising the steps of:
(a) performing an assay method described hereinabove;
(b) identifying one or more ligands capable of binding to a ligand binding domain;
(c) modifying said one or more ligands capable of binding to a ligand binding domain;
(d) performing the assay method described hereinabove;
(e) optionally preparing a pharmaceutical composition comprising said one or more ligands.

The invention also relates to a ligand identified by the method described hereinabove.

Yet another aspect of the invention relates to a pharmaceutical composition comprising a ligand identified by the method described hereinabove.

Another aspect of the invention relates to the use of a ligand identified by the method described hereinabove in the preparation of a pharmaceutical composition for use in the treatment of proliferative disorders.

Preferably, said candidate compound is generated by conventional SAR modification of a compound of the invention.

As used herein, the term "conventional SAR modification" refers to standard methods known in the art for varying a given compound by way of chemical derivatisation.

The above methods may be used to screen for a ligand useful as an inhibitor of the interaction between HDM2 and p53.

### CHEMICAL SYNTHESIS

The compounds of the present invention can be prepared by methods known in the art. In particular, the condensation reaction between arylsulfonyl halides and arylamines can be applied. N-substituted bisarylsulfonamides can be obtained by using secondary arylamines in this reaction or through alkylation of primary bisarylsulfonamide precursors. Alkylation can be achieved with alkyl or aralkyl halides by sulfonylamino-de-halogenation or with alkyl or aralkyl alcohols by e.g. the Tsunoda reaction [Tsunoda T, Otsuka J, Yamamiya Y, Ito S; Chem. Lett. 1994: 539-542]. A number of the bisarylsulfonamides of the present invention contain the thiophenesulfonyl function; appropriate thiophenesulfonyl halide precursors for the sulfonamide condensation reaction can be prepared as described [Cremlyn RJ, Goulding KH, Swinboume FJ, Yung K-M; Phosphorus Sulfur 1951; 10: 111-119; Obafemi CA; Phosphorus Sulfur 1982; 13: 119-131.].

The present invention is further described by way of the following non-limiting examples and with reference to the following figures, wherein:

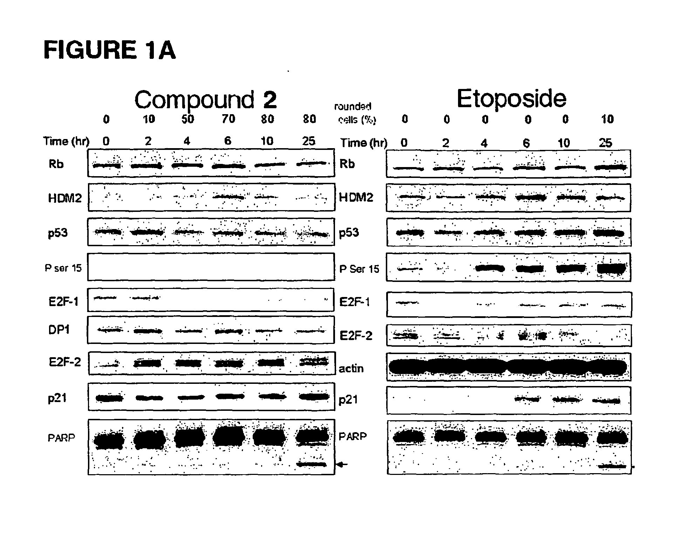

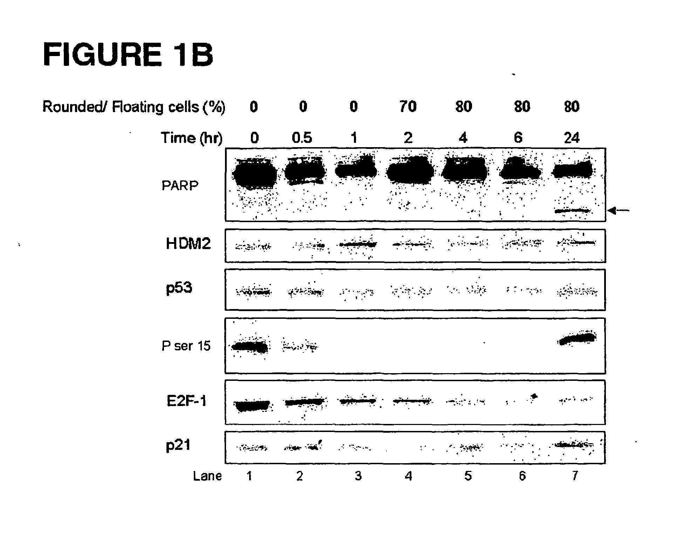

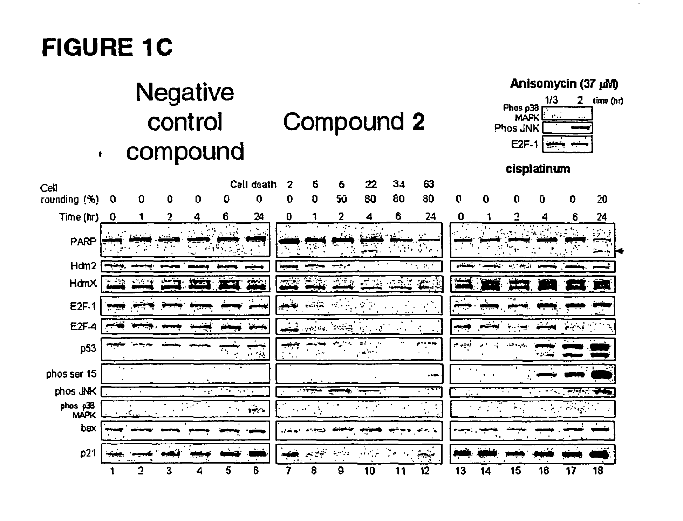

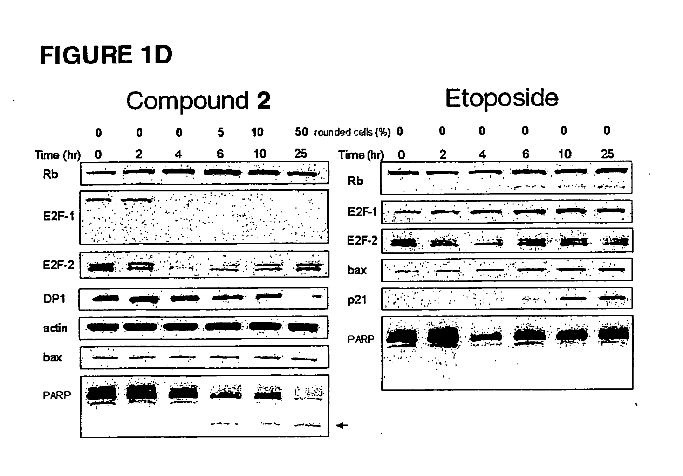

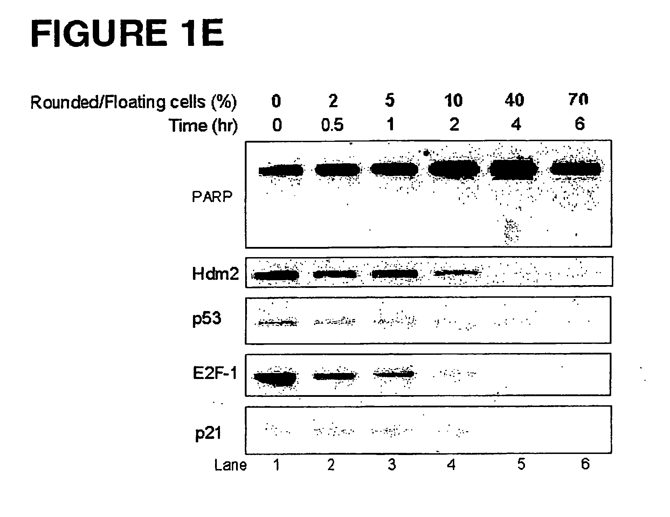

Figure 1 shows the effect of compound **2** or DNA damage inducing agents on the levels of key proteins in the HDM2 pathway and cell morphology in a range of cell lines. In more detail, Figure 1 shows: (**A**) The effect of compound **2** or etoposide on the induction of apoptosis proteins in AGS cells. AGS were treated with 5 times [IC₅₀] of either compound **2** or etoposide and collected at the indicated time points. Equal amounts of cell lysate were separated by SDS-PAGE and analysed by immunoblotting using the indicated antibodies. An estimation of the extent of cell rounding is indicated above each lane. The arrow indicates the position of a 85-kDa cleavage product of PARP, indicative of apoptosis. **(B)** The effect of compound **2** on the levels of the cell cycle proteins in AGS cells (wild-type 53). AGS cells were treated with 5 times [IC₅₀] of either compound **2** or etoposide and collected at the indicated time points. Equal amounts of cell lysate were separated by SDS-PAGE and analysed by immunoblotting using the indicated antibodies. An estimation of the extent of cell rounding is indicated above each lane. **(C)** The effect of negative control compound (refer Example 10), compound **2,** cisplatin, and anisomycin on the levels of key proteins in MCF7 cells. MCF7 cells were treated with either 13.5 µM of negative control compound or compound **2** at 5 times [IC₅₀]. Cisplatinum and anisomycin (a transcription inhibitor) treatments were with 50 µM or 37 µM, respectively. Cells were harvested at various time points; afterwards lysates were prepared and separated by SDS-PAGE and analysed by immunoblotting. The proteins detected are indicated. The extent of rounded or floating cells and the percentage viable cells were assessed. **(D)** The effect of compound **2** or etoposide on the levels of proteins and cell morphology in H1299 cells (p53 null cells). H1299 were treated with 5 times [IC₅₀] of either compound **2** or etoposide and collected at the indicated time points. Equal amounts of cell lysate were separated by SDS-PAGE and analysed by immunoblotting using a range of antibodies. An estimation of the extent of cell rounding is indicated above each lane. **(E)** Effect of 5 times [IC₅₀] of compound **2** on the levels of the cell cycle proteins in SJSA1 cells (overexpress HDM2). SJSA1 cells were treated with 5 times [IC₅₀] of compound **2** and collected at the indicated time points. Equal amounts of cell lysate were separated by SDS-PAGE and analysed by immunoblotting using a range of antibodies. An estimation of the extent of cell rounding is indicated above each lane.

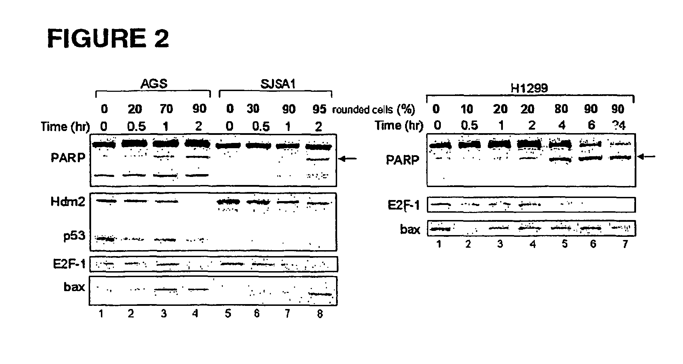

Figure 2 shows the effect of compound **49** on AGS, SJSA1 and H1299 cells. Cells were treated with 5 times [IC₅₀] for each cell line and collected at the indicated time points.
   Equal amounts of cell lysate were separated by SDS-PAGE and analysed by immunoblotting using a range of antibodies. An estimation of the extent of cell rounding is indicated above each lane. The arrow indicates the position of an 85-kDa cleavage product of PARP, indicative of apoptosis.

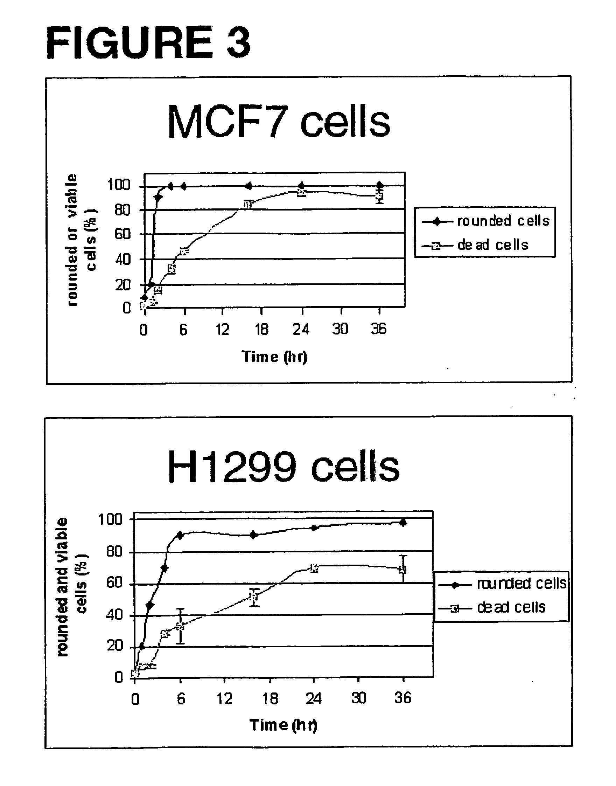

Figure 3 shows the effect of compound **2** on the morphology and cell viability of MCF7 and H1299 cells. Cells were treated with 5 times [IC₅₀] of compound 2 and collected at various time points. The percentage of rounded cells was counted before cells were trypsinised and counted for cell viability using trypan blue.

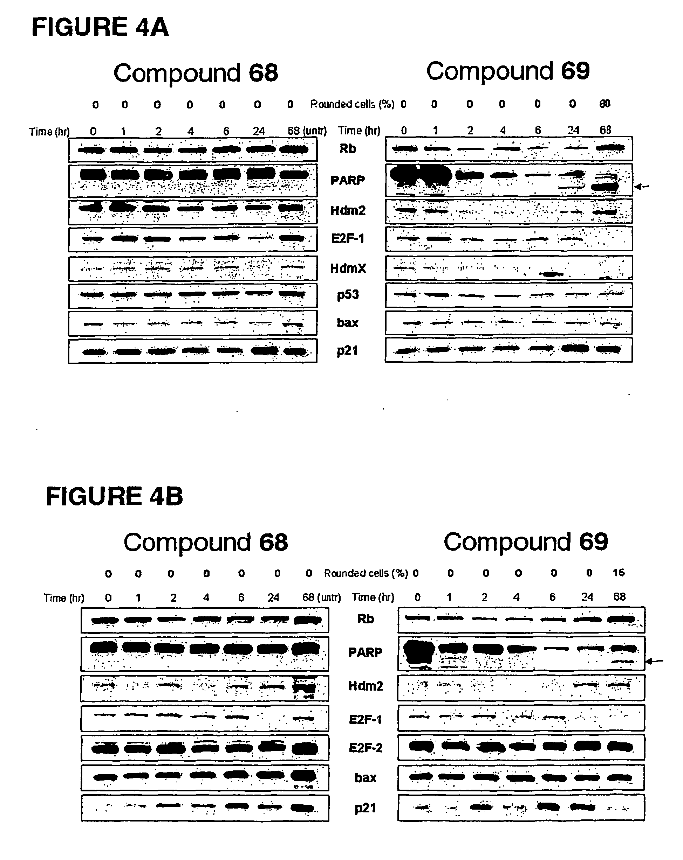

Figure 4 shows the effect of compounds **68** and **69** on MCF7 cells and H1299 cells. In more detail: (A) MCF7 cells were treated with 5 times [IC₅₀] of compound **69** and the same concentration of **68** and collected at the indicated time points. Equal amounts of cell lysate were separated by SDS-PAGE and analysed by immunoblotting using a range of antibodies. An estimation of the extent of cell rounding is indicated above each lane. The arrow indicates the position of an 85-kDa,cleavage product of PARP, indicative of apoptosis. **(B)** H1299 cells were treated with 5 times [IC₅₀] of compound . **69** and the same concentration of **68** and collected at the indicated time points and analysed.

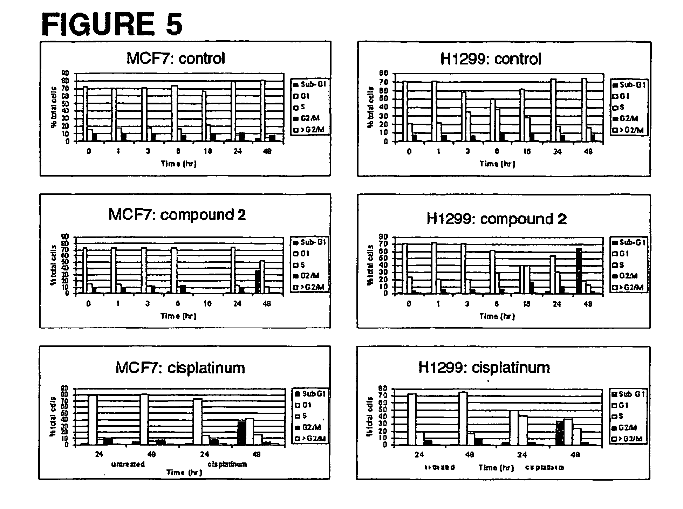

Figure 5 shows the effect of compound **2** and cisplatinum on the cell cycle distribution of MCF7 and H1299 cells. Cells were left untreated, or treated with 5 times [IC₅₀] of either compound **2** or cisplatinum and collected at the indicated time points. Cells were stained with propidium iodide and the cell cycle position was determined using a flow cytometer.

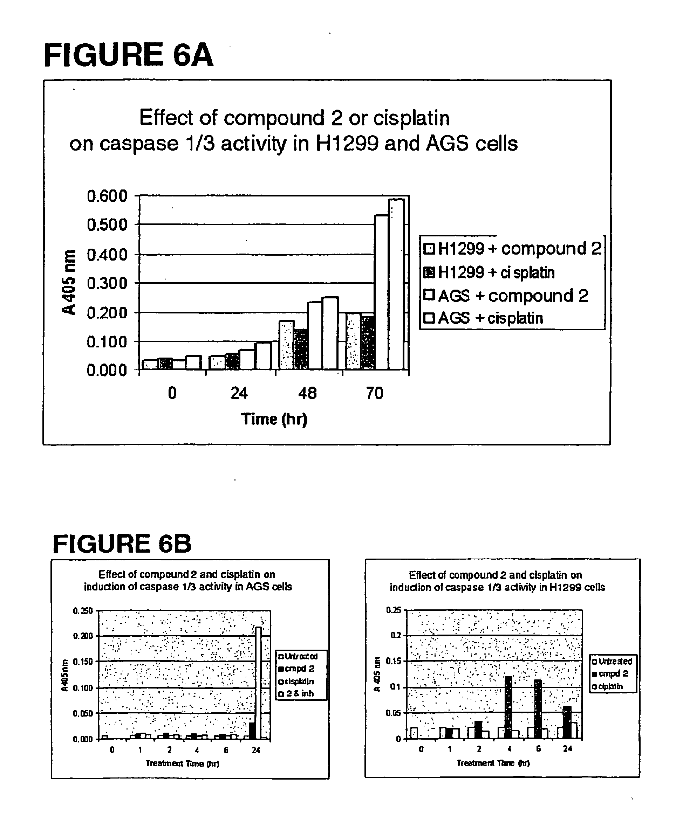

Figure 6 shows the effect of compound **2** on the caspase activation in AGS and H1299 cells. In more detail: **(A)** Cells were treated with 3 times [IC₅₀] of compound **2** or cisplatinum and collected at the time points indicated. Cell lysates were prepared, the caspase activity was determined using the caspACE assay and values were plotted on a graph. (**B**) Cells were treated with 5 times [IC₅₀] of compound **2** or cisplatinum and collected at the time points indicated. AGS cells were also incubated with compound **2** in the presence of a caspase inhibitor Z-VAD.fink. Cell lysates were prepared, the caspase activity was determined using the caspACE assay and values were plotted on a graph.

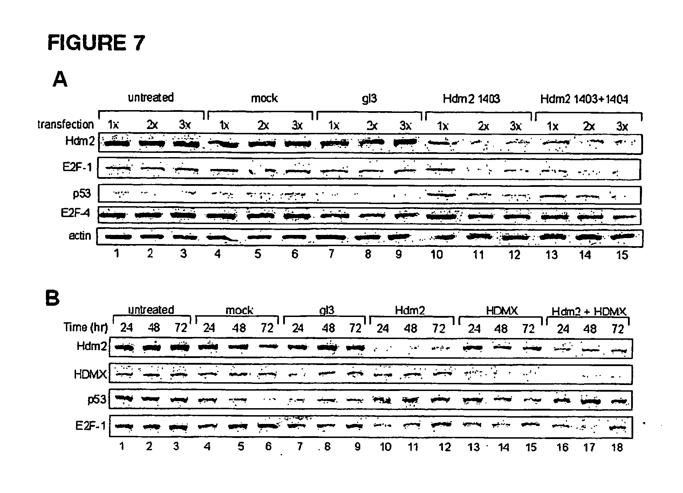

Figure 7 shows the effect of HDM2 and HDMX siRNA on the levels of p53 and E2F-1 in MCF7 cells. In more detail: (A) MCF7 cells were untreated (lanes 1-3) mock transfected (lanes 4-6), transfected with g13 control siRNA (lanes 7-9), HDM2 siRNA 1403 (lanes 10-12), or HDM2 siRNA 1403 and 1404 (lanes 13-15). This was repeated every 24 hours. Cells were collected 24 hours after each transfection so that cells had either been transfected once, twice, or three times and analysed by Western blotting. (B) MCF7 cells were untreated (lanes 1-3) mock transfected (lanes 4-6), transfected with gl3 control siRNA (lanes 7-9), HDM2 siRNA 1403 (lanes 10-12), HDMX siRNA (lanes 13-15) or HDM2 and HDMX siRNA(lanes 16-18). Cells were collected at 24, 48, and 72 hours and analysed by Western blotting.

### EXAMPLES

### General

HPLC retention times (t_{R}) were measured using Vydac 218TP54 columns (C₁₈ reversed-phase stationary phase; 4.5 x 250 mm columns), eluted at 1 mL/min with a linear gradient of acetonitrile in water (containing 0.1 % CF₃COOH) as indicated, followed by isocratic elution. HPLC Method B refers to the following: Supercosil ABZ+Plus column (21.2 x 250 mm), eluted at 20 mL/min using a linear gradient of acetonitrile in water from 5 to 95 % over 10 min, followed by isocratic elution. UV monitors (254 nm) were used. All purification work, unless otherwise stated, was performed using silica gel 60A (particle size 35-70 micron) eluting with hexane/EtOAc (4:1). Thin layer chromatography (TLC) was performed using aluminium sheets precoated with 0.2 mm silica gel 60 F₂₅₄- ¹H NMR spectra were recorded using various different instruments. Chemical shifts are given in ppm using TMS as standard and coupling constants (*J*) are stated in Hz. Mass spectra were recorded under positive or negative ion electrospray conditions.

### Example 1

### General method for the preparation of primary bisarylsulfonamides

The appropriate sulfonyl chloride (1.0 mol eq) was suspended in dichloromethane. The suspension was cooled to 0 °C. While stirring the reaction mixture, the appropriate amino component (aniline, benzylamine, *etc.,* as appropriate) (1.1 mol eq) and pyridine (1.5 mol eq) were added. The reaction mixture was slowly warmed to room temperature. Stirring was continued until TLC [heptane : ethyl acetate (2 : 1)] indicated that the reaction had gone to completion. The product was isolated and purified as follows: the reaction mixture was diluted with dichloromethane and was washed successively with dilute aq HCl, saturated aq NaHCO₃, water, and brine. The organic fraction was dried over MgSO₄ and was concentrated under reduced pressure to yield the crude sulfonamide. Column chromatography [column (Isolute SI; Jones Chromatography), heptane:ethyl acetate (12:1 → 3:1)] afforded the desired bisarylsulfonamide.

The primary bisarylsulfonamide compounds of this invention were prepared in this manner. Analytical details for representative compounds are as follows:
*5-Chloro-4-nitro-thiophene-2-sulfonic acid (4-chloro phenyl)-amide* **2.** TLC R_{F} = 0.72 (heptane:ethyl acetate, 2:1); HPLC t_{R} 17.08 min (20-80 %, 20 min); ¹H NMR (CDCl₃) δ: 6.64 (1 H, br s, NH), 7.05 (2 H, d, *J* 8.5, Ph-H), 7.27 (2 H, d, *J* 8.5, Ph-H), 7.84 (1 H, s, thiophene-H); MS 352.94 (M-H)⁻, C₁₀H₆Cl₂N₂O₄S₂ = 353.20.
*5-Chloro-4-nitro-thiophene-2-sulfonic acid (4-fluoro-phenyl)-amide* **3.** TLC R_{F} = 0.35 (heptane:ethyl acetate, 2:1); ¹H NMR (CDCl₃) δ: 6.83 (1 H, br s, NH), 6.99 (2 H, t, *J* 8.5, 9.0, Ph-H), 7.10 (2 H, dd, *J* 8.5, 9.0 Ph-H), 7.82 (1H, s, thiophene-H).
*5-Chloro-4-nitro-thiophene-2-sulfonic acid (4-trifluoromethyl-phenyl)-amide* **6.** TLC R_{F} = 0.45 (heptane:ethyl acetate, 2:1); ¹H NMR (CD₃OD) δ: 7.37 (2 H, d, *J* 8.5, Ph-H), 7.62 (2 H, d, *J* 8.5, Ph-H), 8.01 (1 H, s, thiophene-H).
*5-Chloro-thiophene-2-sulfonic acid (4-trifluoromethyl-phenyl)-amide* **8.** TLC R_{F} = 0.44 (hexane:EtOAc, 4:1); ¹H NMR (CDCl₃) δ: 6.86 (d, 1 H, *J* 4.1, thiophene-H), 6.95 (s, 1 H, NH), 7.24 (d, 2 H, *J* 8.4, Ph-H), 7.36 (d, 1 H, *J* 4.1, thiophene-H), 7.57 (d, 2 H, *J* 8.4, Ph-H); MS 341 [M]⁺, C11H7ClF3NO2S2 = 341.76.
*4,5-Dibromo-thiophene-2-sulfonic acid* (*3,5-bis-trifluoromethyl-phenyl*)*-amide* **9**. HPLC t_{R} 7.48 min (method B); MS 532.1 (M-H)^{- 79}Br⁸¹Br, C₁₂H₅Br₂F₆NO₂S₂ = 533.10.
*5-Chloro-thiophene-2-sulfonic acid* (*4-chloro-phenyl*)*-amide* 10. HPLC t_{R} 8.45 min (method B); MS 306.1 (M-H)^{- 35}Cl₂, C₁₀H₇Cl₂NO₂S₂ = 308.20.
*5-Chloro-thiophene-2-sulfonic acid* (*3,5*-bis*-trifluoromethyl-phenyl*)*-amide* **11**. HPLC t_{R} 7.31 min (method B); ); ¹H NMR (CDCl₃) δ: 6.85 (d, 1 H, *J* 4.1, thiophene-H), 7.31 (s, 1 H, NH), 7.34 (d, 1 H, *J* 4.1, thiophene-H), 7.54 (s, 2 H, Ph-H), 7.60 (s, 1 H, Ph-H); MS 408.0 (M-H)^{- 35}Cl, C₁₂H₆ClF₆NO₂S₂ = 409.76.
*5-*(*2-Methylsulfanyl-pyrimidin-5-yl*)*-thiophene-2-sulfonic acid* (*3,5-dichloro-phenyl*)-*amide* **12.** HPLC t_{R} 9.01 min (method B); MS 429.9 (M-H)^{- 35}Cl₂, Cl₅H₁₁Cl₂N₃O₂S₃ = 432.3677.
*4-Oxazol-2-yl-N-(4-trifluoromethyl-phenyl)-benzenesulfonamide* **13.** HPLC t_{R} 9.02 min (method B); MS 367.1 (M-H)⁻, C₁₆H₁₁F₃N₂O₃S = 368.33.
*N-(3,5-Bis-trifluoromethyl-phenyl)-4-oxazol-2-yl-benzenesulfonamide* **14.** HPLC t_{R} 9.13 min (method B); MS 435.1 (M-H)⁻, C₁₇H₁₀F₆N₂O₃S = 436.33.
*4-Bromo-5-chloro-thiophene-2-sulfonic acid (4-trifluoromethyl-phenyl)-amide* **15.** HPLC t_{R} 8.25 min (method B); MS 419.9 (M-H)^{- 81}Br³⁵Cl, C₁₁H₆BrClF₃NO₂S₂ = 420.65.
*5-Bromo-thiophene-2-sulfonic acid* (*4-chloro-phenyl*)*-amide* **16.** HPLC t_{R} 9.57 min (method B); MS 352.0 (M-H)^{- 81}Br³⁵Cl, C₁₀H₇BrClNO₂S₂ = 352.66.
*5-Bromo-thiophene-2-sulfonic acid* (*3,5-dichloro-phenyl*)*-amide* **17.** HPLC t_{R} 9.11 min (method B); MS 385.8 (M-H)^{- 81}Br ³⁵Cl₂, C₁₀H₆BrCl₂NO₂S₂ = 387.10.
*5-Bromo-thiophene-2-sulfonic acid* (*3,5-*bis*-trifluoromethyl-phenyl*)*-amide* **18.** HPLC t_{R} 8.07 min (method B); MS 453.9 (M-H)^{- 81}Br, C₁₂H₆BrF₆NO₂S₂ = 454.21.
*N-*(*4-Chloro-phenyl*)*-3-nitro-benzenesulfonamide* **19.** HPLC t_{R} 9.06 min (method B); MS 311.0 (M-H)^{- 35}Cl, C₁₂H₉ClN₂O₄S = 312.73.
*3-Nitro-*N*-*(*4-trifluoromethyl-phenyl*)*-benzenesulfonamide* **20.** MS 345.0 (M-H)⁻, C₁₃H₉F₃N₂O₄S = 346.28.
N*-*(*3,5-*Bis*-trifluoromethyl-phenyl)-3-nitro-benzenesulfonamide* **21**. MS 413.0 (M-H)⁻, C₁₄H₈F₆N₂O₄S = 414.29.
N*-*(*2,4-Dichloro-phenyl*)*-3-nitro-benzenesulfonamide* **22**. MS 344.8 (M-H)^{- 35}Cl₂, C₁₂H₈Cl₂N₂O₄S = 347.17.
*5-Benzenesulfonyl-thiophene-2-sulfonic acid (4-trifluoromethyl-phenyl)-amide* **23**. MS 446.0 (M-H)⁻, C₁₇H₁₂F₃NO₄S₃ = 447.47.
*5-Benzenesulfonyl-thiophene-2-sulfonic acid (4-chloro-phenyl)-amide* **24**. MS 411.9 (M-H)^{- 35}Cl, C₁₆H₁₂ClNO₄S₃ =413.92.
*5-Benzenesulfonyl-thiophene-2-sulfonic acid (3,5-dichloro-phenyl)-amide* **25**. MS 446.0 (M-H)^{- 35}Cl₂, C₁₆H₁₁Cl₂NO₄S₃ = 448.36.
*5-Chloro-thiophene-2-sulfonic acid (3,4-dichloro-phenyl)-amide* 26. MS 339.8 [M-H]⁻³⁵Cl₃, C₁₀H₆Cl₃NO₂S₂ = 342.65.
*4,5-Dibromo-thiophene-2-sulfonic acid (3-trifluoromethyl-phenyl)-amide* 27. ¹H NMR (CDCl₃) δ: 6.69 (s, 1 H, NH), 7.26 (s, 1 H, Ph-H), 7.28 (m, 1 H, Ph-H), 7.33 (s, 1 H, thiophene-H), 7.42 (d, 2 H, J4.2, Ph-H); MS 463.8 (M-H)^{- 79}Brs⁸¹Br, C₁₁H₆Br₂F₃NO₂S₂ = 465.10.
*4,5-Dibromo-thiophene-2-sulfonic acid (3,4-dichloro-phenyl)-amide* 28. MS 465.7 (M-H)- ⁸¹Br⁷⁹Br³⁷Cl³⁵Cl₂, C₁₀H₅Br₂Cl₂NO₂S₂ = 466.00.
N*-(3,5 Bis-trifluoromethyl-phenyl)-4-chloro-3-nitro-benzenesulfonamide* 29. TLC R_{F} = 0.78 (heptane:ethyl acetate, 2:1); ¹H NMR (CDCl₃) δ: 7.53 (3 H, s, Ph-H), 7.60 (1 H, d, *J* 8.5, Ph-H), 7.85 (1 H, m, Ph-H) 8.30 (1 H, s, Ph-H); MS 447.0 (M-H)^{- 35}Cl, C₁₄H₇ClF₆N₂O₄S = 448.72.
*4-Chloro-*N*-(3,4-dichloro-phenyl)-3-nitro-benzenesulfonamide* 30. MS 380.9 (M-H)⁻³⁷Cl³⁵Cl₂, C₁₂H₇Cl₃N₂O₄S = 381.62.
*5-*(*1-Methyl-5-trifluoromethyl-1*H*-pyrazol-4-yl*)*-thiophene-2-sulfonic acid* (*4-trifluoro methylphenyl*)*-amide* **31**. TLC R_{F} = 0.39 (heptane:ethyl acetate, 2:1); HPLC t_{R} 22.14 min (10-70 %, 20 min); ¹H NMR (CD₃OD) δ: 3.915 (3 H, s, *NCH₃),* 6.60 (1 H, s, diazole-H), 7.06 (1 H, d, *J* 3.5*,* thiophene-H), 7.11 (1 H, br s, NH), 7.23 (2 H, d, *J* 8.0, Ph-H), 7.52 (3 H, m, Ph-H & thiophene-H); MS 456.24 (M+H)⁺, C₁₆H₁₁F₆N₃O₂S₂ = 455.40.
*5-Chloro-thiophene-2,4-disulfonic acid bis-[(4-fluoro-phenyl)-amide]* 32. HPLC t_{R} 19.68 min (10-70 %, 20 min); ¹H NMR (CDCl₃) δ: 6.88 (4 H, m, Ph-H), 6.97 (4 H, m, Ph-H), 7.47 (1 H, s, thiophene-H); MS 462.95 (M-2H)⁻, C₁₆H₁₁ClF₂N₂O₄S₃ = 464.92.
*5-Chloro-thiophene-2,4-disulfonic acid bis-[(4-trifluoromethyl)-phenyl)-amide]* **33**. HPLC t_{R} 22.54 min (10-70 %, 20 min); ¹H NMR (CDCl₃) δ: 7.09 (2 H, d, *J* 8.5, Ph-H), 7.14 (2 H, d, *J* 8.5*,* Ph-H), 7.40 (2 H, d, *J* 9.0*,* Ph-H), 7.46 (2 H, d, *J* 9.0*,* Ph-H), 7.66 (1 H, s, thiophene-H); MS 562.88 (M-2H)⁻, C₁₈H₁₁ClF₆N₂O₄S₃ = 564.93.
*4-Methyl-3-nitro-*N*-(4-trifluoromethyl-phenyl)-benzenesulfonamide* **34**. HPLC t_{R} 20.32 min (10-70 %, 20 min); ¹H NMR (CDCl₃) δ: 2.90 (3 H, s, CH₃), 7.15 (2 H, d, *J* 8.0, Ph-H), 7.38 (1 H, d, *J* 8.5, Ph-H), 7.41 (2 H, d, *J* 8.0, Ph-H), 7.81 (1 H, d, *J* 8.5, Ph-H), 8.38 (1 H, s, Ph-H); MS 359.11 (M-H)⁻, C₁₄H₁₁F₃N₂O₄S = 360.31.
*4-Chloro-3-nitro-N-(4-trifluoromethyl-phenyl)-benzenesulfonamide* **35.** TLC R_{F} = 0.40 (heptane:ethyl acetate, 2:1); HPLC t_{R} 15.45 min (20-80 %, 20 min); ¹H NMR (CDCl₃) δ: 7.16 (2 H, d, *J 8.5,* Ph-H), 7.43 (2 H, d, *J 8.5,* Ph-H), 7.57 (1 H, d, *J* 8.5, Ph-H), 7.83 (1 H, dd, *J* 8.5, 8.5, Ph-H), 8.29 (1 H, d, *J* 2.5, Ph-H).
*3-Amino-4-methyl-*N*-*(*4-trifluoromethyl-phenyl*)*-benzenesulfonamide* **36.** HPLC t_{R} 17.14 min (10-70 %, 20 min); ¹H NMR (CD₃OD) δ: 2.07 (3 H, s, CH₃), 7.00 (3 H, m, Ph-H), 7.11 (2 H, d, *J* 8.0, Ph-H), 7.37 (2 H, d, *J* 8.0, Ph-H); MS 329.15 (M-H)⁻, C₁₄H₁₃F₃N₂O₂S = 330.33.
N*-*(*4-Chloro-phenyl*)*-4-methyl-3-nitro-benzenesulfonamide* **37.** ¹H NMR (CDCl₃) δ: 2.58 (3 H, s, CH₃), 6.72 (1 H, br s, NH), 6.96 (2 H, d, *J* 8.0, Ph-H), 7.17 (2 H, d, *J* 8.0, Ph-H), 7.39 (1 H, d, *J* 8.0, Ph-H), 7.74 (1H, d, *J* 8.0, Ph-H), 8.31 (1H, s, Ph-H).
*4-Chloro-*N*-*(*4-chloro-phenyl*)*-3-nitro-benzenesulfonamide* **38.** ¹H NMR (CDCl₃) δ: 6.74 (1 H, br s, NH), 6.98 (2 H, d, *J* 8.0, Ph-H), 7.19 (2 H, d, *J* 8.0, Ph-H), 7.57 (1 H, d, *J* 8.0, Ph-H), 7.71 (1H, d, *J* 8.0, Ph-H), 8.20 (1H, s, Ph-H).
*5-Chloro-4-nitro-thiophene-2-sulfonic acid (3,5-dichloro-phenyl)-amide* **39.** TLC R_{F} = 0.75 (heptane:ethyl acetate, 2:1); HPLC 18.83 min (20-80 %, 20 min); ¹H NMR (CDCl₃) δ: 6.97 (1H, br s, NH), 7.05 (2 H, m, Ph-H), 7.17 (1 H, m, Ph-H), 7.92 (1 H, s, thiophene-H). MS 386.84 (M-H)⁻, C₁₀H₅Cl₃N₂O₄S₂ = 387.65.
*5-Chloro-4-nitro-thiophene-2-sulfonic acid* (*3,5-difluoro-phenyl*)*-amide* **40**. HPLC t_{R} 16.59 min (20-80 %, 20 min); ¹H NMR (CDCl₃) δ: 6.63 (1 H, m, Ph-H), 6.70 (2 H, m, Ph-H), 7.01 (1 H, br s, NH), 7.93 (1 H, s, thiophene-H). MS 353.96 (M-H)⁻, C₁₀H₅ClF₂N₂O₄S₂ = 354.74.
*5-Bromo-6-chloro-pyridine-3-sulfonic acid* (*4-trifluoromethyl-phenyl*)*-amide* **41**. TLC R_{F} = 0.75 (heptane:ethyl acetate, 2:1); HPLC t_{R} 21.09 min (10-70 %, 20 min); ¹H NMR (CDCl₃) δ: 7.17 (2 H, d, J 8.5, Ph-H), 7.47 (2 H, d, J 8.5, Ph-H), 8.23 (1 H, m, pyridine-H), 8.62 (1 H, m, pyridine-H); MS 414.88 (M-H)⁻, C₁₂H₇BrClF₃N₂O₂S = 415.61.
*5-Bromo-6-chloro-pyridine-3-sulfonic acid (3,5-bis-trifluoromethyl-phenyl)-amide* **42**. HPLC t_{R} 23.17 min (10-70 %, 20 min); ¹H NMR (CDCl₃) δ: 7.54 (3 H, s, Ph-H), 8.26 (1 H, m, pyridine-H), 8.65 (1 H, m, pyridine-H); MS 482.80 (M-H)⁻, C₁₃H₆BrClF₆N₂O₂S = 483.61.
*5-(1-Methyl-5-trifluoromethyl-1*H-*pyrazol-4-yl)-thiophene-2-sulfonic acid (3,5-bis-trifluoro-methyl-phenyl)-amide* **43**. TLC R_{F} = 0.63 (heptane:ethyl acetate, 2:1); ¹H NMR (CD₃OD) δ: 3.97 (3 H, s, NCH₃), 6.82 (1 H, s, diazole-H), 7.37 (1 H, dd, *J* 4.0, 4.0, thiophene-H), 7.64 (1 H, dd, *J* 4.0, 4.0, thiophene-H), 7.68 (1 H, s, Ph-H), 7.73 (2 H, s, Ph-H).
*5-Chloro-4-nitro-thiophene-2-sulfonic acid (3,5-bis-trifluoromethyl-phenyl)-amide* **44.** HPLC t_{R} 19.49 min (20-80 %, 20 min); ¹H NMR (CD₃OD) δ: 7.19 (1 H, s, Ph-H), 7.58 (2 H, s, Ph-H), 7.67 (1 H, br s, NH), 7.95 (1 H, s, thiophene-H); MS 452.51 (M-2H)⁻, C₁₂H₅ClF₆N₂O₄S₂ = 454.75.
*5-Chloro-4-nitro-thiophene-2-sulfonic acid 4-fluoro-benzylamide* **45.** HPLC t_{R} 15.89 min (20-80 %, 20 min); TLC R_{F} = 0.64 (heptane:ethyl acetate, 2:1); ¹H NMR (CDCl₃) δ: 4.20 (2 H, d, *J* 6.0, ArCH₂), 6.95 (2 H, t, *J* 8.5, Ph-H), 7.17 (2 H, dd, *J* 8.5, 8.5, Ph-H), 7.82 (1 H, s, thiophene-H); MS 348.96 (M-2H)⁻, C₁₁H₈ClFN₂O₄S₂ = 350.78
*5-Chloro-4-nitro-thiophene-2-sulfonic acid 4-trifluoromethyl-benzylamide* **46.** TLC R_{F} = 0.71 (heptane:ethyl acetate, 2:1); HPLC t_{R} 15.16 min (20-80 %, 20 min); ¹H NMR (CDCl₃) δ: 4.30 (2 H, d, *J* 6.0, ArCH₂), 5.08 (1 H, br s, NH), 7.34 (2 H, d, *J* 8.0, Ph-H), 7.54 (2 H, d, *J* 8.0, Ph-H), 7.86 (1 H, s, thiophene-H); MS 398.91 (M-2H)⁻, C₁₂H₈ClF₃N₂O₄S₂ = 400.78.
*4-Chloro-N-(3,5-dichloro-phenyl)-3-nitro-benzenesulfonamide* **47.** HPLC t_{R} 18.64 min (20-80 %, 20 min); ¹H NMR (CDCl₃) δ: 6.98 (2 H, m, Ph-H), 7.01 (1 H, m, Ph-H), 7.60 (1H, d, *J* 8.5, Ph-H), 7.84 (1H, dd *J* 2.0, 8. 5, Ph-H), 8.27 (1H, d, *J* 2.0, Ph-H); MS 380.91(M-H)⁻, C₁₂H₇Cl₃N₂O₄S = 381.62.
*5-Chloro-4-nitro-thiophene-2-sulfonic acid [2-(1H-indol-3-yl)-ethyl]-amide* **48.** TLC R_{F} = 0.44 (heptane:ethyl acetate, 2:1); HPLC t_{R} 13.48 min (20-80 %, 20 min); ¹H NMR (CD₃OD) *δ:* 2.91 (2 H, t, *J* 6.5, CH₂), 3.43 (2 H, t, *J* 6.5, CH₂), 6.89 (1 H, t, *J* 8.0, indole-H), 6.97 (1 H, t, *J* 8.0, indole-H), 7.01 (1 H, s, indole-H), 7.23 (1 H, d, *J* 8.0, indole-H), 7.38 (1 H, d, *J* 8.0, Ar), 7.47 (1 H, s, thiophene-H); MS 384.21 (M-H)⁻, C₁₄H₁₂ClN₃O₄S₂ = 385.85.
*5-Chloro-4-nitro-thiophene-*2*-sulfonic acid [2-(1*H*-indol-3-yl)-1-methyl-ethyl]-amide* **49.** TLC R_{F} = 0.49 (heptane:ethyl acetate, 2:1); HPLC t_{R} 13.48 min (20-80 %, 20 min); ¹H NMR (CDCl₃) δ: 1.40 (3 H, d, *J* 6.5, CHCH₃), 2.58 (1 H, dd, *J* 10.5, 10.5, CH₂), 2.96 (1 H, dd, *J* 3.5, 3.5, CH₂), 3.64 (1 H, m, CHCH₃), 6.91 (2H, m, indole-H), 7.04 (1 H, t, *J* 8.0, 7.0, indole-H), 7.17 (1 H, d, *J* 8.0, indole-H), 7.25 (2 H, m, indole-H & thiophene-H), 8.105 (1 H, br s, NH); MS 398.63 (M-H)⁻, C₁₅H₁₄ClN₃O₄S₂ = 399.87.
*5-Chloro-4-nitro-thiophene-2-sulfonic acid 3,5-dichloro-benzylamide* **54**. HPLC. t_{R} 18.14 min (20-80 %, 20 min); ¹H NMR (CDCl₃) δ: 4.20 (2 H, d, *J* 6.0, ArCH₂), 5.16 (1 H, br s, NH), 7.07 (2 H, s, Ph-H), 7.23 (1 H, s, Ph-H), 7.84 (1 H, s, thiophene-H); MS 400.86 (M-H)⁻, C₁₁H₇Cl₃N₂O₄S₂ = 401.67.
*5-Chloro-4-nitro-thiophene-2-sulfonic acid 3,5-difluoro-benzylamide* **55.** HPLC t_{R} 16.42 min (20-80 %,20 min); ¹H NMR (CDCl₃) δ: 4.21 (2 H, d, *J* 6.0, ArCH₂), 5.20 (1 H, br s, NH), 6.69 (1 H, m, Ph-H), 6.75 (2 H, m, Ph-H), 7.87 (1 H, s, thiophene-H). MS 367.16 (M-H)⁻, C₁₁H₇ClF₂N₂O₄S₂ = 368.77.
*5-Chloro-4-nitro-thiophene-2-sulfonic acid 4-chloro-benzylamide* **56.** HPLC t_{R} 16.82 min (20-80 %, 20 min); ¹H NMR (CDCl₃) δ: 4.20 (2 H, d, *J* 6.0, ArCH₂), 5.13 (1 H, br s, NH), 7.07 (2 H, d, *J* 8.5, Ph-H), 7.23 (2 H, d, *J* 8.5, Ph-H), 7.82 (1H, s, thiophene-H); MS 366.95 (M-H)', C₁₁H₈Cl₂N₂O₄S₂ = 367.23.
*5-Chloro-4-nitro-thiophene-2-sulfonic acid [1-(4-fluoro-phenyl)-ethyl]-amide* **57***.* HPLC t_{R} 16.25 min (20-80 %, 20 min); ¹H NMR (CDCl₃) δ: 1.45 (3 H, d, *J* 7.0, CH₃), 4.54 (1 H, m, PhCH), 5.18 (1 H, br s, NH), 6.90 (2 H, m, Ph-H), 7.10 (2 H, m, Ph-H), 7.55 (1 H, s, thiophene-H); MS 363.18 (M-H)', C₁₂H₁₀ClFN₂O₄S₂ = 364.80.
*5-Chloro-4-nitro-thiophene-2-sulfonic acid (4-chloro-phenyl)-isobutyl-amide* **58*.*** HPLC t_{R} 20.04 min (20-80 %, 20 min); ¹H NMR (CDCl₃) δ: 0.85 (6 H, d, *J* 7.0, CH₃), 1.53 (1 H, m, CH(CH₃)₂), 5.18 (2 H, d, *J* 7.5, *N*CH₂), 7.08 (2 H, d, *J* 8.5, Ph-H), 7.26 (2 H, d, *J* 8.5, Ph-H), 7.61 (1 H, s, thiophene-H).
*5-Chloro-4-nitro-thiophene-2-sulfonic acid* (*1*H*-benzoimidazol-2-yl*)*-amide* **59**. HPLC t_{R} 17.89 min (0-60 %, 20 min); ¹H NMR (CDCl₃) δ: 7.10 (1 H, t, *J* 8.0, benzimidazole-5/6), 7.23 (2 H, m, benzimidazole-4/7 & 5/6), 7.63 (1 H, d, *J* 8.5, benzimidazole-4/7), 8.15 (1 H, s, thiophene-H); MS 35.9.03 (M+H)⁺, C₁₁H₇ClN₄O₄S₂ =358.78.
*5-Chloro-4-nitro-thiophene-3-sulfonoc acid [2-(6-chloro-1H-indol-3-yl)-ethyl]-amide* 60. HPLC t_{R} 23.16 min (0-60 %, 20 min);¹H NMR (CDCl₃) δ: 2.91 (2 H, t, *J* 6.5, CH₂), 3.38 (2 H, t, *J* 6.5, CH₂), 4.70 (1 H, br s, NH), 6.89 (1 H, t, *J* 8.0, indole-H), 6.95 (2 H, m, indole-H), 7.23 (2 H, m, indole-H), 7.59 (1 H, s, thiophene-H), 8.06 (1 H, br s, indole-NH); MS 419.94 (M-H)⁻, C₁₄H₁₁Cl₂N₃O₄S₂ = 420.29.
*5-Chloro-4-nitro-thiophene-2-sulfonic acid (4-methoxy-phenyl)-amide* **61.** HPLC t_{R} 12.81 min (20-80 %, 20 min); ¹H NMR (DMSO-*d*₆) *δ:* 3.70 (3 H, s, OMe), 6.88 (2 H, d, *J* 8.5, Ph-H), 7.07 (2 H, d, *J* 8.5, Ph-H), 7.82 (1 H, s, thiophene-H); MS 349.26 (M+H)⁺, C₁₁H₉ClN₂O₅S₂ = 348.78.
*5-Chloro-4-nitro-thiophene-2-sulfonic acid phenylamide* **62.** HPLC t_{R} 15.36 min (20-80 %, 20 min); ¹H NMR (CDCl₃) δ: 6.66 (1 H, s, NH), 7.10 (2 H, d, *J* 8.5, Ph-H), 7.20 (1 H, m, Ph-H), 7.28 (2 H, d, *J* 8.5, Ph-H), 7.82 (1 H, s, thiophene-H); MS 317.22 (M-H)⁻, C₁₀H₇ClN₂O₄S₂ = 318.76.
*5-Chloro-4-nitro-thiophene-2-sulfonic acid* p*-tolylamide* **63.** HPLC t_{R} 16.59 min (20-80 %, 20 min);¹H NMR (CDCl₃) δ: 2.27(3 H, s, CH₃), 6.54 (1 H, br s, NH), 6.98 (2 H, d, *J* 7.5*,* Ph-H), 7.08 (2 H, d, *J* 7.5*,* Ph-H), 7.79 (1 H, s, thiophene-H); MS 331.24 (M-H)', C₁₁H₉ClN₂O₄S₂ = 332.78.
*5-Chloro-4-nitro-thiophene-2-sulfonic acid benzylamide* 64. HPLC t_{R} 15.73 min (20-80 %, 20 min);¹H NMR (CDCl₃) *δ:* 4.24 (2 H, d, *J* 5.5, CH₂), 5.05 (1 H, br s, NH), 7.16 (2 H, m, Ph-H), 7.24 (3 H, m, Ph-H), 7.76 (1 H, s, thiophene-H); MS 331.04 (M-H)', C₁₁H₉ClN₂O₄S₂ = 332.78.
*5-Nitro-thiophene-2-sulfonic acid (4-chloro-phenyl)-amide* 68. HPLC t_{R} 22.61 min (0-60 %, 20 min); ¹H NMR (CDCl₃) δ: 7.11 (2 H, d, *J* 9.0, Ph-H), 7.32 (2 H, d, *J* 9.0, Ph-H), 7.38 (1 H, d, *J* 6.0, thiophene 3/4), 7.95 (1 H, d, *J* 6.0, thiophene 3/4); MS 317.02 (M-H)⁻, C₁₀H₇ClN₂O₄S₂ = 318.76.
*4-Nitro-thiophene-2-sulfonic acid (4-chloro-phenyl)-amide* 69. HPLC t_{R} 21.86 min (0-60 %, 20 min); ¹H NMR (CDCl₃) δ: 7.11 (2 H, d, *J* 9.0, Pb-H), 7.32 (2 H, d, *J* 9.0, Ph-H), 8.00 (1 H, s, thiophene 3/5), 8.43 (1 H, s, thiophene 3/5). MS 317.08 (M-H)⁻, C₁₀H₇ClN₂O₄S₂ = 318.76.
*5-Chloro-thiophene-2,4-disulfonic acid bis-[(4-chloro-phenyl)-amide]* **70.** HPLC t_{R} 19.66 min (0-60 %, 20 min); ¹H NMR (CDCl₃) δ. 6.96 (4 H, d, *J* 8.0, Ph-H), 7.04 (1 H, m, NH), 7.19 (5 H, m, Ph-H & NH), 7.57 (1 H, s, thiophene-H). MS 496.83 (M-H)⁻, C₁₆H₁₁Cl₃N₂O₄S_{3 =} 497.83.
*5-Ethyl-4-nitro-thiophene-2-sulfonic acid (4-chloro-phenyl)-amide* **71**. HPLC t_{R} 17.41 min (20-80 %, 20 min); ¹H NMR (CDCl₃) δ: 1.36 (3 H, t, *J* 7.0, CH₂CH₃), 3.28 (2 H, dd, *J* 7.0, 7.0, CH₂H₃), 6.65 (1 H, br s, NH), 7.10 (2 H, d, *J* 8.0, Ph-H), 7.30 (2 H, d, *J* 8.0, Ph-H), 7.96 (1 H, s, thiophene-H); MS 345.05 (M-H)⁻, C₁₂H₁₁ClN₂O₄S₂ = 346.81. *Thioacetic acid S-[5-(4-chloro-phenylsulfamoyl)-3-nitro-thiophen-2-yl] ester* **72***.* HPLC t_{R} 21.56 min (20-80 %, 20 min); ¹H NMR (CD₃OD) δ: 2.59 (3 H, s, CH₃), 7.15 (2 H, d, *J 8.5,* Ph-H), 7.28 (2 H, d, *J* 8.5, Ph-H), 7.91 (1 H, s, thiophene-H); MS 391.15 (M-H)⁻, C₁₂H₉ClN₂O₅S₃ = 392.86.
*5-Methyl-4-nitro-thiophene-2-sulfonic acid* (*4-chloro-phenyl*)*-amide* **73.** HPLC t_{R} 17.95min (20-80 %, 20 min); ¹H NMR (CD₃OD) δ: 2.66 (3 H, s, CH₃), 7.13 (2 H, d, *J* 8.0, Ph-H), 7.32 (2 H, d, *J* 8.0, Ph-H), 7.70 (1 H, s, thiophene-H); MS 333.50 (M-H)⁻, C₁₁H₉ClN₂O₄S₂ = 332.78.
*5-Methyl-thiophene-2,4-disulfonic acid bis-*[(*4-chloro-phenyl*)*-amide*] **74**. HPLC t_{R} 22.19 min (20-80 %, 20 min); ¹H NMR (CDCl₃) δ: 2.31 (3 H; s, CH₃), 7.01 (2 H, d, I 8.0, Ph-H), 7.10 (2 H, d, *J* 8.0, Ph-H), 7.26 (4 H, m, Ph-H), 7.81 (1 H, s, thiophene-H); MS 476.94 (M-H)⁻, C₁₇H₁₄Cl₂N₂O₄S₃ = 477.41.
*4-Nitro-thiophene-1-sulfonic acid (4-trifluoromethyl-phenyl)-amide* **76.** HPLC t_{R} 15.99 min (20-80 %, 20 min); ¹H NMR (CDCl₃) δ: 7.28 (2 H, d, *J* 8.5, Ph-H), 7.53 (2 H, d, *J* 8.5, Ph-H), 8.03 (1 H, s, thiophene 3/5), 8.41 (1 H, s, thiophene 3/5); MS 351.18 (M-H)⁻, C₁₁H₇F₃N₂O₄S₂ = 352.31.
*4-Nitro-thiophene-2-sulfonic acid* [*2-*(*1*H*-indol-3-yl*)*-ethyl*]*-amide* **77.** HPLC t_{R} 14.01 min (20-80 %, 20 min); ¹H NMR (CDCl₃) *δ*: 2.97 (2 H, t, *J* 6.5*,* CH₂), 3.41 (2 H, t, *J* 6.5, CH₂), 7.01 (2 H, m, indole-H), 7.13 (1 H, t, *J* 7.5, indole-H), 7.30 (1 H, d, *J* 7.5, indole-H), 7.38 (1 H, d, *J* 7.5, indole-H), 7.75 (1 H, s, thiophene 3/5), 8.16 (1 H, s, thiophene 3/5); MS 350.24 (M-H)⁻, C₁₄H₁₃N₃O₄S₂ = 351.40.
*5-Morpholin-4-yl-4-nitro-thiophene-2-sulfonic acid* (*4-chloro-phenyl*)*-amide* **79**. HPLC t_{R} 18.52 min (10-70 %, 20 min); ¹H NMR (CDCl₃) δ: 3.33 (4 H, t, *J* 5.0, morpholine-H), 3.88 (4 H, t, *J* 5.0, morpholine-H), 7.03 (1 H, br s, NH), 7.13 (2 H, d, *J* 8.0, Ph-H), 7.29 (2 H, d, *J* 8.0, Ph-H), 7.91 (1 H, s, thiophene-H); MS 404.26 (M+H)⁺, C₁₄H₁₄ClN₃O₅S2 = 403.86.
*5-*(*2-Methoxy-ethylamino*)*-4-nitro-thiophene-2-sulfonic acid* (*4-chloro-phenyl)-amide* **80.** HPLC t_{R} 18.38 min (10-70 %, 20 min); ¹H NMR (CDCl₃) δ: 3.33 (3 H, s, OMe), 3.37 (2 H, t, *J* 5.5, CH₂), 3.58 (2 H, t, *J* 5.5, CH₂), 7.06 (2 H, d, *J* 7.0, Ph-H), 7.21 (2 H, d, *J* 7.0, Ph-H), 7.33 (1 H, br s, NH), 7.70 (1 H, s, thiophene-H), 8.47 (1 H, br s, NH); MS 390.21 (M-H)⁻, C₁₃H₁₄ClN₃O_{S}S₂ = 391.85.
*4-Chloro-*N*-*[*2-*(*5-chloro-1H-indol-3yl*)*-ethyl*]*-3-nitro-benzenesulfonamide* **81.** HPLC t_{R} 16.63 min (20-80 %, 20 min); ¹H NMR (CDCl₃) δ: 2.83 (2 H, t, *J* 6.0, CH₂), 3.29 (2 H, t, *J* 6.0, CH₂), 6.54 (1 H, br s, NH), 6.90 (2 H, m, indole-H), 7.23 (2 H, m, indole-H), 7.34 (1 H, dd, *J* 1.5,8.0, Ph-H), 7.62 (1 H, dd, *J* 1.5, 8.0, Ph-H), 8.15 (1 H, d, *J 1.5,* Ph-H), 9.54 (1 H, br s, indole NH); MS 412.19 (M-2H)⁻, C₁₆H₁₃Cl₂N₃O₄S = 414.26.
*N-[2-(5-Chloro-1H-indol-3 yl)-ethyl]-4-methyl-3-nitro-benzenesulfonamide* **82**. HPLC t_{R} 16.25 min (20-80 %, 20 min);¹H NMR (CDCl₃) *δ:* 2.73 (3 H, s, CH₃), 2.82 (2 H, t, *J* 6.0, CH₂), 3.22 (2 H, t, *J* 6.0, CH₂), 6.86 (1 H, m, indole-H), 6.91 (1 H, s, indole-H), 7.15 (2 H, m, indole-H), 7.20 (1 H, m, Ph-H), 7.63 (1 H, dd, *J* 2.0, 8.0, Ph-H), 8.13 (1 H, d, *J* 2.0*,* Ph-H); MS 392.03 (M-H)⁻, C₁₇H₁₆ClN₃O₄S = 393.85.
*N-(1H-Benzoimidazol-2-yl)-4-chloro-3-nitro-benzenesulfonamide* **83.** HPLC t_{R} 17.55 min (0-60 %, 20 min); ¹H NMR (CDCl₃) δ. 7.07 (1 H, t, *J* 8.0, Ph-H), 7.16 (1 H, t, *J* 8.0, Ph-H), 7.22 (2 H, d, *J* 9.0, Ph-H), 7.64 (2 H, d, *J* 7.5, Ph-H), 7.97 (1H, dd, *J* 2.5, 9.0, Ph-H), 8.42 (1 H, d, *J* 2.5, NH); MS 351.25 (M-H)⁻, C₁₃H₉ClN₄O₄S = 352.75.
*6-Chloro-imidazo[2,1-b]thiazole-5-sulfonic, acid (3,5-bis-trifluoromethyl-phenyl)-amide* **84.** HPLC t_{R} 24.09 min (0-60 %, 20 min); ¹H NMR (CDCl₃) *δ*: 3.40 (1 H, br s, NH), 7.10 (1 H, d, J4.5, thiazole-H), 7.54 (1 H, s, Ph-H), 7.56 (2 H, s, Ph-H), 7.90 (1 H, d, *J* 4.5, thiazole-H); MS 447.86 (M-2H)⁻, C₁₃H₆ClF₆N₃O₂S₂ = 449.78.
*2,3-Dihydro-benzo[1,4]dioxine-6-sulfonic acid* (*4-chloro-phenyl*)*-amide* **85.** HPLC t_{R} 21.20 min (0-60 %, 20 min); ¹H NMR (CDCl₃) δ: 4.27 (4 H, m, CH₂), 6.88 (1 H, d, *J* 8.0, Ph-H), 7.04 (3 H, m, Ph-H), 7.19 (2 H, d, *J* 9.0, Ph-H), 7.25 (1 H, m, Ph-H), 7.31 (1 H, br s, NH); MS 326.11 (M+H)⁺, C₁₄H₁₂ClNO₄S = 325.77.
*2,3-Dihydro-benzo[1,4]dioxine-6-sulfonic acid* (*3,5-*bis*-trifluoromethyl-phenyl*)*-amide* **86.** HPLC t_{R} 24.58 min (0-60 %, 20 min); ¹H NMR (CDCl₃) δ: 4.21 (4 H, m, CH₂), 6.86 (1 H, d, *J* 8.5, Ph-H), 7.27 (1 H, d, *J* 8.5, Ph-H), 7.30 (1 H, d, *J* 2.0, Ph-H), 7.47 (2 H, s, Ph-H), 7.50 (1 H, s, Ph-H), 7.56 (1 H, br-s, NH); MS 425.9 (M-2H)⁻, C₁₆H₁₁F₆NO₄S = 427.32..
*6-Phenoxy-pyridine-3-sulfonic acid (4-chloro-phenyl)-amide* **87.** HPLC t_{R} 23.20 min (10-70 %, 20 min); ¹H NMR (CDCl₃) δ: 6.99 (1 H, d, *J* 8.5, Ph-H), 7.11 (2 H, d, *J* 8.5, Ph-H), 7.26 (1 H, m, Ph-H), 7.41 (2 H, m, Ph-H), 7.57 (3 H, m, Ph-H), 7.63 (1 H, s, Ph-H), 8.03 (1 H, d, *J* 8.5, Ph-H), 8.57 (1 H, br s, NH); MS 463.90 (M+2H)⁺, C₁₇H₁₃ClN₂O₃S = 360.82.
*5-Chloro-3-methyl-benzo[b]thiophene-*2*-sulfonic acid* (*4-chloro-3-nitro-phenyl*)*-amide* **88.** HPLC t_{R} 22.79 min (10-70 %, 20 min); ¹H NMR (CDCl₃) δ: 2.55 (3 H, s, CH₃), 7.38 (2 H, m, Ph-H), 7.42 (1 H, d, *J* 8.0, Ph-H), 7.66 (1 H, s, Ph-H), 7.69 (1 H, d, *J* 9.0, Ph-H), 7.73 (1 H, s, Ph-H); MS 416.91 (M-H)⁻, C₁₅H₁₀Cl₂N₂O₄S₂ = 417.29.
N-(*3*,*5-*Bis*-trifluoromethyl-phenyl*)*-4-pyrazol-1-yl-benzenesulfonamide* **89.** HPLC t_{R} 21.58 min (10-70 %, 20 min); ¹H NMR (CD30D) δ: 6.53 (1 H, s, Ph-H), 7.57 (1 H, s, Ph-H), 7.65 (2 H, s, Ph-H), 7.73 (1 H, s, Ph-H), 7.92 (4 H, s, Ph-H), 8.28 (1 H, s, Ph-H); MS 434.03 (M-H)⁻, C₁₇H₁₁F₆N₃O₂S = 435.34
*4-(4-Chloro-phenylsulfamoyl)-3,5-dimethyl-1H-pyrrole-2-carboxylic acid ethyl ester* **90.** HPLC t_{R} 20.99 min (0-60 %, 20 min); ¹H NMR (CD₃OD) *δ:* 1.32 (3 H, t, J 7.0, CH₂CH₃), 2.30 (3 H, s, CH₃), 2.40 (3 H, s, CH₃), 4.26 (2 H, dd, *J* 7.0*,* 7.0, CH₂CH₃), 7.01 (2H, d, *J* 9.0, Ph-H), 7.20 (2 H, d, J 9.0, Ph-H); MS 355.03 (M-H)⁻, C₁₅H₁₇ClN₂O₄S = 356.83.
*4-(3,5-*Bis*-trifluoromethyl-phenylsulfamoyl)-3,5-dimethyl-1H-pyrrole-2-carboxylic acid* **91.** HPLC t_{R} 23.28 min (0-60 %, 20 min); ¹H NMR (CDCl₃) δ: 2.42 (3 H, s, CH₃), 2.47 (3 H, s, CH₃), 7.45 (3 H, s, Ph-H), 10.35 (1 H, br s, COOH); MS 428.98 (M-H)⁻, C₁₅H₁₂F₆N₂O₄S = 430.32.
*4-(4-Chloro-phenylsulfamoyl)-3,5-dimethyl-1H-pyrrole-2-carboxylic acid* **92.** HPLC t_{R} 19.59 min (0-60 %, 20 min); ¹H NMR (CDCl₃) δ: 2.37 (3 H, s, CH₃), 2.45 (3 H, s, CH₃), 6.99 (2 H, d, *J* 8.5, Ph-H), 7.07 (1 H, br s, NH), 7.21 (2 H, d, *J* 8.5, Ph-H), 9.47 (1 H, br s, COOH); MS 327.26 (M-H)⁻, C₁₃H₁₃ClN₂O₄S = 328.77.
*2-(4-Chloro-phenylsulfamoyl)-4-methyl-thiazole-5-carboxylic acid ethyl ester* **93*.*** HPLC t_{R} 21.74 min (10-70 %, 20 min); ¹H NMR (CDCl₃) δ: 1.26 (3 H, t, *J* 7.5, CH₂CH₃), 2.47 (3 H, s, CH₃), 4.21 (2 H, dd, *J* 7.5, CH₂CH₃), 7.21 (2 H, d, *J* 8.0, Ph-H), 7.28 (2 H, d, *J* 8.0, Ph-H).
*3,5-Dichloro-N-(4-chloro-phenyl)-4-hydroxy-benzenesulfonamide* **94.** HPLC t_{R} 21.65 min (10 - 60%, 20 min); ¹H NMR (CDCl₃) δ: 6.67 (1 H, s, OH), 7.06 (2 H, d, ArH, *J* 8.9), 7.21 (2 H, d, ArH, *J* 8.9), 7.72 (2H, s, ArH), 8.30 (1H, s, NH); MS 352.01 (M-H)⁻, C₁₂H₈Cl₃NO₃S = 352.62.
N-(*3,5-*Bis*-trifuoromethyl-phenyl*)*-3,5-dichloro-4-hydroxy-benzenesulfonamide* **95.** HPLC t_{R} 24.06 min (10 - 60%, 20 min); ¹H NMR (CD₃OD) δ: 7.8 (2 H, d, ArH, *J* 8.7), 7.68 (1 H, s, ArH,), 7.67 (2 H, s, ArH); MS 454.81 (M), C₁₄H₇Cl₂F₆NO₃S = 454.17.
*3,5-Dichloro-4-hydroxy-*N*-*(*4-trifluoromethyl-phenyl*)*-benzenesulfonamid*e **96**. HPLC t_{R} 22.46 min (10 - 60%, 20 min); ¹H NMR (CD₃OD) δ: 7.28 (2 H, d, ArH, *J* 8.7), 7.56 (2 H, d, ArH, *J* 8.7), 7.67 (2 H, s, ArH); MS 386.06 (M), C₁₃H₈Cl₂Cl₂F₃NO₃S = 386.17.
N*-*(*4-Chloro-phenyl*)*-4-nitro-benzenesulfonamide* **97**. HPLC t_{R} 12.24 min (10 - 60%, 20 min); ¹H NMR (CD₃OD) δ: 7.08 (2 H, d, ArH, *J* 8.8), 7.23 (2 H, d, ArH, *J* 8.8), 7.96 (2 H, d, ArH, *J* 8.8), 8.32 (2 H, d, ArH, *J* 8.8). MS 311.33 (M-1)⁻, C₁₂H₉ClN₂O₄S = 312.73.
*N-(3,5-Bis-trifluoromethyl-phenyl)-4-nitro-benzenesulfonamide* **98**. HPLC t_{R} 15.36 min (10 - 60%, 20 min); ¹H NMR (CD₃OD) δ: 7.65 (1 H, s, ArH,), 7.66 (2 H, s, ArH), 8.06 (2H, d, ArH, *J* 9.0) 8.39 (2H, d, ArH, *J* 9.0); MS 413.43 (M-1)⁻, C₁₄H₈F₆N₂O₄S = 414.28.
*4-Amino-N-(3,5-bis-trifluoromethyl-phenyl)-3-chloro-benzenesulfonamide* **99.** HPLC t_{R} 18.38 min (10 - 60%, 20 min); ¹H NMR (CD₃OD) δ: 6.78 (1 H, d, ArH, *J* 7.8), 7.41 (1 H, d, ArH, *J* 7.8), 7.56 (1 H, s, ArH), 7.62 (2 H, s, ArH), 7.72 (1 H, s, ArH); MS 419.77 (M+H)⁺, C₁₄H₉ClF₆N₂O₂S = 418.74.
*3-Nitro-N-(4-trifluoromethyl-phenyl)-benzenesulfonamide* **100.** HPLC t_{R} 23.04 min (10 - 60%, 20 min); ¹H NMR (DMSO-d₆) *δ:* 7.30 (2 H, d, ArH, *J* 8.3), 7.63 (2 H, d, ArH, *J* 8.3), 8.06 (2 H, d, ArH, *J* 8.8), 8.38 (2 H, d, ArH, *J* 8.8), 11.17 (1 H, s, NH); MS 345.05 (M-H)⁻, C₁₃H₉F₃N₂O₄S = 346.28.
*3,5-Dichloro-*N*-(3,5-dichloro-phenyl)-4-hydroxy-benzenesulfonamide* **101.** HPLC t_{R} 16.69 min (10 - 60%, 20 min); ¹H NMR (CD₃OD) δ: 7.08 (2 H, s, ArH), 7.30 (1 H, s, ArH), 7.71 (2 H, s, ArH); MS 383.77 (M-H)⁻, C₁₂H₇Cl₄NO₃S = 384.89.
*4-Amino-3-chloro-*N*-(4-chloro-phenyl)-benzenesulfonamide* **102.** HPLC t_{R} 14.17 min (10 - 60%, 20 min); ¹H NMR (CD₃OD) δ: 6.75 (1 H, d, ArH, *J* 8.3), 7.07, (2 H, d, ArH, *J* 8.8), 7.28 (2 H, d ArH, *J* 8.3), 7.32 (1 H, d, ArH, *J* 8.8), 7.49 (1 H, s, ArH); MS 317.43 (M), C₁₂H₁₀Cl₂N₂O₂S = 317.19.
*3-Chloro-N-(4-chloro-phenyl)-4-methoxy-benzenesulfonamide* **103.** HPLC t_{R} 16.65 min (10 - 60%, 20 min); ¹H NMR (DMSO-*d₆*) δ: 3.31 (3 H, s, OCH₃), 7.08 (2 H, d, ArH, *J* 8.3), 7.27 (1 H, d, ArH, *J* 8.8), 7.30 (2 H, d, ArH, *J* 8.3), 7.64 (1 H, d, ArH, *J* 8.8)*,* 7.74, (1 H, s, ArH), 10.35, (1 H, s, NH); MS 332.02 (M), C₁₃H₁₁Cl₂NO₃S = 332.20.
*N-(3,5-*Bis*-trifluoromethyl-phenyl)-3-chloro-4-methoxy-benzenesulfonamide* **104.** HPLC t_{R} 19.21 min (10 - 60%, 20 min); ¹H NMR (DMSO-d₆) δ: 3.30 (3 H, s, OCH₃), 7.31 (1 H, d, ArH, *J* 8.8), 7.65 (2 H, s, ArH), 7.73 (1 H, dd, ArH, *J* 8.8, 2.4), 7.77 (1 H, s, ArH), 7.80 (1 H, d, ArH, *J* 2.4), 11.12 (1 H, s, NH); MS 433.98 (M), C₁₅H₁₀ClF₆NO₃S = 433.75.
*N-(3-Chloro-4-nitro-phenyl)-3,5-bis-trifluoromethyl-benzenesulfonamide* **105**. HPLC t_{R} 19.56 min (10 - 60%, 20 min); ¹H NMR (DMSO-d₆) δ: 7.38 (1 H, dd, ArH, *J* 8.8, 2.4), 7.66 (1 H, d, ArH, *J* 8.8), 7.76 (1 H, d, ArH, *J* 2.4)*,* 8.34 (2 H, s, ArH), 8.51 (1 H, s, ArH), 11.12 (1 H, s, NH); MS 448.84 (M), C₁₄H₇ClF₆N₂O₄S = 448.73.
*3-(4-Acetyl-piperazin-1-yl)-N-(3,5-*bis*-trifluoromethyl-phenyl)-4-nitro-benzenesulfonamide* **106.** HPLC t_{R} 22.57 min (10 - 60%, 20 min); ¹H NMR (CD₃OD) δ: 2.15 (3 H, s, CH₃), 3.20 (4 H, m, CH₂), 3.68 (2 H, m, CH₂), 3.84 (2 H, m, CH₂), 7.12 (2 H, m, ArH), 7.46 (1 H, s, ArH), 8.26 (1 H, s, ArH), 8.38 (2 H, s, ArH); MS 540.09 (M), C₂₀H₁₈F₆N₄O₅S = 540.44.
*N-(3,5-Bis-trifluoromethyl-phenyl)-2-nitro-benzenesulfonamide* **107.** HPLC t_{R} 16.25 min (10 - 60%, 20 min); ¹H NMR (DMSO-d₆) δ: 7.58 (2 H, s, ArH), 7.82 (1 H, d, ArH, *J* 8.6), 7.88 (1 H, dd, ArH, *J* 8.6, 8.6), 8.13 (1 H, dd, ArH, *J* 8.6, 8.6), 8.37 (1 H, s, ArH), 8.38 (1 H, d, ArH, *J* 8.6), 11.60 (1 H, s, NH); MS 414.85 (M), C₁₄H₈F₆N₂O₄S = 414.28.
*3-(3,5-*Bis*-trifluoromethyl-phenylsulfamoyl)-benzoic acid* **108**. HPLC t_{R} 22.39 min (10 - 60%, 20 min); ¹H NMR (CD₃OD) δ: 7.57 (2 H, s, ArH), 7.62 (2 H, s, ArH), 7.67 (1 H, dd, ArH, *J* 7.8, 7.8), 7.97 (1 H, dd, ArH, *J* 7.8, 1.0), 8.12 (1 H, dd, ArH, *J* 7.8, 1.0), 8.30 (1 H, d, ArH, *J* 1.0); MS 411.99 (M-2H)⁻, C₁₅H₉F₆NO₄S = 413.29.
*3,5-Dichloro-N-(4-chloro-benzyl)-4-hydroxy-benzenesulfonamide* **109.** HPLC t_{R} 24.04 min (10 - 60%, 20 min); ¹H NMR (CD₃OD) δ 4.16 (2 H, s, CH₂), 7.97 (2 H, d, ArH), *J* 8.8), 8.81 (2 H, s, ArH) 8.39 (2 H, d, ArH, *J* 8.8); MS 365.26 (M-H)⁻, C₁₃H₁₀Cl₃NO₃S = 366.65.
*3,5 Dichloro-4-hydroxy-N-(4-trifluoromethyl-benzyl)-benzenesulfonamide* **110.** HPLC t_{R} 23.61 min (10 - 60%, 20 min); ¹H NMR (CD₃OD) δ: 4.24 (2 H, s, CH₂), 7.32 (2 H, d, ArH, *J* 8.7), 7.54 (2 H, d, ArH, *J* 8.7), 7.81 (2 H, s, ArH); MS 400.19 (M), C₁₄H₁₀Cl₂F₃NO₃S = 400.20.
*3,5-Dichloro-4-hydroxy-N-[2-(1H-indol-3-yl)-ethyl]-benzenesulfonamide* **111.** HPLC t_{R} 16.56 min (10- 60%, 20 min); ¹H NMR (CD₃OD) δ: 2.95 (2 H, d, CH₂, *J* 7.2), 3.32 (2 H, s, CH₂, *J* 7.2), 6.99 (1 H, s, ArH), 7.06 (1 H, dd, ArH, *J* 8.1, 8.1), 7.17 (1 H, dd, ArH, *J* 8.1, 8.1), 7.33 (1 H, d, ArH, *J* 8.1), 7.39 (1 H, d, ArH, *J* 8.1), 7.50 (2 H, s, ArH); MS 382.82 (M-2H)⁻, C₁₆H₁₄Cl₂N₂O₃S = 385.27.
*4,5-Dibromo-thiophene-2-sulfonic acid (3,5-dichloro-phenyl)-amide* **112.** HPLC t_{R} 8.21 min (method B); MS 463.9 (M-H)^{- 79}Br⁸¹Br³⁵Cl₂, C₁₀H₅Br₂Cl₂NO₂S₂ = 466.00.
*N-*(*3,5-Dichloro-phenyl*)*-4-oxazol-2-yl-benzenesulfonamide* **113.** HPLC t_{R} 9.04 min (method B); MS 367.0 (M-H)^{- 35}Cl₂, C₁₅H₁₀Cl₂N₂O₃S = 369.22.
*4-Bromo-5-chloro-thiophene-*2*-sulfonic acid* (*3,5-*bis*-trifluoromethyl-phenyl*)*-amide* **114.** HPLC t_{R} 9.22 min (method B); MS 487.9 (M-H)^{- 81}Br³⁵Cl, C₁₂H₅BrClF₆NO₂S₂ = 488.65.
*4-Bromo-5-chloro-thiophene-2-sulfonic acid* (*3,5-dichloro-phenyl*)*-amide* **115.** HPLC t_{R} 9.12 min (method B); MS 419.9 (M-H)^{- 81}Br³⁵Cl₃, C₁₀H₅BrCl₃NO₂S₂ = 421.54.
*5-Bromo-thiophene-2-sulfonic acid* (*4-trifluoromethyl-phenyl*)*-amide* 116. HPLC t_{R} 8.28 min (method B); MS 385.9 (M-H)^{- 81}Br, C₁₁H₇BrF₃NO₂S₂ = 386.21.
*5-Benzenesulfonyl-thiophene-2-sulfonic acid (3,5-*bis*-trifluorometlzyl-phenyl)-amide* **117.** MS 514.2 (M-H)⁻, C₁₈H₁₁F₆NO₄S₃ = 515.47.
*5-Benzenesulfonyl-thiophene-2-sulfonic acid (2,4-dichloro-phenyl)-amide* **118.** MS 446.0 (M-H)^{- 35}Cl², C₁₆H₁₁Cl₂NO₄S₃ = 448.36.
*5-Chloro-3-methyl-benzo[*b*]thiophene-2-sulfonic acid (3,5-*bis*-trifluoromethyl-phenyl)-amide* **119**. ¹H NMR (CDCl₃) δ: 2.51 (s, 3 H, CH₃), 7.40 (dd, 1 H, J 2.0, 8.7, Ar-H), 7.51 (s, 1 H, NH), 7.55 (s, 3 H, Ar-H), 7.67 (d, 1 H, *J* 8.7, Ar-H), 7.69 (s, 1 H, Ar-H); MS (accurate by FAB) 472.97412 (M)^{+ 35}Cl, C₁₇H₁₀ClF₆NO₂S₂ = 472.9746.
*Benzo[*b*]thiophene-2-sulfonic acid (3,5-*bis*-trifluoromethyl-phenyl)-amide* **120**. ¹H NMR (CDCl₃) δ: 7.27 (s, 1 H, NH), 7.45 (m, 5 H, Ar-H), 7.83 (m, 1 H, Ar-H), 8.06 (m, 1 H, Ar-H), 8.24 (s, 1H, Ar-H); MS (accurate by FAB) 426.0049 (MH)⁺ C₁₆H₉F₆NO₂S₂ = 424.9979.
*Benzo[1,2,5]thiadiazole-5-sulfonic acid (4-chloro-phenyl)-amide* **121.** ¹H NMR (CDCl₃) δ: 6.83 (s, 1 H, NH), 7.00 (m, 2 H, Ar-H), 7.16 (m, 2 H, Ar-H), 7.79 (dd, 1 H, *J* 1.8, 9.2, Ar-H), 8.03 (dd, 1 H, *J* 0.7, 9.2, Ar-H), 8.46 (dd, *J* 0.7, 1.8, 1 H, Ar-H); MS (accurate by FAB) 325.9821 (MH)⁺, C₁₂H₈C1N₃O₂S₂ = 324.9746.
*Benzo[1,2,5]thiadiazole-5-sulfonic acid (3,5-bis-trifluoromethyl-phenyl)-amide* **122**. ¹H NMR (CDCl₃) δ: 7.43 (s, 1 H, NH), 7.58 (s, 3 H, Ar-H), 7.84 (dd, 1 H, Ar-H), 8.09 (dd, 1 H, *J* 1.8, 9.2, Ar-H), 8.03 (dd, 1 H, *J* 0.7, 9.2, Ar-H), 8.57 (dd, *J* 0.7, 1.8, 1 H, Ar-H); MS (accurate by FAB) 427.9960 (MH)⁺, C₁₄H₇F₆N₃O₂S₂ = 426.9884.
*Benzo[1,2,5] thiadiazole-5-sulfonic acid (4-trifluoromethyl-phenyl)-amide* **123.** ¹H NMR (CDCl₃) δ: 7.19 (d, 2 H, Ar-H), 7.44 (m, 3 H, Ar-H & NH), 7.87 (dd, 1 H, *J* 1.8, 9.2, Ar-H), 8.06 (dd, 1 H, *J* 0.7, 9.2, AR-H), 8.58 (dd, *J* 0.7, 1.8, 1H, Ar-H); MS (accurate by FAB) 360.0080 (MH)⁺, C₁₃H₈F₃N₃O₂S₂ = 359.0010.
*5-Pyridin-2-yl-thiophene-*2*-sulfonic acid (3,5-*bis*-trifluoromethyl-phenyl)-amide* **124**. ¹H NMR (CDCl₃) δ: 7.21 (m, 1 H, pyridine-H), 7.40 (d, 1 H, *J* 4.0, thiophene-H), 7.54 (s, 3 H, Ph-H), 7.55 (d, 1 H, *J* 4.0, thiophene-H), 7.61 (m, 1 H, pyridine-H), 7.68 (m, 1 H, pyridine-H), 7.80 (s, 1 H, NH), 8.51 (m, 1 H, pyridine-H); MS (accurate by FAB) 453.0164 (MH)⁺, C₁₇H₁₀F₆N₂O₂S₂ = 452.0088.
*4,5-Dibromo-thiophene-2-sulfonic acid (4-chloro phenyl)-amide* **125.** ¹H NMR (CDCl₃) δ: 6.91 (s, 1 H, NH), 7.04 (d, 2 H, *J* 6.7, Ph-H), 7.23 (s, 1 H, thiophene-H), 7.24 (d, 2 H, *J* 6.7, Ph-H); MS (accurate by FAB) 431.7963 (MH)^{+ 81}Br⁷⁹Br³⁵Cl, C₁₀H₆Br₂ClNO₂S₂ = 428.7895.
*4,5-Dibromo-thiophene-2-sulfonic acid (4-trifluoromethyl-phenyl)-amide* **126.** ¹H NMR (CDCl₃) δ: 7.20 (d, 2 H, *J* 8.4, Ph-H), 7.33 (s, 1 H, thiophene-H), 7.35 (s, 1 H, NH), 7.53 (d, 2 H, *J* 8.4, Ph-H); MS (accurate by FAB) 465.82122 (MH)^{+ 79}Br⁸¹Br, C₁₁H₆Br₂F₃NO₂S₂ = 465.1040.

### Example 2

### General method for the N-alkylation of primary bisarylsulfonamides

A primary bisarylsulfonamide from Example 1 (1.0 mol eq) was dissolved in anhydrous acetone and the solution was cooled to 0 °C before triethylamine (5.0 mol eq) was added slowly. After 30 min, alkylating agent (alkyl or aralkyl halide; 5.0 mol eq) was added slowly and the solution was stirred until TLC [heptane : ethyl acetate (2:1)] indicated the reaction to be complete. The reaction mixture was then concentrated, diluted with water, and extracted with ethyl acetate. After concentration of the extract, column chromatography of the residue [column (Isolute SI; Jones Chromatography), heptane : ethyl acetate (12:1 --> 3:1 )] afforded the desired alkylated sulfonamide.

The *N*-alkylated bisarylsulfonamide compounds of this invention were prepared in this manner. Analytical details for representative compounds are as follows:
*5-Chloro-4-nitro-thiophene-2-sulfonic acid* (*4-fluoro-phenyl*)*-methyl-amide* **7.** TLC R_{F} = 0.72 (heptane:ethyl acetate, 2:1); ¹H NMR (CDCl₃) δ: 3.22 (3 H, s, NCH₃), 7.02 (2 H, m, Ph-H) 7.12 (2 H, m, Ph-H), 7.73 (1 H, d, *J* 1.0, thiophene-H).
*5-Chloro-4-nitro-thiophene-2-sulfonic acid methyl-*(*4-trifluoromethyl-phenyl*)*-amide* **50.** ¹H NMR (CDCl3) δ: 3.27 (3 H, s, NMe), 7.30 (2 H, d, *J* 8.5, Ph-H), 7.60 (2 H, d, *J* 8. S, Ph-H), 7.75 (1 H, s, thiophene).
*5-Chloro-4-nitro-thiophene-2-sulfonic acid* (*4-chloro-phenyl*)*-methyl-amide* **51**. TLC R_{F} = 0.69 (heptane:ethyl acetate, 2:1); HPLC t_{R} 18.49 min (20-80 %, 20 min); ¹H NMR (CDCl₃) δ: 3.22 (3 H, s, NCH₃), 7.08 (2 H, d, *J* 8.5, Ph-H), 7.29 (2 H, d, *J* 9.0, Ph-H), 7.38 (1 H, s, thiophene-H).
*5-Chloro-4-nitro-thiophene-2-sulfonic acid methyl-(4-trifluoromethyl-benzyl)-amide* **52.** TLC R_{F} = 0.70 (heptane:ethyl acetate, 2:1); HPLC t_{R} 19.72 min (20-80 %, 20 min); ¹H NMR (CDCl₃) δ: 2.71 (3 H, s, NCH₃), 4.25 (2 H, s, ArCH₂), 7.39 (2 H, d, *J* 8.0, Ph-H), 7.58 (2 H, d, *J* 8.0, Ph-H), 7.91 (1 H, s, thiophene-H).
*5-Chloro-4-nitro-thiophene-2-sulfonic acid benzyl-(4-fluoro-benzyl)-amide* **53.** ¹H NMR (CDCl₃) δ: 4.70 (2 H, s, CH₂), 6.92 (2 H, m, Ph-H), 6.97 (2 H, m, Ph-H), 7.14 (2 H, m, Ph-H), 7.19 (3 H, m, Ph-H), 7.75 (1 H, s, thiophene).
*5-Chloro-4-nitro-thiophene-2-sulfonic acid benzyl-*(*4-chloro-phenyl*)*-amide* **65.** HPLC t_{R} 20.98 min (20-80 %, 20 min); ¹H NMR (CDCl₃) δ: 4.78 (2 H, s, CH₂), 7.00 (2 H, m, Ph-H), 7.20 (2 H, m, Ph-H), 7.26 (5 H, m, Ph-H), 7.76 (1 H, s, thiophene-H).
*5-Chloro-4-nitro-thiophene-2-sulfonic acid benzyl-(4-methoxy-phenyl)-amide* **66.** HPLC t_{R} 22.76 min (0-60 %, 20 min); ¹H NMR (CDCl₃) δ: 3.70 (3 H, s, OMe), 4.69 (2 H, s, CH₂), 6.72 (2 H, d, *J* 8.5, Ph-H), 6.89 (2 H, d, *J* 8.5, Ph-H), 7.19 (5 H, m, Ph-H), 7.74 (1 H, s, thiophene-H).
*5-Chloro-4-nitro-thiophene-2-sulfonic acid (4-chloro-phenyl)-(3-trifluoromethylbenzyl)-amide* **67.** HPLC t_{R} 23.62 min (0-60 %, 20 min); ¹H NMR (CDCl₃) *δ:* 4.77 (2 H, s, CH₂), 6.95 (2 H, d, *J* 8.5, Ph-H), 7.19 (1 H, s, Ph-H), 7.24 (2 H, d, *J* 8.5, Ph-H), 7.37 (3 H, m, Ph-H), 7.46 (1 H, m, Ph-H), 7.79 (1 H, s, thiophene-H).
*5-Chloro-4-nitro-thiophene-2-sulfonic acid (3-trifluoromethyl-benzyl)-(4-trifluoromethylbenzyl)-amide* **75.** HPLC t_{R} 22.73 min (20-80 %, 20 min); ¹H NMR (CDCl₃) δ: 4.38 (4 H, s, CH₂), 7.19 (3 H, m, Ph-H), 7.30 (2 H, m, Ph-H), 7.45 (3 H, m, Ph-H), 7.80 (1 H, s, thiophene-H).
*5-(1-Methyl-5-trifluoromethyl-1*H*-pyrazol-3 yl)-thiophene-2-sulfonic acid (3,5*-bis-*trifluoromethyl-phenyl)-(3-trifluoromethyl-benzyl)-amide* **78.** HPLC t_{R} 24.01 min (10-70 %, 20 min); ¹H NMR (CDCl₃) δ: 4.03 (3 H, s, NCH₃), 4.92 (2 H, s, CH₂), 6.73 (1 H, s, pyrazol-H), 7.23 (1 H, dd, *J* 1.0, 4.0, Ph-H), 7.26 (1 H, d, *J* 1.0, Ph-H), 7.46 (3 H, m, Ph-H), 7.55 (2 H, m, Ph-H), 7.59 (1 H, d, *J* 7.5, Ph-H), 7.80 (1 H, s, Ph-H); MS 682.08 (M+H)⁺, C₂₅H₁₅F₁₂N₃O₂S₂ = 681.52.

### Example 3

### Fluorescence polarisation competitive binding assay

This was carried out using a 96-well microtiter plate (Costar) format. Recombinant HDM2 (1.5 µg per well) in TBS-BSA buffer (50 mM Tris pH 7.4, 150 mM NaCl, and 0.1 % BSA) was incubated for 5 min at room temperature in the presence of serially diluted test compound (in TBS-BSA buffer with a final concentration of 5 % DMSO). Fluorescently labelled p53-derived peptide (fluorescein-Met-Pro-Arg-Phe-Met-Asp-Tyr-Trp-Glu-Gly-Leu-Asn-NH₂, 0.2 µM) was added to each well and the plate was incubated at room tempeature for 45 min. The fluorescence polarisation (excitation 485 nm, emission 520 nm) of the peptide was measured. IC₅₀ values were calculated from dose-response curves.

### ELISA-type competitive binding assay

Streptavidin-coated 96-well plates (Pierce Chemical Co., St. Louis, MO, USA) were washed with TBS/BSA (25 mM Tris-HCl pH 7.5, 150 mM NaCl, 0.05 % Tween 20, 0.1 % BSA). Biotinylated p53-derived peptide (biotin-Ahx-Met-Pro-Arg-Phe-Met-Asp-Tyr-Trp-Glu-Gly-Leu-Asn-NH₂) was diluted to 1 µM with 25 mM Tris-HCl, pH 7.5, and aliquots (0.1 mL/well) were added to each well. After incubation for 1 h at room temperature with constant shaking, the plates were washed extensively with TBS/BSA buffer. HDM2 (50 uL/well of a 10 µg/mL stock solution in TBS/BSA buffer) and serially diluted (in TBS/BSA buffer) test compound (50 µL/well) were mixed and incubated for 30 min at room temperature. The reaction mixtures were then transferred to the peptide-coated streptavidin plates and incubation was continued for 1 h at room temperature, with constant shaking. The plates were then again washed extensively with TBS/BSA buffer. Anti-HDM2 antibody (SMP14 mAb, Santa Cruz Biotechnology, Inc., Santa Cruz, CA, USA; 0.1 mL/well of a 0.125 µg/mL TBS/BSA buffer dilution) was added and incubated for 1 h at room temperature. After renewed washing, secondary antibody (anti-mouse Ab, Sigma Cat. No. A 4789; 0.1 mL/well of a 1:10,000 TBS/BSA buffer dilution) was added and incubated for 1 h at room temperature. After extensive washing, TMB ELISA reagent (Pierce; 0.1 mL/well) was added, incubated for 1 min, and the colour reaction stopped by addition of 2 M aq H₂SO₄ (0.1 mL/well). The absorbance at 450 nm was then measured. IC₅₀ values were calculated from dose-response curves.

### Example 4

Primary screening data of representative bisarylsulfonamide compounds are summarised in Table 1. The fluorescence polarisation competitive HDM2 binding assay described in Example 3 was used. Furthermore, the anti-proliferative potency of the compounds against three human tumour cell lines with different genetic make-up was also determined. The anti-proliferative IC₅₀ values stated in Table 1 were determined using a standard 72-h MTT cytotoxicity assay [Haselsberger K, Peterson DC, Thomas DG, Darling JL; *Anti Cancer Drugs* 1996; **7**: 331-338]. The cell lines used were as follows: AGS, gastric adenocarcinoma, wild-type p53; H1299, large cell lung carcinoma, p53 null, low HDM2; SJSA-1, primitive multipotential bone sarcoma, wild-type p53, overexpressed HDM2.

Compounds active in the fluorescence polarisation competitive HDM2 binding assay were subsequently also tested in the corresponding ELISA-style assay format (Example 3). Similar IC₅₀ values were obtained from both assay formats. In general very good correlation between *in vitro* competitive HDM2-binding and cellular anti-proliferative activities was observed for bisarylsulfonamides. Thus only compounds capable of antagonising p53 peptide binding of HDM2 *in vitro* possessed anti-proliferative activity. Furthermore, the potency ranking of the compounds in the *in vitro* and cellular assays was very similar. These results suggest strongly that the anti-proliferative effects observed are due to the bisarylsulfonamides modulating cellular HDM2 function. This conclusion is supported by the fact that in general the cell line in which HDM2 is overexpressed (SJSA-1) was less sensitive to active bisarylsulfonamide test compounds than the other two cell lines assayed. The fact that anti-proliferative effects can be achieved in tumour cells devoid of functional p53 through modulation of HDM2 is indicated by the fact that the p53^{-/-} cell line H1299 was similarly responsive to the test compounds as the other two cell lines with normal p53.

### Example 5

Representative bisarylsulfonamide compounds were tested on a panel of transformed and non-transformed human cell lines, using a standard 72-h MTT cytotoxicity assay [Haselsberger K *et al, ibid*]. The results are summarised in Table 2.

It can be seen that compounds **2, 3, 6,** & **7** were 3- to 4-fold more potent in their anti-proliferative activity against transformed compared to non-transformed cell lines. Furthermore, potency for these compounds on average was higher against carcinoma compared to sarcoma cell lines, in contrast to the control compound roscovitine, which exerts its anti-proliferative effects through a different mechanism, *viz.* inhibition of cell-cycle cyclin-dependent kinases [Wang S, McClue SJ, Ferguson JR, Hull JD, Stokes S, Parsons S, Westwood R, Fischer PM; Tetrahedron: Asymmetry 2001; 12: 2891-2894]. This differential selectivity further supports the notion that the bisarylsulfonamides modulate cellular HDM2 function. It is known that HDM2 amplification is particularly common in soft tissue sarcomas and osteosarcomas [Momand J *et al, ibid*; Bartel F, Meye A, Wurl P, Kappler M, Bache M, Lautenschlager C, Grunbaum U, Schmidt H, Taubert H; Int. J. Cancer 2001; 95: 168-175].

### Example 6

### p53 reporter gene assay

A dual assay assessing both cell viability and p53 response was employed. A U20S-derived cell line stably transfected with a p53 response element and a luciferase reporter gene was created. The genetic expression cassette construct GC3p53tkaLuc used for this purpose has been described [Zhu J, Gao B, Zhao J, Balmain A; Cancer Gene Ther. 2000; 7: 4-12]. After seeding of cells into 96-well tissue culture plates, treatment with test compounds, and incubation for the desired duration, the cell proliferation reagent WST-1 (Roche Molecular Biochemicals) was used to determine cell viability. The Bright-Glo^{™} Luciferase Assay System (Promega) was used to measure luciferase activity. The data from the cell viability assay was used to normalise data from the luciferase assay. Assay protocols were as previously described [Krausz E, Watt K, Cummings L, Baxter C, Blake DG; Biochemica 2001: 26-27].

### E2F reporter gene assay

A similar system to the p53 reporter assay described above was used, with the exception that it was designed to be responsive to E2F transcription factors rather than p53. An A549-derived cell line stably transfected with an E2F response element and a luciferase reporter gene was created. The genetic expression cassette construct used was from Clontech (Mercury^{™} Cell Cycle Profiling System). Assays were carried out as described above.

### Example 7

Representative bisarylsulfonamides were tested in the p53 gene reporter assay described in Example 6. The results are summarised in Table 3.

It has previously been reported that a p53-derived optimised cell-permeable peptide (the positive control peptide in the table above) was capable of activating the p53 pathway in tumour cells through inhibiting the HDM2/p53 interaction [Chène P, Fuchs J, Bohn J, García-Echeverría C, Furet P, Fabbro D; J. Molec. Biol. 2000; 299: 245-253]. Our results confirm this finding (see above). Furthermore, representative bisarylsulfonamides also caused dramatic induction of p53-responsive luciferase activity in the same assay system. In general it was found that compounds which were inactive in the *in vitro* competitive HDM2-binding assay did not exhibit this ability to induce cellular p53. For example, the effect of the marginally active compound 4 was much less pronounced than that of the more active compounds. The fact that the bisarylsulfonamides were able to activate cellular p53 transactivation activity confirms that they modulate cellular HDM2 activity.

### Example 8

Representative bisarylsulfonamides were tested in the E2F gene reporter assay described in Example 6. The results are summarised in Table 4.

Only bisarylsulfonamides active as HDM2 antagonists were capable of down-regulation of E2F transcriptional activity in the gene reporter assay. This effect was observed regardless of whether an asynchronous cell population or cells synchronised at the late Gl/S phase [Ji C, Mamett LJ, Pietenpol JA; Oncogene 1997; 15: 2749-2753] were used. The fact that bisarylsulfonamides were able to suppress E2F transcriptional activity confirms that they modulate the cellular HDM2/E2F interaction.

### Example 9

It is well established that radiotherapy and most current forms of chemotherapy cause extensive genetic damage and induction of the p53-dependent apoptotic pathway, which determines the important role of this tumor suppressor protein in cancer therapy. HDM2, on the other hand, as a transcriptional product and negative regulator pf p53, would destabilise p53 and diminish its pro-apoptotic function. Inhibitors of the p53-HDM2 negative feedback loop (*e.g*. compounds disrupting the p53-HDM2 complex) are expected to synergise with cytotoxic agents for induction of p53 and subsequent apoptosis. To investigate this hypothesis we have used as a model system three cell lines: AGS (gastric adenocarcinoma, wild type p53), H1299 (colon adenocarcinoma, p53 null) and SJSA-1 (osteosarcoma, wild type p53, over-expressed HDM2) and two cytotoxic agents, cisplatin (DNA alkylating agent) and etoposide (topoisomerase inhibitor), well known to induce p53 and apoptosis. As a representative bisarylsulfonamide inhibitor of the HDM2-p53 interaction, compound **2** was used at the anti-proliferative IC₅₀ concentration determined for each cell line, *i.e*. 1, 5, and 15 µM, respectively. The combination treatment regimen included addition of 2 to the cells at the same time, 6 hours before, or 6 hours after cisplatin or etoposide. Both chemotherapeutic agents were used in the concentration range of 100 - 0.19 µM. The cytotoxic effect of etoposide and cisplatin in the presence and absence of the HDM2-p53 inhibitor was determined using a standard MTT assay after a total of 72 hours incubation time. The cytotoxicity results for cisplatin are shown in Table 5.

Compound 2 increased the cytotoxic effect of cisplatin on all three cell lines used. The synergistic effect was highest when 2 was added prior to the treatment with cisplatin and the potency increase in AGS, H1299 and SJSA-1 cells was 18.5-, 16- and 357-fold, respectively.

Similar synergistic effects of 2 was observed when the compound was used in combination with etoposide. The results are shown in Table 6.

The cytotoxicity of etoposide was significantly increased in the presence of 2, as the highest effect was registered when the inhibitor of the HDM2-p53 interaction was added 6 hours before etoposide. The IC₅₀ values calculated for etoposide in AGS, H1299 and SJSA-1 cells pre-treated with 2 were 3.5-, 25- and 726-fold lower than the ones not receiving 2.

The synergistic effect of bisarylsulfonamides and the cytotoxic agents was highest in SJSA-1 cells. This result was expected as SJSA-1 cells over-express HDM2, suggesting that DNA damage-induced p53 will be destabilised and the effect of the cytotoxic agents diminished. The relative resistance of SJSA-1 towards treatment with etoposide and cisplatin may be due to the anti-apoptotic effect of HDM2. Disrupting the interaction between HDM2 and p53 allows quick accumulation and stabilisation of p53 and induction of cell death. The chemosensitisation effects of bisarylsulfonamides involves a p53-independent mechanism as well sinse it was observed in H1299 cells, which are p53 null.

### Example 10

### Mode of action studies

Many types of cellular stress converge on the p53 pathway and induce its activity. It was therefore important to demonstrate that the compounds of this invention activate p53 directly by targeting the interaction between p53 and HDM2 and not indirectly by targeting the activation of upstream kinases, for example. The biological effect of bisarylsufonamides on cellular level was studied using representative compounds (mainly compound 2) and number of cell lines with different HDM2 and p53 status. These were overexpressing Hdm2 (SJSA1 - osteosarcoma), wild type p53 and normal levels of HDM2 (AGS - gastric adenocarcinoma, MCF7 - breast adenocarcinoma), or p53 null, low levels of HDM2 (H1299 - large cell lung carcinoma). The effects of bisarylsulfonamides were analysed at the cellular levels of key proteins in the HDM2 pathway and were compared with those induced by well-characterised anti-cancer drugs such as etoposide and cisplantinum.

AGS, H1299, MCF7 or SJSA1 cells were treated with five times the anti-proliferative IC₅₀ concentration of compound 2 or in some cases etoposide or cisplatinum (Figure 1). Cells were collected at intervals and morphological changes were noted at the time of collection. Cells were lysed and equal amounts were analysed by SDS-polyacrylamide gel electrophoresis (SDS-PAGE) and immunoblotting. Treatment of AGS or MCF7 cells with etoposide or cisplatinum resulted in classical stress response (Figure 1B, 1C, and ID), inducing p53 phosphorylation of serine 15, as well as increasing the p53 and E2F-1 protein levels. At later time points this correlated with PARP cleavage; production of an 85-kDa cleavage product is a marker for apoptosis that coincided with morphological changes characteristic of cell death. In contrast, treatment of AGS, H2199, MCF7 or SJSAI cells with compound **2** induced a rapid reduction in HDM2 and E2F-1 levels (but not E2F-2) with no induction of p53 nor p53 phosphorylation at serine 15 (Figure 1A, 1B, 1D and 1E). The reduction in E2F-1 levels correlated with reduction in E2F transcriptional activity as determined by an E2F-dependent luciferase reporter assay (refer Example 6). Treatment of MCF7 cells with 4-bromo-5-chloro-thiophene-2-sulfonic acid 4-trifluoromethyl-benzylamide (negative control compound), a compound, which does not inhibit the HDM2 - p53 interaction in *vitro,* but has similar structure to compound 2, did not induce any notable changes in the protein levels tested or cell morphology (Figure 1D).

Compound **2** induced rapid morphological changes that were most dramatic in SJSA1, cells that have a very prominent cytoplasmic component. Rapid cell rounding was detected in all cell types tested.

Analysis of other stress pathways in MCF7 cells after compound **2** treatment showed a transient induction in JNK phosphorylation but no clear induction of the p38 MAPK pathway. Anisomycin (mRNA translation inhibitor) was used as a positive control for the induction of these stress pathways (Figure 1 D). The induction of JNK phosphorylation was not confirmed in the other cell types.

Induction of PARP cleavage was very rapid in H1299 cells but less so in AGS, MCF7 or SJSA1 cells (Figure 1A, 1B, 1C, 1D and 1E). In the MCF7 cells despite the absence of the 85-kDa PARP cleavage product there was a dramatic reduction in PARP protein levels (Figure 1D). The effect of compound **49**, another *in vitro* inhibitor, was also tested on AGS, SJSA1 and H1299 (Figure 2). This induced even more rapid cell rounding as well as similar changes in protein levels seen with compound 2.

It was important to determine whether the rapid cell rounding corresponded to cell death. MCF7 or H1299 cells were treated with compound **2** and the number of rounded cells as well as the number of viable cells were counted at intervals, using trypan blue exclusion. Rapid cell rounding occurred with very different kinetics to cell death in both cell types (Figure 3). This data suggests that cell rounding does not correlate with dead cells.

A pair of compounds, **68** (competitive inhibition IC₅₀ =431 µM) and **69** (IC₅₀ =151 µM; refer Table 1) were analysed for their effects on MCF7 and H1299 cells. Cells were treated at five times the IC₅₀ concentration of the more active compound **69**. Little change in protein levels were detected when cells were treated with **68**. In contrast, **69** induced changes in both morphology and protein levels. Rapid reduction in PARP levels was detected in both cell lines with the production of the 85-kDa cleavage product detectable at later time points (Figure 4A and 4B). HDM2 and HDMX levels decreased in MCF7 cells shortly after treatment with compound **69** (Figure 4A). In H1299 cells compound **69** treatment induced rapid decrease of HDM2 levels, which recovered at a later time point (Figure 4B). E2F-1 levels barely changed compared to controls (Figure 4B), No rapid cell rounding was detected in either cell type. By 68 hours floating MCF7 cells (with morphology corresponding to the one of dead cells) were detected with compound **69** treatment but not with compound **68** treatment. This was distinct from the cell rounding seen with compound 2.

In order to determine whether bisarylsulfonamides induced cell cycle changes, MCF7 or H1299 cells were treated with cisplatin or compound **2** (Figure 5). Both cisplatin and compound 2 induced sub-G1 cells, indicative of apoptosis, with no detectable arrest at any phase of the cell cycle.

To characterise the apoptosis -inducing properties of bisarylsulfonamides caspase 1/3 activty was measured. AGS or H1299 cells were treated with a low concentration of compound **2** or cisplatin corresponding to twice the IC₅₀ value (Figure 6A). Morphological changes were detected in 10-20 % of cells by 72 hours corresponding to an induction of caspase activity first detected at 48 hours, increasing by 72 hours. We also treated AGS and H1299 cells with higher concentrations corresponding to five times the IC₅₀ (Figure 6B). In this experiment we included a specific caspase 3 inhibitor, Z-VAD.fmk to determine the caspase responsible. Caspase activity was detected by four hours in H1299 correlating with the PARP cleavage product detected by Western blotting (Figure 1C), In AGS cells, only weak caspase activity was induced by 24 hours compared with cisplatin treatment, This activity was caspase 3-dependent because the caspase inhibitor reduced the caspase activity to background levels at 24 hours (Figure 6B). Notably, rapid cell rounding was detected in AGS cells despite the presence of the caspase inhibitor, further confirming our results that cell rounding was not an indication of cell death.

Our results consistently show that bisarylsulfonamides, inhibitors of HDM2, can induce a rapid decrease in the levels of E2F-1. To determine if this was an off-target effect or one that could occur by inhibition of HDM2/HDMX activities, two different siRNAs against HDM2 and one agains HDMX were used to transfect MCF7 cells.

First, the cells were transfected with HDM2 or control siRNAs serially every 24 hours and collected 24 hours later. Cells were lysed and equal amounts of protein were separated by SDS-PAGE and analysed by Western blotting. Treatment with control siRNAs had no effect on the levels of HDM2 (Figure 7A lanes 1-9). Treatment with HDM2 siRNA markedly reduced the levels of HDM2 protein at all time points (Figure 7A, lanes 10-15). This corresponded to a decrease in E2F-1 levels but not E2F-4 levels and an induction of p53 protein levels. Actin was used as a loading control. HDMX has been shown to stabilise HDM2 levels. Next, we determined whether HDM2 levels were affected by HDMX levels in MCF7 cells and whether reducing HDMX levels could reduce E2F-1 levels. MCF7 cells were transfected with HDM2 siRNA and HDMX siRNA alone or together. HDM2 levels were reduced at all time points in the presence of HDM2 siRNA (Figure 7B lanes 10-12). Treatment of cells with HDMX siRNA reduced cellular levels of HDMX as well as reducing the levels of HDM2 (Figure 7B lanes 13-15). In both cases E2F-1 levels were also decreased. Combined treatment with HDM2 and HDMX siRNA had an additive effect greatly reducing the levels of E2F-1 at 48 hours. (Figure 7B lane 17). These experiments showe that both HDM2 and HDMX can regulate E2F-1. It is likely that HDMX regulates E2F-1 indirectly by stabilising HDM2.

Various modifications and variations of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the relevant fields are intended to be covered by the present invention.

**Table 1**

| **No.** | **Structure** | **Name** | **IC₅₀(µM)** | | | |
|---|---|---|---|---|---|---|
| | | | ***In vitro* competitive HDM2 binding assay** | **Anti-proliferative effect on cell line** | | |
| | | | | **AGS** | **H 1299** | **SJSA-1** |
| 1 | 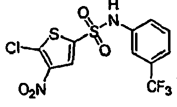 | 5-Chloro-4-nitro-thiophene-2-sulfonic acid (3-trifluoromethyl-phenyl)-amide | 409 ± 84 | n.d. | n.d. | n.d. |
| 2 | 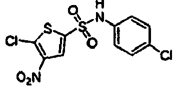 | 5-Chloro-4-nitro-thiophene-2-sulfonic acid (4-chloro-phenyl)-amide | 26.4 ± 3.4 | 1.1 ± 0.2 | 5.1 ± 0.7 | 13.6 ± 1.1 |
| 3 | 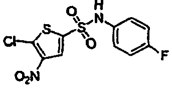 | 5-Chloro-4-nitro-thiophene-2-sulfonic acid (4-fluoro-phenyl)-amide | 41.9 ± 5.8 | 1.5 ± 0.3 | 5.7 ± 0.4 | 17.1 ± 0.4 |
| 4 | 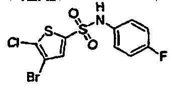 | 4-Bromo-5-chloro-thiophene-2-sulfonic acid (4-fluoro-phenyl)-amide | 378 | n.d. | n.d. | n.d. |
| 5 | 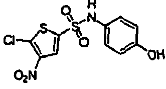 | 5-Chloro-4-nitro-thiophene-2-sulfonic acid (4-hydroxy-phenyl)-amide | 105 ± 26 | 2.2 ± 0.6 | 8.7 ± 22 | 24.0 ± 2.1 |
| 6 | 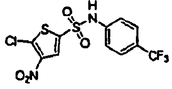 | 5-Chloro-4-nitro-thiophene-2-sulfonic acid (4-trifluoro-methyl-phenyl)-amide | 204 ± 1.4 | 2.0 ± 0.3 | 8.1 ± 1.3 | 22.5 ± 0.6 |
| 7 | 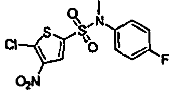 | 5-Chloro-4-nitro-thiophene-2-sulfonic acid (4-fluoro-phenyl)-methyl-amide | 15.4 ± 7.8 | 7.0 ± 13 | 12.9 ± 0.7 | 9.3 ± 2.7 |
| 8 | 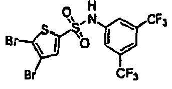 | 4,5-Dibromo-thiophene-2-sulfonic acid (3,5-bis- trifluoromethyl-phenyl)-amide | 407 | n.d. | n.d. | n.d. |
| 9 | 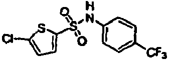 | 5-Chloro-thiopbene-2-sulfonic acid (4- trifluoromethyl-phenyl)-amide | 102 ± 13 | n.d. | n.d. | n.d. |
| 10 | 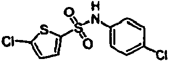 | 5-Chloro-thiophene-2-sulfonic acid (4-chloro-phenyl)-amide | 320 | n.d. | n.d. | n.d. |
| 11 | 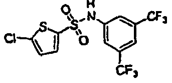 | 5-Chloro-thiophene-2-sulfonic acid (3,5-bis-trifluoromethyl-phenyl)-amide | 435 ± 76 | n.d. | n.d. | n.d. |
| 12 | 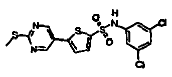 | 5-(2-Methylsulfanyl-pyrimidin-5-yl)-thiophene-2-sulfonic acid (3,5-dichloro-phenyl)-amide | 374 ± 12 | n.d. | n.d. | n.d. |
| 13 | 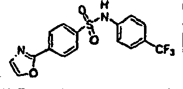 | 4-Oxazol-2-yl-*N*-(4-trifluoromethyl--phenyl)-benzene-sulfonamide | 494 | n.d. | n.d. | n.d. |
| 14 | 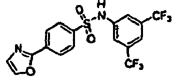 | *N*-(3,5-Bis-trifluoromethyl-phenyl)-4-oxazol-2-yl-benzenesulfonamide | 448 ± 55 | n.d. | n.d. | n.d. |
| 15 | 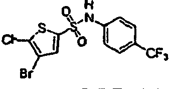 | 4-Bromo-5-chloro-thiophene-2-sulfonic acid (4-trifluoromethyl-phenyl)-amide | 218 ± 15 | n.d. | n.d. | n.d. |
| 16 | 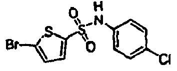 | 5-Bromo-thiophene-2-sulfonic acid (4-chloro-phenyl)-amide | 290 ± 54 | n.d. | n.d. | n.d. |
| 17 | 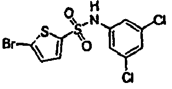 | 5-Bromo-thiophene-2-sulfonic acid (3,5-dichloro-phenyl)-amide | 291 | n.d. | n.d. | n.d. |
| 18 | 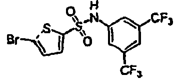 | 5-Bromo-thiophene-2-sulfonic acid (3,5-bis-trifluoromethyl-phenyl)-amide | 172 ± 21 | n.d. | n.d. | n.d. |
| 19 | 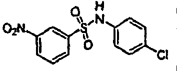 | *N*-(4-Chloro-phenyl)-3-nitro-benzene-sulfonamide | 295 ± 45 | n.d. | n.d. | n.d. |
| 20 | 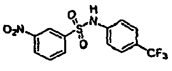 | 3-Nitro-*N*-(4-trifluoromethyl-phenyl)-benzene-sulfonamide | 482 ± 26 | n.d. | n.d. | n.d. |
| 21 | 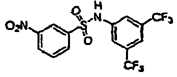 | *N*-(3,5-Bis-trifluoromethyl-phenyl)-3-nitro-benzenesulfonamide | 209 ± 38 | n.d. | n.d. | n.d |
| 22 | 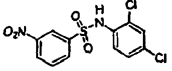 | *N*-(2,4-Dichloro-phenyl)-3-nitro-benzenesulfonamide | 441 ± 69 | n.d. | n.d. | n.d. |
| 23 | 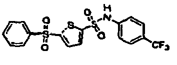 | 5-Benzenesulfonyl-thiophene-2-sulfonic acid (4-trifluoromethyl-phenyl)-amide | 146 ± 5 | n.d. | n.d. | n.d. |
| 24 | 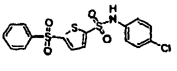 | 5-Benzenesulfonyl-thiophene-2-sulfonic acid (4-chloro-phenyl)-amide | 243 ± 59 | n.d. | n.d. | n.d. |
| 25 | 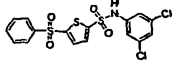 | 5-Benzenesulfonyl-thiophene-2-sulfonic acid (3,5-dichloro-phenyl)-amide | 288 ± 39 | n.d. | n.d. | n.d. |
| 26 | 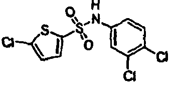 | 5-Chloro-thiophene-2-sulfonic acid (3,4-dichloro-phenyl)-amide | 295 ± 36 | n.d. | n.d. | n.d. |
| 27 | 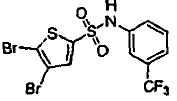 | 4,5-Dibromo-thiophene-2-sulfonic acid (3-trifluoromethyl-phenyl)-amide | 199 ± 28 | n.d. | n.d. | n.d. |
| 28 | 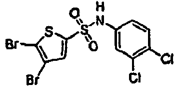 | 4,5-Dibromo-thiophene-2-sulfonic acid (3,4-dichloro-phenyl)-amide | 160 ± 31 | n.d. | n.d. | n.d. |
| 29 | 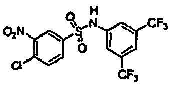 | *N*-(3,5-Bis-trifluoromethyl-phenyl)-4-chloro-3-nitro-benzene-sulfonamide | 129 ± 22 | n.d. | n.d. | n.d. |
| 30 | 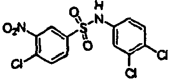 | 4-Chloro-*N*-(3,4-dichloro-phenyl)-3-nitro-benzene-sulfonamide | 427 ± 3 | n.d. | n.d. | n.d. |
| 31 | 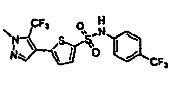 | 5-(1-Methyl-5-trifluoromethyl-1*H*-pyrazol-4-yl)-thiophene-2-sulfonic acid (4-trifluoromethyl-phenyl)-amide | 350 ± 10 | n.d. | n.d. | n.d. |
| 32 | 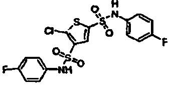 | 5-Chloro-thiophene-2,4-disulfonic acid bis-[(4-fluoro-phenyl)-amide] | 408 ± 31 | n.d. | n.d. | n.d. |
| 33 | 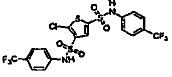 | 5-Chloro-thiophene-2,4-disulfonic acid bis-[(4-trifluoro-methyl-phenyl)-amide] | 65 ± 5 | 68 ± 1 | 69 ± 1 | >100 |
| 34 | 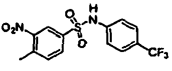 | 4-Methyl-3-nitro-*N*-(4-trifluoromethyl-phenyl)-benzene-sulfonamide | 295 ± 23 | n.d. | n.d. | n.d. |
| 35 | 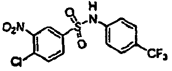 | 4-Chloro-3-nitro-N-(4-trifluoromethyl-phenyl)-benzene-sulfonamide | 192 ± 8 | 13.0 ± 3.3 | 9.3 ± 1.7 | 26.5 ± 1.6 |
| 36 | 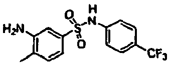 | 3-Amino-4-methyl-*N*-(4-trifluoromethyl-phenyl)-benzene-sulfonamide | 429 | n.d. | n.d. | n.d. |
| 37 | 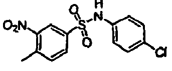 | *N*-(4-Chloro-phenyl)-4-methyl-3-nitro-benzene-sulfonamide | 294 ± 52 | n.d. | n.d. | n.d. |
| 38 | 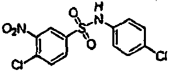 | 4-Chloro-*N*-(4-chloro-phenyl)-3-nitro-benzene-sulfonamide | 232 ± 13 | 16 ± 4 | 11 ± 4 | 22 ± 4 |
| 39 | 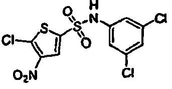 | 5-Chloro-4-nitro-thiophene-2-sulfonic acid (3,5-dichloro-phenyl)-amide | 46.1± 3.4 | 4.9 ± 1.6 | 12.1 ± 4.0 | 27.2 ± 0.4 |
| 40 | 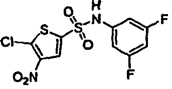 | 5-Chloro-4-nitro-acid (3,5-difluoro-phenyl)-amide | thiophene-2-sulfonic 59.6 ± 10.5 | 4.0 ± 0.3 | 10.6 ± 0.4 | 26.4 ± 0.2 |
| 41 | 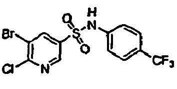 | 5-Bromo-6-chloro-pyridine-3-sulfonic acid (4-trifluoromethyl-phenyl)-amide | 258 ± 12 | 31 ± 2 | 28 ± 0 | 53 ± 3 |
| 42 | 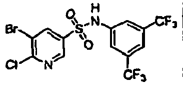 | 5-Bromo-6-chloro-pyridine-3-sulfonic acid (3,5-bis-trifluoromethyl-phenyl)-amide | 278 ± 50 | 30 ± 7 | 32 ± 7 | 65 ± 18 |
| 43 | 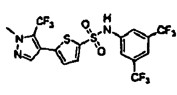 | 5-(1-Methyl-5-trifluoro-methyl-1*H*-pyrazol-4-yl)-thiophene-2-sulfonic acid (3,5-bis-trifluoromethyl-phenyl)-amide | 133 ± 11 | 20.9 ± 1.0 | 18.0 ± 0.8 | 33.7 ± 0.3 |
| 44 | 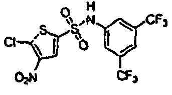 | 5-Chloro-4-nitro-thiophene-2-sulfonic acid (3,5-bis-trifluoromethyl-phenyl)-amide | 25.4 ± 0.2 | 10 ± 0 | 14 ± 2 | 19 ± 3 |
| 45 | 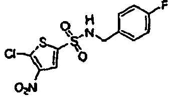 | 5-Chloro-4-nitro-thiophene-2-sulfonic acid 4-fluoro-benzylamide | 16.0 ± 3.7 | 7.5 ± 4.4 | 11.1 ± 3.1 | 11.5 ± 30 |
| 46 | 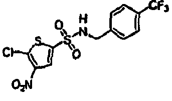 | 5-Chlaro-4-nitro-thiophene-2-sulfonic acid 4-trifluoromethyl-benzylamide | 11.1 ± 1.7 | 1.3 ± 0.5 | 3.4 ± 0.5 | 4.4 ± 1.1 |
| 47 | 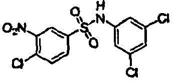 | 4-Chloro-*N*-(3,5-dichloro-phenyl)-3-nitro-benzene-sulfonamide | 234 ± 37 | 23 ± 2 | 18 ± 8 | 37 ± 6 |
| 48 | 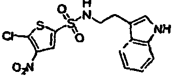 | 5-Chloro-4-nitro-thiophene-2-sulfonic acid [2-1*H*-indol-3-yl)-ethyl]-amide | 25.3 ± 4.6 | 2.6 ± 1.4 | 3.6 ± 0.9 | 3.1 ± 1.0 |
| 49 | 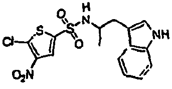 | 5-Chloro-4-nitro-thiophene-2-sulfonic acid[2-(1*H*-indol-3-yl)-1-methyl-ethyl]-amide | 46.3 ± 4.0 | 2.2 ± 2.3 | 1.5 ± 0.4 | 2.0 ± 0.2 |
| 50 | 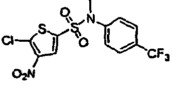 | 5-Chloro-4-nitro-thiophene-2-sulfonic acid methyl-(4-trifluoromethyl-phenyl)-amide | 7.3 ± 0.6 | 30.3 ± 0.3 | 34.7 ± 6.1 | 35.4 ± 11.5 |
| 51 | 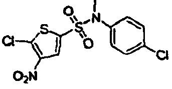 | 5-Chloro-4-nitro-thiophene-2-sulfonic acid (4-chloro-phenyl)-methyl-amide | 5.9 ± 0.7 | 11.3 ± 1.0 | 17.4 ± 3.8 | 15.6 |
| 52 | 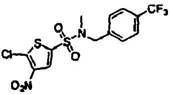 | 5-Chloro-4-nitro-thiophene-2-sulfonic acid methyl-(4-trifluoromethyl-benzyl)-amide | 10.7 ± 0.2 | 23.3 ± 1.4 | 28.5 ± 1.9 | 27.5 ± 1.3 |
| 53 | 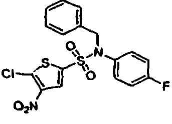 | 5-Chloro-4-nitro-thiophene-2-sulfonic acid benzyl-(4-fluoro-benzyl)-amide | 15 | 3.6 ± 0.3 | 5.6 ± 1.0 | 5.1 ± 0.4 |
| 54 | 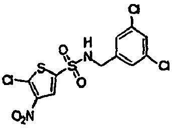 | 5-Chloro-4-nitro-thiophene-2-sulfonic acid 3,5-dichloro-benzylamide | 20.2 ± 1.3 | 5.6 ± 0.2 | 9.7 ± 2.9 | 12.0 ± 0.4 |
| 55 | 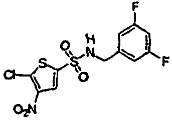 | 5-Chloro-4-nitro-thiophene-2-sulfonic acid 3,5-difluoro-benzylamide | 27.0 ± 10.1 | 7.0 ± 0.0 | 9.3 ± 2.5 | 13.3 ± 2.1 |
| 56 | 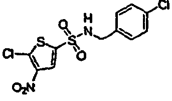 | 5-Chloro-4-nitro-thiophene-2-sulfonic acid 4-chloro-benzylamide | 24.8 ± 5.4 | 4.3 ± 0.6 | 6.3 ± 1.9 | 9.3 ± 2.7 |
| 57 | 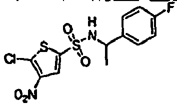 | 5-Chloro-4-nitro-thiophene-2-sulfonic acid [1-(4-fluoro-phenyl)-ethyl]-amide | 31.1 ± 5.6 | 1.7 ± 0.3 | 2.7 ± 0.6 | 5.9 ± 0.2 |
| 58 | 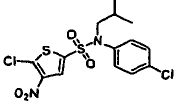 | 5-Chloro-4-nitro-thiophene-2-sulfonic acid (4-chloro-phenyl)- isobutyl-amide | 249 ± 30 | n.d. | n.d. | n.d. |
| 59 | 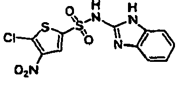 | 5-Chloro-4-nitro-thiophene-2-sulfonic acid (1*H*-benzo- 40 imidazol-2-yl)-amide | 40 ± 1 | n.d. | n.d. | n.d. |
| 60 | 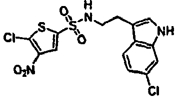 | 5-Chloro-4-nitro-thiophene-2-sulfonic acid [2-(6-chloro-1*H*-indol-3-yl)-ethyl]-amide | 29 ± 6 | n.d. | n.d. | n.d. |
| 61 | 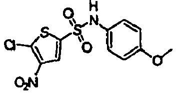 | 5-Chloro-4-nitro-thiophene-2-sulfonic acid (4-methoxy-phenyl)-amide | 45 ± 14 | 1.8 ± 0.4 | 2.7 ± 0.4 | 2.8 ± 0.2 |
| 62 | 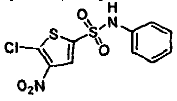 | 5-Chloro-4-nitro-thiophene-2-sulfonic acid phenylamide | 67 ± 1 | n.d. | n.d. | n.d. |
| 63 | 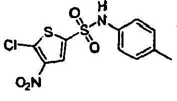 | 5-Cloro-4-nitro-thiophene-2-sulfonic acid *p*-tolylamide | 35 ± 8 | 1.7 ± 0.6 | 3.9 ± 0.9 | 5.7 ± 1.6 |
| 64 | 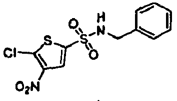 | 5-Chloro-4-nitro-thiophene-2-sulfonic acid benzylamide | 18 ± 0 | 3.6 ± 0.2 | 6.3 ± 2.1 | 7.3 ± 1.7 |
| 65 | 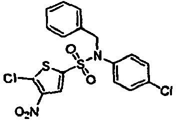 | 5-Chloro-4-nitro-thiophene-2-sulfonic acid benzyl-(4-chloro-phenyl)-amide | 7.4 ± 1.0 | 5.2 ± 2.2 | 10.8 ± 4.3 | 11.1 ± 6.7 |
| 66 | 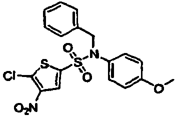 | 5-Chloro-4-nitro-thiophene-2-sulfonic acid benzyl-(4-methoxy-phenyl)-amide | 14.7 ± 0.9 | 2.4 ± 0.2 | 3.9 ± 0.5 | 3.9 ± 1.3 |
| 67 | 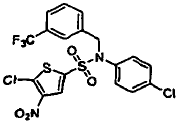 | 5-Chloro-4-nitro thiophene-2-sulfonic acid (4-chloro-phenyl)-(3-trifluoromethyl-benzyl)-amide | 8.5 ± 3.2 | 6.7 ± 0.9 | 12.0 ± 1.0 | 11.8 ± 1.6 |
| 68 | 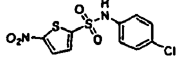 | 5-Nitro-tiophene-2-sulfonic acid (4-chloro-phenyl)-amide | 431 ± 37 | 76 | n.d. | n.d. |
| 69 | 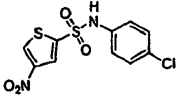 | 4-Nitro-thiophene-2-sulfonic acid (4-chloro-phenyl)-amide | 151 ± 24 | 19 | 30 | 28 |
| 70 | 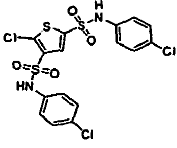 | 5-Chloro-thiophene-2,4-disulfonic acid bis-[(4-chloro-phenyl)-amide] | 135 ± 19 | n.d. | n.d. | n.d. |
| 71 | 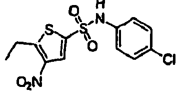 | 5-Ethyl-4-nitro-thiophene-2-sulfonic acid (4-chloro-phenyl)-amide | 237 ± 35 | n.d. | n.d. | n.d. |
| 72 | 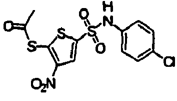 | Thioacetic acid S-[5-(4-chloro-phenylsulfamoyl)-3-nitro-thiophen-2-yl] ester | 16.2 ± 0.0 | 61 | n.d. | n.d. |
| 73 | 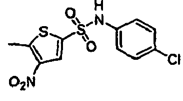 | 5-Methyl-4-nitro-thiophene-2-sulfonic acid (4-chloro-phenyl)-amide | > 167 | 53 | 66 | n.d. |
| 74 | 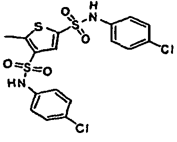 | 5-Methyl-thiophene-2,4-disulfonic acid bis-[(4-chloro-phenyl)-amide] | 123 ± 15 | n.d. | n.d. | n.d. |
| 75 | 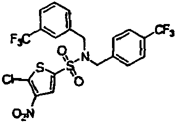 | 5-Chloro-4-nitro-thiophene-2-sulfonic acid (3-trifluoromethyl-benzyl)-(4-trifluoromethyl-benzyl)-amide | 3.5 ± 1.0 | 9 | 16 | 18 |
| 76 | 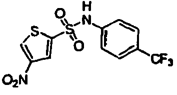 | 4-Nitro-thiophene-2-sulfonic acid (4-trifluoromethyl-phenyl)-amide | 366 ± 7 | n.d. | n.d. | n.d. |
| 77 | 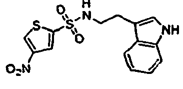 | 4-Nitro-thiophene-2-sulfonic acid [2-(1*H*-indol-3-yl)-ethyl]-amide | 247 ± 61 | n.d. | n.d. | n.d. |
| 78 | 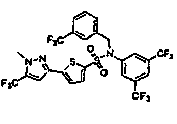 | 5-(1-Methyl-5-trifluoromethyl-1*H*-pyrazol-3-yl)-thiophene-2-sulfonic acid (3,5-bis-trifluoromethyl-phenyl)-(3-trifluoromethyl-benzyl)amide | 315 ± 83 | n.d. | n.d. | n.d. |
| 79 | 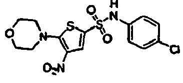 | 5-Morpholin-4-yl-4-nitro-thiophene-2-sulfonic acid (4-chloro-phenyl)-amide | 419 ± 31 | n.d. | n.d. | n.d. |
| 80 | 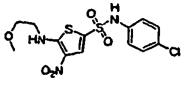 | 5-(2-Methoxy-ethylamino)-4-nitro-thiophene-2-sulfonic acid (4-chloro-phenyl)-amide | 154 ± 27 | n.d. | n.d. | n.d. |
| 81 | 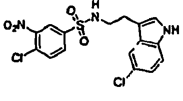 | 4-Chloro-*N*-[2-(5-chloro-1*H*-indol-3-yl) ethyl]-3-nitro-benzenesulfonamide | 311 ± 21 | 29 ± 2 | 39 ± 6 | 51 ± 1 |
| 82 | 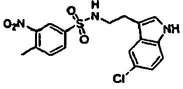 | *N*-[2-(5-Chloro-1*H*-indol-3-yl)-ethyl]-4-methyl-3-nitro-benzenesulfonamide | 338 ± 111 | 50 ± 0 | 49 ± 0 | 65 ± 4 |
| 83 | 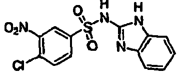 | *N*-(1*H*-Benzoimidazol-2-yl)-4-chloro-3-nitro-benzenesulfonamide | 119 ± 43 | 0.9 ± 0.1 | 1.4 ± 0.2 | 2.3 ± 0.9 |
| 84 | 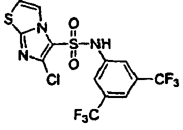 | 6-Chloro-imidazo[2,1-*b*]thiazole-5-sulfonic acid (3,5-bis-trifluoromethyl-phenyl)-amide | 308 ±17 | n.d. | n.d. | n.d. |
| 85 | 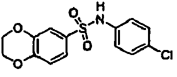 | 2,3-Dihydro-benzo[1,4]dioxine-6-sulfonic acid (4-chloro-phenyl)-amide | 432 ± 7 | n.d. | n.d. | n.d. |
| 86 | 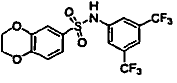 | 2,3-Dihydro-benzo[1,4]dioxine-6-sulfonic acid (3,5-bis-trifluoromethyl-phenyl)-amide | 375 ± 39 | n.d. | n.d. | n.d. |
| 87 | 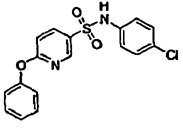 | 6-Phenoxy-pyridine-3-sulfonic acid (4-chloro-phenyl)-amide | 212 ± 27 | n.d. | n.d. | n.d. |
| 88 | 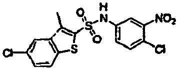 | 5-Chloro-3-methyl-benzo[*b*]thiophene-2-sulfonic acid (4-chloro-3-nitro-phenyl)-amide | 174 ± 11 | n.d. | n.d. | n.d. |
| 89 | 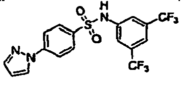 | *N*-(3,5-Bis-trifluoromethyl-phenyl)-4-pyrazol-1-yl-benzenesulfonamide | 275 ± 45 | n.d. | n.d. | n.d. |
| 90 | 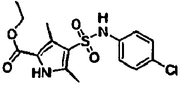 | 4-(4-Chloro-phenylsulfamoyl)-3,5-dimethyl-1*H*-pyrrole-2-carboxylic acid ethyl ester | 331 ± 52 | n.d. | n.d. | n.d. |
| 91 | 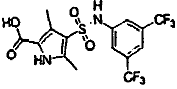 | 4-(3.5-Bis-trifluoromethyl-phenylsulfamoyl)-3,5-dimethyl-1*H*-pyrrole-2-carboxylic acid | 481 | n.d. | n.d. | n.d. |
| 92 | 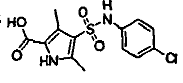 | 4-(4-Chloro-phenylsulfanioyl)-3,5-dimethyl-1*H*-pyrrole-2-carboxylic acid | 418 | n.d. | n.d. | n.d. |
| 93 | 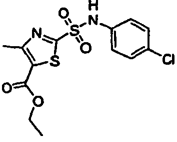 | 3-(4-Chloro-phenylsulfamoyl)4-methyl-thiazole-5-carboxylic acid ethyl ester | 373 ± 373 | n.d. | n.d. | n.d. |
| 94 | 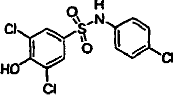 | 3,5-Dichloro-*N*-(4-chloro-phenyl)-4- hydroxy-benzenesulfonamide | 227 ± 7 | n.d. | n.d. | n.d. |
| 95 | 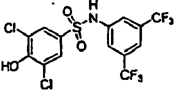 | *N*-(3,5-Bis-trifluoromethyl-phenyl)-3,5-dichloro-4-hydroxy-benzenesulfonamide | 243 ± 11 | n.d. | n.d. | n.d. |
| 96 | 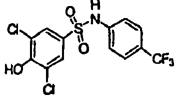 | 3,5-Dichloro-4-hydroxy-*N*-(4-trifluoromethyl-phenyl)-benzenesulfonamide | 449 ± 51 | n.d. | n.d. | n.d. |
| 97 | 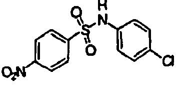 | *N*-(4-Chloro-phenyl)-4-nitro-benzene-sulfonamide | 394 ± 13 | n.d. | n.d. | n.d. |
| 98 | 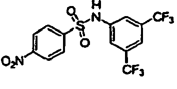 | *N*-(3,5-Bis-trifluoromethyl-phenyl)-4-nitro-benzenesulfonamide | 207 ± 0 | n.d. | n.d. | n.d. |
| 99 | 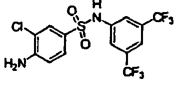 | 4-Amino-*N*-(3,5-bis-trifluoromethyl-phenyl)-3-chloro-benzenesulfonamide | 248 ± 29 | n.d. | n.d. | n.d. |
| 100 | 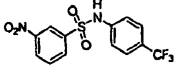 | 3-Nitro-*N*-(4-trifluoromethyl-phenyl)-benzenesulfonamide | 431 ± 5 | n.d. | n.d. | n.d. |
| 101 | 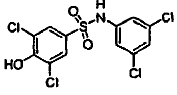 | 3,5-Dichloro-*N*-(3,5-dichloro-phenyl)-4-hydroxy-benzenesulfonamide | 299 ± 31 | n.d. | n.d. | n.d. |
| 102 | 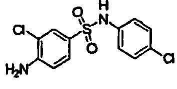 | 4-Arnino-3-chloro-*N*-(4-chloro-phenyl)-benzenesulfonamide | 467 ± 35 | n.d. | n.d. | n.d. |
| 103 | 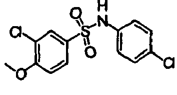 | 3-Chloro-*N*-(4-chloro-phenyl)-4-methoxy-benzenesulfonamide | 408 ± 18 | n.d. | n.d. | n.d. |
| 104 | 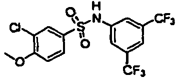 | *N*-(3,5-Bis-trifluoromethyl-phenyl)-3-chloro-4-methoxy-benzene-sulfonamide | 151± 26 | 25 | n.d. | n.d. |
| 105 | 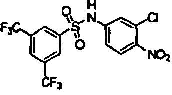 | *N*-(3-Chloro-4-nitro-phenyl)-3,5-bis-trifluoromethyl-benzenesulfonamide | 153 ± 18 | 24 ± 3 | 25 ± 1 | 37 ± 18 |
| 106 | 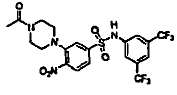 | 3-(4-Acetyl-piperazin-1-yl)-*N*-(3,5-bis-trifluoromethyl-phenyl)-4-nitro-benzenesulfonamide | 241 ± 158 | n.d. | n.d. | n.d. |
| 107 | 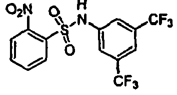 | *N*-(3,5-Bis-trifluoromethyl-phenyl)-2-nitro-benzenesulfonamide | 401 ± 46 | n.d. | n.d. | n.d. |
| 108 | 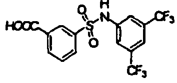 | 3-(3,5-Bis-trifluoromethyl-phenylsulfamoyl)-benzoic acid | 451 ± 118 | n.d. | n.d. | n.d. |
| 109 | 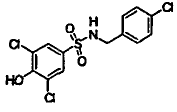 | 3,5-Dichloro-*N*-(4-chloro-benzyl)-4-hydroxy-benzenesulfonamide | 375 ± 41 | n.d. | n.d. | n.d. |
| 110 | 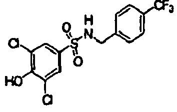 | 3,5-Dichloro-4-hydroxy-*N*-(4-trifluoromethyl-benzyl)-benzenesulfonamide | 242 | n.d. | n.d. | n.d. |
| 111 | 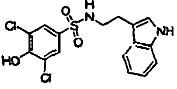 | 3,5-Dichloro-4-hydroxy-N-[2-(1*H*-indol-3-yl-)ethyl]-benzenesulfonamide | 448 | n.d. | n.d. | n.d. |
| 112 | 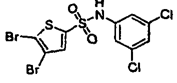 | 4,5-Dibromo-thiophene-2-sulfonic acid (3,5-dichloro-phenyl)-amide | 194 | n.d. | n.d. | n.d. |
| 113 | 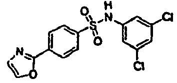 | *N*-(3,5-Dichloro-phenyl)-4-oxazol-2-yl-benzenesulfonamide | 246 | n.d. | n.d. | n.d. |
| 114 | 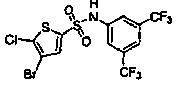 | 4-Bromo-5-chloro-thiophene-2-sulfonic acid (3,5-bis-trifluoromethyl-phenyl)-amide | 293 | n.d. | n.d. | n.d. |
| 115 | 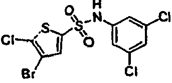 | 4-Bromo-5-chloro-thiophene-2-sulfonic acid (3,5-dichloro-phenyl)-amide | 431 | n.d. | n.d. | n.d. |
| 116 | 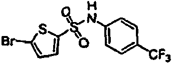 | 5-Bromo-thiopbcne-2-sulfonic acid (4-trifluoromethyl-phenyl)-amide | 152 | n.d. | n.d. | n.d. |
| 117 | 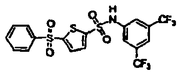 | 5-Benzenesulfonyl-thiophene-2-sulfonic acid (3,5-bis-trifluoromethyl-phenyl)-amide | 147 | n.d. | n.d. | n.d. |
| 118 | 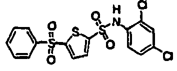 | 5-Benzenesulfonyl-thiophene-2-sulfonic acid (2,4-dichloro-phenyl)-amide | 305 | n.d. | n.d. | n.d. |
| 119 | 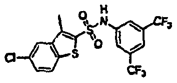 | 5-Chloro-3-methyl-benzo[*b*]thiophene-2-sulfonic acid (3,5-bis-brifluoromethyl-phenyl)-amide | 106 ± 13 | n.d. | n.d. | n.d. |
| 120 | 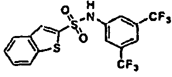 | Benzo[b]thiophene-2-sulfonic acid (3,5-bis-trifluoromethyl-phenyl)-amide | 193 ± 12 | n.d. | n.d. | n.d. |
| 121 | 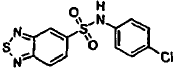 | Benzo[1,2,5]thiadiazol e-5-sulfonic acid (4-chloro-phenyl)-amide | 314 ± 29 | n.d. | n.d. | n.d. |
| 122 | 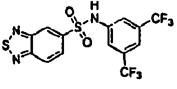 | Benzo[1,2,5]thiadiazol e-5-sulfonic acid (3,5-bis-trifluoromethyl-phenyl)-amide | 165 ± 8 | n.d. | n.d. | n.d. |
| 123 | 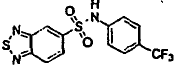 | Benzo[1,2,5]thiadiazol *e*-5-sulfonic acid(4- trifluoromethyl-phenyl)-amide | 329 ± 42 | n.d. | n.d. | n.d. |
| 124 | 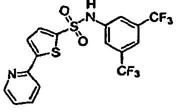 | 5-Pyridin-2-yl-thiophene-2-sulfonic acid (3,5-bis-trifluoromethyl-phenyl)-amide | 265 ± 64 | n.d. | n.d. | n.d. |
| 125 | 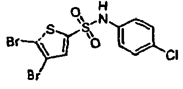 | 4,5-Dibromo-thiophene-2-sulfonic acid (4-chloro-phenyl)-amide | 198 ± 17 | n.d. | n.d. | n.d. |
| 126 | 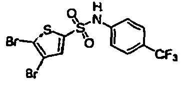 | 4,5-Dibromo-thiophene-2-sulfonic acid (4-trifluoromethyl-phenyl)-amide | 163 ± 27 | n.d. | n.d. | n.d. |
| 127 | 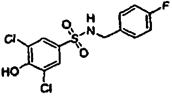 | 3,5-Dichloro-*N*-(4-fluoro-benzyl)-4- hydroxy-benzenesulfonamide | 325 ± 24 | n.d. | n.d. | n.d. |
| 128 | 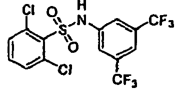 | *N*-(3,5-Bis-trifluoromethyl-phenyl)-2,6-dichloro-benzenesulfonamide | 134 ± 28 | 53 | n.d. | n.d. |
| 129 | 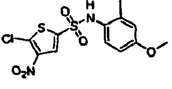 | 5-Chloro-4-nitro-thiophene-2-sulfonic acid (4-methoxy-2-methyl-phenyl)-amide | 278 ± 19 | n.d. | n.d. | n.d. |
| 130 | 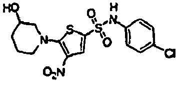 | 5-(3-Hydroxy-piperidin-1-yl)-4-nitro-thiophene-2-sulfonic acid (4-chloro-phenyl)-amide | 64 ± 1 | n.d. | n.d. | n.d. |
| 131 | 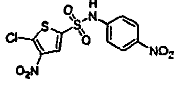 | 5-Chloro-4-nitro-thiophene-2-sulfonic acid (4-nitro-phenyl)-amide | 41 ± 4 | n.d. | n.d. | n.d. |

| | | | | | | |
|---|---|---|---|---|---|---|
| n.d. denoted not determined. | | | | | | |

**Table 2**

| **Cell line** | | **Anti-proliferative activity, 72-h MTT IC₅₀ (µM)** | | | | |
|---|---|---|---|---|---|---|
| | | **2** | **3** | **6** | **7** | **Roscovitine** |
| AGS | Gastric adenocarcinoma | 1.2 | 1.7 | 2.2 | 5.0 | 10.7 |
| DU145 | Prostate carcinoma | 3.1 | 3.6 | 6.1 | 9.1 | 8.5 |
| HT29 | Colon adenocarcinoma | 2.1 | 2.3 | 3.5 | 4.6 | 15.6 |
| Lovo | Colon adenocarcinoma | 2.1 | 2.3 | 3.9 | 8.5 | 13.8 |
| Nci-H460 | Large cell lung carcinoma | 3.3 | 5.2 | 8.0 | 9.6 | 12.8 |
| SK-N-MC | Neuriepithelioma | 3.0 | 3.8 | 2.9 | 3.0 | 7.1 |
| A549 | Lung carcinoma | 5.2 | 5.9 | 10.0 | 19.3 | 9.2 |
| H1299 | Large cell lung carcinoma | 4.4 | 5.2 | 4.9 | 14.0 | 11.9 |
| HCT116 | Colon carcinoma | 2.8 | 3.5 | 6.0 | 12.2 | 9.7 |
| HeLa | Cervical carcinoma | 2.8 | 2.7 | 3.9 | 13.7 | 16.1 |
| MCF7 | Breast adenocarcinoma | 2.7 | 1.9 | 3.3 | 11.2 | 12.1 |
| Messa | Uterus sarcoma | 13.1 | 18.3 | 16.0 | 8.1 | 9.9 |
| Messa-Dx5 | Uterus sarcoma | 9.5 | 12.1 | 15.5 | 7.6 | 5.3 |
| Saos-2 | Osteosracoma | 9.5 | 8.9 | 12.0 | 13.3 | 17.4 |
| NaCat | Human keratinocytes | 9.3 | 10.5 | 10.6 | 12.9 | 16.3 |
| SJSA-1 | Osteosracoma | 13.5 | 14.1 | 17.8 | 17.0 | 17.8 |
| U20S | Osteosracoma | 6.2 | 8.2 | 10.2 | 8.8 | 16.4 |
| SKUT-1 | Uterus leiomyosarcoma | 8.8 | 14.1 | 15.0 | 6.1 | 10.5 |
| HS27 | Foreskin fibroblasts | 17.3 | 15.2 | 21.4 | 39.2 | 43.1 |
| IMR90 | Lung fibroblasts | 23.1 | 19.8 | 29.8 | 41.8 | 44.2 |
| WI38 | Lung fibroblasts | 22.7 | 21.1 | 30.0 | 52.3 | 34.5 |
| Average transformed | | 5.7 ± 3.9 | 6.9 ± 5.0 | 8.4 ± 5.1 | 10.2 ± 4.4 | 12.3 ± 3.7 |
| Average carcinomas | | 3.1 ± 1.2 | 3.8 ± 1.8 | 4.9 ± 2.3 | 9.7 ± 4.7 | 11.2 ± 3.0 |
| Average sarcomas | | 10.2 ± 2.7 | 12.1 ± 3.8 | 13.7 ± 3.1 | 11.3 ± 3.7 | 13.8 ± 5.1 |
| Average non-transformed | | 21.0 ± 3.1 | 18.7 ± 3.1 | 27.1 ± 4.9 | 44.6 ± 6.9 | 40.6 ± 5.3 |

**Table 3**

| **Compound** | **Concentration*^{b}* (µM)** | **Fold induction of luciferase activity*^{c}* assay** | ***In vitro* competitive HDM2 binding*^{d}* IC₅₀(µM)** |
|---|---|---|---|
| Control*^{a}* | 150 - 200 | 4 | 0.19 |
| **2** | 6 | 231 | 26.4 ± 3.4 |
| **3** | 6 | 200 | 41.9 ± 5.8 |
| **4** | 100*^{e}* | < 2 | 378 |
| **6** | 5.5 | 84 | 20.4 ± 1.4 |
| **7** | 9 | 32 | 15.4 ± 7.8 |
| **39** | 15 | 64 | 46.1 ± 3.4 |
| **40** | 13 | 68 | 59.6 ± 10.9 |
| **45** | 15 | 67 | 16 ± 3.7 |
| **46** | 6 | 55 | 11.1 ± 1.7 |
| **48** | 5.5 | 43.5 | 25.3 ± 4.6 |
| **49** | 5.5 | 117 | 46.3 ± 4 |
| **50** | 39 | 21 | 7.3 ± 0.6 |
| **54** | 10 | 90 | 20.2 ± 1.3 |
| **55** | 10 | 53 | 27 ± 10.1 |
| **57** | 7 | 25 | 31.1 ± 5.6 |
| **60** | 7.5 | 48 | 29 ± 6 |
| **61** | 4.8 | 34 | 45 ± 14 |
| **63** | 6.4 | 95 | 35 ± 8 |
| **129** | 100 | 5 | 278 ± 19 |
| **65** | 10 | 74 | 7.4 ± 1 |
| **66** | 4.2 | 65 | 14.7 ± 0.9 |
| **38** | 13 | 4 | 231 ± 13 |
| **83** | 5 | 10 | 119 ± 42.5 |

| | | | |
|---|---|---|---|
| *^{a}* H-Phe-Met-Aib-Pmp-(6-Cl-Trp)-Glu-Ac₃c-Leu-NH₂. *^{b}* Test compound concentration at which the p53 induction was maximal. *^{c}* Compared to basal luciferase activity (16-h time point). *^{d}* As measured in the fluorescence polarisation assay described in Example 3. *^{e}* Highest concentration tested. | | | |

**Table 4**

| **Compound** | **IC₅₀ (µM)*^{a}*** | |
|---|---|---|
| | **Non-synchronised cells** | **Synchronised cells*^{b}*** |
| 2 | 23.2 ± 4.0 | n.d. |
| 3 | 28.8 ± 2.3 | 69 ± 30 |
| 5 | 73.3 ± 33.4 | 72 ± 31 |
| 6 | 37.5 ± 12.9 | 39 |
| 41 | 76.6 | 84 |
| 39 | 90.0 | n.d. |
| 40 | 99.5 | n.d. |
| 46 | 25.5 ± 3.1 | n.d. |
| 48 | 31.3 ± 2.6 | n.d. |
| 49 | 36.3 ± 2.9 | n.d. |
| 60 | 29.2 ± 6.8 | n.d. |
| 61 | 34.6 ± 22.9 | n.d. |
| 64 | 44.6 ± 2.3 | n.d. |
| 83 | 29.4 ± 6.0 | n.d. |

| | | |
|---|---|---|
| *^{a}* After 3-h treatment with test compounds. *^{b}* Synchronisation with 0.3 mM mimosine for 24 h, followed by wash-out. | | |

**Table 5**

| **Administration sequence** | **IC₅₀ (µM)** | | |
|---|---|---|---|
| | *AGS* | *H1299* | *SJSA-1* |
| **2** alone | 1.1 | 5.1 | 14 |
| Cisplatin alone | 3.8 | 2.9 | 6.1 |
| **2** + cisplatin simultaneously | 0.11 | 0.11 | 0.11 |
| Cisplatin + **2** after 6 h | 1.5 | 0.03 | 0.23 |
| **2** + cisplatin 6 h later | 0.21 | 0.18 | 0.02 |

**Table 6**

| **Administration sequence** | IC₅₀ (µM) | | |
|---|---|---|---|
| | *AGS* | *H1299* | *SJSA-1* |
| **2** alone | 1.1 | 5.1 | 14 |
| Etoposide alone | 0.28 | 1.3 | 16 |
| **2** + etoposide simultaneously | 0.20 | 0.04 | 0.19 |
| Etoposide + **2** 6 h later | 0.25 | 0.06 | 1.1 |
| **2** + etoposide 6 h later | 0.08 | 0.05 | 0.02 |

## Claims

1. Use of a compound of formula I, 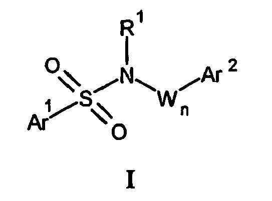 wherein
W is a C₁₋₅ branched or unbranched alkylene group or a C₂₋₅ alkenylene group;
n is 0 or 1;
R¹ is H, a C₁₋₈ branched or unbranched alkyl group, a C₂₋₈ alkenyl group, or an aryl or aralkyl group, each of which may be optionally substituted by one or more halogen or CF₃ groups;
Ar¹ is 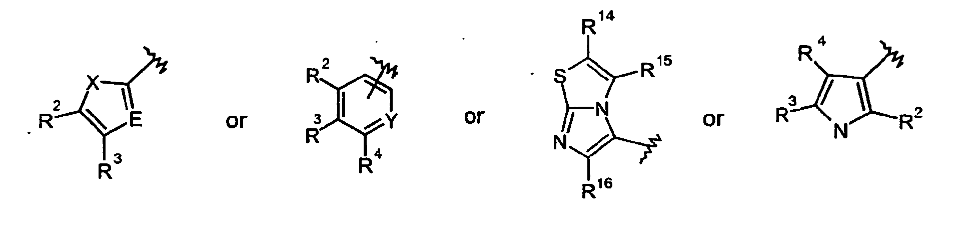 wherein
X is S, O, NH or NR' where R' is a C₁₋₃ alkyl group;
YisCHorN;
E is N or CR⁴;
R², R³, R⁴, and R¹⁴⁻¹⁶ are each independently (A)ₚB, wherein A is C₁₋₃ alkyl, p is 0 or 1, and B is H, halogen, C₁₋₅ alkyl, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f} CONH₂, COONHAr^{d}, CONHR^{g}, COAr^{e}, COR^{h}, S(CO)R^{s}, OR^{t}, OAr^{f}, an alicyclic group optionally containing one or more heteroatoms, optionally substituted by one or more OH, COR^{u}, halogen or CF₃ groups, or a heteroaryl group optionally substituted by one or more C₁₋₅ alkyl, halogen, SRⁱ or CF₃ groups; or
R² and R³ are linked to form a saturated or unsaturated ring system, optionally containing one or more heteroatoms, and optionally substituted by one or more halogen, OH or CF₃ groups;
Ar^{a-f} are each independently aryl groups optionally substituted by one or more C₁₋₅ alkyl, halogen or CF₃ groups;
R^{a-i}, R^{s}, R^{t} and R^{u} are each independently C₁₋₅ alkyl groups optionally substituted by one or more alkoxy, halogen or CF₃ groups;
and with the proviso that at least one of R², R³ and R⁴ is other than H;
Ar² is 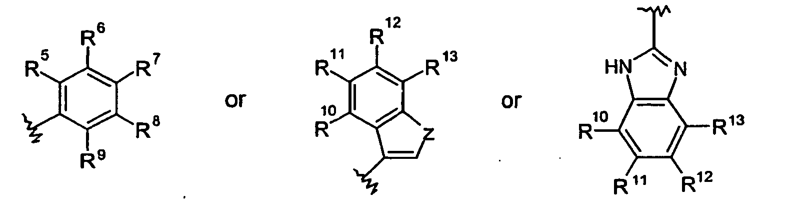 wherein
Z is S, O, NH or NR" where R" is C₁₋₃ alkyl;
R⁵, R⁶, R⁷, R⁸ and R⁹ are each independently (L)_{q}M wherein L is C₁₋₃ alkyl, q is 0 or 1, M is H, C₁₋₅ alkyl, halogen, NO₂, OH, NH₂, NHAr^{g}, NHR^{j}, NR^{k}R^{l}, SO₃H, SO₂NH₂, SO₂NHAr^{h}, SO₂NHR^{m}, SO₂Arⁱ, SO₂Rⁿ, CF₃, CN, COOH, COOR^{p}, CONH₂, CONHAr^{j}, CONHR^{q}, OR^{v} COAr^{k} or COR^{r};
R^{j-r}, R^{v} are each independently C₁₋₅ alkyl groups;
Ar^{g-k} are each independently aryl groups;
and with the proviso that at least one of the substituents R⁵, R⁶, R⁷, R⁸ and R⁹ is other than H;
R¹⁰, R¹¹, R¹² and R¹³ are each independently H, C₁₋₅ alkyl, halogen, NO₂, OH, NH₂ or CF₃;
in the preparation of a medicament for treating a proliferative disorder.

2. Use according to claim wherein Ar¹ is 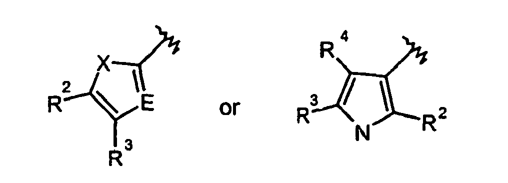 and Ar² is

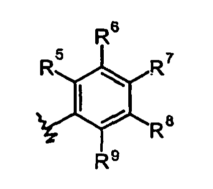

3. Use according to claim 1 wherein Ar¹ is 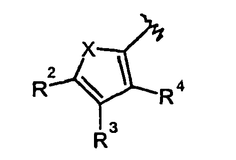 and Ar² is

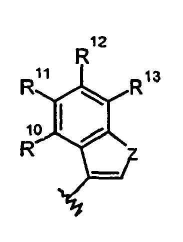

4. Use according to claim 1 wherein Ar¹ is 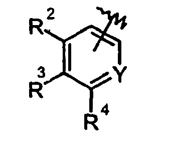 and Ar² is

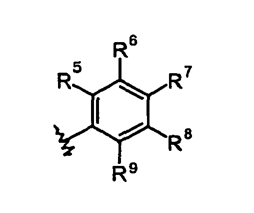

5. Use according to claim 1 wherein Ar¹ is 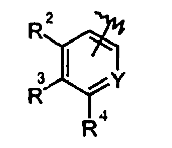 and Ar² is

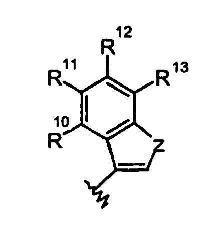

6. Use according to claim 1 wherein Ar¹ is 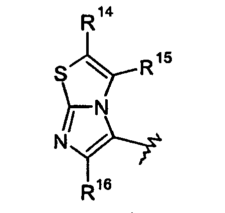 and Ar² is

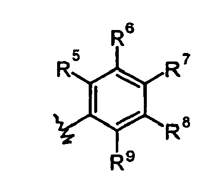

7. Use according to claim 1 wherein Ar¹ is 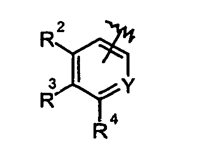 and Ar² is

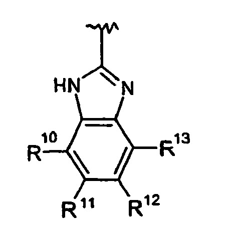

8. Use according to any preceding claim wherein
- R², R³ and R⁴ are each independently (A)ₚB, wherein A is C₁₋₅ alkyl, p is 0 or 1, and B is H, F, Cl, Br, I, C₁₋₅ alkyl, NO₂, OH, NH₂, NHR^{a}, NR^{b}R^{c}, SO₃H, SO₂NH₂, NHPh, SO₂NHAr^{b}, SO₂NHR^{d}, SO₂Ph, SO₂R^{e}, CF₃, CN, COOH, COOR^{f} CONH₂, COONHPh, CONHR^{g}, S(CO)R^{s}, OR^{t}, OAr^{f}, COPh, COR^{h}, a morpholino, piperazino or piperidino group each of which may be optionally substituted by one or more OH or COR^{u} groups, or a heteroaryl group selected from pyridyl, pyrimidyl, oxazolyl, thiazolyl and pyrazolyl, each of which may be optionally substituted by one or more C₁₋₅ alkyl, halogen, SRⁱ or CF₃ groups, or R² and R³ together form a saturated 6-membered ring or an unsaturated 5-membered ring, each of which optionally contain one or more heteroatoms; and
- R⁵, R⁶, R⁷, R⁸, and R⁹ are each independently (L)_{q}M wherein L is C₁₋₅ alkyl, q is 0 or 1, M is H, C₁₋₅ alkyl, halogen, NO₂, OH, NH₂, NHPh, NHR^{j}, NR^{k}R^{l}, SO₃H, SO₂NH₂, SO₂NHPh, SO₂NHR^{m}, SO₂Ph, SO₂Rⁿ, CF₃, CN, COOH, COOR^{p}, CONH₂, CONHPh, CONHR^{q}, OR^{v}, COPh or COR^{r}.

9. Use according to any preceding claim wherein R², R³ and R⁴ are each independently (A)ₚB, wherein A is C₁₋₅ alkyl, p is 0 or 1, and B is H, F, Cl, Br, I, C₁₋₅ alkyl, NO₂, OH, NH₂, NHR^{a}, NR^{b}R^{c}, SO₃H, SO₂NH₂, NHPh, SO₂NHPh, SO₂NHR^{d}, SO₂Ph, SO₂R^{e}, CF₃, CN, COOH, COOR^{f}, CONH₂, COONHPh, CONHR^{g}, S(CO)R^{s}, OR^{t}, OAr^{f}, COPh, COR^{h}, pyridyl, pyrimidyl, 2-methylsulfanylpyrimid-5-yl, oxazol-2-yl, thiazol-2-yl, 1-methyl-5-trifluoromethyl-1*H*-pyrazol-4-yl, morpholin-4-yl, 4-acetylpiperazin-1-yl, 3-hydroxy-piperidin-1-yl, or R² and R³ together form
-OCH₂CH₂O-
-N-S-N-
or
a phenyl group optionally substituted by one or more halogens.

10. Use according to any preceding claim wherein R², R³ and R⁴ are each independently H, halogen, NO₂, SO₂Ph, S(CO)Me, COOH, COOEt, OPh, OMe, NHCH₂CH₂OMe, 1-methyl-5-trifluoromethyl-1*H*-pyrazol-4-yl, 2-methylsulfanyl pyrimid-5-yl, N-(4-fluorophenyl)sulfonamido, N-(4-trifluoromethylphenyl)-sulfonamido, oxazol-2-yl, C₁₋₅ alkyl, NH₂, morpholin-4-yl, 4-acetyl-piperazin-1-yl, 3-hydroxy-piperidin-1-yl, or R² and R³ together form
OCH₂CH₂O-
-N-S-N-
or
a phenyl group optionally substituted by one or more halogens.

11. Use according to any one of claims 1-3 or 8-10 wherein Ar¹ is 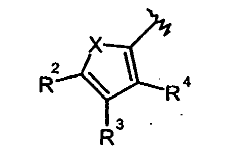
X is S or N;
R², R³ and R⁴ are each independently C₁₋₅ alkyl, S(CO)Me, COOH, NHCH₂CH₂OMe, COOEt, H, halogen, NO₂, SO₂Ph, SO₂NH-(4-chlorophenyl), 1-methyl-5-trifluoromethyl-1*H*-pyrazol-4-yl, morpholin-4-yl, 2-methylsulfanylpyrimid-5-yl, N-(4-fluorophenyl)sulfonamido, N-(4-trifluoro-methylphenyl)-sulfonamido, 3-hydroxy-piperidin-1-yl, pyridin-2-yl; or R² and R³ form a phenyl group optionally substituted by one or more halogens.

12. Use according to claim 11 wherein
- R² is halogen, SO₂Ph, NO₂, Et, SOMe, morpholin-4-yl, NHCH₂CH₂OMe, 3-hydroxy-piperidin-1-yl, 1-methyl-5-trifluoromethyl-1*H*-pyrazol-4-yl or 2-methylsulfanyl-pyrimid-5-yl;
- R³ is halogen, SO₂NH-(4-chlorophenyl), H, NO₂, N-(4-fluorophenyl)sulfonamido or N-(4-trifluoro methylphenyl)-sulfonamido; and
- R⁴ is H.

13. Use according to any one of claims 1, 4, 5, 7-10 wherein Ar¹ is 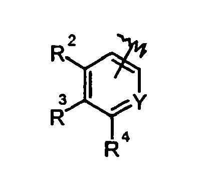
Y is CH or N; and
R², R³ and R⁴ are each independently H, OH, COOH, CF₃, OPh, OMe, NO₂, 4-acetyl-piperazin-1-yl, NH₂, halogen, pyrazol-1-yl, oxazol-2-yl or C₁₋₅ alkyl, or R² and R³ together form -OCH₂CH₂O- or -N-S-N- .

14. Use according to claim 13 wherein
when Y is CH
- R² is H, NO₂ or Cl;
- R³ is NO₂, NH₂, Cl, CF₃, COOH, 4-acetyl-piperazin-1-yl; and
- R⁴ is H, Cl, oxazol-2-yl, OH, NO₂, NH₂, OMe or Me; or
when Y is N
- R² is H;
- R³ is Br;
- R⁴ is Cl or OPh.

15. Use according to any one of claims 1, 2 or 8-10 wherein Ar¹ is 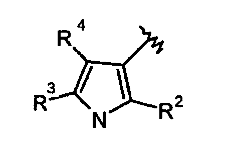
R² and R⁴ are C₁₋₅ alkyl, and R³ is COOH or COOEt.

16. Use according to any preceding claim wherein
- R⁵, R⁶, R⁷, R⁸ and R⁹ are each independently H, halogen, OMe, NO₂, C₁₋₅ alkyl, CF₃ or OH; and
- R¹⁰, R¹¹, R¹² and R¹³ are all H.

17. Use according to claim 16 wherein
- R⁵ is H, C₁₋₅ alkyl, or halogen;
- R⁶ is H, halogen, NO₂, or CF₃;
- R⁷ is H, halogen, OMe, NO₂, OH or CF₃;
- R⁸ is H, halogen or CF₃;
- R⁹ is H.

18. Use according to any preceding claim wherein
- W is CH₂, CH₂CH₂ or CH(CH₃)CH₂; and
- R¹ is H, CH₂Ph, CH₂CH(Me)₂, 3-(trifluoromethyl)benzyl, or Me.

19. Use according to any preceding claim wherein said compound of formula I is selected from
5-Chloro-4-nitrothiophene-2-sulfonic acid (3-trifluoromethylphenyl)amide [1];
5-Chloro-4-nitrothiophene-2-sulfonic acid (4-chlorophenyl)-amide [2];
5-Chloro-4-nitrothiophene-2-sulfonic acid (4-fluorophenyl)amide [3];
4-Bromo-5-chlorothiophene-2-sulfonic acid (4-fluorophenyl)amide [4];
5-Chloro-4-nitrothiophene-2-sulfonic acid (4-hydroxyphenyl)amide [5];
5-Chloro-4-nitrothiophene-2-sulfonic acid (4-trifluoromethylphenyl)amide [6];
5-Chloro-4-nitrothiophene-2-sulfonic acid (4-fluorophenyl)methylamide [7];
4,5-Dibromothiophene-2-sulfonic acid (3,5-bis-trifluoromethylphenyl)amide [8];
5-Chlorothiophene-2-sulfonic acid (4-trifluoromethylphenyl)amide [9];
5-Chlorothiophene-2-sulfonic acid (4-chlorophenyl)amide [10];
5-Chlorothiophene-2-sulfonic acid (3,5-bis-trifluoromethylphenyl)amide [11];
5-(2-Methylsulfanyl-pyrimidin-5-yl)-thiophene-2-sulfonic acid (3,5-dichlorophenyl)-amide [12];
4-Oxazol-2-yl-N-(4-trifluoromethylphenyl)benzenesulfonamide [13];
N-(3,5-Bis-trifluoromethylphenyl)-4-oxazol-2-yl-benzenesulfonamide [14];
4-Bromo-5-chlorothiophene-2-sulfonic acid (4-trifluoromethylphenyl)amide [15];
5-Bromothiophene-2-sulfonic acid (4-chlorophenyl)amide [16];
5-Bromothiophene-2-sulfonic acid (3,5-dichlorophenyl)amide [17];
5-Bromothiophene-2-sulfonic acid (3,5-bis-trifluoromethylphenyl)amide [18];
N-(4-Chlorophenyl)-3-nitrobenzenesulfonamide [19];
3-Nitro-N-(4-trifluoromethylphenyl)benzenesulfonamide [20];
N-(3,5-Bis-trifluoromethylphenyl)-3-nitrobenzenesulfonamide [21];
N-(2,4-Dichlorophenyl)-3-nitrobenzenesulfonamide [22];
5-Benzenesulfonylthiophene-2-sulfonic acid (4-trifluoromethylphenyl)-amide [23];
5-Ben2enesulfonylthiophene-2-sulfonic acid (4-chlorophenyl)amide [24];
5-Benzenesulfonylthiophene-2-sulfonic acid (3,5-dichlorophenyl)amide [25];
5-Chlorothiophene-2-sulfonic acid (3,4-dichlorophenyl)amide [26];
4,5-Dibromothiophene-2-sulfonic acid (3-trifluoromethylphenyl)amide [27];
4,5-Dibromothiophene-2-sulfonic acid (3,4-dichlorophenyl)amide [28];
N-(3,5-Bis-trifluoromethylphenyl)-4-chloro-3-nitrobenzenesulfonamide [29];
4-Chloro-N-(3,4-dichlorophenyl)-3-nitrobenzenesulfonamide [30];
5-(1-Methyl-5-trifluoromethyl-1H-pyrazol-4-yl)-thiophene-2-sulfonic acid (4-trifluoromethylphenyl)-amide [31];
5-Chlorothiophene-2,4-disulfonic acid bis-[(4-fluorophenyl)-amide] [32];
5-Chlorothiophene-2,4-disulfonic acid bis-[(4-trifluoro-methyl-phenyl)-amide] [33];
4-Methyl-3-nitro-N-(4-trifluoromethylphenyl)benzenesulfonamide [34];
4-Chloro-3-nitro-N-(4-trifluoromethylphenyl)benzenesulfonamide [35];
3-Amino-4-methyl-N-(4-trifluoromethyl-phenyl)benzenesulfonamide [36];
N-(4-Chlorophenyl)-4-methyl-3-nitrobenzenesulfonamide [37];
4-Chloro-N-(4-chlorophenyl)-3-nitro-benzenesulfonamide [38];
5-Chloro-4-nitrothiophene-2-sulfonic acid (3,5-dichlorophenyl)-amide [39];
5-Chloro-4-nitrothiophene-2-sulfonic acid (3,5-difluorophenyl)-amide [40];
5-Bromo-6-chloropyridine-3-sulfonic acid (4-trifluoromethylphenyl)amide [41];
5-Bromo-6-chloropyridine-3-sulfonic acid (3,5-bis-trifluoromethylphenyl)amide [42];
5-(1-Methyl-5-trifluoromethyl-1H-pyrazol-4-yl)-thiophene-2-sulfonic acid (3,5-bistrifluoromethylphenyl)amide [43];
5-Chloro-4-nitrothiophene-2-sulfonic acid (3,5-bis-trifluoromethyl-phenyl)-amide [44];
5-Chloro-4-nitrothiophene-2-sulfonic acid 4-fluorobenzylamide [45];
5-Chloro-4-nitrothiophene-2-sulfonic acid 4-trifluoromethylbenzylamide [46];
4-Chloro-N-(3,5-dichlorophenyl)-3-nitro-benzenesulfonamide [47];
5-Chloro-4-nitrothiophene-2-sulfonic acid [2-(1H-indol-3-yl)-ethyl]amide [48];
5-Chloro-4-nitrothiophene-2-sulfonic acid [2-(1H-indol-3-yl)-1-methylethyl]amide [49];
5-Chloro-4-nitrothiophene-2-sulfonic acid methyl-(4-trifluoromethylphenyl)amide [50];
5-Chloro-4-nitrothiophene-2-sulfonic acid (4-chloro-phenyl)-methylamide [51]; and
5-Chloro-4-nitrothiophene-2-sulfonic acid methyl-(4-trifluoromethylbenzyl)amide [52];
5-Chloro-4-nitro-thiophene-2-sulfonic acid benzyl-(4-fluoro-benzyl)-amide [53];
5-Chloro-4-nitro-thiophene-2-sulfonic acid 3,5-dichloro-benzylamide [54];
5-Chloro-4-nitro-thiophene-2-sulfonic acid 3,5-difluoro-benzylamide [55];
5-Chloro-4-nitro-thiophene-2-sulfonic acid 4-chloro-benzylamide [56];
5-Chloro-4-nitro-thiophene-2-sulfonic acid [1-(4-fluoro-phenyl)-ethyl]-amide [57];
5-Chloro-4-nitro-thiophene-2-sulfonic acid (4-chloro-phenyl)-isobutyl-amide [58];
5-Chloro-4-nitro-thiophene-2-sulfonic acid (1H-benzo-imidazol-2-yl)-amide [59];
5-Chloro-4-nitro-thiophene-2-sulfonic acid [2-(6-chloro-1H-indol-3-yl)-ethyl]-amide [60];
5-Chloro-4-nitro-thiophene-2-sulfonic acid (4-methoxy-phenyl)-amide [61];
5-Chloro-4-nitro-thiophene-2-sulfonic acid p-tolylamide [63];
5-Chloro-4-nitro-thiophene-2-sulfonic acid benzyl-(4-chloro-phenyl)-amide [65];
5-Chloro-4-nitro-thiophene-2-sulfonic acid benzyl-(4-methoxy-phenyl)-amide [66];
5-Chloro-4-nitro-thiophene-2-sulfonic acid (4-chloro-phenyl)-(3-trifluoromethyl-benzyl)-amide [67];
5-Nitro-thiophene-2-sulfonic acid (4-chloro-phenyl)-amide [68];
4-Nitro-thiophene-2-sulfonic acid (4-chloro-phenyl)-amide [69];
5-Chloro-thiophene-2,4-disulfonic acid bis-[(4-chloro-phenyl)-amide] [70];
5-Ethyl-4-nitro-thiophene-2-sulfonic acid (4-chloro-phenyl)-amide [71];
Thioacetic acid S-[5-(4-chloro-phenylsulfamoyl)-3-nitro-thiophen-2-yl] ester [72];
5-Methyl-4-nitro-thiophene-2-sulfonic acid (4-chloro-phenyl)-amide [73];
5-Methyl-thiophene-2,4-disulfonic acid bis-[(4-chloro-phenyl)-amide] [74];
5-Chloro-4-nitro-thiophene-2-sulfonic acid (3-trifluoro-methyl-benzyl)-(4-trifluoromethylbenzyl)-amide [75];
4-Nitro-thiophene-2-sulfonic acid (4-trifluoromethyl-phenyl)-amide [76];
4-Nitro-thiophene-2-sulfonic acid [2-(1H-indol-3-yl)-ethyl]-amide [77];
5-(1-Methyl-5-tri-fluoromethyl-1H-pyrazol-3-yl)-thiophene-2-sulfonic acid (3,5-bis-trifluoromethyl-phenyl)-(3-trifluoro-methyl-benzyl)-amide [78];
5-Morpholin-4-yl-4-nitro-thiophene-2-sulfonic acid (4-chloro-phenyl)-amide [79];
S-(2-Methoxy-ethylamino)-4-nitro-thiophene-2-sulfonic acid (4-chloro-phenyl)-amide [80];
4-Chloro-N-[2-(S-chloro-1H-indol-3-yl)-ethyl]-3-nitro-benzenesulfonamide [81];
N-[2-(5-Chloro-1H-indol-3-yl)-ethyl]-4-methyl-3-nitro-benzenesulfonamide [82];
N-(1H-Benzoimidazol-2-yl)-4-chloro-3-nitro-benzenesulfonamide [83];
6-Chloro-imidazo[2,1-b]thiazole-5-sulfonic acid (3,5-bis-trifluoro-methyl-phenyl)-amide [84];
2,3-Dihydro-benzo[1,4]dioxine-6-sulfonic acid (4-chloro-phenyl)-amide [85];
2,3-Dihydro-benzo[1,4]dioxine-6-sulfonic acid (3,5-bis-trifluoromethyl-phenyl)-amide [86];
6-Phenoxy-pyridine-3-sulfonic acid (4-chloro-phenyl)-amide [87];
5-Chloro-3-methyl-benzo[b]thiophene-2-sulfonic acid (4-chloro-3-nitro-phenyl)-amide [88];
N-(3,5-Bis-trifluoromethyl-phenyl)-4-pyrazol-1-yl-benzenesulfonamide [89];
4-(4-Chloro-phenylsulfamoyl)-3,5-dimethyl-1H-pyrrole-2-carboxylic acid ethyl ester [90];
4-(3,5-Bis-trifluoromethyl-phenylsulfamoyl)-3,5-dimethyl-1H-pyrrole-2-carboxylic acid [91];
4-(4-Chloro-phenylsulfamoyl)-3,5-dimethyl-1H-pyrrole-2-carboxylic acid [92];
2-(4-Chloro-phenylsulfamoyl)-4-methyl-thiazole-5-carboxylic acid ethyl ester [93];
3,5-Dichloro-N-(4-chloro-phenyl)-4-hydroxy-benzenesulfonamide [94];
N-(3,5-Bis-trifluoromethyl-phenyl)-3,5-dichloro-4-hydroxy-benzenesulfonamide [95];
3,5-Dichloro-4-hydroxy-N-(4-trifluoromethyl-phenyl)-benzenesulfonamide [96];
N-(4-Chloro-phenyl)-4-nitro-benzene-sulfonamide [97];
N-(3,5-Bis-trifluoromethyl-phenyl)-4-nitro-benzenesulfonamide [98];
4-Amino-N-(3,5-bis-trifluoromethyl-phenyl)-3-chloro-benzenesulfonamide [99];
3-Nitro-N-(4-trifluoromethyl-phenyl)-benzenesulfonamide [100];
3,5-Dichloro-N-(3,5-dichloro-phenyl)-4-hydroxy-benzenesulfonamide [101];
4-Amino-3-chloro-N-(4-chloro-phenyl)-benzenesulfonamide [102];
3-Chloro-N-(4-chloro-phenyl)-4-methoxy-benzenesulfonamide [103];
N-(3,5-Bis-trifluoromethyl-phenyl)-3-chloro-4-methoxy-benzene-sulfonamide [104];
N-(3-Chloro-4-nitro-phenyl)-3,5-bis-trifluoromethyl-benzenesulfonamide [105];
3-(4-Acetyl-piperazin-1-yl)-N-(3,5-bis-trifluoromethyl-phenyl)-4-nitrobenzenesulfonamide [106];
N-(3,5-Bis-trifluoromethyl-phenyl)-2-nitro-benzenesulfonamide [107];
3-(3,5-Bis-trifluoromethyl-phenylsulfamoyl)-benzoic acid [108];
3,5-Dichloro-N-(4-chloro-benzyl)-4-hydroxy-benzenesulfonamide [109];
3,5-Dichloro-4-hydroxy-N-(4-trifluoromethyl-benzyl)-benzenesulfonamide [110];
3,5-Dichloro-4-hydroxy-N-[2-(1H-indol-3-yl)-ethyl]-benzenesulfonamide [111];
4,5-Dibromo-thiophene-2-sulfonic acid (3,5-dichloro-phenyl)-amide [112];
N-(3,5-Dichloro-phenyl)-4-oxazol-2-yl-benzenesulfonamide [113];
4-Bromo-5-chloro-thiophene-2-sulfonic acid (3,5-bis-trifluoromethyl-phenyl)-amide [114];
4-Bromo-5-chloro-thiophene-2-sulfonic acid (3,5-dichloro-phenyl)-amide [115];
5-Bromo-thiophene-2-sulfonic acid (4-trifluoromethyl-phenyl)-amide [116];
5-Benzenesulfonyl-thiophene-2-sulfonic acid (3,5-bis-trifluoromethyl-phenyl)-amide [117]; 5-Benzenesulfonyl-thiophene-2-sulfonic acid (2,4-dichloro-phenyl)-amide [118];
5-Chloro-3-methyl-benzo[b]thiophene-2-sulfonic acid (3,5-bis-trifluoromethyl-phenyl)-amide [119];
Benzo[b]thiophene-2-sulfonic acid (3,5-bis-trifluoromethyl-phenyl)-amide [120];
Benzo[1,2,5]thiadiazole-5-sulfonic acid (4-chloro-phenyl)-amide [121];
Benzo[1,2,5]thiadiazole-5-sulfonic acid (3,5-bis-trifluoromethyl-phenyl)-amide [122];
Benzo[1,2,5]thiadiazole-5-sulfonic acid (4-trifluoromethyl-phenyl)-amide [123];
5-Pyridin-2-yl-thiophene-2-sulfonic acid (3,5-bis-trifluoromethyl-phenyl)-amide [124];
4,5-Dibromo-thiophene-2-sulfonic acid (4-chloro-phenyl)-amide [125];
4,5-Dibromo-thiophene-2-sulfonic acid (4-trifluoromethyl-phenyl)-amide [126];
3,5-Dichloro-N-(4-fluoro-benzyl)-4-hydroxy-benzenesulfonamide [127];
N-(3,5-Bis-trifluoromethyl-phenyl)-2,6-dichloro-benzenesulfonamide [128];
5-Chloro-4-nitro-thiophene-2-sulfonic acid (4-methoxy-2-methyl-phenyl)-amide [129];
5-(3-Hydroxy-piperidin-1-yl)-4-nitro-thiophene-2-sulfonic acid (4-chloro-phenyl)-amide [130]; and
5-Chloro-4-nitro-thiophene-2-sulfonic acid (4-nitro-phenyl)-amide [131].

20. Use according to claim 19 wherein the compound of formula I is selected from the following:
5-Chloro-4-nitrothiophene-2-sulfonic acid (4-chlorophenyl)amide [2];
5-Chloro-4-nitrothiophene-2-sulfonic acid (4-fluorophenyl)amide [3];
5-Chloro-4-nitrothiophene-2-sulfonic acid (4-hydroxyphenyl)amide [5];
5-Chloro-4-nitrothiophene-2-sulfonic acid (4-trifluoromethylphenyl)amide [6];
5-Chloro-4-nitrothiophene-2-sulfonic acid (4-fluorophenyl)methylamide [7];
4-Chloro-3-nitro-N-(4-trifluoromethylphenyl)-benzene-sulfonamide [35];
4-Chloro-N-(4-chlorophenyl)-3-nitrobenzene-sulfonamide [38];
5-Chloro-4-nitrothiophene-2-sulfonic acid (3,5-dichlorophenyl)amide [39];
5-Chloro-4-nitrothiophene-2-sulfonic acid (3,5-difluorophenyl)amide [40];
5-(1-Methyl-5-trifluoromethyl-1H-pyrazol-4-yl)-thiophene-2-sulfonic acid (3,5-bistrifluoromethylphenyl)amide [43];
5-Chloro-4-nitrothiophene-2-sulfonic acid (3,5-bis-trifluoromethyl-phenyl)-amide [44];
5-Chloro-4-nitrothiophene-2-sulfonic acid 4-fluorobenzylamide [45];
5-Chloro-4-nitrothiophene-2-sulfonic acid 4-trifluoromethylbenzylamide [46];
4-Chloro-N-(3,5-dichlorophenyl)-3-nitro-benzenesulfonamide [47];
5-Chloro-4-nitrothiophene-2-sulfonic acid [2-(1H-indol-3-yl)-ethyl]amide [48];
5-Chloro-4-nitrothiophene-2-sulfonic acid [2-(1H-indol-3-yl)-1-methylethyl]amide [49];
5-Chloro-4-nitrothiophene-2-sulfonic acid (4-chlorophenyl)methylamide [51];
5-Chloro-4-nitro-thiophene-2-sulfonic acid benzyl-(4-fluoro-benzyl)-amide [53];
5-Chloro-4-nitro-thiophene-2-sulfonic acid 3,5-dichloro-benzylamide [54];
5-Chloro-4-nitro-thiophene-2-sulfonic acid 3,5-difluoro-benzylamide [55];
5-Chloro-4-nitro-thiophene-2-sulfonic acid 4-chloro-benzylamide [56];
5-Chloro-4-nitro-thiophene-2-sulfonic acid [1-(4-fluoro-phenyl)-ethyl]-amide [57];
5-Chloro-4-nitro-thiophene-2-sulfonic acid (4-methoxy-phenyl)-amide [61];
5-Chloro-4-nitro-thiophene-2-sulfonic acid p-tolylamide [63];
5-Chloro-4-nitro-thiophene-2-sulfonic acid benzyl-(4-chloro-phenyl)-amide [65];
5-Chloro-4-nitro-thiophene-2-sulfonic acid benzyl-(4-methoxy-phenyl)-amide [66];
5-Chloro-4-nitro- thiophene-2-sulfonic acid (4-chloro-phenyl)-(3-trifluoromethyl-benzyl)-amide [67];
4-Nitro-thiophene-2-sulfonic acid (4-chloro-phenyl)-amide [69];
5-Chloro-4-nitro-thiophene-2-sulfonic acid (3-trifluoro-methyl-benzyl)-(4-trifluoromethyl-benzyl)-amide [75]; and
N-(1H-Benzoimidazol-2-yl)-4-chloro-3-nitro-benzenesulfonamide [83].

21. Use according to claim 19 wherein the compound of formula I is selected from the following:
5-Chloro-4-nitrothiophene-2-sulfonic acid (4-chlorophenyl)amide [2];
5-Chloro-4-nitrothiophene-2-sulfonic acid (4-fluorophenyl)amide [3];
5-Chloro-4-nitrothiophene-2-sulfonic acid (4-hydroxyphenyl)amide [5];
5-Chloro-4-nitrothiophene-2-sulfonic acid (4-trifluoromethylphenyl)amide [6];
5-Chloro-4-nitrothiophene-2-sulfonic acid (4-fluorophenyl)methylamide [7];
5-Chlorothiophene-2-sulfonic acid (4-trifluoromethylphenyl)amide [9];
5-Bromothiophene-2-sulfonic acid (3,5-bis-trifluoromethylphenyl)amide [18];
5-Benzenesulfonyl-thiophene-2-sulfonic acid (4-trifluoromethylphenyl)amide [23];
4,5-Dibromothiophene-2-sulfonic acid (3-trifluoromethylphenyl)amide [27];
4,5-Dibromothiophene-2-sulfonic acid (3,4-dichlorophenyl)amide [28];
*N*-(3,5-Bis-trifluoromethylphenyl)-4-chloro-3-nitrobenzene-sulfonamide [29];
5-Chlorothiophene-2,4-disulfonic acid bis-[(4-trifluoromethylphenyl)amide] [33];
4-Chloro-3-nitro-N-(4-trifluoromethylphenyl)benzene-sulfonamide [35];
5-Chloro-4-nitrothiophene-2-sulfonic acid (3,5-dichlorophenyl)amide [39];
5-Chloro-4-nitrothiophene-2-sulfonic acid (3,5-difluorophenyl)amide [40];
5-(1-Methyl-5-trifluoromethyl-1*H*-pyrazol-4-yl)-thiophene-2-sulfonic acid (3,5-bistrifluoromethylphenyl)amide [43];
5-Chloro-4-nitrothiophene-2-sulfonic acid (3,5-bis-trifluoromethylphenyl)amide [44];
5-Chloro-4-nitrothiophene-2-sulfonic acid 4-fluorobenzylamide [45];
5-Chloro-4-nitrothiophene-2-sulfonic acid 4-trifluoromethylbenzylamide [46];
5-Chloro-4-nitrothiophene-2-sulfonic acid [2-(1*H*-indol-3-yl)-ethyl]amide [48];
5-Chloro-4-nitrothiophene-2-sulfonic acid [2-(1*H*-indol-3-yl)-1-methylethyl]amide [49];
5-Chloro-4-nitrothiophene-2-sulfonic acid methyl-(4-trifluoromethylphenyl) amide [50];
5-Chloro-4-nitrothiophene-2-sulfonic acid (4-chlorophenyl)methylamide [51]; and
5-Chloro-4-nitrothiophene-2-sulfonic acid methyl (4-trifluoromethylbenzyl)amide [52]
5-Chloro-4-nitro-thiophene-2-sulfonic acid benzyl-(4-fluoro-benzyl)-amide [53];
5-Chloro-4-nitro-thiophene-2-sulfonic acid 3,5-dichloro-benzylamide [54];
5-Chloro-4-nitro-thiophene-2-sulfonic acid 3,5-difluoro-benzylamide [55];
5-Chloro-4-nitro-thiophene-2-sulfonic acid 4-chloro-benzylamide [56];
5-Chloro-4-nitro-thiophene-2-sulfonic acid [1-(4-fluoro-phenyl)-ethyl]-amide [57];
5-Chloro-4-nitro-thiophene-2-sulfonic acid (1H-benzo-imidazol-2-yl)-amide [59];
5-Chloro-4-nitro-thiophene-2-sulfonic acid [2-(6-chloro-1H-indol-3-yl)-ethyl]-amide [60];
5-Chloro-4-nitro-thiophene-2-sulfonic acid (4-methoxy-phenyl)-amide [61];
5-Chloro-4-nitro-thiophene-2-sulfonic acid p-tolylamide [63];
5-Chloro-4-nitro-thiophene-2-sulfonic acid benzyl-(4-chloro-phenyl)-amide [65];
5-Chloro-4-nitro-thiophene-2-sulfonic acid benzyl-(4-methoxy-phenyl)-amide [66];
5-Chloro-4-nitro-thiophene-2-sulfonic acid (4-chloro-phenyl)-(3-trifluoromethyl-benzyl)-amide [67];
4-Nitro-thiophene-2-sulfonic acid (4-chloro-phenyl)-amide [69];
5-Chloro-thiophene-2,4-disulfonic acid bis-[(4-chloro-phenyl)-amide] [70];
Thioacetic acid S-[5-(4-chloro-phenylsulfamoyl)-3-nitro-thiophen-2-yl] ester [72];
5-Methyl-4-nitro-thiophene-2-sulfonic acid (4-chloro-phenyl)-amide [73];
5-Methyl-thiophene-2,4-disulfonic acid bis-[(4-chloro-phenyl)-amide] [74];
5-Chloro-4-nitro-thiophene-2-sulfonic acid (3-trifluoro-methyl-benzyl)-(4-trifluoromethylbenzyl)-amide [75];
5-(2-Methoxy-ethylamino)-4-nitro-thiophene-2-sulfonic acid (4-chloro-phenyl)-amide [80];
N-(1H-Benzoimidazol-2-yl)-4-chloro-3-nitro-benzenesulfonamide [83];
5-Chloro-3-methyl-benzo[b]thiophene-2-sulfonic acid (4-chloro-3-nitro-phenyl)-amide [88];
N-(3,5-Bis-trifluoromethyl-phenyl)-3-chloro-4-methoxy-benzene-sulfonamide [104];
N-(3-Chloro-4-nitro-phenyl)-3,5-bis-trifluoromethyl-benzenesulfonamide [105];
4,5-Dibromo-thiophene-2-sulfonic acid (3,S-dichloro-phenyl)-amide [112];
5-Bromo-thiophene-2-sulfonic acid (4-trifluoromethyl-phenyl)-amide [116];
5-Benzenesulfonyl-thiophene-2-sulfonic acid (3,5-bis-trifluoromethyl-phenyl)-amide [117];
5-Chloro-3-methyl-benzo[b]thiophene-2-sulfonic acid (3,5-bis-trifluoromethyl-phenyl)-amide [119];
Benzo[b]thiophene-2-sulfonic acid (3,5-bis-trifluoromethyl-phenyl)-amide [120];
Benzo[1,2,5]thiadiazole-5-sulfonic acid (3,5-bis-trifluoromethyl-phenyl)-amide [122];
4,5-Dibromo-thiophene-2-sulfonic acid (4-chloro-phenyl)-amide [125];
4,5-Dibromo-thiophene-2-sulfonic acid (4-trifluoromethyl-phenyl)-amide [126];
N-(3,5-Bis-trifluoromethyl-phenyl)-2,6-dichloro-benzenesulfonamide [128];
5-(3-Hydroxy-piperidin-1-yl)-4-nitro-thiophene-2-sulfonic acid (4-chloro-phenyl)-amide [130]; and
5-Chloro-4-nitro-thiophene-2-sulfonic acid (4-nitro-phenyl)-amide [131].

22. Use according to any preceding claim wherein the proliferative disorder is cancer.

23. Use according to any preceding claim wherein said compound is administered in an amount sufficient to inhibit the interaction between HDM2 and p53 and/or HDM2 and E2F transcription factors.

24. A compound of formula **Ic**, 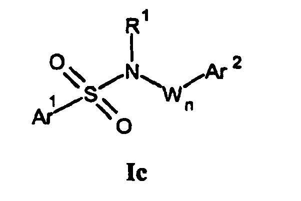 wherein
W is a C₁₋₅ branched or unbranched alkylene group or a C₂₋₅ alkenylene group;
n is 0 or 1;
R¹ is H, a C₁₋₈ branched or unbranched alkyl group, a C₂₋₈ alkenyl group, or an aryl or aralkyl group, each of which may be optionally substituted by one or more halogen or CF₃ groups;
Ar¹ is 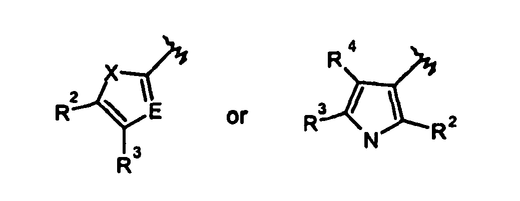 wherein
X is O, NH or NR' where R' is a C₁₋₃ alkyl group;
E is N or CR⁴;
Y is N;
R², R³ and R⁴ are each independently (A)ₚB, wherein A is C₁₋₃ alkyl, p is 0 or 1, and B is H, halogen, C₁₋₅ alkyl, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, an alicyclic group optionally containing one or more heteroatoms, optionally substituted by one or more OH, COR^{u}, halogen or CF₃ groups, or a heteroaryl group optionally substituted by one or more C₁₋₅ alkyl, halogen, SRⁱ or CF₃ groups; or
R² and R³ are linked to form a saturated or unsaturated ring system, optionally containing one or more heteroatoms, and optionally substituted by one or more halogen, OH or CF₃ groups;
Ar^{a-f} are each independently aryl groups optionally substituted by one or more C₁₋₅ alkyl, halogen or CF₃ groups;
R^{a-i}, R^{s}, R^{t} and R^{u} are each independently C₁₋₅ alkyl groups optionally substituted by one or more alkoxy, halogen or CF3 groups;
and with the proviso that at least one of R², R³ and R⁴ is other than H;
Ar² is 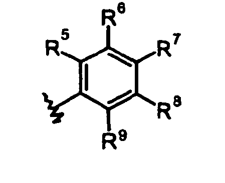 wherein
Z is S, O, NH or NR" where R" is C₁₋₃ alkyl;
R⁵, R⁶, R⁷, R⁸ and R⁹ are each independently (L)_{q}M wherein L is C₁₋₃ alkyl, q is 0 or 1, M is H, C₁₋₅ alkyl, halogen, NO₂, OH, NH₂, NHAr^{g}, NHR^{j}, NR^{k}R^{l}, SO₃H, SO₂NH₂, SO₂NHAr^{h}, SO₂NHR^{m}, SO₂Arⁱ, SO₂Rⁿ, CF₃, CN, COOH, COOR^{p}, CONH₂, CONHAr^{j}, CONHR^{q}, OR^{v}, COAr^{k} or COR^{r};
R^{j-r}, R^{v} are each independently C₁₋₅ alkyl groups;
Ar^{g-k} are each independently aryl groups;
and with the proviso that at least one of the substituents R⁵, R⁶, R⁷, R⁸ and R⁹ is other than H;
with the proviso that said compound is other than
5-[(4-chlorophenyl)amino]sulfonyl-2-furancarboxylic acid.

25. A compound of formula Id 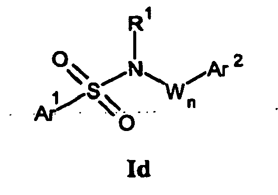 wherein
W is a C₁₋₅ branched or unbranched alkylene group or a C₂₋₅ alkenylene group;
n is 0 or 1;
R¹ is H, a C₁₋₈ branched or unbranched alkyl group, a C₂₋₈ alkenyl group, or an aryl or aralkyl group, each of which may be optionally substituted by one or more halogen or CF₃ groups;
Ar¹ is 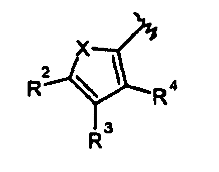
wherein X is O, NH or NR' where R' is a C₁₋₃ alkyl group;
R², R³ and R⁴ are each independently (A)ₚB, wherein A is C₁₋₃ alkyl, p is 0 or 1, and B is H, halogen, C₁₋₅ alkyl, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, an alicyclic group optionally containing one or more heteroatoms, optionally substituted by one or more OH, COR^{u}, halogen or CF₃ groups, or a heteroaryl group optionally substituted by one or more C₁₋₅ alkyl, halogen, SRⁱ or CF₃ groups; or
R² and R³ are linked to form a saturated or unsaturated ring system, optionally containing one or more heteroatoms, and optionally substituted by one or more halogen, OH or CF₃ groups;
Ar^{a-f} are each independently aryl groups optionally substituted by one or more C₁₋₅ alkyl, halogen or CF₃ groups;
R^{a-i}, R^{s}, R^{t} and R^{u} are each independently C₁₋₅ alkyl groups optionally substituted by one or more alkoxy, halogen or CF3 groups;
and with the proviso that at least one of R², R³ and R⁴ is other than H;
and Ar² is 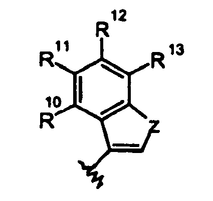
wherein Z is S, O, NH or NR" where R" is C₁₋₃ alkyl; and
R¹⁰, R¹¹, R¹² and R¹³ are each independently H, C₁₋₅ alkyl, halogen, NO₂, OH, NH₂ or CF₃.

26. A compound of formula Ie 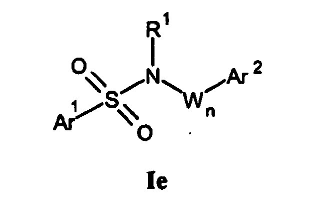 wherein
W is a C₁₋₅ branched or unbranched alkylene group or a C₂₋₅ alkenylene group;
n is 0 or 1;
R¹ is H, a C₁₋₈ branched or unbranched alkyl group, a C₂₋₈ alkenyl group, or an aryl or aralkyl group, each of which may be optionally substituted by one or more halogen or CF₃ groups;
Ar¹ is 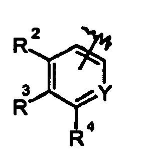 wherein Y is N;
R², R³ and R⁴ are each independently (A)ₚB, wherein A is C₁₋₃ alkyl, p is 0 or 1, and B is H, halogen, C₁₋₅ alkyl, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, an alicyclic group optionally containing one or more heteroatoms, optionally substituted by one or more OH, COR^{u}, halogen or CF₃ groups, or a heteroaryl group optionally substituted by one or more C₁₋₅ alkyl, halogen, SRⁱ or CF₃ groups; or
R² and R³ are linked to form a saturated or unsaturated ring system, optionally containing one or more heteroatoms, and optionally substituted by one or more halogen, OH or CF₃ groups;
Ar^{a-f} are each independently aryl groups optionally substituted by one or more C₁₋₅ alkyl, halogen or CF₃ groups;
R^{a-i}, R^{s}, R^{t} and R^{u} are each independently C₁₋₅ alkyl groups optionally substituted by one or more alkoxy, halogen or CF3 groups;
and with the proviso that at least one of R², R³ and R⁴ is other than H;
and Ar² is 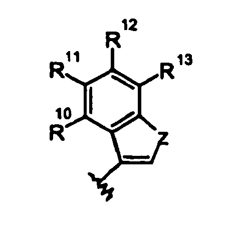
wherein Z is S, O, NH or NR" where R" is C₁₋₃ alkyl; and
R¹⁰, R¹¹, R¹² and R¹³ are each independently H, C₁₋₅ alkyl, halogen, NO₂, OH, NH₂ or CF₃.

27. A compound of formula If 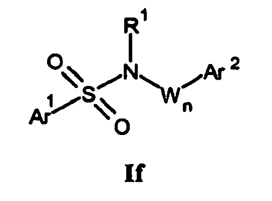 wherein
W is a C₁₋₅ branched or unbranched alkylene group or a C₂₋₅ alkenylene group;
n is 0 or 1;
R¹ is H, a C₁₋₈ branched or unbranched alkyl group, a C₂₋₈ alkenyl group, or an aryl or aralkyl group, each of which may be optionally substituted by one or more halogen or CF₃ groups;
Ar¹ is 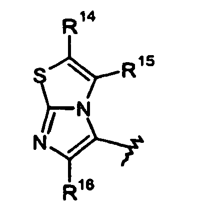
wherein R¹⁴, R¹⁵ and R¹⁶ are each independently (A)ₚB, wherein A is C₁₋₃ alkyl, p is 0 or 1, and B is H, halogen, C₁₋₅ alkyl, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, an alicyclic group optionally containing one or more heteroatoms, optionally substituted by one or more OH, COR^{u}, halogen or CF₃ groups, or a heteroaryl group optionally substituted by one or more C₁₋₅ alkyl, halogen, SRⁱ or CF₃ groups; or
R² and R³ are linked to form a saturated or unsaturated ring system, optionally containing one or more heteroatoms, and optionally substituted by one or more halogen, OH or CF₃ groups;
Ar^{a-f} are each independently aryl groups optionally substituted by one or more C₁₋₅ alkyl, halogen or CF₃ groups;
R^{a-i}, R^{s}, R^{t} and R^{u} are each independently C₁₋₅ alkyl groups optionally substituted by one or more alkoxy, halogen or CF3 groups;
and Ar² is 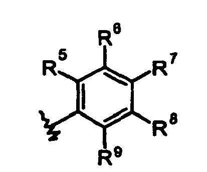
wherein R⁵, R⁶, R⁷, R⁸ and R⁹ are each independently (L)_{q}M wherein L is C₁₋₃ alkyl, q is 0 or 1, M is H, C₁₋₅ alkyl, halogen, NO₂, OH, NH₂, NHAr^{g}, NHR^{j}, NR^{k}R^{l}, SO₃H, SO₂NH₂, SO₂NHAr^{h}, SO₂NHR^{m}, SO₂Arⁱ, SO₂Rⁿ, CF₃, CN, COOH, COOR^{p}, CONH₂, CONHAr^{j}, CONHR^{q}, OR^{v}, COAr^{k} or COR^{r};
R^{j-r}, R^{v} are each independently C₁₋₅ alkyl groups;
Ar^{g-k} are each independently aryl groups;
and with the proviso that at least one of the substituents R⁵, R⁶, R⁷, R⁸ and R⁹ is other than H;

28. A compound of formula Ig 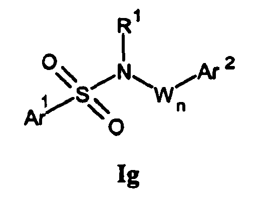 wherein
W is a C₁₋₅ branched or unbranched alkylene group or a C₂₋₅ alkenylene group;
n is 0 or 1;
R¹ is H, a C₁₋₈ branched or unbranched alkyl group, a C₂₋₈ alkenyl group, or an aryl or aralkyl group, each of which may be optionally substituted by one or more halogen or CF₃ groups;
Ar¹ is 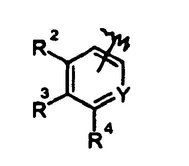 wherein Y is N;
R², R³ and R⁴ are each independently (A)ₚB, wherein A is C₁₋₃ alkyl, p is 0 or 1, and B is H, halogen, C₁₋₅ alkyl, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, an alicyclic group optionally containing one or more heteroatoms, optionally substituted by one or more OH, COR^{u}, halogen or CF₃ groups, or a heteroaryl group optionally substituted by one or more C₁₋₅ alkyl, halogen, SRⁱ or CF₃ groups; or
R² and R³ are linked to form a saturated or unsaturated ring system, optionally containing one or more heteroatoms, and optionally substituted by one or more halogen, OH or CF₃ groups;
Ar^{a-f} are each independently aryl groups optionally substituted by one or more C₁₋₅ alkyl, halogen or CF₃ groups;
R^{a-i}, R^{s}, R^{t} and R^{u} are each independently C₁₋₅ alkyl groups optionally substituted by one or more alkoxy, halogen or CF3 groups;
and with the proviso that at least one of R², R³ and R⁴ is other than H;
and Ar² is 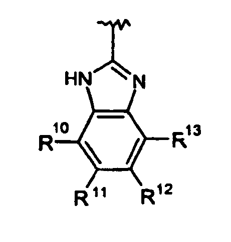
wherein R¹⁰, R¹¹, R¹² and R¹³ are each independently H, C₁₋₅ alkyl, halogen, NO₂, OH, NH₂ or CF₃.

29. A compound of formula Ib, 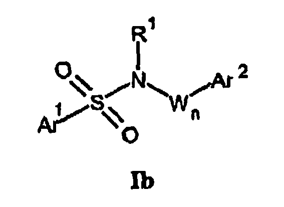 wherein
W is a C₁₋₅ branched or unbranched alkylene group or a C₂₋₅ alkenylene group;
n is 0 or 1;
R¹ is H, a C₁₋₈ branched or unbranched alkyl group, a C₂₋₈ alkenyl group, or an aryl or aralkyl group, each of which may be optionally substituted by one or more halogen or CF₃ groups;
Ar¹ is 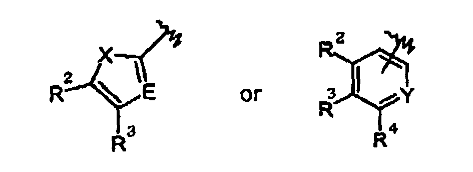 wherein
X is S, O, NH or NR' where R' is a C₁₋₃ alkyl group;
E is N or CR⁴;
Y is CH or N;
R², R³ and R⁴ are each independently (A)ₚB, wherein A is C₁₋₃ alkyl, p is 0 or 1, and B is H, halogen, C₁₋₅ alkyl, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, an alicyclic group optionally containing one or more heteroatoms, optionally substituted by one or more OH, COR^{u}, halogen or CF₃ groups, or a heteroaryl group optionally substituted by one or more C₁₋₅ alkyl, halogen, SRⁱ or CF₃ groups; or
R² and R³ are linked to form a saturated or unsaturated ring system, optionally containing one or more heteroatoms, and optionally substituted by one or more halogen, OH or CF₃ groups;
Ar^{a-f} are each independently aryl groups optionally substituted by one or more C₁₋₅ alkyl, halogen or CF₃ groups;
R^{a-i}, R^{s}, R^{t} and R^{u} are each independently C₁₋₅ alkyl groups optionally substituted by one or more alkoxy, halogen or CF3 groups;
and with the proviso that at least one of R², R³ and R⁴ is other than H;
Ar² is 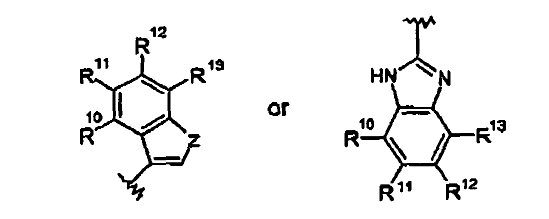 wherein
Z is S, O, NH or NR" where R" is C₁₋₃alkyl;
R¹⁰, R¹¹, R¹² and R¹³ are each independently H, C₁₋₅ alkyl, halogen, NO₂, OH, NH₂ or CF₃;
wherein said compound is other than 5-methoxy-N-methyl-N-(p-toluenesulfonyl-tryptamine, N-methyl-p-toluenesulfonyl-tryptamine N-(p-toluenesulfonyl)-7-methoxy-tryptamine, p-toluenesulfonyl-tryptamine or 3-sulphanilamidoethylindole.

30. A compound according to any one of claims 24 to 29 wherein
- R², R³ and R⁴ are each independently (A)ₚB, wherein A is C₁₋₅alkyl, p is 0 or 1, and B is H, F, Cl, Br, I, C₁₋₅ alkyl, NO₂, OH, NH₂, NHR^{a}, NR^{b}R^{c}, SO₃H, SO₂NH₂, NHPh, SO₂NHAr^{b}, SO₂NHR^{d}, SO₂Ph, SO₂R^{e}, CF₃, CN, COOH, COOR^{f}, CONH₂, COONHPh, CONHR^{g}, S(CO)R^{s}, OR^{t}, OAr^{f}, COPh, COR^{h}, a morpholino, piperazino or piperidino group each of which may be optionally substituted by one or more OH or COR^{u} groups, or a heteroaryl group selected from pyridyl, pyrimidyl, oxazolyl, thiazolyl and pyrazolyl, each of which may be optionally substituted by one or more C₁₋₅ alkyl, halogen, SRⁱ or CF₃ groups, or R² and R³ together form a saturated 6-membered ring or an unsaturated 5-membered ring, each of which optionally contain one or more heteroatoms; and
- R⁵, R⁶, R⁷, R⁸, and R⁹ are each independently (L)_{q}M wherein L is C₁₋₅ alkyl, q is 0 or 1, M is H, C₁₋₅ alkyl, halogen, NO₂, OH, NH₂, NHPh, NHR^{j}, Nk^{k}R^{l}, SO₃H, SO₂NH₂, SO₂NHPh, SO₂NHR^{m}, SO₂Ph, SO₂Rⁿ, CF₃, CN, COOH, COOR^{p}, CONH₂, CONHPh, CONHR^{q}, OR^{v}, COPh or COR^{r}.

31. A compound according to any one of claims 24 to 29 wherein R², R³ and R⁴ are each independently (A)ₚB, wherein A is C₁₋₅ alkyl, p is 0 or 1, and B is H, F, Cl, Br, I, C₁₋₅ alkyl, NO₂, OH, NH₂, NHR^{a}, NR^{b}R^{c}, SO₃H, SO₂NH₂, NHPh, SO₂NHPh, SO₂NHR^{d}, SO₂Ph, SO₂R^{e}, CF₃, CN, COOH, COOR^{f} ; CONH₂, COONHPh, CONHR^{g}, S(CO)R^{s}, OR^{t}, OAr^{f}, COPh, COR^{h}, pyridyl, pyrimidyl, 2-methylsulfanylpyrimid-5-yl, oxazol-2-yl, thiazol-2-yl, 1-methyl-5-trifluoromethyl-1*H*-pyrazol-4-yl, morpholin-4-yl, 4-acetyl-piperazin-1-yl, 3-hydroxy-piperidin-1-yl, or R² and R³ together form
-OCH₂CH₂O-
-N-S-N-
or
a phenyl group optionally substituted by one or more halogens.

32. A compound according to any one of claims 24 to 29 wherein R², R³ and R⁴ are each independently H, halogen, NO₂, SO₂Ph, S(CO)Me, COOH, COOEt, OPh, OMe, NHCH₂CH₂OMe, 1-methyl-5-trifluoromethyl-1*H*-pyrazol-4-yl, 2-methylsulfanyl pyrimid-5-yl, N-(4-fluorophenyl)sulfonamido, N-(4-trifluoromethylphenyl)-sulfonamido, oxazol-2-yl, C₁₋₅ alkyl, NH₂, morpholin-4-yl, 4-acetyl-piperazin-1-yl, 3-hydroxy-piperidin-1-yl, or R² and R³ together form
OCH₂CH₂O-
-N-S-N-
or
a phenyl group optionally substituted by one or more halogens.

33. A compound according to any one of claims 24, 25 or 29-32 wherein Ar¹ is 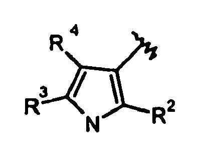
R² and R⁴ are C₁₋₅ alkyl, and R³ is COOH or COOEt.

34. A compound according to any one of claims 26, 28 or 29 to 32 wherein Ar¹ is 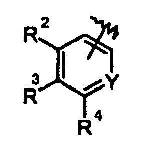
R², R³ and R⁴ are each independently H, OH, COOH, CF₃, OPh, OMe, NO₂, 4-acetyl-piperazin-1-yl, NH₂, halogen, pyrazol-1-yl, oxazol-2-yl or C₁₋₅ alkyl, or R² and R³ together form -OCH₂CH₂O- or -N-S-N-.

35. A compound according to claim 34 wherein
when Y is CH
- R² is H, NO₂ or Cl;
- R³ is NO₂, NH₂, Cl, CF₃, COOH, 4-acetyl-piperazin-1-yl; and
- R⁴ is H, Cl, oxazol-2-yl, OH, NO₂, NH₂, OMe or Me; or
when Y is N
- R² is H;
- R³ is Br;
- R⁴ is Cl or OPh.

36. A compound according to any one of claims 24 to 35 wherein
- R⁵, R⁶, R⁷, R⁸ and R⁹ are each independently H, halogen, OMe, NO₂, C₁₋₅ alkyl, CF₃ or OH; and
- R¹⁰, R¹¹, R¹² and R¹³ are all H.

37. A compound according to claim 36 wherein
- R⁵ is H, C₁₋₅ alkyl, or halogen;
- R⁶ is H, halogen, NO₂, or CF₃;
- R⁷ is H, halogen, OMe, NO₂, OH or CF₃;
- R⁸ is H, halogen or CF₃;
- R⁹ is H.

38. A compound according to any one of claims 24 to 37 wherein
- W is CH₂, CH₂CH₂ or CH(CH₃)CH₂; and
- R¹ is H, CH₂Ph, CH₂CH(Me)₂, 3-(trifluoromethyl)benzyl, or Me.

39. A compound selected from the following:
5-Chloro-4-nitrothiophene-2-sulfonic acid (3-trifluoromethylphenyl)amide [1];
5-Chloro-4-nitrothiophene-2-sulfonic acid (4-chlorophenyl)-amide [2];
5-Chloro-4-nitrothiophene-2-sulfonic acid (4-fluorophenyl)amide [3];
4-Bromo-5-chlorothiophene-2-sulfonic acid (4-fluorophenyl)amide [4];
5-Chloro-4-nitrothiophene-2-sulfonic acid (4-hydroxyphenyl)amide [5];
5-Chloro-4-nitrothiophene-2-sulfonic acid (4-trifluoromethylphenyl)amide [6];
5-Chloro-4-nitrothiophene-2-sulfonic acid (4-fluorophenyl)methylamide [7];
4,5-Dibromothiophene-2-sulfonic acid (3,5-bis-trifluoromethylphenyl)amide [8];
5-Chlorothiophene-2-sulfonic acid (3,5-bis-trifluoromethylphenyl)amide [11];
5-(2-Methylsulfanyl-pyrimidin-5-yl)-thiophene-2-sulfonic acid (3,5-dichlorophenyl)-amide [12];
4-Oxazol-2-yl-N-(4-trifluoromethylphenyl)benzenesulfonamide [13];
N-(3,5-Bis-trifluoromethylphenyl)-4-oxazol-2-yl-benzenesulfonamide [14];
4-Bromo-5-chlorothiophene-2-sulfonic acid (4-trifluoromethylphenyl)amide [15];
5-Bromothiophene-2-sulfonic acid (4-chlorophenyl)amide [16];
5-Bromothiophene-2-sulfonic acid (3,5-dichlorophenyl)amide [17];
5-Bromothiophene-2-sulfonic acid (3,5-bis-trifluoromethylphenyl)amide [18];
3-Nitro-N-(4-trifluoromethylphenyl)benzenesulfonamide [20];
N-(3,5-Bis-trifluoromethylphenyl)-3-nitrobenzenesulfonamide [21];
5-Benzenesulfonylthiophene-2-sulfonic acid (4-trifluoromethylphenyl)-amide [23];
5-Benzenesulfonylthiophene-2-sulfonic acid (4-chlorophenyl)amide [24];
5-Benzenesulfonylthiophene-2-sulfonic acid (3,5-dichlorophenyl)amide [25];
5-Chlorothiophene-2-sulfonic acid (3,4-dichlorophenyl)amide [26];
4,5-Dibromothiophene-2-sulfonic acid (3-trifluoromethylphenyl)amide [27];
4,5-Dibromothiophene-2-sulfonic acid (3,4-dichlorophenyl)amide [28];
5-(1-Methyl-5-trifluoromethyl-1H-pyrazol-4-yl)-thiophene-2-sulfonic acid (4-trifluoromethylphenyl)-amide [31];
5-Chlorothiophene-2,4-disulfonic acid bis-[(4-fluorophenyl)-amide] [32];
5-Chlorothiophene-2,4-disulfonic acid bis-[(4-trifluoro-methyl-phenyl)-amide] [33];
4-Methyl-3-nitro-N-(4-trifluoromethylphenyl)benzenesulfonamide [34];
4-Chloro-3-nitro-N-(4-trifluoromethylphenyl)benzenesulfonamide [35];
3-Amino-4-methyl-N-(4-trifluoromethyl-phenyl)benzenesulfonamide [36];
N-(4-Chlorophenyl)-4-methyl-3-nitrobenzenesulfonamide [37];
4-Chloro-N-(4-chlorophenyl)-3-nitro-benzenesulfonamide [38];
5-Chloro-4-nitrothiophene-2-sulfonic acid (3,5-dichlorophenyl)-amide [39];
5-Chloro-4-nitrothiophene-2-sulfonic acid (3,5-difluorophenyl)-amide [40];
5-Bromo-6-chloropyridine-3-sulfonic acid (4-trifluoromethylphenyl)amide [41];
5-Bromo-6-chloropyridine-3-sulfonic acid (3,5-bis-trifluoromethylphenyl)amide [42];
5-(1-Methyl-5-trifluoromethyl-1H-pyrazol-4-yl)-thiophene-2-sulfonic acid (3,5-bistrifluoromethylphenyl)amide [43];
5-Chloro-4-nitrothiophene-2-sulfonic acid (3,5-bis-trifluoromethyl-phenyl)-amide [44];
5-Chloro-4-nitrothiophene-2-sulfonic acid 4-fluorobenzylamide [45];
5-Chloro-4-nitrothiophene-2-sulfonic acid 4-trifluoromethylbenzylamide [46];
4-Chloro-N-(3,5-dichlorophenyl)-3-nitro-benzenesulfonamide [41];
5-Chloro-4-nitrothiophene-2-sulfonic acid [2-(1H-indol-3-yl)-ethyl]amide [48];
5-Chloro-4-nitrothiophene-2-sulfonic acid [2-(1H-indol-3-yl)-1-methylethyl]amide [49];
5-Chloro-4-nitrothiophene-2-sulfonic acid methyl-(4-trifluoromethylphenyl)amide [50];
5-Chloro-4-nitrothiophene-2-sulfonic acid (4-chloro-phenyl)-methylamide [51]; and
5-Chloro-4-nitrothiophene-2-sulfonic acid methyl-(4-trifluoromethylbenzyl)amide [52];
5-Chloro-4-nitro-thiophene-2-sulfonic acid benzyl-(4-fluoro-benzyl)-amide [53];
5-Chloro-4-nitro-thiophene-2-sulfonic acid 3,5-dichloro-benzylamide [54];
5-Chloro-4-nitro-thiophene-2-sulfonic acid 3,5-difluoro-benzylamide [55];
5-Chloro-4-nitro-thiophene-2-sulfonic acid 4-chloro-benzylamide [56];
5-Chloro-4-nitro-thiophene-2-sulfonic acid [1-(4-fluoro-phenyl)-ethyl]-amide [57];
5-Chloro-4-nitro-thiophene-2-sulfonic acid (4-chloro-phenyl)-isobutyl-amide [58];
5-Chloro-4-nitro-thiophene-2-sulfonic acid (1H-benzo-imidazol-2-yl)-amide [59];
5-Chloro-4-nitro-thiophene-2-sulfonic acid [2-(6-chloro-1H-indol-3-yl)-ethyl]-amide [60];
5-Chloro-4-nitro-thiophene-2-sulfonic acid (4-methoxy-phenyl)-amide [61];
5-Chloro-4-nitro-thiophene-2-sulfonic acid phenylamide [62];
5-Chloro-4-nitro-thiophene-2-sulfonic acid p-tolylamide [63];
5-Chloro-4-nitro-thiophene-2-sulfonic acid benzylamide [64];
5-Chloro-4-nitro-thiophene-2-sulfonic acid benzyl-(4-chloro-phenyl)-amide [65];
5-Chloro-4-nitro-thiophene-2-sulfonic acid benzyl-(4-methoxy-phenyl)-amide [66];
5-Chloro-4-nitro-thiophene-2-sulfonic acid (4-chloro-phenyl)-(3-trifluoromethyl-benzyl)-amide [67];
5-Nitro-thiophene-2-sulfonic acid (4-chloro-phenyl)-amide [68];
4-Nitro-thiophene-2-sulfonic acid (4-chloro-phenyl)-amide [69];
5-Chloro-thiophene-2,4-disulfonic acid bis-[(4-chloro-phenyl)amide] [70];
5-Ethyl-4-nitro-thiophene-2-sulfonic acid (4-chloro-phenyl)-amide [71];
Thioacetic acid S-[5-(4-chloro-phenylsulfamoyl)-3-nitro-thiophen-2-yl]ester[72];
5-Methyl-4-nitro-thiophene-2-sulfonic acid (4-chloro-phenyl)-amide [73];
5-Methyl-thiophene-2,4-disulfonic acid bis-[(4-chloro-phenyl)-amide] [74];
5-Chloro-4-nitro-thiophene-2-sulfonic acid (3-trifluoro-methyl-benzyl)-(4-trifluoromethylbenzyl)-amide [75];
4-Nitro-thiophene-2-sulfonic acid (4-trifluoromethyl-phenyl)-amide [76];
4-Nitro-thiophene-2-sulfonic acid [2-(1H-indol-3-yl)-ethyl]-amide [77];
5-(1-Methyl-5-tri-fluoromethyl-1H-pyrazol-3-yl)-thiophene-2-sulfonic acid (3,5-bis-trifluoromethyl-phenyl)-(3-trifluoro-methyl-benzyl)-amide [78];
5-Morpholin-4-yl-4-nitro-thiophene-2-sulfonic acid (4-chloro-phenyl)-amide [79];
5-(2-Methoxy-ethylamino)-4-nitro-thiophene-2-sulfonic acid (4-chloro-phenyl)-amide [80];
4-Chloro-N-[2-(5-chloro-1H-indol-3-yl)-ethyl]-3-nitro-benzenesulfonamide [81];
N-[2-(5-Chloro-1H-indol-3-yl)-ethyl]-4-methyl-3-nitro-benzenesulfonamide [82];
N-(1H-Benzoimidazol-2-yl)-4-chloro-3-nitro-benzenesulfonamide [83];
6-Chloro-imidazo[2,1-b]thiazole-5-sulfonic acid (3,5-bis-trifluoro-methyl-phenyl)-amide [84];
2,3-Dihydro-benzo[1,4]dioxine-b-sulfonic acid (4-chloro-phenyl)-amide [85];
2,3-Dihydro-benzo[1,4]dioxine-6-sulfonic acid (3,5-bis-trifluoromethyl-phenyl)-amide [86];
6-Phenoxy-pyridine-3-sulfonic acid (4-chlom-phenyl)-amide [87];
5-Chloro-3-methyl-benzo[b]thiophene-2-sulfonic acid (4-chloro-3-nitro-phenyl)-amide [88];
N-(3,5-Bis-trifluoromethyl-phenyl)-4-pyrazol-1-yl-benzenesulfonamide [89];
4-(4-Chloro-phenylsulfamoyl)-3,5-dimethyl-1H-pyrrole-2-carboxylic acid ethyl ester [90];
4-(3,5-Bis-trifluoromethyl-phenylsulfamoyl)-3,5-dimethyl-1H-pyrrole-2-carboxylic acid [91];
4-(4-Chloro-phenylsulfamoyl)-3,5-dimethyl-1H-pyrrole-2-carboxylic acid [92];
2-(4-Chloro-phenylsulfamoyl)-4-methyl-thiazole-5-carboxylic acid ethyl ester [93];
3,5-Dichloro-N-(4-chloro-phenyl)-4-hydroxy-benzenesulfonamide [94];
N-(3,5-Bis-trifluoromethyl-phenyl)-3,5-dichloro-4-hydroxy-benzenesulfonamide [95];
3,5-Dichloro-4-hydroxy-N-(4-trifluoromethyl-phenyl)-benzenesulfonamide [96];
N-(3,5-Bis-trifluoromethyl-phenyl)-4-nitro-benzenesulfomide [96];
4-Amino-N-(3,5-bis-trifluoromethyl-phenyl)-3-chloro-benzenesulfonwnide [99];
3-Nitro-N-(4-trifluoromethyl-phenyl)-benzenesulfonamide [100];
3,5-Dichloro-N-(3,5-dichloro-phenyl)-4-hydroxy-benzenesulfonamide [101];
4-Amino-3-chloro-N-(4-chloro-phenyl)-benzenesulfonamide [102];
3-Chloro-N-(4-chloro-phenyl)-4-methoxy-benzenesulfonamide [103];
N-(3,5-Bis-trifluoromethyl-phenyl)-3-chloro-4-methoxy-benzene-sulfonamide [104];
N-(3-Chloro-4-nitro-phenyl)-3,5-bis-trifluoromethyl-benzenesulfonamide [105];
3-(4-Acetyl-piperazin-1-yl)-N-(3,5-bis-trifluoromethyl-phenyl)-4-nitrobenzenesulfonamide [106];
N-(3,5-Bis-trifluoromethyl-phenyl)-2-nitro-benzenesulfonamide [107];
3-(3,5-Bis-trifluoromethyl-phenylsulfamoyl)-benzoic acid [108];
3,5-Dichloro-N-(4-chloro-benzyl)-4-hydroxy-benzenesulfonamide [109];
3,5-Dichloro-4-hydroxy-N-(4-trifluoromethyl-benzyl)-benzenesulfonamide [110];
3,5-Dichloro-4-hydroxy-N-[2-(1H-indol-3-yl)-ethyl]-benzenesulfonamide [111];
4,5-Dibromo-thiophene-2-sulfonic acid (3,5-dichloro-phenyl)-amide [112];
N-(3,5-Dichloro-phenyl)-4-oxazol-2-yl-benzenesulfonamide [113];
4-Bromo-5-chloro-thiophene-2-sulfonic acid (3,5-bis-trifluoromethyl-phenyl)-amide [114];
4-Bromo-5-chloro-thiophene-2-sulfonic acid (3,5-dichloro-phenyl)-amide [115];
5-Benzenesulfonyl-thiophene-2-sulfonic acid (3,5-bis-trifluoromethyl-phenyl)-amide [117];
5-Benzenesulfonyl-thiophene-2-sulfonic acid (2,4-dichloro-phenyl)-amide [118];
5-Chloro-3-methyl-benzo[b]thiophene-2-sulfonic acid (3,5-bis-trifluoromethyl-phenyl)-amide [119];
Benzo[b]thiophene-2-sulfonic acid (3,5-bis-trifluoromethyl-phenyl)-amide [120];
Benzo[1,2,5]thiadiazole-5-sulfonic acid (4-chloro-phenyl)-amide [121];
Benzo[1,2,5]thiadiazole-5-sulfonic acid (3,5-bis-trifluoromethyl-phenyl)-amide [122];
Benzo[1,2,5]thiadiazole-5-sulfonic acid (4-trifluoromethyl-phenyl)-aroide [123];
5-Pyridin-2-yl-thiophene-2-sulfonic acid (3,5-bis-trifluoromethyl-phenyl)-amide [124];
4,5-Dibromo-thiophene-2-sulfonic acid (4-chloro-phenyl)-amide [125];
4,5-Dibromo-thiophene-2-sulfonic acid (4-trifluoromethyl-phenyl)-amide [126];
3,5-Dichloro-N-(4-fluoro-benzyl)-4-hydroxy-benzenesulfonamide [127];
N-(3,5-Bis-trifluoromethyl-phenyl)-2,6-dichloro-benzenesulfonamide [128];
5-Chloro-4-nitro-thiophene-2-sulfonic acid (4-methoxy-2-methyl-phenyl)-amide [129];
5-(3-Hydroxy-piperidin-1-yl)-4-nitro-thiophene-2-sulfonic acid (4-chloro-phenyl)-amide [130]; and
5-Chloro-4-nitro-thiophene-2-sulfonic acid (4-nitro-phenyl)-amide [131].

40. A compound according to claim 39 which is selected from the following:
5-Chloro-4-nitrothiophene-2-sulfonic acid (4-chlorophenyl)amide [2];
5-Chloro-4-nitrothiophene-2-sulfonic acid (4-fluorophenyl)amide [3];
5-Chloro-4-nitrothiophene-2-sulfonic acid (4-hydroxyphenyl)amide [5];
5-Chloro-4-nitrothiophene-2-sulfonic acid (4-trifluoromethylphenyl)amide [6];
5-Chloro-4-nitrothiophene-2-sulfonic acid (4-fluorophenyl)methylamide [7];
4-Chloro-3-nitro-N-(4-trifluoromethylphenyl)-benzene-sulfonamide [35];
4-Chloro-N-(4-chlorophenyl)-3-nitrobenzene-sulfonamide [38];
5-Chloro-4-nitrothiophene-2-sulfonic acid (3,5-dichlorophenyl)amide [39];
5-Chloro-4-nitrothiophene-2-sulfonic acid (3,5-difluorophenyl)amide [40];
5-(1-Methyl-5-trifluoromethyl-1H-pyrazol-4-yl)-thiophene-2-sulfonic acid (3,5-bistrifluoromethylphenyl)amide [43];
5-Chloro-4-nitrothiophene-2-sulfonic acid (3,5-bis-trifluoromethyl-phenyl)-amide [44];
5-Chloro-4-nitrothiophene-2-sulfonic acid 4-fluorobenzylamide [45];
5-Chloro-4-nitrothiophene-2-sulfonic acid 4-trifluoromethylbenzylamide [46];
5-Chloro-4-nitrothiophene-2-sulfonic acid [2-(1H-indol-3-yl)-ethyl]amide [48];
5-Chloro-4-nitrothiophene-2-sulfonic acid [2-(1H-indol-3-yl)-1-methylethyl]amide [49];
5-Chloro-4-nitrothiophene-2-sulfonic acid (4-chlorophenyl)methylamide [51];
5-Chloro-4-nitro-thiophene-2-sulfonic acid benzyl-(4-fluoro-benzyl)-amide [53];
5-Chloro-4-nitro-thiophene-2-sulfonic acid 3,5-dichloro-benzylamide [54];
5-Chloro-4-nitro-thiophene-2-sulfonic acid 3,5-difluoro-benzylamide [55];
5-Chloro-4-nitro-thiophene-2-sulfonic acid 4-chloro-benzylamide [56];
5-Chloro-4-nitro-thiophene-2-sulfonic acid [1-(4-fluoro-phenyl)-ethyl]-amide [57];
5-Chloro-4-nitro-thiophene-2-sulfonic acid (4-methoxy-phenyl)-amide [61];
5-Chloro-4-nitro-thiophene-2-sulfonic acid p-tolylamide [63];
5-Chloro-4-nitro-thiophene-2-sulfonic acid benzylamide [64];
5-Chloro-4-nitro-thiophene-2-sulfonic acid benzyl-(4-chloro-phenyl)-amide [65];
5-Chloro-4-nitro-thiophene-2-sulfonic acid benzyl-(4-methoxy-phenyl)-amide [66];
5-Chloro-4-nitro-thiophene-2-sulfonic acid (4-chloro-phenyl)-(3-trifluoromethyl-benzyl)-amide [67];
4-Nitro-thiophene-2-sulfonic acid (4-chloro-phenyl)-amide [69];
5-Chloro-4-nitro-thiophene-2-sulfonic acid (3-trifluoro-methyl-benzyl)-(4-trifluoromethyl-benzyl)-amide [75]; and
N-(1H-Benzoimidazol-2-yl)-4-chloro-3-nitro-benzenesulfonamide [83].

41. A compound according to claim 39 which is selected from the following:
5-Chloro-4-nitrothiophene-2-sulfonic acid (4-chlorophenyl)amide [2];
5-Chloro-4-nitrothiophene-2-sulfonic acid (4-fluorophenyl)amide [3];
5-Chloro-4-nitrothiophene-2-sulfonic acid (4-hydroxyphenyl)amide [5];
5-Chloro-4-nitrothiophene-2-sulfonic acid (4-trifluoromethylphenyl)amide [6];
5-Chloro-4-nitrothiophene-2-sulfonic acid (4-fluorophenyl)methylamide [7];
5-Bromothiophene-2-sulfonic acid (3,5-bis-trifluoromethylphenyl)amide [18];
5-Benzenesulfonyl-thiophene-2-sulfonic acid (4-trifluoromethylphenyl)amide [23];
4,5-Dibromothiophene-2-sulfonic acid (3-trifluoromethylphenyl)amide [27];
4,5-Dibromothiophene-2-sulfonic acid (3,4-dichlorophenyl)amide [28];
5-Chlorothiophene-2,4-disulfonic acid bis-[(4-trifluoromethylphenyl)amide] [33];
4-Chloro-3-nitro-N-(4-trifluoromethylphenyl)benzene-sulfonamide [35];
5-Chloro-4-nitrothiophene-2-sulfonic acid (3,5-dichlorophenyl)amide [39];
5-Chloro-4-nitrothiophene-2-sulfonic acid (3,5-difluorophenyl)amide [40];
5-(1-Methyl-5-trifluoromethyl-1*H*-pyrazol-4-yl)-thiophene-2-sulfonic acid (3,5-bistrifluoromethylphenyl)amide [43];
5-Chloro-4-nitrothiophene-2-sulfonic acid (3,5-bis-trifluoromethylphenyl)amide [44];
5-Chloro-4-nitrothiophene-2-sulfonic acid 4-fluorobenzylamide [45];
5-Chloro-4-nitrothiophene-2-sulfonic acid 4-trifluoromethylbenzylamide [46];
5-Chloro-4-nitrothiophene-2-sulfonic acid [2-(1*H*-indol-3-yl)-ethyl]amide [48];
5-Chloro-4-nitrothiophene-2-sulfonic acid [2-(1*H*-indol-3-yl)-1-methylethyl]amide [49];
5-Chloro-4-nitrothiophene-2-sulfonic acid methyl-(4-trifluoromethylphenyl) amide [50];
5-Chloro-4-nitrothiophene-2-sulfonic acid (4-chlorophenyl)methylamide [51]; and
5-Chloro-4-nitrothiophene-2-sulfonic acid methyl (4-trifluoromethylbenzyl)amide [52]
5-Chloro-4-nitro-thiophene-2-sulfonic acid benzyl-(4-fluoro-benzyl)-amide [53];
5-Chloro-4-nitro-thiophene-2-sulfonic acid 3,5-dichloro-benzylamide [54];
5-Chloro-4-nitro-thiophene-2-sulfonic acid 3,5-difluoro-benzylamide [55];
5-Chloro-4-nitro-thiophene-2-sulfonic acid 4-chloro-benzylamide [56];
5-Chloro-4-nitro-thiophene-2-sulfonic acid [1-(4-fluoro-phenyl)-ethyl]-amide [57];
5-Chloro-4-nitro-thiophene-2-sulfonic acid (1H-benzo-imidazol-2-yl)-amide [59];
5-Chloro-4-nitro-thiophene-2-sulfonic acid [2-(6-chloro-1H-indol-3-yl)-ethyl]-amide [60];
5-Chloro-4-nitro-thiophene-2-sulfonic acid (4-methoxy-phenyl)-amide [61];
5-Chloro-4-nitro-thiophene-2-sulfonic acid phenylamide [62];
5-Chloro-4-nitro-thiophene-2-sulfonic acid p-tolylamide [63];
5-Chloro-4-nitro-thiophene-2-sulfonic acid benzylamide [64];
5-Chloro-4-nitro-thiophene-2-sulfonic acid benzyl-(4-chloro-phenyl)-amide [65];
5-Chloro-4-nitro-thiophene-2-sulfonic acid benzyl-(4-methoxy-phenyl)-amide [66];
5-Chloro-4-nitro-thiophene-2-sulfonic acid (4-chloro-phenyl)-(3-trifluoromethyl-benzyl)-amide [67];
4-Nitro-thiophene-2-sulfonic acid (4-chloro-phenyl)-amide [69];
5-Chloro-thiophene-2,4-disulfonic acid bis-[(4-chloro-phenyl)-amide] [70];
Thioacetic acid S-[5-(4-chloro-phenylsulfamoyl)-3-nitro-thiophen-2-yl] ester [72];
5-Methyl-4-nitro-thiophene-2-sulfonic acid (4-chloro-phenyl)-amide [73];
5-Methyl-thiophene-2,4-disulfonic acid bis-[(4-chloro-phenyl)-amide] [74];
5-Chloro-4-nitro-thiophene-2-sulfonic acid (3-trifluoro-methyl-benzyl)-(4-trifluoromethylbenzyl)-amide [75];
5-(2-Methoxy-ethylamino)-4-nitro-thiophene-2-sulfonic acid (4-chloro-phenyl)-amide [80];
N-(1H-Benzoimidazol-2-yl)-4-chloro-3-nitro-benzenesulfonamide [83];
5-Chloro-3-methyl-benzo[b]thiophene-2-sulfonic acid (4-chloro-3-nitro-phenyl)-amide [88];
N-(3,5-Bis-trifluoromethyl-phenyl)-3-chloro-4-methoxy-benzene-sulfonamide [104];
N-(3-Chloro-4-nitro-phenyl)-3,5-bis-trifluoromethyl-benzenesulfonamide [105];
4,5-Dibromo-thiophene-2-sulfonic acid (3,-5-dichloro-phenyl)-amide [112];
5-Benzenesulfonyl-thiophene-2-sulfonic acid (3,5-bis-trifluoromethyl-phenyl)-amide [117];
5-Chloro-3-methyl-benzo[b]thiophene-2-sulfonic acid (3,5-bis-trifluoromethyl-phenyl)-amide [119];
Benzo[b]thiophene-2-sulfonic acid (3,5-bis-trifluoromethyl-phenyl)-amide [120];
Benzo[1,2,5]thiadiazole-5-sulfonic acid (3,5-bis-trifluoromethyl-phenyl)-amide [122];
4,5-Dibromo-thiophene-2-sulfonic acid (4-chloro-phenyl)-amide [125];
4,5-Dibromo-thiophene-2-sulfonic acid (4-trifluoromethyl-phenyl)-amide [126];
N-(3,5-Bis-trifluoromethyl-phenyl)-2,6-dichloro-benzenesulfonamide [128];
5-(3-Hydroxy-piperidin-1-yl)-4-nitro-thiophene-2-sulfonic acid (4-chloro-phenyl)-amide [130]; and
5-Chloro-4-nitro-thiophene-2-sulfonic acid (4-nitro-phenyl)-amide [131].

42. A pharmaceutical composition comprising a compound as defined in any one of claims 24 to 41 admixed with one or more pharmaceutically acceptable diluents, excipients or carriers.

43. Use of a compound as defined in any one of claims 1 to 41 in an assay for determining binding to HDM2.

44. Use according to claim 43 wherein said assay is capable of identifying candidate compounds that inhibit the interaction between HDM2 and p53 and/or E2F.

45. Use according to any one of claims 43 or 44 wherein said assay is a competitive binding assay.

46. Use according to claim 45 wherein said competitive binding assay comprises contacting a compound of formula I as defined in any one of claims 1 to 22 with HDM2 in the presence of a p53-derived peptide and detecting any change in the interaction between HDM2 and said p53-derived peptide.

47. Use according to claim 46 wherein said p53-derived peptide is a fluorescently labelled or biotinylated p53-derived peptide.

48. A method of detecting the binding of a ligand to HDM2, said method comprising the steps of:
(i) contacting a ligand with HDM2 in the presence of a p53-derived peptide; and
(ii) detecting any change in the interaction between HDM2 and said p53-derived peptide;
and wherein said ligand is a compound as defined in any one of claims 1 to 22.

49. A combination comprising at least one compound as defined in any one of claims 1 to 41 and at least one cytotoxic agent.

50. A combination according to claim 49 wherein said cytotoxic agent is a chemotherapeutic agent.

51. A combination according to claim 50 wherein said chemotherapeutic agent is cisplatin or etoposide.

52. A pharmaceutical composition comprising at least one compound as defined in any one of claims 1 to 41, and one or more cytotoxic agents, admixed with a pharmaceutically acceptable diluent, excipient or carrier.

53. Use of at least one compound as defined in any one of claims 1 to 41, in the preparation of a medicament for treating a proliferative disorder, wherein said compound is for consecutive, simultaneous or sequential use with one or more other cytotoxic agents.

54. Use of at least one compound as defined in any one of claims 1 to 41, in the preparation of a medicament for treatinga proliferative disorder, wherein said compound is for consecutive, simultaneous or sequential use with radiotherapy.

## Patentansprüche

1. Verwendung einer Verbindung der Formel I 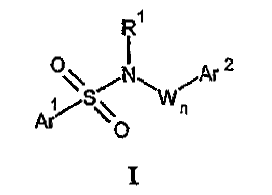 wobei
W eine verzweigte oder unverzweigte C₁₋₅-Alkylengruppe oder eine C₂₋₅-Alkenylengruppe ist,
n 0 oder 1 ist,
R¹ H, eine verzweigte oder unverzweigte C₁₋₈-Alkylgruppe, eine C₂₋₈-Alkenylgruppe oder eine Aryl- oder Aralkylgruppe ist, von denen jede optional mit einer oder mehreren Halogen- oder CF₃-Gruppen substituiert sein kann,
Ar¹ folgendes ist 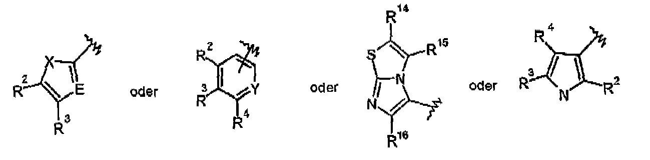 wobei
X S, O, NH oder NR' ist, wobei R' eine C₁₋₃-Alkylgruppe ist,
Y CH oder N ist,
E N oder CR⁴ ist
R², R³, R⁴ und R¹⁴⁻¹⁶ jeweils unabhängig voneinander (A)ₚB sind, wobei A C₁₋₃-Alkyl ist, p 0 oder 1 ist und B H, Halogen, C₁₋₅-Alkyl, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COON-HAr^{d}, CONHR^{g}, COAr^{e}, COR^{h}, S(CO)R^{e}, OR^{t}, OAr^{r}, eine alizyklische Gruppe, die optional ein oder mehrere Heteroatome enthält, optional substituiert mit einer oder mehreren OH-, COR^{u}-, Halogen- oder CF₃-Gruppen, oder eine Heteroarylgruppe, optional substituiert mit einer oder mehreren C₁₋₅-Alkyl-, Halogen-, SR'- oder CF₃-Gruppen, ist oder
R² und R³ miteinander verknüpft sind unter Bildung eines gesättigten oder ungesättigten Ringsystems, weiches optional ein oder mehrere Heteroatome enthält und optional mit einer oder mehreren Halogen-, OH- oder CF₃-Gruppen substituiert ist,
Ar^{a-f} jeweils unabhängig voneinander Arylgruppen, optional substituiert mit einer oder mehreren C₁₋₅-Alkyl-, Halogen- oder CF₃-Gruppen, sind,
R^{a-i}, R^{s}, R^{t} und R^{u} jeweils unabhängig voneinander C₁₋₅-Alkylgruppen, optional substituiert mit einer oder mehreren Alkoxy-, Halogen- oder CF₃-Gruppen, sind
und mit der Maßgabe, daß wenigstens einer von R², R³ und R⁴ etwas anderes als H ist,
Ar² folgendes ist 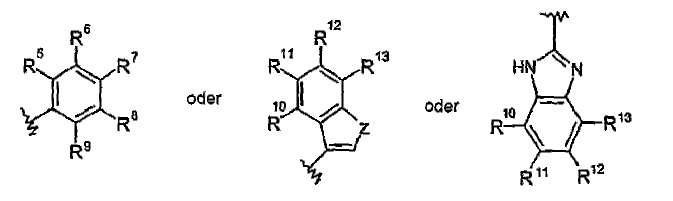 wobei
Z S, O, NH oder NR" ist, wobei R" C₁₋₃-Alkyl ist,
R⁵, R⁶, R⁷, R⁸ und R⁹ jeweils unabhängig voneinander (L)_{q}M sind, wobei L C₁₋₃-Alkyl ist, q 0 oder 1 ist, M H, C₁₋₅-Alkyl, Halogen, NO₂, OH, NH₂, NHAr^{g}, NHR^{j}, NR^{k}R^{l}, SO₃H, SO₂NH₂, SO₂NHArⁿ, SO₂NHR^{m}, SO₂Ar^{j}, SO₂Rⁿ, CF₃, CN, COOH, COOR^{p}, CONH₂, CONHAr^{j}, CONHR^{q}, OR^{v}, COAr^{k} oder COR^{r} ist,
R^{j-r}, R^{v} jeweils unabhängig voneinander C₁₋₅-Alkylgruppen sind,
Ar^{g-k} jeweils unabhängig voneinander Arylgruppen sind,
und mit der Maßgabe, daß wenigstens einer der Substituenten R⁵, R⁶, R⁷, R⁸ und R⁹ etwas anderes als H ist,
R¹⁰, R¹¹, R¹² und R¹³ jeweils unabhängig voneinander H, C₁₋₅-Alkyl, Halogen, NO₂, OH, NH₂ oder CF₃ sind,
bei der Herstellung eines Medikaments zur Behandlung einer proliferativen Störung.

2. Verwendung nach Anspruch 1, wobei Ar¹ folgendes ist 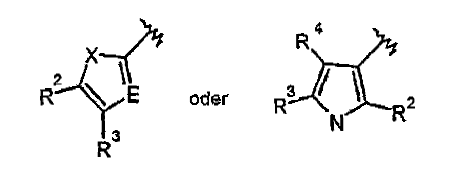 und Ar² folgendes ist

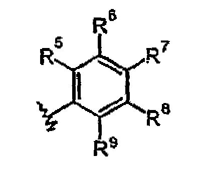

3. Verwendung nach Anspruch 1, wobei Ar¹ folgendes ist 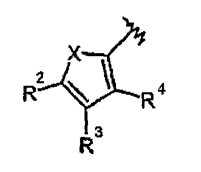 und Ar² folgendes ist

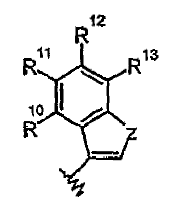

4. Verwendung nach Anspruch 1, wobei Ar¹ folgendes ist 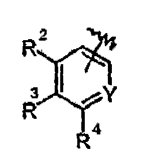 und Ar² folgendes ist

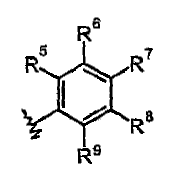

5. Verwendung nach Anspruch 1, wobei Ar¹ folgendes ist 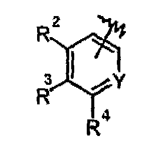 und Ar² folgendes ist

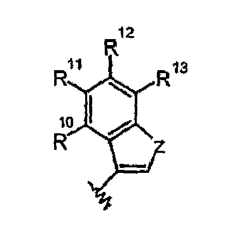

6. Verwendung nach Anspruch 1, wobei Ar¹ folgendes ist 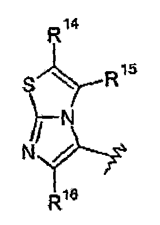 und Ar² folgendes ist

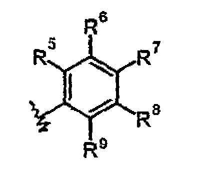

7. Verwendung nach Anspruch 1, wobei Ar¹ folgendes ist 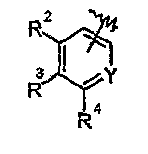 und Ar² folgendes ist

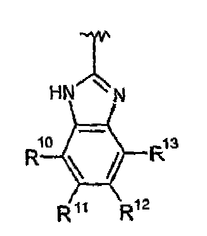

8. Verwendung nach einem der vorangegangenen Ansprüche, wobei
- R², R³ und R⁴ jeweils unabhängig voneinander (A)ₚB sind, wobei A C₁₋₅-Alkyl ist, p 0 oder 1 ist und B H, F, Cl, Br, l, C₁₋₅-Alkyl, NO₂, OH, NH₂, NHR^{a}, NR^{b}R^{c}, SO₃H, SO₂NH₂, NHPh, SO₂NHA^{b}, SO₂NHR^{d}. SO₂Ph, SO₂R^{e}, CF₃, CN, COOH, COOR^{f}, CONH₂, COONHPh, CONHR^{g}, S(CO)R^{s}, OR^{t}, OAr^{f}, COPh, COR^{h}, eine Morpholino-, Piperazino- oder Piperidinogruppe, von denen jede optional mit einer oder mehreren OH- oder COR"-Gruppen substituiert sein kann, oder eine Heteroarylgruppe, ausgewählt unter Pyridyl, Pyrimidyl, Oxazolyl, Thiazolyl und Pyrazolyl, von denen jede optional mit einer oder mehreren C₁₋₅-Alkyl-, Halogen-, SR'- oder CF₃-Gruppen substituiert sein kann, ist, oder R² und R³ zusammen einen gesättigten sechsgliedrigen Ring oder einen ungesättigten fünfgliedrigen Ring bilden, von denen jeder optional ein oder mehrere Heteroatome enthält, und
- R⁵, R⁶, R⁷, R⁸ und R⁹ jeweils unabhängig voneinander (L)_{q}M sind, wobei L C₁₋₆-Alkyl ist, q 0 oder 1 ist, M H, C₁₋₅-Alkyl, Halogen, NO₂, OH, NH₂, NHPh, NHR^{j}, NR^{k}R^{l}, SO₃H, SO₂NH₂, SO₂NHPh, SO₂NHR^{m}, SO₂Ph, SO₂Rⁿ, CF₃, CN, COOH, COOR^{p}, CONH₂, CONHPh, CONHR^{q}, OR^{v}, COPh oder COR^{r} ist.

9. Verwendung nach einem der vorangegangenen Ansprüche, wobei R², R³ und R⁴ jeweils unabhängig voneinander (A)ₚB sind, wobei A C₁₋₅-Alkyl ist, p 0 oder 1 ist und B H, F, Cl, Br, 1, C₁₋₅-Alkyl, NO₂, OH, NH₂, NHR^{a}, NR^{b}R^{c}, SO₃H, SO₂NH₂, NHPh, SO₂NHPh, SO₂NHR^{d}, SO₂Ph, SO₂-R^{e}, CF₃, CN, COOH, COOR^{f}, CONH₂, COOHNPh, CONHR^{g}, S(CO)R^{s}, OR^{t}, OAr^{f}, COPh, COR^{h}, Pyridyl, Pyrimidyl, 2-Methylsulfanylpyrimid-5-yl, Oxazol-2-yl, Thiazol-2-yl, 1-Methyl-5-trifluormethyl-1*H-*pyrazol-4-yl, Morpholin-4-yl, 4-Acetylpiperazin-1-yl, 3-Hydroxypiperidin-1-yl ist oder R² und R³ zusammen
-OCH₂CH₂O-
-N-S-N- oder
eine Phenylgruppe, optional substituiert mit einem oder mehreren Halogenen, bilden.

10. Verwendung nach einem der vorangegangenen Ansprüche, wobei R², R³ und R⁴ jeweils unabhängig voneinander H, Halogen, NO₂, SO₂Ph, S(CO)Me, COOH, COOEt, OPh, OMe, NHCH₂CH₂OMe, 1-Methyl-5-trifluormethyl-1*H*-pyrazol-4-yl, 2-Methylsulfanylpyrimid-5-yl, N-(4-Fluorphenyl)-sulfonamido, N-(4-Trifluormethylphenyl)-sulfonamido, Oxazol-2-yl, C₁₋₅-Alkyl, NH₂, Morpholin-4-yl, 4-Acetylpiperazin-1-yl, 3-Hydroxypiperidin-1-yl sind oder R² und R³ zusammen
OCH₂CH₂O-
-N-S-N- oder
eine Phenylgruppe, optional substituiert mit einem oder mehreren Halogenen, bilden.

11. Verwendung nach einem der Ansprüche 1-3 oder 8-10, wobei Ar¹ folgendes ist 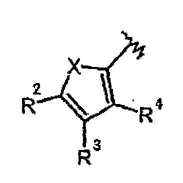
X S oder N ist,
R² R³ und R⁴ jeweils unabhängig voneinander C₁₋₅-Alkyl, S(CO)Me, COOH, NHCH₂CH₂OMe, COOEt, H, Halogen, NO₂, SO₂Ph, SO₂NH-(4-Chlorphenyl), 1-Methyl-5-trifluormethyl-1*H*-pyrazol-4-yl, Morpholin-4-yl, 2-Methylsulfanylpyrimid-5-yl, N-(4-Fluorphenyl)-sulfonamido, N-(4-Trifluormethylphenyl)-sulfonamido, 3-Hydroxypiperidin-1-yl, Pyridin-2-yl sind oder R² und R³ eine Phenylgruppe, optional substituiert mit einem oder mehreren Halogenen, bilden.

12. Verwendung nach Anspruch 11, wobei
- R² Halogen, SO₂Ph, NO₂, Et, SOMe, Morpholin-4-yl, NHCH₂CH₂OMe, 3-Hydroxypiperidin-1-yl, 1-Methyl-5-trifluormethyl-1*H*-pyrazol-4-yl oder 2-Methylsulfanyl-pyrimid-5-yl ist,
- R³ Halogen, SO₂NH-(4-Chlorphenyl), H, NO₂, N-(4-Fluorphenyl)-sulfonamido oder N-(4-Trifluormethylphenyl)-sulfonamido ist, und
- R⁴ H ist.

13. Verwendung nach einem der Ansprüche 1, 4, 5, 7-10, wobei Ar¹ folgendes ist 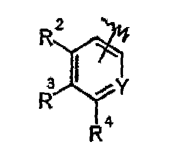
Y CH oder N ist und
R², R³ und R⁴ jeweils unabhängig voneinander H, OH, COOH, CF₃, OPh, OMe, NO₂, 4-Acetyl-piperazin-1-yl, NH₂, Halogen, Pyrazol-1-yl, Oxazol-2-yl oder C₁₋₅-Alkyl sind oder R² und R³ zusammen -OCH₂CH₂O- oder N-S-N- bilden.

14. Verwendung nach Anspruch 13, wobei,
wenn Y CH ist,
- R² H, NO₂ oder Cl ist,
- R³ NO₂, NH₂, Cl, CF₃, COOH, 4-Acetylpiperazin-1-yl ist und
- R⁴ H, Cl, Oxazol-2-yl, OH, NO₂, NH₂, OMe oder Me ist, oder,
wenn Y N ist,
- R² H ist,
- R³ Br ist,
- R⁴ Cl oder OPh ist.

15. Verwendung nach einem der Ansprüche 1, 2 oder 8-10, wobei Ar¹ folgendes ist 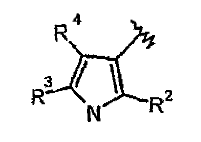
R² und R⁴ C₁₋₅-Alkyl sind und R³ COOH oder COOEt ist.

16. Verwendung nach einem der vorangegangenen Ansprüche, wobei
- R⁵, R⁶, R⁷, R⁸ und R⁹ jeweils unabhängig voneinander H, Halogen, OMe, NO₂, C₁₋₅-Alkyl, CF₃ oder OH sind und
- R¹⁰, R¹¹, R¹² und R¹³ alle H sind.

17. Verwendung nach Anspruch 16, wobei
- R⁵ H, C₁₋₅-Alkyl oder Halogen ist,
- R⁶ H, Halogen, NO₂ oder CF₃ ist,
- R⁷ H, Halogen, OMe, NO₂, OH oder CF₃ ist,
- R⁸ H, Halogen oder CF₃ ist,
- R⁸ H ist.

18. Verwendung nach einem der vorangegangenen Ansprüche, wobei
- W CH₂, CH₂CH₂ oder CH(CH₃)CH₂ ist und
- R¹ H, CH₂Ph, CH₂CH(Me)₂, 3-(Trifluormethyl)-benzyl oder Me ist.

19. Verwendung nach einem der vorangegangenen Ansprüche, wobei die Verbindung der Formel ausgewählt ist unter
5-Chlor-4-nitrothiophen-2-sulfonsäure-(3-trifluormethylphenyl)-amid [1],
5-Chlor-4-nitrothiophen-2-sulfonsäure-(4-chlorphenyl)-amid [2],
5-Chlor-4-nitrothiophen-2-sulfonsäure-(4-fluorphenyl)-amid [3],
4-Brom-5-chlorthiophen-2-sulfonsäure-(4-fluorphenyl)-amid [4],
5-Chlor-4-nitrothiophen-2-sulfonsäure-(4-hydroxyphenyl)-amid [5],
5-Chlor-4-nitrothiophen-2-sulfonsäure-(4-trifluormethylphenyl)-amid [6],
5-Chlor-4-nitrothiophen-2-sulfonsäure-(4-fluorphenyl)-methylamid [7],
4,5-Dibromthiophen-2-sulfonsäure-(3,5-bis-trifluormethylphenyl)-amid [8],
5-Chlorthiophen-2-sulfonsäure-(4-trifluormethylphenyl)-amid [9],
5-Chlorthiophen-2-sulfonsäure-(4-chlorphenyl)-amid [10],
5-Chlorthiophen-2-sulfonsäure-(3,5-bis-trifluormethylphenyl)-amid [11],
5-(2-Methylsulfanylpyrimidin-5-yl)-thiophen-2-sulfonsäure-(3,5-dichlorphenyl)-amid [12],
4-Oxazol-2-yl-N-(4-trifluormethylphenyl)-benzensulfonamid [13],
N-(3,5-Bis-trifluormethylphenyl)-4-oxazol-2-yl-benzensulfonamid [14],
4-Brom-5-chlorthiophen-2-sulfonsäure-(4-trifluormethylphenyl)-amid [15],
5-Bromthiophen-2-sulfonsäure-(4-chlorphenyl)-amid [16],
5-Bromthiophen-2-sulfonsäure-(3,5-dichlorphenyl)-amid [17],
5-Bromthiophen-2-sulfonsäure-(3,5-bis-trifluormethylphenyl)-amid [18],
N-(4-Chlorphenyl)-3-nitrobenzensulfonamid [19],
3-Nitro-N-(4-trifluormethylphenyl)-benzensulfonamid [20],
N-(3,5-Bis-trifluormethylphenyl)-3-nitrobenzensulfonamid [21],
N-(2,4-Dichlorphenyl)-3-nitrobenzensulfonamid [22],
5-Benzensulfonylthiophen-2-sulfonsäure-(4-trifluormethylphenyl)-amid [23],
5-Benzensulfonylthiophen-2-sulfonsäure-(4-chlorphenyl)-amid [24],
5-Benzensulfonylthiophen-2-sulfonsäure-(3,5-dichlorphenyl)-amid [25],
5-Chlorthiophen-2-sulfonsäure-(3,4-dichlorphenyl)-amid [26],
4,5-Dibromthiophen-2-sulfonsäure-(3-trifluormethylphenyl)-amid [27],
4,5-Dibromthiophen-2-sulfonsäure (3,4-dichlorphenyl)-amid [28],
N-(3,5-Bis-trifluormethylphenyl)-4-chlor-3-nitrobenzensulfonamid [29],
4-Chlor-N-(3,4-dichlorphenyl)-3-nitrobenzensulfonamid [30],
5-(1-Methyl-5-trifluormethyl-1*H*-pyrazol-4-yl)-thiophen-2-sulfonsäure-(4-trifluormethylphenyl)-amid [31],
5-Chlorthiophen,2,4-disulfonsäure-bis-[(4-fluorphenyl)-amid] [32],
5-Chlorthiophen-2,4-disulfonsäure-bis-[(4-trifluormethylphenyl)-amid] [33],
4-Methyl-3-nitro-N-(4-trifluormethylphenyl)-benzensulfonamid [34],
4-Chlor-3-nitro-N-(4-trifluormethylphenyl)-behzensulfonamid [35],
3-Amino-4-methyl-N-(4-trifluormethylphenyl)-benzensulfonamid [36],
N-(4-Chlorphenyl)-4-methyl-3-nitrobenzensulfonamid [37],
4-Chlor-N-(4-chlorphenyl)-3-nitrobenzensulfonamid [38],
5-Chlor-4-nitrothiophen-2-sulfonsäure-(3,5-dichlorphenyl)-amid [39],
5-Chlor-4-nitrothiophen-2-sulfonsäure-(3,5-difluorphenyl)-amid [40],
5-Brom-6-chlorpyridin-3-sulfonsäure-(4-trifluormethylphenyl)-amid [41],
5-Brom-6-chlorpyridin-3-sulfonsäure-(3,5-bis-trifluormethylphenyl)-amid [42],
5-(1-Methyl-5-trifluormethyl-1*H*-pyrazol-4-yl)-thiophen-2-sulfonsäure-(3,5-bis-trifluormethylphenyl)-amid [43],
5-Chlor-4-nitrothiophen-2-sulfonsäure-(3,5-bis-trifluormethylphenyl)-amid [44],
5-Chlor -4-nitrothiophen-2 -sulfonsäure-4-fluorbenzylamid [45].
5-Chlor-4-nitrothiophen-2-sulfonsäure-4-trifluormethylbenzylamid [46],
4-Chlor-N-(3,5-dichlorphenyl)-3-nitrobenzensulfonamid [47],
5-Chlor-4-nitrothlophen-2-sulfonsäure-[2-(1*H*-indol-3-yl)-ethyl]-amid [48],
6-Chlor-4-nitrothiophen-2-sulfonsäure-[2-(1*H*-indol-3-yl)-1-methylethyl]-amid [49],
5-Chlor-4-nitrothiophen-2-sulfonsäure-(4-trifluormethylphenyl)-amid [50],
5-Chlor-4-nitrothiophen-2-sulfonsäure-(4-chlorphenyl)-methylamid [51] und
5-Chlor-4-nitrothiophen-2-sulfonsäuremethyl-(4-trifluormethylbenzyl)-amid [52],
5-Chlor-4-nitrothiophen-2-sulfonsäurebenzyl-(4-fluorbenzyl)-amid [53],
5-Chlor-4-nitrothiophen-2-sulfonsäure-3,5-dichlorbenzylamid [54],
5-Chlor-4-nitrothiophen-2-sulfonsäure-3,5-difluorbenzylamid [55],
5-Chlor-4-nitrothiophen-2-sulfonsäure-4-chlorbenzylamid [56],
5-Chlor-4-nitrothiophen-2-sulfonsäure-[1-(4-fluorphenyl)-ethyl]-amid [57],
5-Chlor-4-nitrothiophen-2-sulfonsäure-(4-chlorphenyl)-isobutylamid [58],
5-Chlor-4-nitrothiophen-2-sulfonsäure-(1*H*-benzoimidazol-2-yl)-amid [59],
5-Chlor-4-nitrothiophen-2-sulfonsäure-[2-(6-chlor-1*H*-indol-3-yl)-ethyl]-amid [60],
5-Chlor-4-nitrothiophen-2-sulfonsäure-(4-methoxyphenyl)-amid [61],
5-Chlor-4-nitrothiophen-2-sulfonsäure-p-tolylamid [63],
5-Chlor-4-nitrothiophen-2-sulfonsäurebenzyl-(4-chlorphenyl)-amid [65],
5-Chlor-4-nitrothiophen-2-sulfonsäurebenzyl-(4-methoxyphenyl)-amid [66],
5-Chlor-4-nitrothiophen-2-sulfonsäure-(4-chlorphenyl)-(3-trifluormethylbenzyl)-amid [67],
5-Nitrothiophen-2-sUlfonsäure-(4-chlorphenyl)-amid [68],
4-Nitrothiophen-2-sulfonsäure-(4-chlorphenyl)-amid [69],
5-Chlorthiophen-2,4-disulfonsäure-bis-[(4-chlorphenyl)-amid] [70],
5-Ethyl-4-nitrothiophen-2 -sulfonsäure-(4-chlorphenyl)-amid [71],
Thioessigsäure-S-[5-(4-chlorphenylsulfamoyl)-3-nitrothiophen-2-yl]-ester [72],
5-Methyl-4-nitrothiophen-2-sulfonsäure-(4-chlorphenyl)-amid [73],
5-Methylthiophen-2,4-disulfonsäure-bis-[(4-chlorphenyl)-amid] [74],
5-Chlor-4-nitrothiophen-2-sulfonsäuro-(3-trifluormethylbenzyl)-(4-trifluortnethylbenzyl)-amid [75],
4-Nitrothiophen-2-sulfonsäure-(4-trifluorrnethylphenyl)-amid [76],
4-Nitrothiophen-2-sulfonsäure-[2-(1H-indol-3-yl)-ethyl]-amid [77],
5-(1-Methyl-5-trifluormethyl-1*H*-pyrazol-3-yl)-thiophen-2-sulfonsäure-(3,5-bis-trifluormethylphenyl)-(3-trifluormethylbenzyl)-amid [78],
5-Morpholin-4-yl-4-nitrothiophen-2-sulfonsäure-(4-chlorphenyl)-amid [79],
5-(2-Methoxyethylamino)-4-nitrothiophen-2-sulfonsäure-(4-chlorphenyl)-amid [80],
4-Chlor-N-[2-(5-chlor-1*H*-indol-3-yl)-ethyl]-3-nitrobenzensulfonamid [81],
N-[2-(5-Chlor-1 H-indol-3-yl)-ethyl]-4-methyl-3-nitrobenzensulfonamid [82],
N-(1*H*-Benzoimidazol-2-yl)-4-chlor-3-nitrobenzensulfonamid [83],
6-Chlorimidazo[2,1,b]thiazol-5-sulfonsäure-(3,5-bis-trifluormethylphenyl)amid [84],
2,3-Dihydrobenzo[1,4]dioxin-6-sulfonsäure-(4-chlorphenyl)-amid [85],
2,3-Dihydrobenzo[1,4]dioxin-6-sulfonsäure-(3,5-bis-trifluormethylphenyl)-amid [86],
6-Phenoxypyridin-3-sulfonsäure-(4-chlorphenyl)-amid [87],
5-Chlor-3-methylbenzo[b]thiophen-2-sulfonsäure-(4-chlor-3-nitrophenyl)-amid [88],
N-(3,5-Bis-trifluormethylphenyl)-4-pyrazol-1-yl-benzensulfonamid [89],
4-(4-Chlorphenylsulfamoyl)-3,5-dimethyl-1*H*-pyrrol-2-carbonsäureethylester [90],
4-(3,5-Bis-trifluormethylphenylsulfamoyl)-3,5-dimethyl-1*H*-pyrrol-2-carbonsäure [91],
4-(4-Chlorphenylsulfamoyl)-3,5-dimethyl-1*H*-pyrrol-2-carbonsäure [92],
2-(4-Chlorphenylsulfamoyl)-4-methylthiazol-5-carbonsäureethylester [93],
3,5-Dichlor-N-(4-chlorphenyl)-4-hydroxybenzensulfonamid [94],
N-(3,5-Bis-trifluormethylphenyl)-3,5-dichlor-4-hydroxybenzensulfonamid [95],
3,5-Dichlor-4-hydroxy-N-(4-trifluormethylphenyl)-benzensulfonamid [96],
N-(4-Chlorphenyl)-4-nitrobenzensulfonamid [97],
N-(3,5-Bis-trifluormethylphenyl)-4-nitrobenzensulfonamid [98],
4-Amino-N-(3,5-Bis-trifluormethylphenyl)-3-chlorbenzensulfonamid [99],
3-Nitro-N-(4-trifluormethylphenyl)-benzensulfonamid [100],
3.5-Dichlor-N-(3,5-dichlorphenyl)-4-hydroxybenzensulfonamid [101],
4-Amino-3-chlor-N-(4-chlorphenyl)-benzensulfonamid [102],
3-Chlor-N-(4-chlorphenyl)-4-methoxybenzensulfonamid [103],
N-(3,5-Bis-trifluormethylphenyl)-3-chlor-4-methoxybenzensulfonamid [104],
N-(3-Chlor-4-nitrophenyl)-3,5-bis-trifluormethylbenzensulfonamid [105],
3-(4-Acetylpiperazin-1-yl)-N-(3,5-bis-trifluormethylphenyl)-4-nitrobenzensulfonamid [106],
N-(3,5-Bis-trifluormethylphenyl)-2-nitrobenzensulfonamid [107],
3-(3,5-Bis-trifluormethylphenylsulfamoyl)-benzoesäure [108],
3,5-Dichlor-N-(4-chlorbenzyl)-4-hydroxybenzensulfonamid [109],
3,5-Dichlor-4-hydroxy-N-(4-trifluormethylbenzyl)-benzensulfonamid [110],
3,6-Dichlor-4-hydroxy-N-[2-(1*H*-Indol-3-yl)-ethyl]-benzensulfonamid [111],
4,5-Dibromthiophen-2-sulfonsäure-(3,5-dichlorphenyl)-amid [112],
N-(3,5-Dichlorphenyl)-4-oxazol-2-yl-benzensulfonamid [113],
4-Brom-5-chlorthiophen-2-sulfonsäure-(3,5-bis-trifluormethylphenyl)-amid [114],
4-Brom-5-chlorthiophen-2-sulfonsäure-(3,5-dichlorphenyl)-amid [115],
5-Bromthiophen-2-sulfonsäure-(4-trifluormethylphenyl)-amid [116],
5-Benzensulfonylthiophen-2-sulfonsäure-(3,5-bis-trifluormethylphenyl)-amid [117],
5-Benzensulfonylthiophen-2-sulfonsäure-(2,4-dichlorphenyl)-amid [118],
5-Chlor-3-methylbenzo[b]thiophen-2-sulfonsäure-(3,5-bis-trifluormethylphenyl)-amid [119],
Benzo[b]thiophen-2-sulfonsäure-(3,5-bis-trifluormethylphenyl)-amid [120],
Benzo[1,2,5]thiadiazol-5-sulfonsäure-(4-chlorphenyl)-amid [121],
Benzo[1,2,5]thiadiazol-5-sulfonsäure-(3,5-bis-trifluormethylphenyl)-amid [122],
Benzo[1,2,5]thiadiazol-5-sulfonsäure-(4-trifluormethylphenyl)-amid [123],
5-Pyridin-2-yl-thiophen-2-sulfonsäure-(3,5-bis-trifluormethylphenyl)-amid [124],
4,5-Dibromthiophen-2-sulfonsäure-(4-chlorphenyl)-amid [125],
4,5-Dibromthiophen-2-sulfonsäure-(4-trifluormethylphenyl)-amid [126],
3,5-Dichlor-N-(4-fluorbenzyl)-4-hydroxybenzensulfonamid [127],
N-(3,5-Bis-trifluormethylphenyl)-2,6-dichlorbenzensulfonamid [128],
5-Chlor-4-nitrothiophen-2-sulfonsäure-(4-methoxy-2-methylphenyl)-amid [129],
5-(3-Hydroxypiperidin-1-yl)-4-nitrothiophen-2-sulfonsäure-(4-chlorphenyl)-amid [130] und
5-Chlor-4-nitrothiophen-2-sulfonsäure-(4-nitrophenyl)-amid [131].

20. Verwendung nach Anspruch 19, wobei die Verbindung der Formel I unter den folgenden ausgewählt ist:
5-Chlor-4-nitrothiophen-2-sulfonsäure-(4-chlorphenyl)-amid [2],
5-Chlor-4-nitrothiophen-2-sulfonsäure-(4-fluorphenyl)-amid [3],
5-Chlor-4-nitrothiophen-2-sulfonsäure-(4-hydroxyphenyl)-amid [5],
5-Chlor-4-nitrothiophen-2-sulfonsäure-(4-trifluormethylphenyl)-amid [6],
5-Chlor-4-nitrothiophen-2-sulfonsäure-(4-fluorphenyl)-methylamid [7],
4-Chlor-3-nitro-N-(4-trifluormethylphenyl)-benzensulfonamid [35],
4-Chlor-N-(4-chlorphenyl)-3-nitrobenzensulfonamid [38],
5-Chlor-4-nitrothiophen-2-sulfonsäure-(3,5-dichlorphenyl)-amid [39],
5-Chlor-4-nitrothiophen-2-sulfonsäure-(3,5-difluorphenyl)-amid [40],
5-(1-Methyl-5-trifluormethyl-1*H*-pyrazol-4-yl)-thiophen-2-sulfonsäure-(3,5-bis-trifluormethylphenyl)-amid [43],
5-Chlor-4-nitrothiophen-2-sulfonsäure-(3,5-bis-trifluormethylphenyl)-amid [44],
5-Chlor-4-nitrothiophen-2-sulfonsäure-4-fluorbenzylamid [45],
5-Chlor-4-nitrothiophen-2-sulfonsäure-4-trifluormethylbenzylamid [46],
4-Chlor-N-(3,5-dichlorphenyl)-3-nitrobenzensulfonamid [47],
5-Chlor-4-nitrothiophen-2-sulfonsäure-[2-(1*H*-indol-3-yl)-ethyl]-amid [48],
5-Chlor-4-nitrothiophen-2-sulfonsäure-[2-(1*H*-indol-3-yl)-1-methylethyl]-amid [49],
5-Chlor-4-nitrothiophen-2-sulfonsäure-(4-chlorphenyl)-methylamid [51]
5-Chlor-4-nitrothiophen-2-sulfonsäurebenzyl-(4-fluorbenzyl)-amid [53],
5-Chlor-4-nitrothiophen-2-sulfonsäure-3,5-dichlorbenzylamid [54],
5-Chlor-4-nitrothiophen-2-sulfonsäure-3,5-difluorbenzylamid [55],
5-Chlor-4-nitrothiophen-2-sulfonsäure-4-chlorbenzylamid [56],
5-Chlor-4-nitrothiophen-2-sulfonsäure-[1-(4-fluorphenyl)-ethyl]-amid [57],
5-Chlor-4-nitrothiophen-2-sulfonsäure-(4-methoxyphehyl)-amid [61],
5-Chlor-4-nitrothiophen-2-sulfonsäure-p-tolylamid [63],
5-Chlor-4-nitrothiophen-2-sulfonsäurebenzyl-(4-chlorphenyl)-amid [65],
5-Chlor-4-nitrothiophen-2-sulfonsäurebenzyl-(4-methoxyphenyl)-amid [66],
5-Chlor-4-nitrothiophen-2-sulfonsäure-(4-chlorphenyl)-(3-trifluormethylbenzyl)-amid [67],
4-Nitrothiophen-2-sulfonsäure-(4-chlorphenyl)-amid [69],
5-Chlor-4-nitrothiophen-2-sulfonsäure-(3-trifluormethylbenzyl)-(4-trifluormethylbenzyl)-amid [75] und
N-(1*H*-Benzoimidazol-2-yl)-4-chlor-3-nitrobenzensulfonamid [83].

21. Verwendung nach Anspruch 19, wobei die Verbindung der Formel I unter den folgenden ausgewählt ist:
5-Chlor-4-nitrothiophen-2-sulfonsäure-(4-chlorphenyl)-amid [2],
5-Chlor-4-nitrothiophen-2-sulfonsäure-(4-fluorphenyl)-amid [3],
5-Chlor-4-nitrothiophen-2-sulfonsäure-(4-hydroxyphenyl)-amid [5],
5-Chlor-4-nitrothiophen-2-sulfonsäure-(4-trifluormethylphenyl)-amid [6],
5-Chlor-4-nitrothiophen-2-sulfonsäure-(4-fluorphenyl)-methylamid [7],
5-Chlorthiophen-2-sulfonsäure-(4-trifluormethylphenyl)-amid [9],
5-Bromthiaphen-2-sulfonsäure-(3,5-bis-trifluormethylphenyl)-amid [18],
5-Benzensulfonylthiophen-2-sulfonsäure-(4-trifluormethylphenyl)-amid [23],
4,5-Dibromthiophen-2-sulfonsäure-(3-trifluormethylphenyl)-amid [27],
4,5-Dibromthiophen-2-sulfonsäure (3,4-dichlorphenyl)-amid [28],
N-(3,5-Bis-trifluormethylphenyl)-4-chlor-3-nitrobenzensulfonamid [29],
5-Chlorthiophen-2,4-disulfonsäure-bis-[(4-trifluormethylphenyl)-amid] [33],
4-Chlor-3-nitro-N-(4-trifluormethylphenyl)-benzensulfonamid [35],
5-Chlor-4-nitrothiophen-2-sulfonsäure-(3,5-dichlorphenyl)-amid [39],
5-Chlor-4-nitrothiophen-2-sulfonsäure-(3,5-difluorphenyl)-amid [40],
5-(1-Methyl-5-trifluormethyl-1*H*-pyrazol-4-yl)-thiophen-2-sulfonsäure-(3,5,bis-trifluormethylphenyl)-amid [43],
5-Chlor-4-nitrothiophen-2-sulfonsäure-(3,5-bis-trifluormethylphenyl)-amid [44],
5-Chlor-4-nitrothiophen-2-sulfonsäure-4-fluorbenzylamid [45],
5-Chlor-4-nitrothiophen-2-sulfonsäure-4-trifluormethylbenzylamid [46],
5-chlor-4-nitrothiophen-2-sulfonsäure-[2-(1*H*-indol-3-yl)-ethyl]-amid [48],
5-Chlor-4-nitrothiophen-2-sulfonsäure-[2-(1*H*-indol-3-yl)-1-methylethyl]-amid [49],
5-Chlor-4-nitrothiophen-2-sulfonsäure-(4-trifluormethylphenyl)-amid [50],
5-Chlor-4-nitrothiophen-2-sulfonsäure-(4-chlorphenyl)-methylamid [51] und
5-Chlor-4-nitrothiophen-2-sulfonsäuremethyl-(4-trifluormethylbenzyl)-amid [52],
5-Chlor-4-nitrothiophen-2-sulfonsäurebenzyl-(4-fluorbenzyl)-amid [53],
5-Chlor-4-nitrothiophen-2-sulfonsäure-3,5-dichlorbenzylamid [54],
5-Chlor-4-nitrothiophen-2-sulfonsäure-3,5-difluorbenzylamid [55],
5-Chlor-4-nitrothiophen-2-sulfonsäure-4-chlorbenzylamid [56],
6-Chlor-4-nitrothiophen-2-sulfonsäure-[1-(4-fluorphenyl)-ethyl]-amid [57],
5-Chlor-4-nitrothiophen-2-sulfonsäure-(1*H*-benzoimidazol-2-yl)-amid [59],
5-Chlor-4-nitrothiophen-2-sulfonsäure-[2-(6-chlor-1*H*-indol-3-yl)-ethyl]-amid [60],
5-Chlor-4-nitrothiophen-2-sulfonsäure-(4-methoxyphenyl)-amid [61],
5-Chlor-4-nitrothiophen-2-sulfonsäure-p-tolylamid [63],
5-Chlor-4-nitrothiophen-2-sulfonsäurebenzyl-(4-chlorphenyl)-amid [65],
5-Chlor-4-nitrothiophen-2-sulfonsäurebenzyl-(4-methoxyphenyl)-amid [66].
5-Chlor-4-nitrothiophen-2-sulfonsäure-(4-chlorphenyl)-(3-trifluormethylbenzyl)-amid [67],
4-Nitrothiophen-2-sulfonsäure-(4-chlorphenyl)-amid [69],
5-Chlorthiophen-2,4-disulfonsäure-bis-[(4-chlorphenyl)-amid] [70],
Thioessigsäure-S-[5-(4-chlorphenylsulfamoyl)-3-nitrothiophen-2-yl]-ester [72],
5-Methyl-4-nitrothiophen-2-sulfonsäure-(4-chlorphenyl)-amid [73],
5-Methylthiophen-2,4-disulfonsäure-bis-[(4-chlorphenyl)-amid] [74],
5-Chlor-4-nitrothiophen-2-sulfonsäure-(3-trifluormethylbenzyl)-(4-trifluormethylbenzyl)-amid [75],
5-(2-Methoxyethylamino)-4-nitrothiophen-2-sulfonsäure-(4-chlorphenyl)-amid [80],
N-(1*H*-Benzoimidazol-2-yl)-4-chlor-3-nitrobenzensulfonamid [83],
5-Chlor-3-methylbenzo[b]thiophen-2-sulfonsäure-(4-chlor-3-nitrophenyl)-amid [88],
N-(3,5-Bis-trifluormethylphenyl)-3-chlor-4-methoxybenzensulfonamid [104],
N-(3-Chlor-4-nitrophenyl)-3,5-bis-trifluormethylbenzensulfonamid [105],
4,5-Dibromthiophen-2-sulfonsäure-(3,5-dichlorphenyl)-amid [112],
5-Bromthiophen-2-sulfonsäure-(4-trifluormethylphenyl)-amid [116],
5-Benzensulfonylthiophen-2-sulfonsäure-(3,5-bis-trifluormethylphenyl)-amid [117],
5-Chlor-3-methylbenzo[b]thiophen-2-sulfonsäure-(3,5-bis-trifluormethylphenyl)-amid [119],
Benzo[b]thiophen-2-sulfonsäure-(3,5-bis-trifluormethylphenyl)-amid [120],
Benzo[1,2,5]thiadiazol-5-sulfonsäure-(3,5-bis-trifluormethylphenyl)-amid [122],
4,5-Dibromthiophen-2-sulfonsäure-(4-chlorphonyl)-amid [125],
4,5-Dibromthiophen-2-sulfonsäure-(4-trifluormethylphenyl)-amid [126],
N-(3,5-Bis-trifluormethylphenyl)-2,6-dichlorbenzensulfonamid [128],
5-(3-Hydroxypiperidin-1-yl)-4-nitrothiophen-2-sulfonsäure-(4-chlorphenyl)-amid [130] und
5-Chlor-4-nitrothiophen-2-sulfonsäure-(4-nitrophenyl)-amid [131],

22. Verwendung nach einem der vorangegangenen Ansprüche, wobei die proliferative Störung Krebs ist.

23. Verwendung nach einem der vorangegangenen Ansprüche, wobei die Verbindung in einer Menge verabreicht wird, die ausreichend ist, um die Wechselwirkung zwischen HDM2- und p53- und/oder HDM2- und E2F-Transkriptionsfaktoren zu hemmen.

24. Verbindung der Formel Ic, 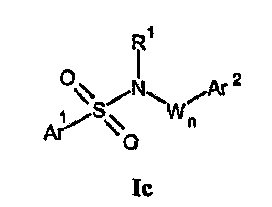 wobei
W eine verzweigte oder unverzweigte C₁₋₅-Alkylengruppe oder eine C₂₋₅-Alkenylengruppe ist,
n 0 oder 1 ist,
R¹ H, eine verzweigte oder unverzweigte C₁₋₈-Alkylgruppe, eine C₂₋₈-Alkenylgruppe oder eine Aryl- oder Aralkylgruppe ist, von denen jede optional mit einer oder mehreren Halogen- oder CF₃-Gruppen substituiert sein kann,
Ar¹ folgendes ist 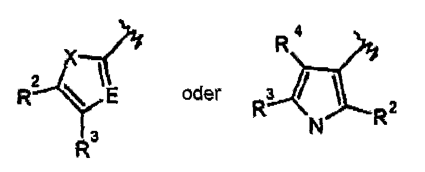 wobei
X O, NH oder NR' ist, wobei R' eine C₁₋₃-Alkylgruppe ist,
E N oder CR⁴ ist,
Y N ist,
R², R³ und R⁴ jeweils unabhängig voneinander (A)ₚB sind, wobei A C₁₋₃-Alkyl ist, p 0 oder 1 ist und B H, Halogen, C₁₋₅-Alkyl, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, eine alizyklische Gruppe, die optional ein oder mehrere Heteroatome enthält, optional substituiert mit einer oder mehreren OH-, COR^{u}-, Halogen- oder CF₃-Gruppen, oder eine Heteroarylgruppe, optional substituiert mit einer oder mehreren C₁₋₅-Alkyl-, Halogen-, SR'- oder CF₃-Gruppen, ist oder
R² und R³ miteinander verknüpft sind unter Bildung eines gesättigten oder ungesättigten Ringsystems, welches optional ein oder mehrere Heteroatome enthält und optional mit einer oder mehreren Halogen-, OH- oder CF₃-Gruppen substituiert ist,
Ar^{a-f} jeweils unabhängig voneinander Arylgruppen, optional substituiert mit einer oder mehreren C₁₋₅-Alkyl-, Halogen-oder CF₃-Gruppen, sind,
R^{a-i}, R^{s}, R^{t} und R^{u} jeweils unabhängig voneinander C₁₋₅-Alkylgruppen, optional substituiert mit einer oder mehreren Alkoxy-, Halogen- oder CF₃-Gruppen, sind
und mit der Maßgabe, daß wenigstens einer von R², R³ und R⁴ etwas anderes als H ist,
Ar² folgendes ist 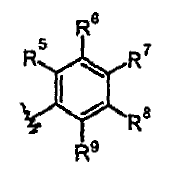 wobei
Z S, O, NH oder NR" ist, wobei R" C₁₋₃-Alkyl ist,
R⁵, R⁶, R⁷, R⁸ und R⁹ jeweils unabhängig voneinander (L)_{q}M sind, wobei L C₁₋₃-Alkyl ist, q 0 oder 1 ist, M H, C₁₋₅-Alkyl, Halogen, NO₂, OH, NH₂, NHAr^{g}, NHR^{j}, NR^{k}R^{l}, SO₃H, SO₂NH₂, SO₂NHAr^{h}, SO₂NHR^{m}, SO₂Arⁱ, SO₂Rⁿ, CF₃, CN, COOH, COOR^{p}, CONH₂₋ CONHAr^{j}, CONHR^{q}, OR^{v}, COAr^{k} oder COR^{r} sind,
R^{j-r}, R^{v} jeweils unabhängig voneinander C₁₋₅-Alkylgruppen sind,
Ar^{g-k} jeweils unabhängig voneinander Arylgruppen sind,
und mit der Maßgabe, daß wenigstens einer der Substituenten R⁵, R⁶, R⁷, R⁸ und R⁹ etwas anderes als H ist,
mit der Maßgabe, daß die Verbindung nicht
5-[(4-Chlorphenyl)-amino]-sulfonyl-2-furancarbonsäure ist.

25. Verbindung der Formel **Id** 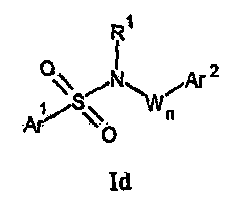 wobei
W eine verzweigte oder unverzweigte C₁₋₅-Alkylengruppe oder eine C₂₋₅-Alkenylengruppe ist,
n 0 oder 1 ist,
R¹ H, eine verzweigte oder unverzweigte C₁₋₈-Alkylgruppe, eine C₂₋₈-Alkenylgruppe oder eine Aryl- oder Aralkylgruppe ist, von denen jede optional mit einer oder mehreren Halogen- oder CF₃-Gruppen substituiert sein kann,
Ar¹ folgendes ist 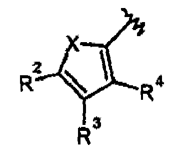 wobei
X O, NH oder NR' ist, wobei R' eine C₁₋₃-Alkylgruppe ist,
R², R³ und R⁴ jeweils unabhängig voneinander (A)ₚB sind, wobei A C₁₋₃-Alkyl ist, p 0 oder 1 ist und B H, Halogen, C₁₋₅-Alkyl, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, eine alizyklische Gruppe, die optional ein oder mehrere Heteroatome enthält, optional substituiert mit einer oder mehreren OH-, COR^{u}-, Halogen- oder CF₃-Gruppen, oder eine Heteroarylgruppe, optional substituiert mit einer oder mehreren C₁₋₅-Alkyl-, Halogen-, SR^{l}- oder CF₃-Gruppen, ist oder
R² und R³ miteinander verknüpft sind unter Bildung eines gesättigten oder ungesättigten Ringsystems, welches optional ein oder mehrere Heteroatome enthält und optional mit einer oder mehreren Halogen-, OH- oder CF₃-Gruppen substituiert ist,
Ar^{a-f} jeweils unabhängig voneinander Arylgruppen, optional substituiert mit einer oder mehreren C₁₋₅-Alkyl-, Halogen- oder CF₃-Gruppen, sind,
R^{a-i}, R^{s}, R^{t} und R^{u} jeweils unabhängig voneinander C₁₋₅-Alkylgruppen, optional substituiert mit einer oder mehreren Alkoxy-, Halogen- oder CF₃-Gruppen, sind
und mit der Maßgabe, daß wenigstens einer von R², R³ und R⁴ etwas anderes als H ist,
und Ar² folgendes ist 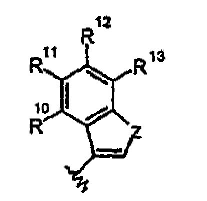
wobei Z S, O, NH oder NR" ist, wobei R" C₁₋₃-Alkyl ist, und
R¹⁰, R¹¹, R¹² und R¹³ jeweils unabhängig voneinander H, C₁₋₅-Alkyl, Halogen, NO₂, OH, NH₂ oder CF₃ sind.

26. Verbindung der Formel le 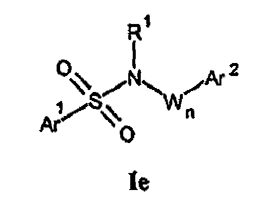 wobei
W eine verzweigte oder unverzweigte C₁₋₅-Alkylengruppe oder eine C₂₋₅-Alkenylengruppe ist,
n 0 oder 1 ist,
R¹ H, eine verzweigte oder unverzweigte C₁₋₈-Alkylgruppe, eine C₂₋₈-Alkenylgruppe oder eine Aryl- oder Aralkylgruppe ist, von denen jede optional mit einer oder mehreren Halogen- oder CF₃-Gruppen substituiert sein kann,
Ar¹ folgendes ist 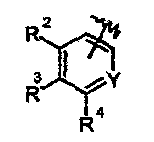
wobei Y N ist,
R², R³ und R⁴ jeweils unabhängig voneinander (A)ₚB sind, wobei A C₁₋₃-Alkyl ist, p 0 oder 1 ist und B H, Halogen, C₁₋₅-Alkyl, NO₂ OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, eine alizyklische Gruppe, die optional ein oder mehrere Heteroatome enthält, optional substituiert mit einer oder mehreren OH-, COR^{u}-, Halogen- oder CF₃-Gruppen, oder eine Heteroarylgruppe, optional substituiert mit einer oder mehreren C₁₋₅-Alkyl-, Halogen-, SRⁱ- oder CF₃-Gruppen, ist oder
R² und R³ miteinander verknüpft sind unter Bildung eines gesättigten oder ungesättigten Ringsystems, welches optional ein oder mehrere Heteroatome enthält und optional mit einer oder mehreren Halogen-, OH- oder CF₃-Gruppen substituiert ist,
Ar^{a-f} jeweils unabhängig voneinander Arylgruppen, optional substituiert mit einer oder mehreren C₁₋₅-Alkyl-, Halogen- oder CF₃-Gruppen, sind,
R^{a-i}, R^{s}, R^{t} und R^{u} jeweils unabhängig voneinander C₁₋₅-Alkylgruppen, optional substituiert mit einer oder mehreren Alkoxy-, Halogen- oder CF₃-Gruppen, sind
und mit der Maßgabe, daß wenigstens einer von R², R³ und R⁴ etwas anderes als H ist,
und Ar² folgendes ist 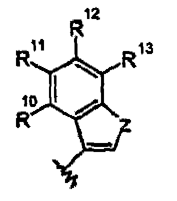
wobei Z S, O, NH oder NR" ist, wobei R" C₁₋₃-Alkyl ist, und
R¹⁰, R¹¹, R¹² und R¹³ jeweils unabhängig voneinander H, C₁₋₅-Alkyl, Halogen, NO₂, OH, NH₂ oder CF₃ sind.

27. Verbindung der Formel **If** 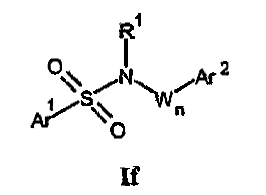 wobei
W eine verzweigte oder unverzweigte C₁₋₅-Alkylengruppe oder eine C₂₋₅-Alkenylengruppe ist,
n 0 oder 1 ist,
R¹ H, eine verzweigte oder unverzweigte C₁₋₈-Alkylgruppe, eine C₂₋₈-Alkenylgruppe oder eine Aryl- oder Aralkylgruppe ist, von denen jede optional mit einer oder mehreren Halogen- oder CF₃-Gruppen substituiert sein kann,
Ar¹ folgendes ist 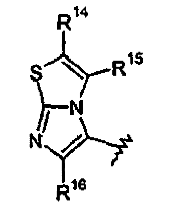
wobei R¹⁴, R¹⁵ und R¹⁶ jeweils unabhängig voneinander (A)ₚB sind, wobei A C₁₋₃-Alkyl ist, p 0 oder 1 ist und B H, Halogen, C₁₋₅-Alkyl, NO₂, OH, NH₂. NHR^{a}, NR^{b}R^{c}, SO₃H, SO₂NH₂, NHr^{a}, SO₂NHAr^{b}, SO₂NHR^{d}, SO₂Ar^{c}, SO₂R^{e}, CF₃. CN, COOH, COOR^{f}, CONH₂, COON-HAr^{d}, CONHR^{g}, COAr^{e}, COR^{h}, S(CO)R^{s}, OR^{t}, OAr^{f}, eine alizyklische Gruppe, die optional ein oder mehrere Heteroatome enthält, optional substituiert mit einer oder mehreren OH-, COR^{u}-, Halogen- oder CF₃-Gruppen, oder eine Heteroarylgruppe, optional substituiert mit einer oder mehreren C₁₋₅-Alkyl-, Halogen-, SR^{l}- oder CF₃-Gruppen, ist oder
R² und R³ miteinander verknüpft sind unter Bildung eines gesättigten oder ungesättigten Ringsystems, welches optional ein oder mehrere Heteroatome enthält und optional mit einer oder mehreren Halogen-, OH- oder CF₃-Gruppen substituiert ist,
Ar^{a-f} jeweils unabhängig voneinander Arylgruppen, optional substituiert mit einer oder mehreren C₁₋₅-Alkyl-, Halogen- oder CF₃-Gruppen, sind,
R^{a-i}, R^{s} R^{t} und R^{u} jeweils unabhängig voneinander C₁₋₅-Alkylgruppen, optional substituiert mit einer oder mehreren Alkoxy-, Halogen- oder CF₃-Gruppen, sind,
und Ar² folgendes ist 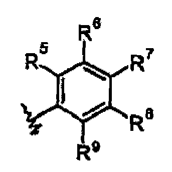
wobei R⁵, R⁶, R⁷, R⁸ und R⁹ jeweils unabhängig voneinander (L)_{q}M sind, wobei L C₁₋₃-Alkyl ist, q 0 oder 1 ist, M H, C₁₋₅-Alkyl, Halogen, NO₂, OH, NH₂, NHAr^{g}, NHR^{j}, NR^{k}R^{l}, SO₃H, SO₂NH₂, SO₂NHAr^{h}, SO₂NHR^{m}, SO₂Ar^{l}, SO₂Rⁿ, CF₃, CN, COOH, COOR^{p}, CONH₂, CONHA^{j}, CONHR^{q}, OR^{v}, COAr^{k} oder COR^{r} sind,
R^{j-r}, R^{v} jeweils unabhängig voneinander C₁₋₅-Alkylgruppen sind,
Ar^{g-k} jeweils unabhängig voneinander Arylgruppen sind,
und mit der Maßgabe, daß wenigstens einer der Substituenten R⁵, R⁶, R⁷, R⁸ und R⁹ etwas anderes als H ist.

28. Verbindung der Formel **Ig** 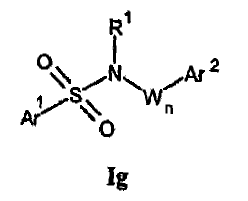 wobei
W eine verzweigte oder unverzweigte C₁₋₅-Alkylengruppe oder eine C₂₋₅-Alkenylengruppe ist,
n 0 oder 1 ist,
R¹ H, eine verzweigte oder unverzweigte C₁₋₈-Alkylgruppe, eine C₂₋₈-Alkenylgruppe oder eine Aryl- oder Aralkylgruppe ist, von denen jede optional mit einer oder mehreren Halogen- oder CF₃-Gruppen substituiert sein kann,
Ar¹ folgendes ist 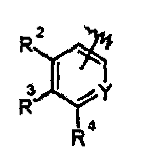
wobei Y N ist,
R², R³ und R⁴jeweils unabhängig voneinander (A)ₚB sind, wobei A C₁₋₃-Alkyl ist, p 0 oder 1 ist und B H, Halogen, C₁₋₅-Alkyl, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, eine alizyklische Gruppe, die optional ein oder mehrere Heteroatome enthält, optional substituiert mit einer oder mehreren OH-, COR^{u}-, Halogen- oder CF₃-Gruppen, oder eine Heteroarylgruppe, optional substituiert mit einer oder mehreren C₁₋₅-Alkyl-, Halogen-, SR'- oder CF₃-Gruppen, ist oder
R² und R³ miteinander verknüpft sind unter Bildung eines gesättigten oder ungesättigten Ringsystems, welches optional ein oder mehrere Heteroatome enthält und optional mit einer oder mehreren Halogen-, OH- oder OF₃-Gruppen substituiert ist,
Ar^{a-f} jeweils unabhängig voneinander Arylgruppen, optional substituiert mit einer oder mehreren C₁₋₆-Alkyl-, Halogen- oder CF₃-Gruppen, sind,
R^{a-i}, R^{s}, R^{t} und R^{u} jeweils unabhängig voneinander C₁₋₅-Alkylgruppen, optional substituiert mit einer oder mehreren Alkoxy-, Halogen- oder CF₃-Gruppen, sind
und mit der Maßgabe, daß wenigstens einer von R², R³ und R⁴ etwas anderes als H ist,
und Ar² folgendes ist 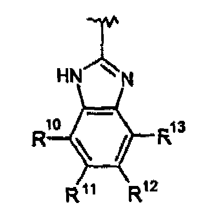
wobei R¹⁰, R¹¹, R¹² und R¹³ jeweils unabhängig voneinander H, C₁₋₅-Alkyl, Halogen, NO₂, OH, NH₂ oder CF₃ sind.

29. Verbindung der Formel **Ib** 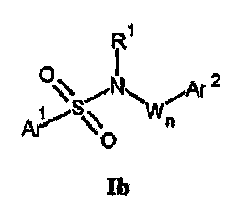 wobei
W eine verzweigte oder unverzweigte C₁₋₅-Alkylengruppe oder eine C₂₋₅-Alkenylengruppe ist,
n 0 oder 1 ist,
R¹ H, eine verzweigte oder unverzweigte C₁₋₈-Alkylgruppe, eine C₂₋₈-Alkenylgruppe oder eine Aryl- oder Aralkylgruppe ist, von denen jede optional mit einer oder mehreren Halogen- oder CF₃-Gruppen substituiert sein kann,
Ar¹ folgendes ist 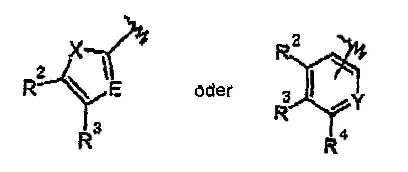 wobei
X S, O, NH oder NR' ist, wobei R' eine C₁₋₃-Alkylgruppe ist,
E N oder CR⁴ ist,
Y CH oder N ist,
R², R³ und R⁴ jeweils unabhängig voneinander (A)ₚB sind, wobei A C₁₋₃-Alkyl ist, p 0 oder 1 ist und B H, Halogen, C₁₋₅-Alkyl, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, eine alizyklische Gruppe, die optional ein oder mehrere Heteroatome enthält, optional substituiert mit einer oder mehreren OH-, COR^{u}-, Halogen- oder CF₃-Gruppen, oder eine Heteroarylgruppe, optional substituiert mit einer oder mehreren C₁₋₅-Alkyl-, Halogen-, SRⁱ- oder CF₃-Gruppen, ist, oder
R² und R³ miteinander verknüpft sind unter Bildung eines gesättigten oder ungesättigten Ringsystems, welches optional ein oder mehrere Heteroatome enthält und optional mit einer oder mehreren Halogen-, OH- oder CF₃-Gruppen substituiert ist,
Ar^{a-f} jeweils unabhängig voneinander Arylgruppen, optional substituiert mit einer oder mehreren C₁₋₅-Alkyl-, Halogen- oder CF₃-Gruppen, sind,
R^{a-l}, R^{s}, R^{t} und R^{u} jeweils unabhängig voneinander C₁₋₅-Alkylgruppen, optional substituiert mit einer oder mehreren Alkoxy-, Halogen- oder CF₃-Gruppen, sind
und mit der Maßgabe, daß wenigstens einer von R², R³ und R⁴ etwas anderes als H ist,
Ar² folgendes ist 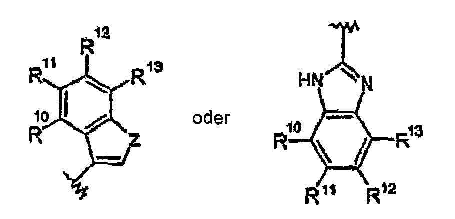 wobei
Z S, O, NH oder NR" ist, wobei R" C₁₋₃-Alkyl ist, und
R¹⁰, R¹¹, R¹² und R¹³ jeweils unabhängig voneinander H, C₁₋₅-Alkyl, Halogen, NO₂, OH, NH₂ oder CF₃ sind,
wobei die Verbindung etwas anderes ist als 5-Methoxy-N-methyl-N-(p-toluensulfonyltryptamin), N-Methyl-p-toluensulfonyltryptamin, N-(p-Toluensulfonyl)-7-methoxytryptamin, p-Toluensulfonyltryptamin oder 3-Sulfanilamidoethylindol.

30. Verbindung nach einem der Ansprüche 24 bis 29, wobei
- R² , R³ und R⁴ jeweils unabhängig voneinander (A)ₚB sind, wobei A C₁₋₅-Alkyl ist, p 0 oder 1 ist und B H, F, Cl, Br, 1, C₁₋₅-Alkyl, NO₂, OH, NH₂, NHR^{a}, NR^{b}R^{c}, SO₃H, SO₂NH₂, NHPh, SO₂NHAr^{b}, SO₂NHR^{d}, SO₂Ph, SO₂R^{a}, CF₃, CN, COOH, COOR^{f}, CONH₂, COONHP^{a}, CONHR^{g}, S(CO)R^{s}, OR^{t}, OAr^{f}, COPh, COR^{h}, eine Morpholino-, Piperazino- oder Piperidinogruppe, von denen jede optional mit einer oder mehreren OH- oder COR^{u}-Gruppen substituiert sein kann, oder eine Heteroarylgruppe, ausgewählt unter Pyridyl, Pyrimidyl, Oxazolyl, Thiazolyl und Pyrazolyl, von denen jede optional mit einer oder mehreren C₁₋₅-Alkyl-, Halogen-, SR'- oder CF₃-Gruppen substituiert sein kann, ist, oder R² und R³ zusammen einen gesättigten sechsgliedrigen Ring oder einen ungesättigten fünfgliedrigen Ring bilden, von denen jeder optional ein oder mehrere Heteroatome enthält, und
- R⁵, R⁶, R⁷, R⁸ und R⁹ jeweils unabhängig voneinander (L)_{q}M sind, wobei L C₁₋₅-Alkyl ist, q 0 oder 1 ist, M H, C₁₋₅-Alkyl, Halogen, NO₂, OH, NH₂, NHPh, NHR^{j}, NR^{k}R^{l}, SO₃H, SO₂NH₂, SO₂NHPh, SO₂NHR^{m}, SO₂Ph, SO₂Rⁿ, CF₃, CN, COOH, COOR^{p}, CONH₂, CONHPh, CONHR^{q}, OR^{v}, COPh oder COR^{r} ist.

31. Verbindung nach einem der Ansprüche 24 bis 29, wobei R², R³ und R⁴ jeweils unabhängig voneinander (A)ₚB sind, wobei A C₁₋₅-Alkyl ist, p 0 oder 1 ist und B H, F, Cl, Br, l, C₁₋₅-Alkyl, NO₂, OH, NH₂, NHR^{a}, NR^{b}R^{c}, SO₃H, SO₂NH₂, NHPh, SO₂NHPh, SO₂NHR^{d}, SO₂Ph, SO₂R^{e}, CF₃, CN, COOH, COOR^{f}, CONH₂, COOHNPh, CONHR^{g}, S(CO)R^{s}, OR^{t}, OAr^{f}, COPh, COR^{h}, Pyridyl, Pyrimidyl, 2-Methylsulfanylpyrimid-5-yl, Oxazol-2-yl, Thiazol-2-yl, 1-Methyl-5-trifluormethyl-1*H*-pyrazol-4-yl, Morpholin-4-yl, 4-Acetylpiperazin-1-yl, 3-Hydroxypiperidin-1-yl ist, oder R² und R³ zusammen
-OCH₂CH₂O-
-N-S-N- oder
eine Phenylgruppe, optional substituiert mit einem oder mehreren Halogenen, bilden.

32. Verbindung nach einem der Ansprüche 24 bis 29, wobei R², R³ und R⁴ jeweils unabhängig voneinander H, Halogen. NO₂, SO₂Ph, S(CO)Me, COOH, COOEt, OPh, OMe, NHCH₂CH₂OMe, 1-Methyl-5-trifluormethyl-1*H*-pyrazol-4-yl, 2-Methylsulfanylpyrimid-5-yl, N-(4-Fluorphenyl)-sulfonamido, N-(4-Trifluormethylphenyl)-sulfonamido, Oxazol-2-yl, C₁₋₅-Alkyl, NH₂, Morpholin-4-yl, 4-Acetylpiperazin-1-yl, 3-Hydroxypiperidin-1-yl sind, oder R² und R³ zusammen
OCH₂CH₂O-
-N-S-N- oder
eine Phenylgruppe, optional substituiert mit einem oder mehreren Halogenen, bilden.

33. Verbindung nach einem der Ansprüche 24, 25 oder 29-32, wobei Ar¹ folgendes ist 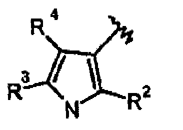
R² und R⁴ C₁₋₅-Alkyl sind und R³ COOH oder COOEt ist.

34. Verbindung nach einem der Ansprüche 26, 28 oder 29 bis 32, wobei Ar¹ folgendes ist 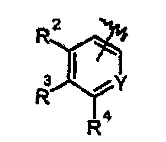
R², R³ und R⁴ jeweils unabhängig voneinander H, OH, COOH, CF₃, OPh, OMe, NO₂, 4-Acetyl-piperazin-1-yl, NH₂, Halogen, Pyrazol-1-yl, Oxazol-2-yl oder C₁₋₆-Alkyl sind oder R² und R³ zusammen -OCH₂CH₂O- oder N-S-N- bilden.

35. Verbindung nach Anspruch 34, wobei
wenn Y CH ist,
- R² H, NO₂ oder Cl ist,
- R³ NO₂, NH₂, Cl, CF₃, COOH. 4-Acetylpiperazin-1-yl ist und
- R⁴ H, Cl, Oxazol-2-yl, OH, NO₂, NH₂, OMe oder Me ist, oder,
wenn Y N ist,
- R² H ist,
- R³ Br ist,
- R⁴ Cl oder OPh ist.

36. Verbindung nach einem der Ansprüche 24 bis 35, wobei
- R⁵, R⁶, R⁷, R⁸ und R⁹ jeweils unabhängig voneinander H, Halogen, OMe, NO₂, C₁₋₅-Alkyl, CF₃ oder OH sind und
- R¹⁰, R¹¹, R¹² und R¹³ alle H sind.

37. Verbindung nach Anspruch 36, wobei
- R⁵ H, C₁₋₅-Alkyl oder Halogen ist,
- R⁶ Halogen, NO₂ oder CF₃ ist,
- R⁷ H, Halogen, OMe, NO₂, OH oder CF₃ ist,
- R⁶ H, Halogen oder CF₃ ist,
- R⁹ H ist.

38. Verbindung nach einem der Ansprüche 24 bis 37, wobei
- W CH₂, CH₂CH₂ oder CH(CH₃)CH₂ ist und
- R¹ H, CH₂Ph, CH₂CH(Me)₂, 3-(Trifluormethyl)-benzyl oder Me ist.

39. Verbindung, ausgewählt unter den folgenden:
5-Chlor-4-nitrothiophen-2-sulfonsäure-(3-trifluormethylphenyl)-amid [1],
5-Chlor-4-nitrothiophen-2-sulfonsäure-(4-chlorphenyl)-amid [2],
S-Chlor-4-nitrothiophen-2-sulfonsäure-(4-fluorphenyl)-amld [3],
4-Brom-5-chlorthiophen-2-sulfonsäure-(4-fluorphenyl)-amid [4],
S-Chlor-4-nitrothiophen-2-sulfonsäure-(4-hydroxyphenyl)-amid [5],
5-Chlor-4-nitrothiophen-2-sulfonsäure-(4-trifluormethylphenyl)-amid [6],
5-Chlor-4-nitrothiophen-2-sulfonsäure-(4-fluorphenyl)-methylamid [7],
4,5-Dibromthiophen-2-sulfonsäure-(3,5-bis-trifluormethylphenyl)-amid [8],
5-Chlorthiophen-2-sulfonsäure-(3,5-bis-trifluormethylphenyl)-amid [11],
(5-(2-Methylsulfanylpyrimidin-5-yl)-thiophen-2-sulfonsäure-(3,5-dichlorphenyl)-amid [12],
4-Oxazol-2-yl-N-(4-trifluormethylphenyl)-benzensulfonamid [13],
N-(3,5-Bis-trifluormethylphenyl)-4-oxazol-2-yl-benzensulfonamid [14],
4-Brom-5-chlorthiophen-2-sulfonsäure-(4-trifluormethylphenyl)-amid [15],
5-Bromthiophen-2-sulfonsäure-(4-chlorphenyl)-amid [16],
5-Bromthiophen-2-sulfonsäure-(3,5-dichlorphenyl)-amid [17],
5-Bromthiophen-2-sulfonsäure-(3,5-bis-trifluormethylphenyl)-amid [18],
3-Nitro-N-(4-trifluormethylphenyl)-benzensulfonamid [20],
N-(3,5-Bis-trifluormethylphenyl)-3-nitrobenzensulfonamid [21],
5-Benzensulfonylthiophen-2-sulfonsäure-(4-trifluormethylphenyl)-amid [23],
5-Benzensulfonylthiophen-2-sulfonsäure-(4-chlorphenyl)-amid [24],
5-Benzensulfonylthiophen-2-sulfonsäure-(3,5-dichlorphenyl)-amid [25],
5-Chlorthiophen-2-sulfonsäure-(3,4-dichlorphenyl)-amid [26],
4,5-Dibromthiophen-2-sulfonsäure-(3-trifluormethylphenyl)-amid [27],
4,5-Dibromthiophen-2-sulfonsäure (3,4-dichlorphenyl)-amid [28],
5-(1-Methyl-5-trifluormethyl-1*H*-pyrazol-4-yl)-thiophen-2-sulfonsäure-(4-trifluormethylphenyl)-amid [31].
5-Chlorthiophen,2,4-disulfonsäure-bis-[(4-fluorphenyl)-amid] [32],
5-Chlorthiophen-2,4-disulfonsäure-bis-[(4-trifluormethylphenyl)-amid] [33],
4-Methyl-3-nitro-N-(4-trifluormethylphenyl)-benzensulfonamid [34],
4-Chlor-3-nitro-N-(4-trifluormethylphenyl)-benzensulfonamid [35],
3-Amino-4-methyl-N-(4-trifluormethylphenyl)-benzensulfonamid [36],
N-(4-Chlorphenyl)-4-methyl-3-nitrobenzensulfonamid [37],
4-Chlor-N-(4-chlorphenyl)-3-nitrobenzensulfonamid [38],
5-Chlor-4-nitrothiophen-2-sulfonsäure-(3,5-dichlorphenyl)-amid [39],
5-Chlor-4-nitrothiophen-2-sulfonsäure-(3,5-difluorphenyl)-amid [40],
5-Brom-6-chlorpyridin-3-sulfonsäure-(4-trifluormethylphenyl)-amid [41],
5-Brom-6-chlorpyridin-3-sulfonsäure-(3,5-bis-trifluormethylphenyl)-amid [42],
5-(1-Methyl-5-trifluormethyl-1*H*-pyrazol-4-yl)-thiophen-2-sulfonsäure-(3,5-bis-trifluormethylphenyl)-amid [43],
5-Chlor-4-nitrothiophen-2-sulfonsäure-(3,5-bis-trifluormethylphenyl)-amid [44],
5-Chlor-4-nitrothiophen-2-sulfonsäure-4-fluorbenzylamid [45],
5-Chlor-4-nitrothiophen-2-sulfonsäure-4-trifluormethylbenzylamid [46],
4-Chlor-N-(3,5-dichlorphenyl)-3-nitrobenzensulfonamid [47],
5-Chlor-4-nitrothiophen-2-sulfonsäure-[2-(1*H*-indol-3-yl)-ethyl]-amid [48],
5-Chlor-4-nitrothiophen-2-sulfonsäure-[2-(1*H*-indol-3-yl)-1-methylethyl]-amid [49],
5-Chlor-4-nitrothiophen-2-sulfonsäure-(4-trifluormethylphenyl)-amid [50],
5-Chlor-4-nitrothiophen-2-sulfonsäure-(4-chlorphenyl)-methylamid [51] und
5-Chlor-4-nitrothiophen-2-sulfonsäuremethyl-(4-trifluormethylbenzyl)-amid [52],
5-Chlor-4-nitrothiophen-2-sulfonsäurebenzyl-(4-fluorbenzyl)-amid [53],
5-Chlor-4-nitrothiophen-2-sulfonsäure-3,5-dichlorbenzylamid [54],
5-Chlor-4-nitrothiophen-2-sulfonsäure-3,5-difluorbenzylamid [55],
5-Chlor-4-nitrothiophen-2-sulfonsäure-4-chlorbenzylamid [56],
5-Chlor-4-nitrothiophen-2-sulfonsäure-[1-(4-fluorphenyl)-ethyl]-amid [57],
5-Chlor-4-nitrothiophen-2-sulfonsäure-(4-chlorphenyl)-isobutylamid [58],
5-Chlor-4-nitrothiophen-2-sulfonsäure-(1*H*-benzoimidazol-2-yl)-amid [59],
5-Chlor-4-nitrothiophen-2-sulfonsäure-[2-(6-chlor-1*H*-indol-3-yl)-ethyl]-amid [60],
5-Chlor-4-nitrothiophen-2-sulfonsäure-(4-methoxyphenyl)-amid [61],
5-Chlor-4-nitrothiophen-2-sulfonsäurephenylamid [62],
5-Chlor-4-nitrothiophen-2-sulfonsäure-p-tolylamid [63],
5-Chlor-4-nitrothiophen-2-sulfonsäurebenzylamid [64],
5-Chlor-4-nitrothiophen-2-sulfonsäurebenzyl-(4-chlorphenyl)-amid [65],
5-Chlor-4-nitrothiophen-2-sulfonsäurebenzyl-(4-methoxyphenyl)-amid [66],
5-Chlor-4-nitrothiophen-2-sulfonsäure-(4-chlorphenyl)-(3-trifluormethylbenzyl)-amid [67],
5-Nitrothiophen-2-sulfonsäure-(4-chlorphenyl)-amid [68],
4-Nitrothiophen-2-sulfonsäure-(4-chlorphenyl)-amid [69],
5-Chlorthiophen-2,4-disulfonsäure-bis-[(4-chlorphenyl)-amid] [70],
5-Ethyl-4-nitrothiophen-2-sulfonsäure-(4-chlorphenyl)-amid [71],
Thioessigsäure-S-[5-(4-chlorphenylsulfamoyl)-3-nitrothiophen-2-yl]-ester [72],
5-Methyl-4-nitrothiophen-2-sulfonsäure-(4-chlorphenyl)-amid [73],
5-Methylthiophen-2,4-disulfonsäure-bi5-[(4-chlorphenyl)-amid] [74],
5-Chlor-4-nitrothiophen-2-sulfonsäure-(3-trifluormethylbenzyl)-(4-trifluomethylbenzyl)-amid [75],
4-Nitrothiophen-2-sulfonsäure-(4-trifluomethylphenyl)-amid [76],
4-Nitrothiophen-2-sulfonsäure-[2-(1H-indol-3-yl)-ethyl]-amid [77],
5-(1-Methyl-5-trifluormethyl-1*H*-pyrazol-3-yl)-thiophen-2-sulfonsäure-(3,5-bis-trifluormethylphenyl)-(3-trifluormethylbenzyl)-amid [78],
5-Morpholin-4-yl-4-nitrothiophen-2-sulfonsäure-(4-chlorphenyl)-amid [79],
5-(2-Methoxyethylamino)-4-nitrothiophen-2-sulfonsäure-(4-chlorphenyl)-amid [80],
4-Chlor-N-[2-(5-chlor-1*H*-indol-3-yl)-ethyl]-3-nitrobenzensulfonamid [81],
N-[2-(5-Chlor-1*H*-indol-3-yl)-ethyl]-4-methyl-3-nitrobenzensulfonamid [82],
N-(1*H*-Benzoimidazol-2-yl)-4-chlor-3-nitrobenzensulfonamid [83],
6-Chlorimidazo[2,1-b]thiazol-5-sulfonsäure-(3,5-bis-trifluormethylphenyl)-amid [84],
2,3-Dihydrobenzo[1,4]dioxin-6-sulfonsäure-(4-chlorphenyl)-amid [85],
2,3-Dihydrobenzo[1,4]dioxin-6-sulfonsäure-(3,5-bis-trifluormethylphenyl)-amid [86],
6-Phenoxypyridin-3-sulfonsäure-(4-chlorphenyl)-amid [87].
5-Chlor-3-methylbenzo[b]thiophen-2-sulfonsäure-(4-chlor-3-nitrophenyl)-amid [88],
N-(3,5-Bis-trifluormethylphenyl)-4-pyrazol-1-yl-benzensulfonamid [89].
4-(4-Chlorphenylsulfamoyl)-3,5-dimethyl-1*H*-pyrrol-2-carbonsäureethylester [90],
4-(3,5-Bis-trifluormethylphenylsulfamoyl)-3,5-dimethyl-1*H*-pyrrol-2-carbonsäure [91],
4-(4-Chlorphenylsulfamoyl)-3,5-dimethyl-1*H*-pyrrol-2-carbonsäure [92],
2-(4-Chlorphenylsulfamoyl)-4-methylthiazol-5-carbonsäureethylester [93],
3,5-Dichlor-N-(4-chlorphenyl)-4-hydroxybenzensulfonamid [94],
N-(3,5-Bis-trifluormethylphenyl)-3,5-dichlor-4-hydroxybenzensulfonamid [95],
3,5-Dichlor-4-hydroxy-N-(4-trifluormethylphenyl)-benzensulfonamid [96],
N-(3,6-Bis-trifluormethylphenyl)-4-nitrobenzensulfonamid [98],
4-Amino-N-(3,5-bis-trifluormethylphenyl)-3-chlorbenzensulfonamid [99],
3-Nitro-N-(4-trifluormethylphenyl)-benzensulfonamid [100],
3,5-Dichlor-N-(3,5-dichlorphenyl)-4-hydroxybenzensulfonamid [101],
4-Amino-3-chlor -N-(4-chlorphenyl)-benzensulfonamid [102],
3-Chlor-N-(4-chlorphenyl)-4-methoxybenzensulfonamid [103],
N-(3,5-Bis-trifluormethylphenyl)-3-chlor-4-methoxybenzensulfonamid [104],
N-(3-Chlor-4-nitrophenyl)-3,5-bis-trifluormethylbenzensulfonamid [105],
3-(4-Acetylpiperazin-1-yl)-N-(3,5-bis-trifluormethylphenyl)-4-nitrobenzensulfonamid [106].
N-(3,5-Bis-trifluormethylphenyl)-2-nitrobenzensulfonamid [107],
3-(3,5-Bis-trifluormethylphenylsulfamoyl)-benzoesäure [108],
3,5-Dichlor-N-(4-chlorbenzyl)-4-hydroxybenzensulfonamid [109],
3,5-Dichlor-4-hydroxy-N-(4-trifluormethylbenzyl)-benzensulfonamid [110],
3,5-Dichlor-4-hydroxy-N-[2-(1H-indol-3-yl)-ethyl]-benzensulfonamid [111],
4,5-Dibromthiophen-2-sulfonsäure-(3,5-dichlorphenyl)-amid [112],
N-(3,5-Dichlorphenyl)-4-oxazol-2-yl-benzensulfonamid [113],
4-Brom-5-chlorthiophen-2-sulfonsäure-(3 ,5-bis-trifluormethylphenyl)-amid [114],
4-Brom-5-chlorthiophen-2-sulfonsäure-(3,5-dichlorphenyl)-amid [115],
5-Benzensulfonylthiophen-2-sulfonsäure-(3,5-bis-trifluormethylphenyl)-amid [117],
5-Benzensulfonylthiophen-2-sulfonsäure-(2,4-dichlorphenyl)-amid [118],
5-Chlor-3-methylbenzo[b]thiophen-2-sulfonsäure-(3,5-bis-trifluormethylphenyl)-amid [119],
Benzo[b]thiophen-2-sulfonsäure-(3,5-bis-trifluormethylphenyl)-amid [120],
Benzo[1,2,5]thiadiazol-5-sulfonsäure-(4-chlorphenyl)-amid [121],
Benzo[1,2,5]thiadiazol-5-sulfonsäure-(3,5-bis-trifluormethylphenyl)-amid [122],
Benzo[1,2,5]thiadiazol-5-sulfonsäure-(4-trifluormethylphenyl)-amid [123],
5-Pyridin-2-yl-thiophen-2-sulfonsäure-(3,5-bis-trifluormethylphenyl)-amid [124],
4,5-Dibromthiophen-2-sulfonsäure-(4-chlorphenyl)-amid [125],
4,5-Dibromthiophen-2-sulfonsäure-(4-trifluormethylphenyl)-amid [126],
3,5-Dichlor-N-(4-fluorbenzyl)-4-hydroxybenzensulfonamid [127],
N-(3,5-Bis-trifluormethylphenyl)-2,6-dichlorbenzensulfonamid [128],
5-Chlor-4-nitrothiophen-2-sulfonsäure-(4-methoxy-2-methylphenyl)-amid [129],
5-(3-Hydroxypiperidin-1-yl)-4-nitrothiophen-2-sulfonsäure-(4-chlorphenyl)-amid [130] und
5-Chlor-4-nitrothiophen-2-sulfonsäure-(4-nitrophenyl)-amid [131].

40. Verbindung nach Anspruch 39, welche unter den folgenden ausgewählt ist:
5-Chlor-4-nitrothiophen-2-sulfonsäure-(4-chlorphenyl)-amid [2],
5-Chlor-4-nitrothiophen-2-sulfonsäure-(4-fluorphenyl)-amid [3],
5-Chlor-4-nitrothiophen-2-sulfonsäure-(4-hydroxyphenyl)-amid [5],
5-Chlor-4-nitrothiophen-2-sulfonsäure-(4-trifluormethylphenyl)-amid [6],
5-Chlor-4-nitrothiophen-2-sulfonsäure-(4-fluorphenyl)-methylamid [7],
4-Chlor-3-nitro-N-(4-trifluormethylphenyl)-benzensulfonamid [35],
4-Chlor-N-(4-chlorphenyl)-3-nitrobenzensulfonamid [38],
5-Chlor-4-nitrothiophen-2-sulfonsäure-(3,5-dichlorphenyl)-amid [39],
5-Chlor-4-nitrothiophen-2-sulfonsäure-(3,5-difluorphenyl)-amid [40],
5-(1-Methyl-5-trifluormethyl-1*H*-pyrazol-4-yl)-thiophen-2-sulfonsäure-(3,5-bis-trifluormethylphenyl)-amid [43],
5-Chlor-4-nitrothiophen-2-sulfonsäure-(3,5-bis-trifluormethylphenyl)-amid [44],
5-Chlor-4-nitrothiophen-2-sulfonsäure-4-fluorbenzylamid [45],
5-Chlor-4-nitrothiophen-2-sulfonsäure-4-trifluormethylbenzylamid [46],
5-Chlor-4-nitrothiophen-2-sulfonsäure-[2-(1*H*-indol-3-yl)-ethyl]-amid [48],
5-Chlor-4-nitrothiophen-2-sulfonsäure-[2-(1*H*-indol-3-yl)-1-methylethyl]-amid [49],
5-Chlor-4-nitrothiophen-2-sulfonsäure-(4-chlorphenyl)-methylamid [51]
5-Chlor-4-nitrothiophen-2-sulfonsäurebenzyl-(4-fluorbenzyl)-amid [53],
5-Chlor-4-nitrothiophen-2-sulfonsäure-3,5-dichlorbenzylamid [54],
5-Chlor-4-nitrothiophen-2-sulfonsäure-3,5-difluorbenzylamid [55],
5-Chlor -4-nitrothiophen-2 -sulfonsäure-4-chlorbenzylamid [56],
5-Chlor-4-nitrothiophen-2-sulfonsäure-[1-(4-fluorphenyl)-ethyl]-amid [57],
5-Chlar-4-nitrothiophen-2-sulfonsäure-(4-methoxyphenyl)-amid [61],
5-Chlor-4-nitrothiophen-2-sulfonsäure-p-tolylamid [63],
5-Chlor-4-nitrothiophen-2-sulfonsäurebenzylamid [64],
5-Chlor-4-nitrothiophen-2-sulfonsäurebenzyl-(4-chlorphenyl)-amid [65],
5-Chlor-4-nitrothiophen-2-sulfonsäurebenzyl-(4-methoxyphenyl)-amid [66],
5-Chlor-4-nitrothiophen-2-sulfonsäure-(4-chlorphenyl)-(3-trifluomlethylbenzyl)-amid [67],
4-Nitrothiophen-2-sulfonsäure-(4-chlorphenyl)-amid [69],
5-Chlor-4-nitrothiophen-2-sulfonsäure-(3-trifluormethylbenzyl)-(4-trifluormethylbenzyl)-amid [75] und N-(1*H*-Benzoimidazol-2-yl)-4-chlor-3-nitrobenzensulfonamid [83].

41. Verbindung nach Anspruch 39, welche unter den folgenden ausgewählt ist:
5-Chlor-4-nitrothiophen-2-sulfonsäure-(4-chlorphenyl)-amid [2],
5-Chlor-4-nitrothiophen-2-sulfonsäure-(4-fluorphenyl)-amid [3],
5-Chlor-4-nitrothiophen-2-sulfonsäure-(4-hydroxyphenyl)-amid [5],
5-Chlor-4-nitrothiophen-2-sulfonsäure-(4-trifluormethylphenyl)-amid [6],
5-Chlor-4-nitrothiophen-2-sulfonsäure-(4-fluorphenyl)-methylamid [7],
5-Bromthiophen-2-sulfonsäure-(3,5-bis-trifluormethylphenyl)-amid [18],
5-Benzensulfonylthiophen-2-sulfonsäure-(4-trifluormethylphenyl)-amid [23],
4,5-Dibromthiophen-2-sulfonsäure-(3-trifluormethylphenyl)-amid [27],
4,5-Dibromthiophen-2-sulfonsäure (3,4-dichlorphenyl)-amid [28],
5-Chlorthiophen-2,4-disulfonsäure-bis-[(4-trifluormethylphenyl)-amid] [33],
4-Chlor-3-nitro-N-(4-trifluormethylphenyl)-benzensulfonamid [35],
5-Chlor-4-nitrothiophen-2-sulfonsäure-(3,5-dichlorphenyl)-amid [39],
5-Chlor-4-nitrothiophen-2-sulfonsäure-(3,5-difluorphenyl)-amid [40],
5-(1-Methyl-5-trifluormethyl-1 H-pyrazol-4-yl)-thiophen-2-sulfonsäure-(3,5-bis-trifluormethylphenyl)-amid [43],
5-Chlor-4-nitrothiophen-2-sulfonsäure-(3,5-bis-trifluormethylphenyl)-amid [44],
5-Chlor-4-nitrothiophen-2-sulfonsäure-4-fluorbenzylamid [45],
5-Chlor-4-nitrothiophen-2-sulfonsäure-4-trifluormethylbenzylamid [46],
5-Chlor-4-nitrothiophen-2-sulfonsäure-[2-(1H-indol-3-yl)-ethyl]-amid [48],
5-Chlor-4-nitrothiophen-2-sulfonsäure-[2-(1H-indol-3-yl)-1-methylethyl]-amid [49],
5-Chlor-4-nitrothiophen-2-sulfonsäure-(4-trifluormethylphenyl)-amid [50],
5-Chlor-4-nitrothiophen-2-sulfonsäure-(4-chlorphenyl)-methylamid [51] und
5-Chlor-4-nitrothiophen-2-sulfonsäuremethyl-(4-trifluormethylbenzyl)-amid [52],
5-Chlor-4-nitrothiophen-2-sulfonsäurebenzyl-(4-fluorbenzyl)-amid [53],
6-Chlor-4-nitrothiophen-2-sulfonsäure-3,5-dichlorbenzylamid [54],
5-Chlor-4-nitrothiophen-2-sulfonsäure-3,5-difluorbenzylamid [55],
5-Chlor-4-nitrothiophen-2-sulfonsäure-4-chlorbenzylamid [56],
5-Chlor-4-nitrothiophen-2-sulfonsäure-[1-(4-fluorphenyl)-ethyl]-amid [57],
5-Chlor-4-nitrothiophen-2-sulfonsäure-(1*H*-benzoimidazol-2-yl)-amid [59],
5-Chlor-4-nitrothiophen-2-sulfonsäure-[2-(6-chlor-1*H*-indol-3-yl)-ethyl]-amid [60],
5-Chlor-4-nitrothiophen-2-sulfonsäure-(4-methoxyphenyl)-amid [61],
5-Chlor-4-nitrothiophen-2-sulfonsäurephenylamid [62],
5-Chlor-4-nitrothiophen-2-sulfonsäure-p-tolylamid [63],
5-Chlor-4-nitrothiophen-2-sulfonsäurebenzylamid [64],
5-Chlor-4-nitrothiophen-2-sulfonsäurebenzyl-(4-chlorphenyl)-amid [65],
5-Chlor-4-nitrothiophen-2-sulfonsäurebenzyl-(4-methoxyphenyl)-amid [66],
5-Chlor-4-nitrothiophen-2-sulfonsäure-(4-chlorphenyl)-(3-trifluormethylbenzyl)-amid [67],
4-Nitrothiophen-2-sulfonsäure-(4-chlorphenyl)-amid [69],
5-Chlorthiophen-2,4-disulfonsäure-bis-[(4-chlorphenyl)-amid] [70],
Thioessigsäure-5-[5-(4-chlorphenylsulfamoyl)-3-nitrothiophen-2-yl]-ester [72],
5-Methyl-4-nitrothiophen-2-sulfonsäure-(4-chlorphenyl)-amid [73],
5-Methylthiophen-2,4-disulfonsäure-bis-[(4-chlorphenyl)-amid] [74],
5-Chlor-4-nitrothiophen-2-sulfonsäure-(3-trifluormethylbenzyl)-(4-trifluormethylbenzyl)-amid [75],
5-(2-Methoxyethylamino)-4-nitrothiophen-2-sulfonsäure-(4-chlorphenyl)-amid [80],
N-(1*H*-Benzoimidazol-2-yl)-4-chlor-3-nitrobenzensulfonamid [83],
5-Chlor-3-methylbenzo[b]thiophen-2-sulfonsäure-(4-chlor-3-nitrophenyl)-amid [88],
N-(3,5-Bis-trifluormethylphenyl)-3-chlor-4-methoxybenzensulfonamid [104],
N-(3-Chlor-4-nitrophenyl)-3,5-bis-trifluormethylbenzensulfonamid [105],
4,5-Dibromthiophen-2-sulfonsäure-(3,5-dichlorphenyl)-amid [112],
5-Benzensulfonylthiophen-2-sulfonsäure-(3,5-bis-trifluormethylphenyl)-amid [117],
5-Chlor-3-methylbenzo[b]thiophen-2-sulfonsäure-(3,5-bis-trifluormethylphenyl)-amid [119],
Benzo[b]thiophen-2-sulfonsäure-(3,5-bis-trifluormethylphenyl)-amid [120],
Benzo[1,2,5]thiadiazol-5-sulfonsäure-(3,5-bis-trifluormethylphenyl)-amid [122],
4,5-Dibromthiophen-2-sulfonsäure-(4-chlorphenyl)-amid [125],
4,5-Dibromthiophen-2-sulfonsäure-(4-trifluormethylphenyl)-amid [126],
N-(3,5-Bis-trifluormethylphenyl)-2,6-dichlorbenzensulfonamid [128],
5-(3-Hydroxypiperidin-1 -yl)-4-nitrathiophen-2-sulfonsäure-(4-chlorphenyl)-arnid [130] und 5-Chlor-4-nitrothiophen-2-sulfonsäure-(4-nitrophenyl)-amid [131].

42. Pharmazeutische Zusammensetzung, welche eine Verbindung, wie sie in einem der Ansprüche 24 bis 41 definiert ist, im Gemisch mit einem oder mehreren pharmazeutisch verträglichen Verdünnungsmitteln, Hilfsstoffen oder Trägern umfaßt.

43. Verwendung einer Verbindung, wie sie in einem der Ansprüche 1 bis 41 definiert ist, in einem Test zur Bestimmung der Bindung an HDM2.

44. Verwendung nach Anspruch 43, wobei der Test Kandidatenverbindungen bestimmen kann, die in der Lage sind, die Wechselwirkung zwischen HDM2 und p53 und/oder E2F zu hemmen.

45. Verwendung nach einem der Ansprüche 43 oder 44, wobei der Test ein kompetitiver Bindungstest ist.

46. Verwendung nach Anspruch 45, wobei der kompetitive Bindungstest das Inkontaktbringen einer Verbindung der Formel I, wie sie in einem der Ansprüche 1 bis 22 definiert ist, mit HDM2 in der Gegenwart eines von p53 abgeleiteten Peptids und das Detektieren irgendeiner Veränderung in der Wechselwirkung zwischen HDM2 und dem von p53 abgeleiteten Peptid umfaßt.

47. Verwendung nach Anspruch 46, wobei das von p53 abgeleitete Peptid ein fluoreszenzmarkiertes oder biotinyliertes von p53 abgeleitetes Peptid ist,

48. Verfahren zum Detektieren der Bindung eines Liganden an HDM2, wobei das Verfahren die folgenden Stufen umfaßt.
(i) Inkontaktbringen eines Liganden mit HDM2 in der Gegenwart eines von p53 abgeleiteten Peptids und
(ii) Detektieren irgendeiner Veränderung in der Wechselwirkung zwischen HDM2 und dem von p53 abgeleiteten Peptid,
und wobei der Ligand eine Verbindung ist, wie sie in einem der Ansprüche 1 bis 22 definiert ist.

49. Kombination, welche wenigstens eine Verbindung, wie sie in einem der Ansprüche 1 bis 41 definiert ist, und wenigstens ein zytotoxisches Mittel umfaßt.

50. Kombination nach Anspruch 49, wobei das zytotoxische Mittel ein chemotherapeutisches Mittel ist.

51. Kombination nach Anspruch 50, wobei das chemotherapeutische Mittel Cisplatin oder Etoposid ist.

52. Pharmazeutische Zusammensetzung, welche wenigstens eine Verbindung, wie sie in einem der Ansprüche 1 bis 41 definiert ist, und ein oder mehrere zytotoxische Mittel im Gemisch mit einem pharmazeutisch verträglichen Verdünnungsmittel, Hilfsstoff oder Träger umfaßt.

53. Verwendung wenigstens einer Verbindung, wie sie in einem der Ansprüche 1 bis 41 definiert ist, bei der Herstellung eines Medikaments zur Behandlung einer proliferativen Störung, wobei die Verbindung für die aufeinanderfolgende, gleichzeitige oder sequentielle Verwendung mit einem oder mehreren anderen zytotoxischen Mitteln bestimmt ist.

54. Verwendung wenigstens einer Verbindung, wie sie in einem der Ansprüche 1 bis 41 definiert ist, bei der Herstellung eines Medikaments zur Behandlung einer proliferativen Störung, wobei die Verbindung für die aufeinanderfolgende, gleichzeitige oder sequentielle Verwendung mit Radiotherapie bestimmt ist.

## Revendications

1. Utilisation d'un composé de formule I, 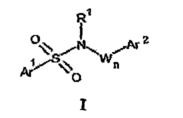 dans laquelle
W est un groupe alkylène en C₁ à C₅ ramifié ou non ramifié ou un groupe alcénylène en C₂ à C₅ ;
n est 0 ou 1 ;
R¹ est H, un groupe alkyle en C₁ à C₈ ramifié ou non ramifié, un groupe alcényle en C₂ à C₈, ou un groupe aryle ou aralkyle, chacun pouvant être facultativement substitué par un ou plusieurs groupes halogène ou CF₃ ;
Ar¹ est 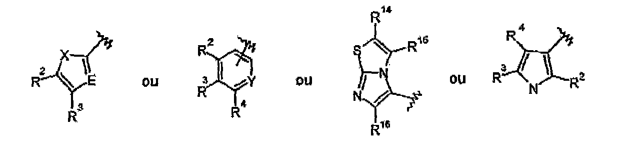 où
X est S, O, NH ou NR' où R' est un groupe alkyle en C₁ à C₃;
Y est CH ou N ;
E est N ou CR⁴ ;
R², R³, R⁴ et R¹⁴⁻¹⁶ sont chacun indépendamment (A)ₚB, où A est un groupe alkyle en C₁ à C₃, p est 0 ou 1, et B est H, un halogène, un groupe alkyle en C₁ à C₅, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, un groupe alicyclique contenant facultativement un ou plusieurs hétéroatomes, facultativement substitué par un ou plusieurs groupes OH, COR^{u}, halogéno ou CF₃, ou un groupe hétéroaryle facultativement substitué par un ou plusieurs groupes alkyle en C₁ à C₅, halogéno, SRⁱ ou CF₃; ou bien
R² et R³ sont reliés pour former un système cyclique saturé ou insaturé, contenant facultativement un ou plusieurs hétéroatomes, et facultativement substitué par un ou plusieurs groupes halogéno, OH ou CF₃ ;
Ar^{a-f} sont chacun indépendamment des groupes aryle facultativement substitués par un ou plusieurs groupes alkyle en C₁ à C₅, halogéno ou CF₃;
R^{a-i}, R^{s}, R^{t} et R^{u} sont chacun indépendamment des groupes alkyle en C₁ à C₅ facultativement substitués par un ou plusieurs groupes alkoxy, halogéno ou CF₃;
et à condition qu'au moins l'un de R², R³ et R⁴ soit autre chose que H;
Ar² est 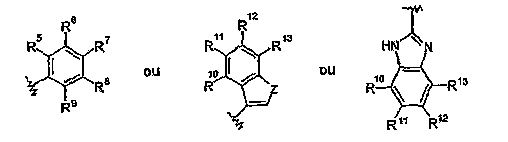 où
Z est S, O, NH ou NR" où R" est un groupe alkyle en C₁ à C₃ ;
R⁵, R⁶, R⁷, R⁸ et R⁹ sont chacun indépendamment (L)_{q}M où L est un groupe alkyle en C₁ à C₃, q est 0 ou 1, M est H, un groupe alkyle en C₁ à C₅, un halogène, NO₂, OH, NH₂, NHAr^{g}, NHR^{j}, NR^{k}R^{l}, SO₃H, SO₂NH₂, SO₂NHAr^{h}, SO₂NHR^{m}, SO₂Arⁱ, SO₂Rⁿ, CF₃, CN, COOH, COOR^{p}, CONH₂, CONHAr^{j}, CONHR^{q}, OR^{v}, COAr^{k} ou COR^{r};
R^{j-r}, R^{v} sont chacun indépendamment des groupes alkyle en C₁ à C₅ ;
Ar^{g-k} sont chacun indépendamment des groupes aryle :
et à condition qu'au moins l'un des substituants R⁵, R⁶, R⁷, R⁸ et R⁹ soit autre chose que H ;
R¹⁰, R¹¹, R¹² et R¹³ sont chacun indépendamment H, un groupe alkyle en C₁ à C₅, un halogène, NO₂, OH, NH₂ ou CF₃ ;
dans la préparation d'un médicament destiné au traitement d'un trouble prolifératif.

2. Utilisation selon la revendication, dans laquelle Ar¹ est 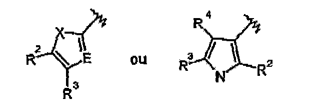 et Ar² est

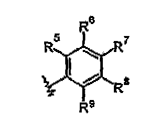

3. Utilisation selon la revendication 1, dans laquelle Ar¹ est 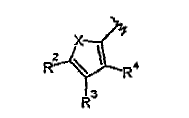 et Ar² est

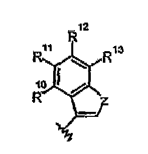

4. Utilisation selon la revendication 1, dans laquelle Ar¹ est 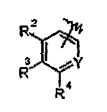 et Ar² est

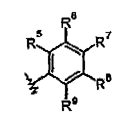

5. Utilisation selon la revendication 1, dans laquelle Ar¹ est 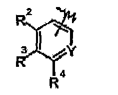 et Ar² est

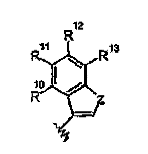

6. Utilisation selon la revendication 1, dans laquelle Ar¹ est 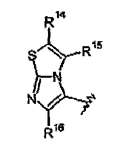 et Ar² est

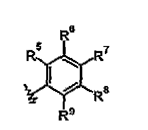

7. Utilisation selon la revendication 1, dans laquelle Ar¹ est 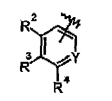 et Ar² est

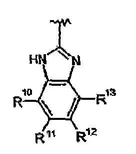

8. Utilisation selon l'une quelconque des revendications précédentes, dans laquelle
R² , R³ et R⁴ sont chacun indépendamment (A)ₚB, où A est un groupe alkyle en C₁ à C₅, p est 0 ou 1, et B est H, F, Cl, Br, I, un groupe alkyle en C₁ à C₅, NO₂, OH, NH₂, NHR^{a}, NR^{b}R^{c}, SO₃H, SO₂NH₂, NHPh, SO₂NHAr^{b}, SO₂NHR^{d}, SO₂Ph, SO₂R^{e}, CF₃, CN, COOH, COOR^{f}, CONH₂, COONHPh, CONHR^{g}, S(CO)R^{s}, OR^{t}, OAr^{f}, COPh, COR^{h}, un groupe morpholino, pipérazino ou pipéridino dont chacun peut être facultativement substitué par un ou plusieurs groupes OH ou COR^{u}, ou un groupe hétéroaryle choisi parmi les groupes pyridyle, pyrimidyle, oxazolyle, thiazolyle et pyrazolyle, dont chacun peut être facultativement substitué par un ou plusieurs groupes alkyle en C₁ à C₅, halogéno, SRⁱ ou CF₃, ou bien R² et R³ forment ensemble un cycle hexagonal saturé ou un cycle pentagonal insaturé, dont chacun peut contenir facultativement un ou plusieurs hétéroatomes ; et
R⁵, R⁶, R⁷, R⁶ et R⁹ sont chacun indépendamment (L)_{q}M où L est un groupe alkyle en C₁ à C₅, q est 0 ou 1, M est H, un groupe alkyle en C₁ à C₅, un halogène, NO₂, OH, NH₂, NHPh, NHR^{j}, NR^{k}R^{l} SO₃H, SO₂NH₂, SO₂NHPh, SO₂NHR^{m}, SO₂Ph, SO₂Rⁿ, CF₃, CN, COOH, COOR^{p}, CONH₂, CONHPH, CONHR^{q}, OR^{v}, COPh ou COR^{r}.

9. Utilisation selon l'une quelconque des revendications précédentes, dans laquelle R², R³ et R⁴ sont chacun indépendamment (A)ₚB où A est un groupe alkyle en C₁ à C₅, p est 0 ou 1, et B est H, F, Cl, Br, I, un groupe alkyle en C₁ à C₅, NO₂, OH, NH₂, NHR^{a} NR^{b}R^{c}, SO₃H, SO₂NH₂, NHPh, SO₂NHPh, SO₂NHR^{d}, SO₂Ph, SO₂R^{e}, CF₃, CN, COOH, COOR^{f}, CONH₂, COONHPh, CONHR^{g}, S(CO)R^{s}, OR^{t}, OAr^{f}, COPh, COR^{h}, pyridyle, pyrimidyle, 2-méthylsulfanylpyrimid-5-yle, oxazole-2-yle, thiazole-2-yle, 1-methyl-5-trifluoromethyl-1*H*-pyrazole-4-yle, morpholine-4-yle, 4-acétylpipérazine-1-yle, 3-hydroxypipéridine-1-yle, ou bien R² et R³ forment ensemble un groupe
-OCH₂CH₂O-
-N-S-N- ou
un groupe phényle facultativement substitué par un ou plusieurs halogènes.

10. Utilisation selon l'une quelconque des revendications précédentes, dans laquelle R², R³ et R⁴ sont chacun indépendamment H, un halogène, NO₂, SO₂Ph, S(CO)Me, COOH, COOEt, OPh, OMe, NHCH₂CH₂OMe, un groupe 1-méthyl-5-trifluorométhyl-1*H*-pyrazole-4-yle, 2-méthylsulfanyl-pyrimid-5-yle, N-(4-fluorophényl)sulfonamido, N-(4-trifluorométhylphényl)sulfonamido, oxazole-2-yle, alkyle en C₁ à C₅, NH₂, morpholine-4-yle, 4-acétyl-pipérazine-1-yle, 3-hydroxy-pipéridine-1-yle, ou bien R² et R³ forment ensemble
OCH₂CH₂O-
-N-S-N- ou
un groupe phényle facultativement substitué par un ou plusieurs halogènes.

11. Utilisation selon l'une quelconque des revendications 1 à 3 ou 8 à 10, dans laquelle Ar¹ est 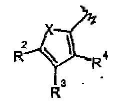
X est S ou N ;
R², R³ et R⁴ sont chacun indépendamment un groupe alkyle en C₁ à C₅, S(CO)Me, COOH, NHCH₂CH₂OMe, COOEt, H, un halogène, NO₂, SO₂Ph, un groupe SO₂NH-(4-chlorophényle), 1-méthyl-5-trifluorométhyl-1*H*-pyrazole-4-yle, morpholine-4-yle, 2-méthylsulfanylpyrimid-5-yle, N-(4-fluorophényl)sulfonamido, N-(4-trifluorométhylphényl)sulfonamido, 3-hydroxypipéridine-1-yle, pyridine-2-yle ; ou bien R² et R³ forment un groupe phényle facultativement substitué par un ou plusieurs halogènes.

12. Utilisation selon la revendication 11, dans laquelle
- R² est un halogène, SO₂Ph, NO₂, Et, SOMe, un groupe morpholine-4-yle, NHCH₂CH₂OMe, 3-hydroxypipéridine-1-yle, 1-méthyl-5-trifluorométhyl-1H-pyrazole-4-yle ou 2-méthylsulfanylpyrimid-5-yle ;
- R³ est un halogène, un groupe SO₂NH-(4-chloxophényle), H, NO₂, N-(4-fluorophényl)sulfonamido ou N-(4-trifluorométhylphényl)sulfonamido ; et
- R⁴ est H.

13. Utilisation selon l'une quelconque des revendications 1, 4, 5, 7 à 10, où Ar¹ est 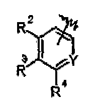
Y est CH ou N ; et
R², R³ et R⁹ sont chacun indépendamment H, un groupe OH, COOH, CF₃, OPh, OMe, NO₂, 4-acétylpipérazine-1-yle, NH₂, un halogène, un groupe pyrazole-1-yle, oxazole-2-yle ou alkyle en C₁ à C₅, ou bien R² et R³ forment ensemble -OCH₂CH₂O- ou -N-S-N-.

14. Utilisation selon la revendication 13, dans laquelle
lorsque Y est CH
- R² est H, NO₂ ou Cl ;
- R³ est NO₂, NH₂, Cl, CF₃, COOH, un groupe 4-acétyl-pipérazine-1-yle ; et
- R⁴ est H, Cl, un groupe oxazole-2-yle, OH, NO₂, NH₂, OMe ou Me ; ou bien
lorsque Y est N
- R² est H;
- R³ est Br ;
- R⁴ est Cl ou OPh.

15. Utilisation selon l'une quelconque des revendications 1, 2 ou 8 à 10, dans laquelle Ar¹ est 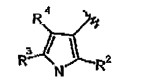
R² et R⁴ sont des groupes alkyle en C₁ à C₅, et R³ est COOH ou COOEt.

16. Utilisation selon l'une quelconque des revendications précédentes, dans laquelle
- R⁵, R⁶, R⁷, R⁸ et R⁹ sont chacun indépendamment H, un halogène, OMe, NO₂, un groupe alkyle en C₁ à C₅, CF₃ ou OH ; et
- R¹⁰, R¹¹, R¹² et R¹³ sont tous H.

17. Utilisation selon la revendication 16, dans laquelle
- R⁵ est H, un groupe alkyle en C₁ à C₅ ou un halogène ;
- R⁶ est H, un halogène, NO₂ ou CF₃ ;
- R⁷ est H, un halogène, OMe, NO₂, OH ou CF₃ :
- R⁸ est H, un halogène ou CF₃ ;
- R⁹ est H.

18. Utilisation selon l'une quelconque des revendications précédentes, dans laquelle
- W est CH₂, CH₂CH₂ ou CH(CH₃)CH₂ ; et
- R¹ est H, CH₂Ph, CH₂CH (Me)., un groupe 3- (trifluorométhyl)benzyle ou Me.

19. Utilisation selon l'une quelconque des revendications précédentes, dans laquelle ledit composé de formule I est choisi parmi les suivants :
(3-trifluorométhylphényl)amide d'acide 5-chloro-4-nitrothiophène-2-sulfonique [1];
(4-chlorophényl)amide d'acide 5-chloro-4-nitrothiophène-2-sulfonique [2];
(4-fluorophényl)amide d'acide 5-chloro-4-nitrothiophène-2-sulfonique [3];
(4-fluorophényl)amide d'acide 4-bromo-5-chlorothiophène-2-sulfonique [4];
(4-hydroxyphényl)amide d'acide 5-chloro-4-nitrothiophène-2-sulfonique [5] ;
(4-trifluorométhylphényl)amide d'acide 5-chloro-4-nitrothiophène-2-sulfonique [6] ;
(4-fluorophényl)méthylamide d'acide 5-chloro-4-nitrothiophène-2-sulfonique [7] ;
(3,5-bis-trifluorométhylphényl)amide d'acide 4,5-dibromothiophène-2-sulfonique [8] ;
(4-trifluorométhylphényl)amide d'acide 5-chlorothiophène-2-sulfonique [9] ;
(4-chlorophényl)amide d'acide 5-chlorothiophène-2-sulfonique [10] ;
(3,5-bis-trifluorométhylphényl) amide d'acide 5-chlorothiophéne-2-sulfonique [11] ;
(3,5-dichlorophényl)amide d'acide 5-(2-mèthylsulfanylpyrimidine-5-yl)thiophène-2-sulfonique [12] ;
4-oxazole-2-yl-N-(4-trifluorométhylphényl)benzènesulfonamide [13] ;
N-(3,5-bis-trifluorométhylphényl)-4-oxazole-2-yl-benzènesulfonamide [14] ;
(4-trifluorométhylphényl)amide d'acide 4-bromo-5-chlorothiophène-2-sulfonique [15] ;
(4-chlorophényl)amide d'acide 5-bromothiophène-2-sulfonique [16] ;
(3,5-dichlorophényl)amide d'acide 5-bromothiophène-2-sulfonique [17] ;
(3,5-bis-trifluorométhylphényl)amide d'acide 5-bromothiophène-2-sulfonique [18] ;
N-(4-chlorophényl)-3-nitrobenzènesulfonamide [19] ;
3-nitro-N-(4-trifluoraméthylphényl)benzènesulfonamide [20] ;
N-(3,5-bis-trifluorométhylphényl)-3-nitrobenzènesulfonamide [21] ;
N-(2,4-dichlorophényl)-3-nitrobenzènesulfonamide [22] ;
(4-trifluorométhylphényl)amide d'acide 5-benzènesulfonylthiophène-2-sulfonique [23] ;
(4-chlorophényl)amide d'acide 5-benzènesulfonylthiophène-2-sulfonique [24] ;
(3,5-dichlorophényl)amide d'acide 5-benzènesulfonylthiophène-2-sulfonique [25] ;
(3,4-dichlorophényl)amide d'acide 5-chlorothiophène-2-sulfonique [26] ;
(3-trifluorométhylphényl)amide d'acide 4,5-dibromothiophène-2-sulfonique [27] ;
(3,4-dichlorophényl)amide d'acide 4,5-dibromothiophène-2-sulfonique [28];
N-(3,5-bis-trifluorométhylphényl)-4-chloro-3-nitrobenzénesulfonamide [29] ;
4-chloro-N-(3,4-dichlorophényl)-3-nitrobenzènesulfonamide [30] ;
(4-trifluorométhylphényl)amide d'acide 5-(1-méthyl-5-trifluorométhyl-1H-pyrazole-4-yl)thiophène-2-sulfonique [31] ;
bis[(4-fluorophényl)amide] d'acide 5-chlorothiophène-2,4-disulfonique [32];
bis[(4-trifluorométhylphényl)amide] d'acide 5-chlorothiophène-2,4-disulfonique [33];
4-méthyl-3-nitro-N-(4-trifluorométhylphényl)benzènesulfonamide [34] ;
4-chloro-3-nitro-N-(4-trifluorométhylphényl)benzènesulfonamide [35];
3-amino-4-méthyl-N-(4-trifluorométhylphényl)benzènesulfonamide [36] ;
N-(4-chlorophényl)-4-méthyl-3-nitrobenzènesulfonamide [37] ;
4-chloro-N-(4-chlorophényl)-3-nitrobenzènesulfonamide [38] ;
(3,5-dichlorophényl)amide d'acide 5-chloro-4-nitrothiophène-2-sulfonique [39] ;
(3,5-difluorophényl)amide d'acide 5-chloro-4-nitrothiophène-2-sulfonique [40] ;
(4-trifluorométhylphényl)amide d'acide 5-bromo-6-chloropyridine-3-sulfonique [41] ;
(3,5-bis-trifluorométhylphényl)amide d'acide 5-bromo-6-chloropyridine-3-sulfonique [42] ;
(3,5-bis-trifluorométhylphényl)amide d'acide 5-(1-méthyl-5-trifluorométhyl-1*H*-pyrazole-4-yl)thiophène-2-sulfonique [43] ;
(3,5-bis-trifluorométhylphényl)amide d'acide 5-chloro-4-nitxothiophène-2-sulfonique [44] ;
4-fluorobenzylamide d'acide 5-chloro-4-nitrothiophène-2-sulfonique [45] ;
4-trifluorométhylbenzylamide d'acide 5-chloro-4-nitrothiophène-2-sulfonique [46] ;
4-chloro-N-(3,5-dichloraphényl)-3-nitrobenzènesulfonamide [47] ;
[2-(1*H*-indole-3-yl)éthyl]amide d'acide 5-chloro-4-nitrothiophène-2-sulfonique [48] ;
[2-(1H-indole-3-yl)-1-méthyléthyl]amide d'acide 5-chloro-4-nitzothiophène-2-sulfonique [49];
méthyl-(4-trifluorométhylphënyl)amide d'acide 5-chloro-4-nitrothiophène-2-sulfonique [50] ;
(4-chloxophényl)méthylamide d'acide 5-chloro-4-nitrothiophène-2-sulfonique [51] ; et
méthyl-(4-trifluorométhylbenzyl)amide d'acide 5-chloro-4-nitrothiophène-2-sulfonique [52];
benzyl-(4-fluorobenzyl)amide d'acide 5-chloro-4-nitrothiophène-2-sulfonique [53] ;
3,5-dichlorobenzylamide d'acide 5-chloro-4-nitrothiophène-2-sulfonique [54] ;
3,5-difluorobenzylamide d'acide 5-chloro-4-nitrothiophène-2-sulfonique [55] ;
4-chlorobenzylamide d'acide 5-chloro-4-nitrothiophène-2-sulfonique [56] ;
[1-(4-fluorophényl)éthyl]amide d'acide 5-chloro-4-nitrothiophène-2-sulfonique [57] ;
(4-chlorophényl)isobutylamide d'acide 5-chloro-4-nitrothiophène-2-sulfonique [58] ;
(1H-benzo-imidazole-2-yl)amide d'acide 5-chloro-4-nitrothiophène-2-sulfonique [59] ;
[2-(6-chloro-1*H*-indole-3-yl)éthyl]amide d'acide 5-chloro-4-nitrothiophène-2-sulfonique [60] ;
(4-méthoxyphényl)amide d'acide 5-chloro-4-nitrothiophène-2-sulfonique [61] ;
*p*-tolylamide d'acide 5-chloro-4-nitrothiophène-2-sulfonique [63] ;
benzyl-(4-chlorophényl)amide d'acide 5-chloro-4-nitrothiophène-2-sulfonique [65] ;
benzyl-(4-méthoxyphényl)amide d'acide 5-chloro-4-nitrothiophène-2-sulfonique [66] ;
(4-chlorophényl)-(3-trifluorométhylbenzyl)amide d'acide 5-chloro-4-nitrothiophène-2-sulfonique [67] ;
(4-chlorophényl)amide d'acide 5-nitrothiophène-2-sulfonique [68] ;
(4-chlorophényl)amide d'acide 4-nitrothiophène-2-sulfonique [69] ;
bis[(4-chlorophényl)amide] d'acide 5-chloro-thiophène-2,4-disulfonique [70] ;
(4-chlorophényl)amide d'acide 5-éthyl-4-nitrothiophène-2-sulfonique [71] ;
ester 5-[5-(4-chlorophénylsulfamoyl)-3-nitrothiophène-2-ylique] d'acide thioacétique [72] ;
(4-chlorophényl)amide d'acide 5-méthyl-4-nitrothiaphène-2-sulfonique [73] ;
bis[(4-chlorophényl)amide] d'acide 5-méthyl-thiophène-2,4-disulfonique [74];
(3-trifluorométhylbenzyl)-(4-trifluorométhylbenzyl)-amide d'acide 5-chloro-4-nitrothiophène-2-sulfonique [75];
(4-trifluorométhylphényl)amide d'acide 4-nitrothiophène-2-sulfonique (76] ;
[2-(1*H*-indole-3-yl)éthyl]amide d'acide 4-nitrothiophène-2-sulfonique [77] ;
(3,5-bis-trifluorométhylphényl)-(3-trifluorométhylbenzyl)amide d'acide 5-(1-méthyl-5-trifluorométhyk-1*H*-pyrazole-3-yl)thiophène-2-sulfonique [78] ;
(4-chlorophényl)amide d'acide 5-morpholine-4-yl-4-nitrothiophène-2-sulfonique [79] ;
(4-chlorophényl)amide d'acide 5-(2-méthoxyéthylamino)-4-nitrothiophène-2-sulfonique [80] ;
4-chloro-N-[2-(5-chloro-1*H*-indole-3-yl)éthyl]-3-nitrobenzènesulfonamide [81] ;
N-[2-(5-chloro-1*H*-indole-3-yl)éthyl]-4-méthyl-3-nitrobenzènesulfonamide [82] ;
N-(1*H*-benzoimidazole-2-yl)-4-chloro-3-nitrobenzènesulfonamide [83] ;
(3,5-bis-trifluorométhylphényl)amide d'acide 6-chloroimidazo[2,1-b]thiazole-5-sulfonique [84] ;
(4-chlorophényl)amide d'acide 2,3-dihydrobenzo-[1,4]dioxine-6-sulfonique [85] ;
(3,5-bis-trifluorométhylphényl)amide d'acide 2,3-dihydrobenzo[1,4]dioxine-6-sulfonique [86] ;
(4-chlorophényl)amide d'acide 6-phénoxypyridine-3-sulfonique [87] ;
(4-chloro-3-nitrophényl)amide d'acide 5-chloro-3-méthylbenzo[b]thiophène-2-sulfonique [88] ;
N-(3,5-bis-trifluorométhylphényl)-4-pyrazole-1-yl-benzènesulfonamide (89] ;
ester éthylique d'acide 4-(4-chlorophénylsulfamoyl)-3,5-diméthyl-1*H*-pyrrole-2-carboxylique [90];
acide 4-(3,5-bis-trifluorométhylphénylsulfamoyl)-3,5-diméthyl-1*H*-pyrrole-2-carboxylique [91];
acide 4-(4-chlorophénylsulfamoyl)-3,5-diméthyl-1*H*-pyrrole-2-carboxylique [92];
ester éthylique d'acide 2-(4-chlorophénylsulfamoyl)-4-méthyl-thiazole-5-carboxylique [93] ;
3,5-dichloro-N-(4-chlorophényl)-4-hydroxybenzènesulfonamide [94] ;
N-(3,5-bis-trifluorométhylphényl)-3,5-dichloro-4-hydroxybenzènesulfonamide [95] ;
3,5-dichloro-4-hydroxy-N-(4-trifluorométhylphényl)-benzènesulfonamide [96] ;
N-(4-chlorophényl)-4-nitrobenzènesulfonamide [97];
N-(3,5-bis-trifluorométhylphényl)-4-nitrobenzènesulfonamide [98] ;
4-amino-N-(3,5-bis-trifluorométhylphényl)-3-chlorobenzènesulfonamide [99] ;
3-nitro-N-(4-trifluorométhylphényl)benzènesulfonamide [100] ;
3,5-dichloro-N-(3,5-dichlorophényl)-4-hydroxybenzènesulfonamide [101] ;
9-amino-3-chloro-N-(4-chlorophényl)benzènesulfonamide [102] ;
3-chloro-N-(4-chlorophényl)-4-méthoxybenzènesulfonamide [103] ;
N-(3,5-bis-trifluorométhylphényl)-3-chloro-4-méthoxybenzènesulfonamide [104] ;
N-(3-chloro-4-nitrophényl)-3,5-bis-trifluorométhylbenzènesulfonamide [105] ;
3-(4-acétylpipérazine-1-yl)-N-(3,5-bis-trifluorométhylphényl)-4-nitrobenzènesulfonamide [106] ;
N-(3,5-bis-trifluorométhylphényl)-2-nitrobenzènesulfonamide [107] ;
acide 3-(3,5-bis-trifluorométhylphénylsulfamoyl)-benzoïque [108] ;
3,5-dichloro-N-(4-chlorobenzyl)-4-hydroxybenzènesulfonamide [109];
3,5-dichloro-4-hydroxy-N-(4-trifluorométhylbenzyl)-benzènesulfonamide [110];
3,5-dichloro-4-hydroxy-N-[2-(1*H*-indole-3-yl)éthyl]-benzènesulfonamide [111];
(3,5-dichlorophényl)amide d'acide 4,5-dibromothiophène-2-sulfonique [112];
N-(3,5-dichlorophényl)-4-oxazole-2-yl-benzènesulfonamide [113];
(3,5-bis-trifluorométhylphényl)amide d'acide 4-bromo-5-chlorothiophène-2-sulfonique [114] ;
(3,5-dichlorophényl)amide d'acide 4-bromo-5-chlorothiophène-2-sulfonique [115] ;
(4-trifluorométhylphényl)amide d'acide 5-bromothiophène-2-sulfonique [116] ;
(3,5-bis-trifluorométhylphényl) amide d'acide 5-benzènesulfonylthiophène-2-sulfonique [117] ;
(2,4-dichlorophényl)amide d'acide 5-benzènesulfonylthiophène-2-sulfonique [118] ;
(3,5-bis-trifluorométhylphényl)amide d'acide 5-chloro3-méthylbenzo[b]thiophène-2-sulfonique [119] ;
(3,5-bis-trifluorométhylphényl)amide d'acide benzo[b]-thiophène-2-sulfonique [120] ;
(4-chlorophényl)amide d'acide benzo[1,2,5]thiadiazole-5-sulfonique [121] ;
(3,5-bis-trifluorométhylphényl)amide d'acide benzo-[1,2,5]thiadiazole-5-sulfonique [122] ;
(9-trifluorométhylphényl)amide d'acide benzo[1,2,5]-thiadiazole-5-sulfonique [123] ;
(3,5-bis-trifluorométhylphényl)amide d'acide 5-pyridine-2-yl-thiophène-2-sulfonique [124] ;
(4-chlorophényl)amide d'acide 4,5-dibromothiophène-2-sulfonique [125] ;
(4-trifluorométhylphényl)amide d'acide 4,5-dibromothiophène-2-sulfonique [126] ;
3,5-dichloro-N-(4-fluorobenzyl)-4-hydroxybenzènesulfonamide [127] ;
N-(3,5-bis-trifluorométhylphényl)-2,6-dichlorobenzènesulfonamide [128] ;
(4-méthoxy-2-méthylphényl)amide d'acide 5-chloro-4-nitrothiophène-2-sulfonique [129] ;
(4-chlorophényl) amide d'acide 5-(3-hydroxypipéridine-1-yl)-4-nitrothiophène-2-sulfonique [130] ; et
(4-nitrophényl)amide d'acide 5-chloxo-4-nitrothiophène-2-sulfonique [131].

20. Utilisation selon la revendication 19, dans laquelle le composé de formule I est choisi parmi les suivants :
(4-chlorophényl)amide d'acide 5-chloro-4-nitrothiophène-2-sulfonique [2] ;
(4-fluorophényl)amide d'acide 5-chloro-4-nitrothiophène-2-sulfonique [3) ;
(4-hydroxyphényl)amide d'acide 5-chloro-4-nitrothiophène-2-sulfonique [5] ;
(4-trifluorométhylphènyl)amide d'acide 5-chloro-4-nitrothiophène-2-sulfonique [6] ;
(4-fluorophényl)méthylamide d'acide 5-chloro-4-nitrothiophène-2-sulfonique [7] ;
4-chloro-3-nitro-N-(4-trifluorométhylphényl)benzènesulfonamide [35] ;
4-chloro-N-(4-chlorophényl)-3-nitrobenzènesulfonamide [38] ;
(3,5-dichlorophényl)amide d'acide 5-chloro-4-nitrothiophène-2-sulfonique [39] ;
(3,5-difluorophényl)amide d'acide 5-chloro-4-nitrothiophène-2-sulfonique [40] ;
(3,5-bis-trifluorométhylphényl)amide d'acide 5-(1-méthyl-5-trifluorométhyl-1*H*-pyrazole-4-yl)thiophène-2-sulfonique [43] ;
(3,5-bis-trifluorométhylphényl)amide d'acide 5-chloro-4-nitrothiophène-2-sulfonique [44] ;
4-fluozobenzylamide d'acide 5-chloro-4-nitrothiophène-2-sulfonique [45] ;
4-trifluorométhylbenzylamide d'acide 5-chloro-4-nitrothiophène-2-sulfonique [46] ; 1
4-chloro-N-(3,5-dichlorophényl)-3-nitrobenzènesulfonamide [47] ;
[2-(1H-indole-3-yl)éthyl]amide d'acide 5-chloro-4-nitrothiophène-2-sulfonique [48] ;
[2-(1H-indole-3-yl)-1-méthyléthyl]amide d'acide 5-chloro-4-nitrothiophène-2-sulfonique [49] ;
(4-chlorophényl)méthylamide d'acide 5-chloro-9-nitrothiophène-2-sulfonique [51] ;
benzyl-(4-fluorobenzyl)amide d'acide 5-chloro-4-nitrothiophène-2-sulfonique [53] ;
3,5-diahlorobenzylamide d'acide 5-chloro-4-nitrothiophène-2-sulfonique [54] ;
3,5-difluorobenzylamide d'acide 5-chloro-4-nitrothiophène-2-sulfonique [55] ;
4-chlorobenzylamide d'acide 5-chloro-4-nitrothiophène-2-sulfonique [56] ;
[1-(4-fluorophényl)éthyl]amide d'acide 5-chloro-4-nitrothiophène-2-sulfonique [57] ;
(4-méthoxyphényl)amide d'acide 5-chloro-4-nitrothiophène-2-sulfonique [61] ;
p-tolylamide d'acide 5-chloro-4-nitrothiophène-2-sulfonique [63] ;
benzyl-(4-chlorophényl)amide d'acide 5-chloro-4-nitrothiophène-2-sulfonique [65] ;
benzyl-(4-méthoxyphényl)amide d'acide 5-chloro-4-nitrothiophène-2-sulfonique [66] ;
(4-chlorophényl)-(3-trifluorométhylbenzyl)amide d'acide 5-chloro-4-nitrothiophène-2-sulfonique [67] ;
(4-chlorophényl)amide d'acide 4-nitrothiophene-2-sulfonique [69];
(3-trifluorométhylbenzyl)-(4-trifluorométhylbenzyl)-amide d'acide 5-chloro-4-nitrothiophène-2-sulfonique [75] ; et
N-(1*H*-benzoimidazole-2-yl)-4-chloro-3-nitrobenzènesulfonamide [83].

21. Utilisation selon la revendication 19, dans laquelle le composé de formule I est choisi parmi les suivants:
(4-chlorophényl)amide d'acide 5-chloro-4-nitrothiophène-2-sulfonique [2];
(4-fluorophényl)amide d'acide 5-chloro-4-nitrothiophène-2-sulfonique [3);
(4-hydroxyphényl)amide d'acide 5-chloro-4-nitrothiophène-2-sulfonique [5] ;
(4-trifluorométhylphényl)amide d'acide 5-chloro-4-nitrothiophène-2-sulfonique [6] ;
(4-fluorophényl)méthylamide d'acide 5-chloro-4-nitrothiophène-2-sulfonique [7] ;
(4-trifluorométhylphényl)amide d'acide 5-chlorothiophène-2-sulfonique [9] ;
(3,5-bis-trifluorométhylphényl)amide d'acide 5-bromothiophène-2-sulfonique [18] ;
(4-trifluorométhylphényl)amide d'acide 5-benzènesulfonylthiophène-2-sulfonique [23] ;
(3-trifluorométhylphényl)amide d'acide 4,5-dibromothiophène-2-sulfonique [27] ;
(3,4-dichlorophényl)amide d'acide 4,5-dibromothiophène-2-sulfonique [28] ;
N-(3,5-bis-trifluorométhylphényl)-4-chloro-3-nitrobenzènesulfonamide [29] ;
bis[(4-trifluorométhylphényl)amide] d'acide 5-chlorothiophène-2,4-disulfonique [33] ;
4-chloro-3-nitro-N-(4-trifluorméthylphényl)benzènesulfonamide [35] ;
(3,5-dichlorophényl)amide d'acide 5-chloro-4-nitrothiophène-2-sulfonique [39] ;
(3,5-difluoraphényl)amide d'acide 5-chloro-4-nitrothiophène-2-sulfonique [40] ;
(3,5-bis-trifluorométhylphényl)amide d'acide 5-(1-méthyl-5-trifluorométhyl-1*H*-pyrazole-4-yl)thiophène-2-sulfonique [43] ;
(3,5-bis-trifluoraméthylphényl)amide d'acide 5-chloro-4-nitrothiophène-2-sulfonique [44] ;
4-fluorobenzylamide d'acide 5-chloro-4-nitrothiophène-2-sulfonique [45] ;
4-trifluorométhylbenzylamide d'acide 5-chloro-4-nitrothiophène-2-sulfonique [46] ;
[2-(1H-indole-3-yl)éthyl]amide d'acide 5-chloro-4-nitrothiophène-2-sulfonique [48] ;
[2-(1*H*-indole-3-yl)-1-méthyléthyl]amide d'acide 5-chloro-4-nitrothiophène-2-sulfonique [49] ;
méthyl-(9-trifluorométhylphényl)amide d'acide 5-chloro-4-nitrothiophène-2-sulfonique [50] ;
(4-chlorophényl)méthylamide d'acide 5-chloro-4-nitrothiophène-2-sulfonique [51] ; et
méthyl-(4-trifluorométhylbenzyl)amide d'acide 5-chloro-4-nitrothzophène-2-sulfonique [52] ;
benzyl-(4-fluorobenzyl)amide d'acide 5-chloro-4-nitrothiophène-2-sulfonique [53] ;
3,5-dichlorobenzylamide d'acide 5-chloro-4-nitrothiophène-2-sulfonique [54] ;
3,5-difluorobenzylamide d'acide 5-chloro-4-nitrothiophène-2-sulfonique [55] ;
4-chlorobenzylamide d'acide 5-chloro-4-nitrothiophène-2-sulfonique [56] ;
[1-(4-fluorophényl)éthyl]amide d'acide 5-chloro-4-nitrothiophène-2-sulfonique [57] ;
(1*H*-benzo-imidazole-2-yl)amide d'acide 5-chloro-4-nitrothiophène-2-sulfonique [59] ;
[2-(6-chloro-1*H*-indole-3yl)éthyl]amide d'acide 5-chloro-4-nitrothiophène-2-sulfonique [60] ;
(4-méthoxyphényl)amide d'acide 5-chloro-4-nitrothiophène-2-sulfonique [61] ;
*p*-tolylamide d'acide 5-chloro-4-nitrothiophène-2-sulfonique [63] ;
benzyl-(4-chlorophényl)amide d'acide 5-chloro-4-nitrothiophène-2-sulfonique [65] ;
benzyl-(4-méthoxyphényl)amide d'acide 5-chloro-4-nitrothiophène-2-sulfonique [66] ;
(4-chlorophényl)-(3-trifluorométhylbenzyl)amide d'acide 5-chloro-4-nitrothiophène-2-sulfonique [67] ;
(4-chlorophényl)amide d'acide 4-nitrothiophène-2-sulfonique [69] ;
bis[(4-chlorophényl)amide] d'acide 5-chloro-thiophène-2,4-disulfonique [70] ;
ester S-[5-(4-chlorophénylsulfamoyl)-3-nitrothiophène-2-ylique] d'acide thioacétique [72] ;
(4-chlorophényl)amide d'acide 5-méthyl-4-nitrothiophène-2-sulfonique [73];
bis[(4-chlorophényl)amide] d'acide 5-méthyl-thiophène-2,4-disulfonique [74] ;
(3-trifluorométhylbenzyl)-(4-trifluorométhylbenzyl)-amide d'acide 5-chloro-4-nitrothiophène-2-sulfonique [75] ;
(4-chlorophényl)amide d'acide 5-(2-méthoxyéthylamino)-4-nitrothiophène-2-sulfonique [80] ;
N-(1*H*-benzoimidazole-2-yl)-4-chloro-3-nitrobenzènesulfonamide [83] ;
(4-chloro-3-nitrophényl)amide d'acide 5-chloro-3-méthylbenzo[b]thiophène-2-sulfonique [88] ;
N-(3,5-bis-trifluorométhylphényl)-3-chloro-4-méthoxybenzènesulfonamide [104] ;
N-(3-chloro-4-nitrophényl)-3,5-bis-trifluorométhylbenzènesulfonamide [105] ;
(3,5-dichlorophényl)amide d'acide 4,5-dibromothiophène-2-sulfonique [112] ;
(4-trifluorométhylphényl)amide d'acide 5-bromothiophène-2-sulfonique [116] ;
(3,5-bis-trifluorométhylphényl)amide d'acide 5-benzènesulfonylthiophène-2-sulfonique [117] ;
(3,5-bis-trifluorométhylphényl)amide d'acide 5-chloro-3-méthylbenzo[b]thiophène-2-sulfonique [119] ;
(3,5-bis-trifluorométhylphényl)amide d'acide benzo[b]-thiophène-2-sulfonique [120] ;
(3,5-bis-trifluorométhylphényl)amide d'acide benzo-[1,2,5]thiadiazole-5-sulfonique [122] ;
(4-chlorophényl)amide d'acide 4,5-dibromothiophène-2-sulfonique [125] ;
(4-trifluorométhylphényl)amide d'acide 4,5-dibromothiophène-2-sulfonique [126] ;
N-(3,5-bis-trifluorométhylphényl)-2,6-dichlorobenzènesulfonamide [128] ;
(4-chlorophényl)amide d'acide 5-(3-hydroxypipéridine-1-yl)-4-nitrothiophène-2-sulfonique [130] ; et
(4-nitrophényl)amide d'acide 5-chloro-4-nitrothiophène-2-sulfonique [131].

22. Utilisation selon l'une quelconque des revendications précédentes, dans laquelle le trouble prolifératif est un cancer.

23. Utilisation selon l'une quelconque des revendications précédentes, dans laquelle ledit composé est administré en une quantité suffisante pour inhiber l'interaction entre HDM2 et p53 et/ou HDM2 et les facteurs de transcription E2F.

24. Composé de formule **Ic**, 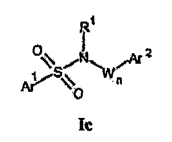 dans laquelle
W est un groupe alkylène en C₁ à C₅ ramifié ou non ramifié ou un groupe alcénylène en C₂ à C₅ ;
n est 0 ou 1 ;
R¹ est H, un groupe alkyle en C₁ à C₈ ramifié ou non ramifié, un groupe alcényle en C₂ à C₈. ou un groupe aryle ou aralkyle, chacun pouvant être facultativement substitué par un ou plusieurs groupes halogéno ou CF₃ ;
Ar¹ est 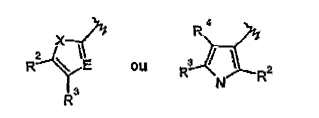 où
X est O, NH ou NR' où R' est un groupe alkyle en C₁ à C₃ ;
E est N ou CR⁴;
Y est N;
R², R³ et R⁴ sont chacun indépendamment (A)ₚB, où A est un groupe alkyle en C₁ à C₃, p est 0 ou 1, et B est H, un halogène, un groupe alkyle en C₁ à C₅, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAR^{e}, COR^{h}, S(CO)R^{s}, OR^{t}, OAr^{f}, un groupe alicyclique contenant facultativement un ou plusieurs hétéroatomes, facultativement substitué par un ou plusieurs groupes OH, COR^{u}, halogéno ou CF₃, ou un groupe hétéroaryle facultativement substitué par un ou plusieurs groupes alkyle en C₁ à C₅, halogéno, SRⁱ ou CF₃ ; ou bien
R² et R³ sont reliés pour former un système cyclique saturé ou insaturé, contenant facultativement un ou plusieurs hétéroatomes, et facultativement substitué par un ou plusieurs groupes halogéno, OH ou CF₃ ;
Ar^{a-f} sont chacun indépendamment des groupes aryle facultativement substitués par un ou plusieurs groupes alkyle en C₁ à C₅, halogéno ou CF₃
R^{a-i}, R^{s}, R^{t} et R^{u} sont chacun indépendamment des groupes alkyle en C₁ à C₅ facultativement substitués par un ou plusieurs groupes alkoxy, halogéno ou CF₃ ;
et à condition qu'au moins l'un de R², R³ et R⁴ soit autre chose que H;
Ar² est 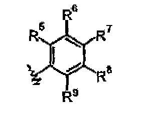 où
Z est S, O, NH ou NR" où R" est un groupe alkyle en C₁ à C₃ ;
R⁵, R⁶, R⁷, R⁸ et R⁹ sont chacun indépendamment (L)_{q}M où L est un groupe alkyle en C₁ à C₃, q est 0 ou 1, M est H, un groupe alkyle en C₁ à C₅, un halogène, NO₂, OH, NH₂, NHAr⁹, NHR^{j}, NR^{k}R¹, SO₃H, SO₂NH₂, SO₂NHAr^{h}, SO₂NHR^{m}, SO₂Arⁱ, SO₂Rⁿ, CF₃, CN, COOH, COOR^{p}, CONH₂, CONHAr^{j}, CONHR^{q}, OR^{v}, COAr^{k} ou COR^{r} ;
R^{j-r}, R^{v} sont chacun indépendamment des groupes alkyle en C₁ à C₅ ;
Ar^{g-k} sont chacun indépendamment des groupes aryle ;
et à condition qu'au moins l'un des substituants R⁵, R⁶,
R⁷, R⁸ et R⁹ soit autre chose que H;
à condition que ledit composé soit autre chose que l'acide 5-[(4-chlorophényl)amino]sulfonyl-2-furannecarboxylique.

25. Composé de formule Id 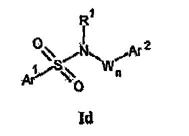 où
W est un groupe alkylène en C₁ à C₅ ramifié ou non ramifié ou un groupe alcénylène en C₂ à C₅ ;
n est 0 ou 1 ;
R¹ est H, un groupe alkyle en C₁ à C₈ ramifié ou non ramifié, un groupe alcényle en C₂ à C₈, ou un groupe aryle ou aralkyle, chacun pouvant être facultativement substitué par un ou plusieurs groupes halogéno ou CF₃ ;
Ar¹ est 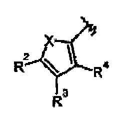 où
X est O, NH ou NR' où R' est un groupe alkyle en C₁ à C₃ ; N
R², R³ et R⁴ sont chacun indépendamment (A)ₚB, où A est un groupe alkyle en C₁ à C₃, p est 0 ou 1, et B est H, un halogène, un groupe alkyle en C₁ à C₅, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, un groupe alicyclique contenant facultativement un ou plusieurs hétéroatomes, facultativement substitué par un ou plusieurs groupes OH, COR^{u}, halogéno ou CF₃, ou un groupe hétéroaryle facultativement substitué par un ou plusieurs groupes alkyle en C₁ à C₅, halogéno, SRⁱ ou CF₃ ; ou bien
R² et R³ sont reliés pour former un système cyclique saturé ou insaturé, contenant facultativement un ou plusieurs hétéroatomes, et facultativement substitué par un ou plusieurs groupes halogéno, OH ou CF₃ ;
Ar^{a-f} sont chacun indépendamment des groupes aryle facultativement substitués par un ou plusieurs groupes alkyle en C₁ à C₅, halogéno ou CF₃ ;
R^{a-i}, R^{s}, R^{t} et R^{u} sont chacun indépendamment des groupes alkyle en C₁ à C₅ facultativement substitués par un ou plusieurs groupes alkoxy, halogéno ou CF₃ ;
et à condition qu'au moins l'un de R², R³ et R⁴ soit autre chose que H ;
et Ar² est 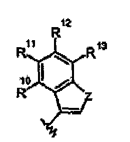 où
Z est S, O, NH ou NR" où R" est un groupe alkyle en C₁ à C₃ ; et
R¹⁰, R¹¹, R¹² et R¹³ sont chacun indépendamment H, un groupe alkyle en C₁ à C₅, un halogène, NO₂, OH, NH₂ ou CF₃.

26. Composé de formule **Ie** 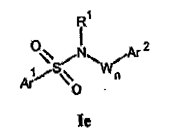 dans laquelle
W est un groupe alkylène en C₁ à C₅ ramifié ou non ramifié ou un groupe alcénylène en C₂ à C₅ ;
n est 0 ou 1 ;
R¹ est H, un groupe alkyle en C₁ à C₈ ramifié ou non ramifié, un groupe alcényle en C₂ à C₈, ou un groupe aryle ou aralkyle, chacun pouvant être facultativement substitué par un ou plusieurs groupes halogéno ou CF₃;
Ar¹ est 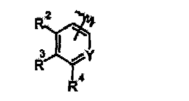 où
Y est N ;
R², R³ et R⁴ sont chacun indépendamment (A)ₚB, où A est un groupe alkyle en C₁ à C₃, p est 0 ou 1, et B est H, un halogène, un groupe alkyle en C₁ à C₅, NO₂, OH, NH₂, NHR^{a}, NR^{b}R^{c}, SO₃H, SO₂NH₂, NHAr^{a} SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, un groupe alicyclique contenant facultativement un ou plusieurs hétéroatomes, facultativement substitué par un ou plusieurs groupes OH, COR^{u}, halogéno ou CF₃, ou un groupe hétéroaryle facultativement substitué par un ou plusieurs groupes alkyle en C₁ à C₅, halogéno, SR^{f} ou CF₃ ; ou bien
R² et R³ sont reliés pour former un système cyclique saturé ou insaturé, contenant facultativement un ou plusieurs hétéroatomes, et facultativement substitué par un ou plusieurs groupes halogéno, OH ou CF₃;
Ar^{a-f} sont chacun indépendamment des groupes aryle facultativement substitués par un ou plusieurs groupes alkyle en C₁ à C₅, halogéno ou CF₃ ;
R^{a-i}, R^{s}, R^{t} et R^{u} sont chacun indépendamment des groupes alkyle en C₁ à C₅ facultativement substitués par un ou plusieurs groupes alkoxy, halogéno ou CF₃ ;
et à condition qu'au moins l'un de R², R³ et R⁴ soit autre chose que H;
et Ar² est 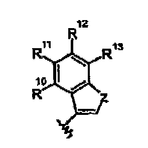 où
Z est S, O, NH ou NR" où R" est un groupe alkyle en C₁ à C₃ ; et
R¹⁰, R¹¹, R¹² et R¹³ sont chacun indépendamment H, un groupe alkyle en C₁ à C₅, un halogène, NO₂, OH, NH₂ ou CF₃.

27. Composé de formule If 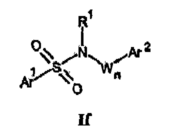 dans laquelle
W est un groupe alkylène en C₁ à C₅ ramifié ou non ramifié ou un groupe alcénylène en C₂ à C₅ ;
n est 0 ou 1 ;
R¹ est H, un groupe alkyle en C₁ à C₈ ramifié ou non ramifié, un groupe alcényle en C₂ à C₈, ou un groupe aryle ou aralkyle, chacun pouvant être facultativement substitué par un ou plusieurs groupes halogéno ou CF₃ ;
Ar¹ est 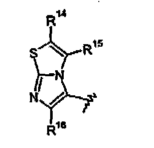 où
R¹⁴, R¹⁵ et R¹⁶ sont chacun indépendamment (A)ₚB, où A est un groupe alkyle en C₁ à C₃, p est 0 ou 1, et B est H, un halogène, un groupe alkyle en C₁ à C₅, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{s}, COR^{h}, S(CO)R^{s}, OR^{t}, OAr^{f}, un groupe alicyclique contenant facultativement un ou plusieurs hétéroatomes, facultativement substitué par un ou plusieurs groupes OH, COR^{u}, halogéno ou CF₃, ou un groupe hétéroaryle facultativement substitué par un ou plusieurs groupes alkyle en C₁ à C₅, halogéno, SRⁱ ou CF₃ ; ou bien
R² et R³ sont reliés pour former un système cyclique saturé ou insaturé, contenant facultativement un ou plusieurs hétéroatomes, et facultativement substitué par un ou plusieurs groupes halogéno, OH ou CF₃ ;
Ar^{a-f} sont chacun indépendamment des groupes aryle facultativement substitués par un ou plusieurs groupes alkyle en C₁ à C₅, halogéno ou CF₃;
R^{a-i}, R^{s}, R^{t} et R^{u} sont chacun indépendamment des groupes alkyle en C₁ à C₅ facultativement substitués par un ou plusieurs groupes alkoxy, halogéno ou CF₃;
et Ar² est 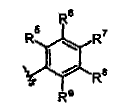 où
R⁵, R⁶, R⁷, R⁸ et R⁹ sont chacun indépendamment (L)_{q}M où L est un groupe alkyle en C₁ à C₃, q est 0 ou 1, M est H, un groupe alkyle en C₁ à C₅, un halogène, NO₂, OH, NH₂, NHAr^{g}, NHR^{j}, NR^{k}R^{l}, SO₃H, SO₂NH₂, SO₂NHAr^{h}, SO₂NHR^{m}, SO₂Arⁱ, SO₂Rⁿ, CF₃, CN, COOH, COOR^{p}, CONH₂, CONHAr^{j}, CONHR^{q}, OR^{v}, COAr^{k} ou COR^{r} ;
R^{j-r}, R^{v} sont chacun indépendamment des groupes alkyle en C₁ à C₅ ;
Ar^{g-k} sont chacun indépendamment des groupes aryle ;
et à condition qu'au moins l'un des substituants R⁵, R⁶, R⁷, R⁸ et R⁹ soit autre chose que H.

28. Composé de formule **Ig** 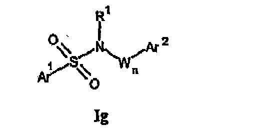 dans laquelle
W est un groupe alkylène en C₁ à C₅ ramifié ou non ramifié ou un groupe alcénylène en C₂ à C₅ ;
n est 0 ou 1 ;
R¹ est H, un groupe alkyle en C₁ à C₈ ramifié ou non ramifié, un groupe alcényle en C₂ à Ce, ou un groupe aryle ou aralkyle, chacun pouvant être facultativement substitué par un ou plusieurs groupes halogéno ou CF₃ ;
Ar¹ est 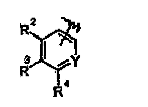 où
Y est N ;
R², R³ et R⁴ sont chacun indépendamment (A)ₚB, où A est un groupe alkyle en C₁ à C₃, p est 0 ou 1, et B est H, un halogène, un groupe alkyle en C₁ à C₅, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, un groupe alicyclique contenant facultativement un ou plusieurs hétéroatomes, facultativement substitué par un ou plusieurs groupes OH, COR^{u}, halogéno ou CF₃, ou un groupe hétéroaryle facultativement substitué par un ou plusieurs groupes alkyle en C₁ à C₅, halogéno, SRⁱ ou CF₃ ; ou bien
R² et R³ sont reliés pour former un système cyclique saturé ou insaturé, contenant facultativement un ou plusieurs hétéroatomes, et facultativement substitué par un ou plusieurs groupes halogéno, OH ou CF₃ ;
Ar^{a-f} sont chacun indépendamment des groupes aryle facultativement substitués par un ou plusieurs groupes alkyle en C₁ à C₅, halogéno ou CF₃ ;
R^{a-i}, R^{s}, R^{t} et R^{u} sont chacun indépendamment des groupes alkyle en C₁ à C₅ facultativement substitués par un ou plusieurs groupes alkoxy, halogéno ou CF₃ ;
et à condition qu'au moins l' un de R², R³ et R⁴ soit autre chose que H ;
et Ar² est 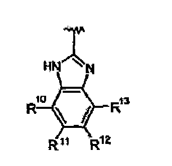 où
R¹⁰, R¹¹, R¹² et R¹³ sont chacun indépendamment H, un groupe alkyle en C₁ à C₅, halogéno, NO₂, OH, NH₂ ou CF₃.

29. Composé de formule **Ib,** 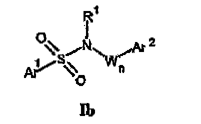 dans laquelle
W est un groupe alkylène en C₁ à C₅ ramifié ou non ramifié ou un groupe alcénylène en C₂ à C₅ ;
n est 0 ou 1 ;
R¹ est H, un groupe alkyle en C₁ à C₈ ramifié ou non ramifié, un groupe alcényle en C₂ à C₈, ou un groupe aryle ou aralkyle, chacun pouvant être facultativement substitué par un ou plusieurs groupes halogéno ou CF₃ ;
Ar¹ est 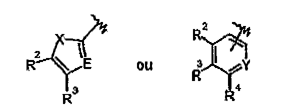 où
X est S, O, NH ou NR' où R' est un groupe alkyle en C₁ à C₃ ;
E est N ou CR⁴ ;
Y est CH ou N ;
R², R³ et R⁴ sont chacun indépendamment (A)ₚB, où A est un groupe alkyle en C₁ à C₃, p est 0 ou 1, et B est H, un halogène, un groupe alkyle en C₁ à C₅, NO₂, OH, NH₂, NHR^{a}, NR^{b}R^{c}, SO₃H, SO₂NH₂, NHAr^{a}, SO₂NHAr^{b}, SO₂NHR^{d}, SO₂Ar^{c}, SO₂R^{e}, CF₃, CN, COOH, COOR^{f}, CONH₂, COONHAr^{d}, CONHR^{g}, COAr^{e}, COR^{h}, S(CO)R^{s}, OR^{t}, OAr^{f}, un groupe alicyclique contenant facultativement un ou plusieurs hétéroatomes, facultativement substitué par un ou plusieurs groupes OH, COR^{u}, halogéno ou CF₃, ou un groupe hétéroaryle facultativement substitué par un ou plusieurs groupes alkyle en C₁ à C₅, halogéno, SRⁱ ou CF₃ ; ou bien
R² et R³ sont reliés pour former un système cyclique saturé ou insaturé, contenant facultativement un ou plusieurs hétéroatomes, et facultativement substitué par un ou plusieurs groupes halogéno, OH ou CF₃;
Ar^{a-f} sont chacun indépendamment des groupes aryle facultativement substitués par un ou plusieurs groupes alkyle en C₁ à C₅, halogéno ou CF₃
R^{a-i}, R^{s}, R^{t} et R^{u} sont chacun indépendamment des groupes alkyle en C₁ à C₅ facultativement substitués par un ou plusieurs groupes alkoxy, halogène ou CF₃ ;
et à condition qu'au moins l'un de R², R³ et R⁴ soit autre chose que H ;
Ar² est 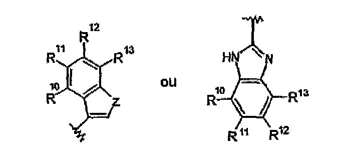 où
Z est S, O, NH ou NR" où R" est un groupe alkyle en C₁ à C₃ ;
R¹⁰, R¹¹, R¹² et R¹³ sont chacun indépendamment H, un groupe alkyle en C₁ à C₅, un halogène, NO₂, OH, NH₂ ou CF³;
ledit composé étant autre chose que la 5-méthoxy-N-méthyl-N-(*p-*toluènesulfonyl)tryptamine, la N-méthyl-*p*-toluènesulfonyl-tryptamine, la N-(*p*-toluènesulfonyl)-7-méthoxy-tryptamine, la *p*-toluènesulfonyl-tryptamine ou le 3-sulfanilamidoéthylindole.

30. Composé selon l'une quelconque des revendications 24 à 29, dans lequel
R², R³ et R⁴ sont chacun indépendamment (A)ₚB, où A est un groupe alkyle en C₁ à C₅, p est 0 ou 1, et B est H, F, Cl, Br, I, un groupe alkyle en C₁ à C₅, NO₂, OH, NH₂, NHR^{a}, NR^{b}R^{c}, SO₃H, SO₂NH₂, NHPh, SO₂NHAr^{b}, SO₂NHR^{d}, SO₂Ph, SO₂R^{e}, CF₃, CN, COOH, COOR^{f}, CONH₂, COONHPh, CONHR^{g}, S(CO)R^{s}, OR^{t}, OAr^{f}, COPh, COR^{h}, un groupe morpholino, pipérazino ou pipéridino dont chacun peut être facultativement substitué par un ou plusieurs groupes OH ou COR^{u}, ou un groupe hétéroaryle choisi parmi les groupes pyridyle, pyrimidyle, oxazolyle, thiazolyle et pyrazolyle, dont chacun peut être facultativement substitué par un ou plusieurs groupes alkyle en C₁ à C₅, halogéno, SRⁱ ou CF₃, ou bien R² et R³ forment ensemble un cycle hexagonal saturé ou un cycle pentagonal insaturé dont chacun contient facultativement un ou plusieurs hétéroatomes ; et
R⁵, R⁶, R⁷, R⁸ et R⁹ sont chacun indépendamment (L)_{q}M où L est un groupe alkyle en C₁ à C₅, q est 0 ou 1, M est H, un groupe alkyle en C₁ à C₅, un halogène, NO₂, OH, NH₂, NHPh, NHR^{j}, NR^{k}R^{l}, SO₃H, SO₂NH₂, SO₂NHPh, SO₂NHR^{m}, SO₂Ph, SO₂Rⁿ, CF₃, CN, COOH, COOR^{P}, CONH₂, CONHPh, CONHR^{q}, OR^{v}, COPh ou COR^{r}.

31. Composé selon l'une quelconque des revendications 24 à 29, dans lequel R², R³ et R⁴ sont chacun indépendamment (A)ₚB, où A est un groupe alkyle en C₁ à C₅, p est 0 ou 1, et B est H, F, Cl, Br, I, un groupe alkyle en C₁ à C₅, NO₂, OH, NH₂, NHR^{a}, NR^{b}R^{c}, SO₃H, SO₂NH₂, NHPh, SO₂NHPh, SO₂NHR^{d}, SO₂Ph, SO₂R^{e}, CF₃, CN, COOH, COOR^{f}, CONH₂, COONHPh, CONHR^{g}, S(CO)R^{s}, OR^{t}, OAr^{f}, COPh, COR^{h}, un groupe pyridyle, pyrimidyle, 2-méthylsulfanylpyrimid-5-yle, oxazole-2-yle, thiazole-2-yle, 1-méthyl-5-trifluorométhyl-1*H*-pyrazole-4-yle, morpholine-4-yle, 4-acétyl-pipérazine-1-yle, 3-hydroxy-pipéridine-1-yle, ou bien R² et R³ forment ensemble
-OCH₂CH₂O-
-N-S-N- ou
un groupe phényle facultativement substitué par un ou plusieurs halogènes.

32. Composé selon l'une quelconque des revendications 24 à 29, dans lequel R², R³ et R⁴ sont chacun indépendamment H, un halogène, NO₂, SO₂Ph, 5(CO)Me, COOH, COOEt, OPh, OMe, NHCH₂CH₂OMe, un groupe 1-méthyl-5-trifluorométhyl-1H-pyrazole-4-yle, 2-méthylsulfanyl-pyrimid-5-yle, N-(4-fluorophényl)sulfonamido, N-(4-trifluorométhylphényl)sulfonamido, oxazole-2-yle, alkyle en C₁ à C₅, NH₂, morpholine-4-yle, 4-acétyl-pipérazine-1-yle, 3-hydroxy-pipéridine-1-yle, ou bien R² et R³ forment ensemble
-OCH₂CH₂O-
-N-S-N- ou
un groupe phényle facultativement substitué par un ou plusieurs halogènes.

33. Composé selon l'une quelconque des revendications 24, 25 ou 29 à 32, dans lequel Ar¹ est 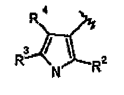
R² et R⁴ sont des groupes alkyle en C₁ à C₅, et R³ est COOH ou COOEt.

34. Composé selon l'une quelconque des revendications 26, 28 ou 29 à 32, dans lequel Ar¹ est 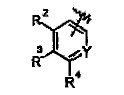
R², R³ et R⁴ sont chacun indépendamment H, OH, COOH, CF₃, OPh, OMe, NO₂, un groupe 4-acétyl-pipérazine-1-yle, NH₂, un halogène, un groupe pyrazole-1-yle, oxazole-2-yle ou alkyle en C₁ à C₅, ou bien R² et R³ forment ensemble un groupe -OCH₂CH₂O- ou -N-S-N-.

35. Composé selon la revendication 34, dans lequel lorsque Y est CH
- R² est H, NO₂ ou Cl ;
- R³ est NO₂, NH₂, Cl, CF₃, COOH, un groupe 4-acétyl-pipérazine-1-yle ; et
- R⁴ est H, Cl, un groupe oxazole-2-yle, OH, NO₂, NH₂, OMe ou Me ; ou bien lorsque Y est N
- R² est H ;
- R³ est Br ;
- R⁴ est Cl ou OPh.

36. composé selon l'une quelconque des revendications 24 à 35, dans lequel
- R⁵, R⁶, R⁷, R⁸ et R⁹ sont chacun indépendamment H, un halogène, OMe, NO₂, un groupe alkyle en C₁ à C₅, CF₃ ou OH; et
- R¹⁰, R¹¹, R¹² et R¹³ sont tous H.

37. Composé selon la revendication 36, dans lequel
- R⁵ est H, un groupe alkyle en C₁ à C₅ ou un halogène;
- R⁶ est H, un halogène, NO₂ ou CF₃;
- R⁷ est H, un halogène, OMe, NO₂, OH ou CF₃ ;
- R⁸ est H, un halogène ou CF₃ ;
- R⁹ est H.

38. Composé selon l'une quelconque des revendications 24 à 37, dans lequel
- W est CH₂, CH₂CH₂ ou CH(CH₃)CH₂ ; et
- R¹ est H, CH₂Ph, CH₂CH(Me)₂, un groupe 3-(trifluorométhyl)benzyle, ou Me.

39. Composé choisi parmi les suivants :
(3-trifluorométhylphényl)amide d'acide 5-chloro-4-nitrothiophène-2-sulfonique [1] ;
(4-chlorophényl)amide d'acide 5-chloro-4-nitrothiophène-2-sulfonique [2] ;
(4-fluorophényl)amide d'acide 5-chloro-4-nitrothiophène-2-sulfonique [3) ;
(4-fluorophényl)amide d'acide 4-bromo-5-chlorothiophène-2-sulfonique [4] ;
(4-hydroxyphényl)amide d'acide 5-chloro-4-nitrothiophène-2-sulfonique [5] ;
(4-trifluorométhylphényl)amide d'acide 5-chloro-4-nitrothiophène-2-sulfonique [6] ;
(4-fluorophényl)méthylamide d'acide 5-chloro-4-nitrothiophène-2-sulfonique [7] ;
(3,5-bis-trifluorométhylphényl)amide d'acide 4,5-dibromothiophène-2-sulfonique [8] ;
(3,5-bis-trifluorométhylphényl)amide d'acide 5-chlorothiophène-2-sulfonique [11] ;
(3,5-dichlorophényl)amide d'acide 5-(2-méthylsulfanylpyrimidine-5-yl)thiophène-2-sulfonique [12] ;
4-oxazole-2-yl-N-(4-trifluorométhylphényl)benzènesulfonamide [13] ;
N-(3,5-bis-trifluorométhylphényl)-4-oxazole-2-yl-benzènesulfonamide [14] ;
(4-trifluorométhylphényl)amide d'acide 4-bromo-5-chlorothiophène-2-sulfonique [15] ;
(4-chlorophényl)amide d'acide 5-bromathiophène-2-sulfonique [16] ;
(3,5-dichlorophényl)amide d'acide 5-bromothiophène-2-sulfonique [17] ;
(3,5-bis-trifluorométhylphényl)amide d'acide 5-bromothiophène-2-sulfonique [18] ;
3-nitro-N-(4-trifluorométhylphényl)benzènesulfonamide [20] ;
N-(3,5-bis-trifluorométhylphényl)-3-nitrobenzènesulfonamide [21] ;
(4-trifluorométhylphényl)amide d'acide 5-benzènesulfonylthiophène-2-sulfonique [23] ;
(4-chloxophényl)amide d'acide 5-benzènesulfonylthiophène-2-sulfonique [24] ;
(3,5-dichlorophényl)amide d'acide 5-benzènesulfonylthiophène-2-sulfonique [25] ;
(3,4-dichlorophényl)amide d'acide 5-chlorothiophène-2-sulfonique [26] ;
(3-trifluorométhylphényl)amide d'acide 4,5-dibromothiophène-2-sulfonique [27] ;
(3,4-dichlorophényl)amide d'acide 4,5-dibromothiophène-2-sulfonique [28] ;
(4-trifluorométhylphényl)amide d'acide 5-(1-méthyl-5-trifluorométhyl-1H-pyrazole-4-yl)thiophène-2-sulfonique [31] ;
bis[(4-fluorophényl)amide] d'acide 5-chlorothiophène-2,4-disulfonique [32] ;
bis[(4-trifluorométhylphényl)amide] d'acide 5-chlorothiophène-2,4-disulfonique [33] ;
4-méthyl-3-nitro-N-(4-trifluorométhylphényl)benzènesulfonamide [34] ;
4-chloro-3-nitro-N-(4-trifluorométhylphényl)benzènesulfonamide [35] ;
3-amino-4-méthyl-N-(4-trifluorométhylphényl)benzènesulfonamide [36] ;
N-(4-chlorophényl)-4-méthyl-3-nitrobenzènesulfonamide [37] ;
4-chloro-N-(4-chlorophényl)-3-nitrobenzènesulfonamide [38] ;
(3,5-dichlorophényl)amide d'acide 5-chloro-4-nitrothiophène-2-sulfonique [39] ;
(3,5-difluorophényl)amide d'acide 5-chloro-4-nitrothiophène-2-sulfonique [40] ;
(4-trifluorométhylphényl)amide d'acide 5-bromo-6-chloropyridine-3-sulfonique [41] ;
(3,5-bis-trifluorométhylphényl)amide d'acide 5-bromo-6-chloropyridine-3-sulfonique [42];
(3,5-bis-trifluorométhylphényl)amide d'acide 5-(1-méthyl-5-trifluorométhyl-1H-pyrazvle-4-yl)thiophène-2-sulfonique [43] ;
(3,5-bis-trifluorométhylphényl)amide d'acide 5-chloro-4-nitrothiophène-2-sulfonique [44] ;
4-fluorobenzylamide d'acide 5-chloro-4-nitrothiophène-2-sulfonique [45] ;
4-trifluorométhylbenzylamide d'acide 5-chloro-4-nitrothiophène-2-sulfonique [46] ;
4-chloro-N-(3,5-dichlorophényl)-3-nitrobenzènesulfonamide [47] ;
[2-(1H-indole-3-yl)éthyl]amide d'acide 5-chloro-4-nitrothiophène-2-sulfonique [48] ;
[2-(1H-indole-3-yl)-1-méthyléthyl]amide d'acide 5-chloro-4-nitrothiophène-2-sulfonique [49] ;
méthyl-(4-trifluorométhylphényl)amide d'acide 5-chloro-4-nitrothiophène-2-sulfonique [50] ;
(4-chlorophényl)méthylamide d'acide 5-chloro-4-nitrothiophène-2-sulfonique [51] ; et
méthyl-(4-trifluorométhylbenzyl)amide d'acide 5-chloro-4-nitrothiophène-2-sulfonique [52] ;
benzyl-(4-fluorobenzyl)amide d'acide 5-chloro-4-nitrothiophène-2-sulfonique [53] ;
3,5-dichlorobenzylamide d'acide 5-chloro-9-nitrothiophène-2-sulfonique [54] ;
3,5-difluorobenzylamide d'acide 5-chloro-4-nitrothiophène-2-sulfonique [55] ;
4-chlorobenzylamide d'acide 5-chloro-4-nitrothiophène-2-sulfonique [56] ;
[1-(4-fluorophényl)éthyl]amide d'acide 5-chloro-4-nitrothiophène-2-sulfonique [57] ;
(4-chlorophényl)isobutylamide d'acide 5-chloro-4-nitrothiophène-2-sulfonique [58] ;
(1*H*-benzo-imidazole-2-yl)amide d'acide 5-chloro-4-nitrothiophène-2-sulfonique [59] ;
[2-(6-chloro-1*H*-indole-3-yl)éthyl]amide d'acide 5-chloro-4-nitrothiophène-2-sulfonique [60] ;
(4-méthoxyphényl)amide d'acide 5-chloro-4-nitrothiophène-2-sulfonique [61] ;
phénylamide d'acide 5-chloro-4-nitrothiophène-2-sulfonique [62] ;
p-tolylamide d'acide 5-chloro-4-nitrothiophène-2-sulfonique [63] ;
benzylamide d'acide 5-chloro-4-nitrothiophène-2-sulfonique [64] ;
benzyl-(4-chlorophényl)amide d'acide 5-chloro-4-nitrothiophène-2-sulfonique [65] ;
benzyl-(4-méthoxyphényl)amide d'acide 5-chloro-4-nitrothiophène-2-sulfonique [66];
(4-chlorophényl)-(3-trifluorométhylbenzyl)amide d'acide 5-chloro-4-nitrothiophène-2-sulfonique [67] ;
(4-chlorophényl)amide d'acide 5-nitrothiophène-2-sulfonique [68] ;
(4-chlorophényl)amide d'acide 4-nitrothiophène-2-sulfonique [69];
bis[(4-chlorophényl)amide] d'acide 5-chloro-thiophène-2,4-disulfonique [70] ;
(4-chlorophényl)amide d'acide 5-éthyl-4-nitrothiophène-2-sulfonique [71] ;
ester S-[5-(4-chlorophénylsulfamoyl)-3-nitrothiophène-2-ylique] d'acide thioacétique [72] ;
(4-chlorophényl)amide d'acide 5-méthyl-4-nitrothiophène-2-sulfonique [73] ;
bis[(4-chlorophényl)amide] d'acide 5-méthyl-thiophène-2,4-disulfonique [74] ;
(3-trifluorométhylbenzyl)-(4-trifluorométhylbenzyl)-amide d'acide 5-chloro-4-nitrothiophène-2-sulfonique [75];
(4-trifluorométhylphényl)amide d'acide 4-nitrothiophène-2-sulfonique (76] ,
[2-(1*H*-indole-3-yl)éthyl]amide d'acide 4-nitrothiophène-2-sulfonique [77];
(3,5-bis-trifluorométhylphényl)-(3-trifluorométhylbenzyl)amide d'acide 5-(1-méthyl-5-trifluorométhyl-1*H*-pyrazole-3-yl)thiophène-2-sulfonique [78];
(4-chlorophényl)amide d'acide 5-morpholine-4-yl-4-nitrothiophène-2-sulfonique [79] ;
(4-chlorophényl)amide d'acide 5-(2-méthoxyethylamino)-4-nitrothiophène-2-sulfonique [80];
4-chloro-N-[2-(5-chloro-1*H*-indole-3-yl)éthyl]-3-nitrobenzènesulfonamide [81];
N-[2-(5-chloro-1*H*-indole-3-yl)éthyl]-4-méthyl-3-nitrobenzènesulfonamide [82] ;
N-(1*H*-benzoimidazole-2-yl)-4-chloro-3-nitrobenzènesulfonamide [83];
(3,5-bis-trifluorométhylphényl)amide d'acide 6-chloroimidazo[2,1-b]thiazole-5-sulfonique [84] ;
(4-chlorophényl)amide d'acide 2,3-dihydrobenzo[1,4]-dioxine-6-sulfonique [85] ;
(3,5-bis-trifluorométhylphényl)amide d'acide 2,3-dihydrobenzo[1,9]dioxine-6-sulfonique [86] ;
(4-chlorophényl)amide d'acide 6-phénoxypyridine-3-sulfonique [87] ;
(4-chlozo-3-nitrophényl)amide d'acide 5-chloro-3-méthylbenzo[b]thiophène-2-sulfonique [88] ;
N-(3,5-bis-trifluorométhylphényl)-4-pyrazole-1-yl-benzènesulfonamide (89] ;
ester éthylique d'acide 4-(4-chlorophénylsulfamoyl)-3,5-diméthyl-1*H*-pyrrole-2-carboxylique [90] ;
acide 4-(3,5-bis-trifluorométhylphénylsulfamoyl)-3,5-diméthyl-1*H*-pyrrole-2-carboxylique [91] ;
acide 4-(4-chlorophénylsulfamoyl)-3,5-diméthyl-1*H*-pyrrole-2-carboxylique [92] ;
ester éthylique d'acide 2-(4-chlorophénylsulfamoyl)-4-méthyl-thiazole-5-carboxylique [93] ;
3,5-dichloro-N-(4-chlorophényl)-4-hydroxybanzénesulfonamide [94] ;
N-(3,5-bis-trifluorométhylphényl)-3,5-dichloro-4-hydroxybenzènesulfonamide [95] ;
3,5-dichloro-4-hydroxy-N-(4-trifluorométhylphényl)-benzènesulfonamide [96] ;
N-(3,5-bis-trifluorométhylphényl)-9-nitrobenzènesulfonamide [98] ;
4-amino-N-(3,5-bis-trifluorométhylphényl)-3-chlorobenzènesulfonamide [99] ;
3-nitro-N-(4-trifluorométhylphényl)benzènesulfonamide [100] ;
3,5-dichloro-N-(3,5-dichlorophényl)-4-hydroxybenzènesulfonamide [101] ;
4-amino-3-chloro-N-(4-chlorophényl)benzènesulfonamide [102] ;
3-chloro-N-(4-chlorophényl)-4-méthoxybenzènesulfonamide [103] ;
N-(3,5-bis-trifluorométhylphényl)-3-chloro-4-méthoxybenzènesulfonamide [104] ;
N-(3-chloro-4-nitrophényl)-3,5-bis-trifluorométhylbenzènesulfonamide [105] ;
3-(4-acétylpipérazine-1-yl)-N-(3,5-bis-trifluorométhylphényl)-4-nitrobenzènesulfonamide [106] ;
N-(3,5-bis-trifluorométhylphényl)-2-nitrobenzènesulfonamide [107] ;
acide 3-(3,5-bis-trifluorométhylphénylsulfamoyl)-benzoïque [108] ;
3,5-dichloro-N-(4-chlorabenzyl)-4-hydroxybenzènesulfonamide [109] ;
3,5-dichloro-9-hydroxy-N-(4-trifluorométhylbenzyl)-benzènesulfonamide [110] ;
3,5-dichloro-4-hydroxy-N-[2-(1H-indole-3-yl) éthyl]-benzènesulfonamide [111] ;
(3,5-dichlorophényl)amide d'acide 4,5-dibromothiophène-2-sulfonique [112] ;
N-(3,5-dichlorophényl)-4-oxazole-2-yl-benzènesulfonamide [113] ;
(3,5-bis-trifluorométhylphényl)amide d'acide 4-bromo-5-chlorothiophène-2-sulfonique [114] ;
(3,5-dichlorophényl)amide d'acide 4-bromo-5-chlorothiophène-2-sulfonique [115];
(3,5-bis-trifluorométhylphényl)amide d'acide 5-benzènesulfonylthiophène-2-sulfonique [117] ;
(2,4-dichlorophényl)amide d'acide 5-benzènesulfonylthiophène-2-sulfonique [118] ;
(3,5-bis-trifluorométhylphényl)amide d'acide 5-chloro-3-méthylbenzo[b]thiophène-2-sulfonique [119] ;
(3,5-bis-trifluorométhylphényl)amide d'acide benzo[b]-thiophène-2-sulfonique [120] ;
(4-chlorophényl)amide d'acide benzo[1,2,5]thiadiazole-5-sulfonique [121] ;
(3,5-bis-trifluorométhylphényl)amide d'acide benzo-[1,2,5]thiadiazole-5-sulfonique [122] ;
(4-trifluorométhylphényl)amide d'acide benzo[1,2,5]-thiadiazole-5-sulfonique [123] ;
(3,5-bis-trifluorométhylphényl)amide d'acide 5-pyridine-2-yl-thiophène-2-sulfonique [124] ;
(4-chlorophényl)amide d'acide 4,5-dibromothiophène-2-sulfonique [125] ;
(4-trifluorométhylphényl)amide d'acide 4,5-dibromothiophène-2-sulfonique [126] ;
3,5-dichloro-N-(4-fluorobonzyl)-4-hydroxybenzènesulfonamide [127] ;
N-(3,5-bis-trifluorométhylphényl)-2,6-dichlorobenzènesulfonamide [128] ;
(4-méthoxy-2-méthylphényl)amide d'acide 5-chloro-4-nitrothiophène-2-sulfonique [129] ;
(4-chlorophényl)amide d'acide 5-(3-hydroxypipéridine-1-yl)-4-nitrothiophène-2-sulfonique [130] ; et
(4-nitrophényl)amide d'acide 5-chloro-4-nitrothiophène-2-sulfonique [131].

40. Composé selon la revendication 39, qui est choisi parmi les suivants :
(4-chlorophényl)amide d'acide 5-chloro-4-nitrothiophène-2-sulfonique [2] ;
(4-fluorophényl) amide d'acide 5-chloro-4-nitrothiophène-2-sulfonique [3) ;
(4-hydroxyphényl) amide d'acide 5-chloro-4-nitrothiophène-2-sulfonique [5] ;
(4-trifluorométhylphényl) amide d'acide 5-chloro-4-nitrothiophène-2-sulfonique [6] ;
(4-fluorophényl) méthylamide d'acide 5-chloro-4-nitrothiophène-2-sulfonique [7] ;
4-chloro-3-nitro-N-(4-trifluorométhylphényl)benzènesulfonamide [35] ;
4-chloro-N-(4-chlorophényl)-3-nitrobenzènesulfonamide [38] ;
(3,5-dichlorophényl)amide d'acide 5-chloro-4-nitrothiophène-2-sulfonique [39] ;
(3,5-difluorophényl)amide d'acide 5-chloro-4-nitrothiophène-2-sulfonique [40] ;
(3,5-bis-trifluorométhylphényl)amide d'acide 5-(1-méthyl-5-trifluorométhyl-1*H*-pyrazole-4-yl)thiophène-2-sulfonique [43] ;
(3,5-bis-trifluorométhylphényl)amide d'acide 5-chloro-4-nitrothiophène-2-sulfonique [44] ;
4-fluorobenzylamide d'acide 5-chloro-4-nitrothiophène-2-sulfonique [45] ;
4-trifluorométhylbenzylamide d'acide 5-chloro-4-nitrothiophène-2-sulfonique [46] ;
[2-(1*H*-indole-3-yl)éthyl]amide d'acide 5-chloro-4-nitrothiophène-2-sulfonique [48] ;
[2-(1*H*-indole-3-yl)-1-méthyléthyl]amide d'acide 5-chloro-4-nitrothiophène-2-sulfonique [49] ;
(4-chlorophényl)méthylamide d'acide 5-chloro-4-nitrothiophène-2-sulfonique [51] ;
benzyl-(4-fluorobenzyl)amide d'acide 5-chloro-4-nitrothiophène-2-sulfonique [53] ;
3,5-dichlorobenzylamide d'acide 5-chloro-9-nitrothiophène-2-sulfonique [54] ;
3,5-difluorobenzylamide d'acide 5-chloro-4-nitrothiophène-2-sulfonique [55] ;
4-chlorobenzylamide d'acide 5-chloro-4-nitrothiophène-2-sulfonique [56] ;
[1-(4-fluorophényl)éthyl]amide d'acide 5-chloro-4-nitrothiophène-2-sulfonique [57] ;
(4-méthoxyphényl)amide d'acide 5-chloro-4-nitrothiophène-2-sulfonique [61] ;
*p*-tolylamide d'acide 5-chloro-4-nitrothiophène-2-sulfonique [63] ;
benzylamide d'acide 5-chloro-4-nitrothiophène-2-sulfonique [64] ;
benzyl-(4-chlorophényl)amide d'acide 5-chloro-4-nitrothiophène-2-sulfonique [65] ;
benzyl-(4-méthoxyphényl)amide d'acide 5-chloro-4-nitrothiophène-2-sulfonique [66] ;
(4-chlorophényl)-(3-trifluorométhylbenzyl)amide d'acide 5-chloro-4-nitrothiophène-2-sulfonique [67] ;
(4-chlorophényl)amide d'acide 4-nitrothiophène-2-sulfonique [69] ;
(3-trifluorométhylbenzyl)-(4-trifluorométhylbenzyl)-amide d'acide 5-chloro-4-nitrothiophène-2-sulfonique [75] ; et
N-(1*H*-benzoimidazole-2-yl)-4-chloro-3-nitrobenzènesulfonamide [83].

41. Composé selon la revendication 39, qui est choisi parmi les suivants :
(4-chlorophényl)amide d'acide 5-chloro-4-nitrothiophène-2-sulfonique [2] ;
(4-fluorophényl)amide d'acide 5-chloro-4-nitrothiophène-2-sulfonique [3) ;
(9-hydroxyphényl)amide d'acide 5-chloro-4-nitrothiophène-2-sulfonique [5] ;
(4-trifluorométhylphényl)amide d'acide 5-chloro-4-nitrothiophène-2-sulfonique [6] ;
(4-fluorophényl)méthylamide d'acide 5-chloro-4-nitrothiophéne-2-sulfonique [7] ;
(3,5-bis-trifluorométhylphényl)amide d'acide 5-bromothiophène-2-sulfonique [18] ;
(4-trifluorométhylphényl)amide d'acide 5-benzènesulfonylthiophène-2-sulfonique [23] ;
(3-trifluorométhylphényl)amide d'acide 4,5-dibromothiophène-2-sulfonique [27] ;
(3,4-dichlorophényl)amide d'acide 4,5-dibromothiophène-2-sulfonique [28] ;
bis[(4-trifluorométhylphényl)amide] d'acide 5-chlorothiophène-2,4-disulfonique [33] ;
4-chloro-3-nitro-N-(4-trifluorométhylphényl)benzènesulfonamide [35] ;
(3,5-dichlorophényl)amide d'acide 5-chloro-4-nitrothiophène-2-sulfonique [39] ;
(3,5-diflucrophényl)amide d'acide 5-chloro-4-nitrothiophène-2-sulfonique [40] ;
(3,5-bis-trifluorométhylphényl)amide d'acide 5-(1-méthyl-5-trifluorométhyl-1*H*-pyrazole-4-yl)thiophène-2-sulfonique [43] ;
(3,5-bis-trifluorométhylphényl)amide d'acide 5-chloro-4-nitrothiophène-2-sulfonique [44] ;
4-fluorobenzylamide d'acide 5-chloro-4-nitrothiophène-2-sulfonique [45] ;
4-trifluorométhylbenzylamide d'acide 5-chloro-4-nitrothiophène-2-sulfonique [46] ;
[2-(1H-indole-3-yl)éthyl]amide d'acide 5-chloro-4-nitrothiophène-2-sulfonique [48] ;
[2-(1H-indole-3-yl)-1-méthyléthyl]amide d'acide 5-chloro-4-nitrothiophène-2-sulfonique [49] ;
méthyl-(9-trifluorométhylphényl)amide d'acide 5-chloro-4-nitrothiophène-2-sulfonique [50] ;
(4-chlorophényl)méthylamide d'acide 5-chloro-4-nitrothiophène-2-sulfonique [51] ; et
méthyl-(4-trifluorométhylbenzyl) amide d'acide 5-chloro-4-nitrothiophène-2-sulfonique [52] ;
benzyl-(4-fluorobenzyl)amide d'acide 5-chloro-4-nitrothiophène-2-sulfonique [53] ;
3,5-dichlorobenzylamide d'acide 5-chloro-4-nitrothiophène-2-sulfonique [54] ;
3,5-difluorobenzylamide d'acide 5-chloro-4-nitrothiophène-2-sulfonique [55] ;
4-chlorobenzylamide d'acide 5-chloro-4-nitrothiophène-2-sulfonique [56] ;
[1-(4-fluorophényl)éthyl]amide d'acide 5-chloro-4-nitrothiophène-2-sulfonique [57] ;
(1*H*-benzo-imidazole-2-yl)amide d'acide 5-chloro-4-nitrothiophéne-2-sulfonique [59] ;
[2-(6-chloro-1*H*-indole-3-yl)éthyl]amide d'acide 5-chloro-4-nitrothiophène-2-sulfonique [60] ;
(4-méthoxyphényl)amide d'acide 5-chloro-4-nitrothiophène-2-sulfonique [61] ;
phénylamide d'acide 5-chloro-4-nitrothiophène-2-sulfonique [62] ;
*p*-tolylamide d'acide 5-chloro-4-nitrothiophène-2-sulfonique [63] ;
benzylamide d'acide 5-chloro-4-nitrothiophène-2-sulfonigue [64] ;
benzyl-(4-chlorophényl) amide d'acide 5-chloro-4-nitrothiophène-2-sulfonique [65] ;
benzyl-(4-méthoxyphényl)amide d'acide 5-chloro-4-nitrothiophène-2-sulfonique [66] ;
(4-chlorophényl)-(3-trifluorométhylbenzyl)amide d'acide 5-chloro-4-nitrothiophène-2-sulfonique [67] ;
(4-chlorophényl) amide d'acide 4-nitrothiophène-2-sulfonique [69] ;
bis [(4-chlorophényl)amide] d'acide 5-chloro-thiophène-2,4-disulfonique [70] ;
ester S-[5-(4-chlorophénylsulfamoyl)-3-nitrothiophène-2-ylique] d'acide thioacétique [72] ;
(4-chlorophényl)amide d'acide 5-méthyl-4-nitrothiophène-2-sulfonique [73] ;
bis[(4-chlorophényl)amide] d'acide 5-méthyl-thiophène-2,4-disulfonique [74] ;
(3-trifluorométhylbenzyl)-(4-trifluorométhylbenzyl)-amide d'acide 5-chloro-4-nitrothiophène-2-sulfonique [75] ;
(4-chlorophényl)amide d'acide 5-(2-méthoxyéthylamino)-4-nitrothiophène-2-sulfonique [80] ;
N-(1*H*-benzoimidazole-2-yl)-4-chloro-3-nitrobenzènesulfonamide [83] ;
(4-chloro-3-nitrophényl)amide d'acide 5-chloro-3-méthylbenzo[b]thiophène-2-sulfonique [88] ;
N-(3,5-bis-trifluorométhylphényl)-3-chloro-4-méthoxybenzènesulfonamide [104] ;
N-(3-chloro-4-nitrophényl)-3,5-bis-trifluorométhylbenzènesulfonamide [105] ;
(3,5-dichlorophényl)amide d'acide 4,5-dibromothiophène-2-sulfonique [112] ;
(3,5-bis-trifluorométhylphényl)amide d'acide 5-benzènesulfonylthiophène-2-sulfonique [117] ;
(3,5-bis-trifluorométhylphényl)amide d'acide 5-chloro-3-méthylbenzo[b]thiophène-2-sulfonique [119] ;
(3,5-bis-trifluorométhylphényl)amide d'acide benzo[b]-thiophène-2-sulfonique [120] ;
(3,5-bis-trifluorométhylphényl)amide d'acide benzo-[1,2,5]thiadiazole-5-sulfonique [122] ;
(4-chlorophényl)amide d'acide 4,5-dibromothiophène-2-sulfonique [125] ;
(4-trifluorométhylphényl)amide d'acide 4,5-dibromothiophène-2-sulfonique [126] ;
N-(3,5-bis-trifluorométhylphényl)-2,6-dichlorobenzènesulfonamide [128] ;
(4-chlorophényl)amide d'acide 5-(3-hydroxypipéridine-1-yl)-4-nitrothiophène-2-sulfonique [130] ; et
(4-nitrophényl)amide d'acide 5-chloro-4-nitrothiophène-2-sulfonique [131].

42. Composition pharmaceutique comprenant un composé tel que défini dans l'une quelconque des revendications 24 à 41 en mélange avec un ou plusieurs diluants, excipients ou supports pharmaceutiquement acceptables.

43. Utilisation d'un composé tel que défini dans l'une quelconque des revendications 1 à 41 dans un essai pour déterminer la liaison à HDM2.

44. Utilisation selon la revendication 43, dans laquelle ledit essai est capable d'identifier des composés candidats qui inhibent l'interaction entre HDM2 et p53 et/ou E2F.

45. Utilisation selon l'une quelconque des revendications 43 ou 44, dans laquelle ledit essai est un essai de liaison par compétition.

46. Utilisation selon la revendication 45, dans laquelle ledit essai de liaison par compétition comprend la mise en contact d'un composé de formule I tel que défini dans l'une quelconque des revendications 1 à 22 avec HDM2 en présence d'un peptide dérivé de p53 et la détection de tout changement dans l'interaction entre HDM2 et ledit peptide dérivé de p53.

47. Utilisation selon la revendication 46, dans laquelle ledit peptide dérivé de p53 est un peptide dérivé de p53 marqué par fluorescence ou biotinylé.

48. Méthode de détection de la liaison d'un ligand à HDM2, ladite méthode comprenant les étapes de :
(i) mise en contact d'un ligand avec HDM2 en présence d'un peptide dérivé de p53 ; et
(ii) détection de tout changement dans l'interaction entre HDM2 et ledit peptide dérivé de p53 ;
et dans laquelle ledit ligand est un composé tel que défini dans l'une quelconque des revendications 1 à 22.

49. Association comprenant au moins un composé tel que défini dans l'une quelconque des revendications 1 à 41 et au moins un agent cytotoxique.

50. Association selon la revendication 49, dans laquelle ledit agent cytotoxique est un agent chimiothérapeutique.

51. Association selon la revendication 50, dans laquelle ledit agent chimiothérapeutique est le cisplatine ou l'étoposide.

52. Composition pharmaceutique comprenant au moins un composé tel que défini dans l'une quelconque des revendications 1 à 41, et un ou plusieurs agents cytotoxiques, en mélange avec un diluant, excipient ou support pharmaceutiquement acceptable.

53. Utilisation d'au moins un composé tel que défini dans l'une quelconque des revendications 1 à 41, dans la préparation d'un médicament destiné à traiter un trouble prolifératif, dans laquelle ledit composé est destiné à une utilisation consécutive, simultanée ou séquentielle avec un ou plusieurs autres agents cytotoxiques.

54. Utilisation d'au moins un composé tel que défini dans l'une quelconque des revendications 1 à 41, dans la préparation d'un médicament destiné à traiter un trouble prolifératif, dans laquelle ledit composé est destiné à une utilisation consécutive, simultanée ou séquentielle avec une radiothérapie.
